# EUROPEAN PATENT APPLICATION

(11) **EP 4 361 157 A1**
(43) Date of publication of application: **01.05.2024**
(21) Application number: 22827544.2
(22) Date of filing: 21.06.2022
(51) Int. Cl.: C07D 498/22, A61K 31/553, A61P 35/00

(54) **FUSED TETRACYCLIC COMPOUND, PREPARATION METHOD THEREFOR AND APPLICATION THEREOF IN MEDICINE**

(30) Priority: 21.06.2021 CN 202110687643; 02.08.2021 CN 202110880105; 14.09.2021 CN 202111073775; 21.10.2021 CN 202111225928
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); SHEN, Feng, Shanghai 200245 (CN); DONG, Huaide, Shanghai 200245 (CN); CHEN, Yang, Shanghai 200245 (CN); HE, Feng, Shanghai 200245 (CN); TAO, Weikang, Shanghai 200245 (CN)
(74) Representative: Regimbeau
(86) International application number: PCT/CN2022/100016
(87) International publication number: WO 2022/268051

(57) **Abstract**

The present disclosure relates to a fused tetracyclic compound, a preparation method therefor and an application thereof in medicine. Specifically, the present disclosure relates to a fused tetracyclic compound as shown in general formula (I), a preparation method therefor, a pharmaceutical composition containing the compound, and use thereof as a therapeutic agent, in particular use thereof in preparation of drugs for inhibiting KRAS G12D. The groups in general formula (I) are as defined in the description.

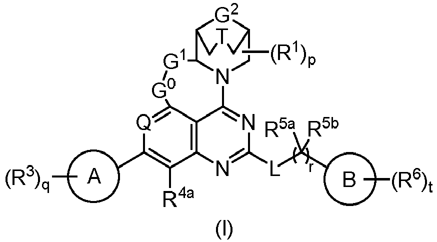

## Description

### TECHNICAL FIELD

The present disclosure belongs to the field of pharmaceutics, and relates to a fused tetracyclic compound, a preparation method therefor and medical use thereof. In particular, the present disclosure relates to fused tetracyclic compounds of general formula (I), a preparation method therefor, a pharmaceutical composition containing the compounds, and use thereof in preparing a medicament for inhibiting KRAS G12D.

### BACKGROUND

RAS is one of the oncogenes with the highest mutation rate in tumors, and about 30% of human malignancies are associated with mutations of the RAS gene. The RAS family includes KRAS, NRAS and HRAS, and KRAS mutations are the most common and account for approximately 85%. KRAS mutations are common in solid tumors, with high-frequency mutations in the three fatal cancers in humans: lung cancer (17%), colorectal cancer (33%) and pancreatic cancer (61%). A total of 97% of the KRAS gene mutations involve mutation of amino acid residue No. 12 or 13, and G12D is an important mutation. Data analysis on the European and American population shows that G12D mutation is found in 36%, 12% and 4% of pancreatic cancer, colorectal cancer and non-small cell lung cancer patients, respectively.

After KRAS is activated, it regulates multiple functions such as cell proliferation, survival, migration and metabolism through a plurality of downstream signaling pathways represented by RAF-MEK-ERK, PI3K-AKT-mTOR and TIAM1-RAc. After mutation of KRAS gene, the protein is continuously activated, resulting in continuous activation of downstream signaling pathways and thereby promoting tumorigenesis.

KRAS protein is considered as an undruggable drug target for a long time because it lacks conventional small molecule binding sites on its surface and it is extremely difficult to inhibit due to its ultrahigh affinity for guanylic acid. However, based on the importance and prevalence of abnormal KRAS activation in cancer progression, KRAS has been and remains a target of high interest for drug development. Currently, except for inhibitors against KRAS G12C, there is a lack of KRAS inhibitors that are effective against other mutations, such that most patients with KRAS mutation remain non-medicated. G12D is a mutant widely expressed in multiple tumors, and it is of important clinical significance to develop inhibitors against it.

Currently, related patent applications disclosed include WO2021041671A1, WO2020146613A1, WO2017172979A1, WO2020238791A1, WO2021000885A1, and the like.

### SUMMARY

The object of the present disclosure is to provide a compound of general formula (I) or a pharmaceutically acceptable salt thereof: 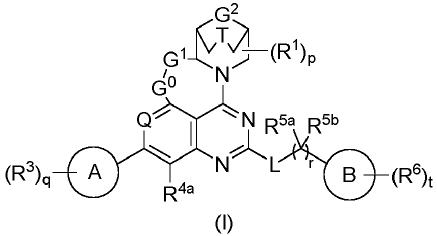 wherein:
G⁰ is selected from the group consisting of O, S, S(O), S(O)₂, CR^{G0a}R^{G0b} and NR^{G0c};
G¹ is selected from the group consisting of CR^{G1a}R^{G1b}, CR^{G1a}R^{G1b}CR^{G1c}R^{G1d}, C=O and C(O)CR^{G1a}R^{G1b};
G² is NR^{d};
T is a chemical bond or is selected from the group consisting of CR^{a}R^{b}, NR^{T} and O; Q is N or CR^{2a};
ring A is aryl or heteroaryl;
ring B is selected from the group consisting of cycloalkyl, heterocyclyl, aryl and heteroaryl;
L is selected from the group consisting of a single bond, O and NR^{e};
R^{a}, R^{b}, R^{G0a}, R^{G0b}, R^{G1a}, R^{G1b}, R^{G1c} and R^{G1d} are identical or different and are each independently selected from the group consisting of a hydrogen atom, halogen, alkyl, alkoxy, haloalkyl, haloalkoxy, cyano, amino, nitro, hydroxy, hydroxyalkyl, cycloalkyl and heterocyclyl; or, R^{G1a} and R^{G1b}, together with the carbon atom to which they are attached, form cycloalkyl; or, R^{G1c} and R^{G1d}, together with the carbon atom to which they are attached, form cycloalkyl;
each R¹ is identical or different and is independently selected from the group consisting of a hydrogen atom, halogen, alkyl, alkenyl, alkynyl, alkoxy, haloalkyl, haloalkoxy, cyano, amino, -(CH₂)ᵤ-NR^{f}R^{g}, hydroxy and hydroxyalkyl;
R^{2a} and R^{4a} are identical or different and are each independently selected from the group consisting of a hydrogen atom, halogen, alkyl, alkenyl, alkynyl, alkoxy, haloalkyl, haloalkoxy, cyano, amino, -(CH₂)ᵥ-NR^{h}Rⁱ, hydroxy, hydroxyalkyl and cycloalkyl;
each R³ and R⁶ are identical or different and are each independently selected from the group consisting of a hydrogen atom, halogen, alkyl, alkenyl, alkynyl, alkoxy, haloalkyl, haloalkoxy, cyano, amino, -(CH₂)_{w}-NR^{j}R^{k}, -(CH₂)_{w1}-(O)_{z1}-C(O)NR^{j1}R^{k1}, -(CH₂)_{w2}-(O)_{z2}-C(O)OR^{j2}, nitro, hydroxy, hydroxyalkyl, cycloalkyl, heterocyclyl, aryl and heteroaryl;
R^{5a} and R^{5b} are identical or different and are each independently selected from the group consisting of a hydrogen atom, halogen, alkyl, haloalkyl, cyano, hydroxy and hydroxyalkyl; or
R^{5a} and R^{5b}, together with the carbon atom to which they are attached, form cycloalkyl or heterocyclyl, and the cycloalkyl or heterocyclyl is independently optionally substituted with one or more of the identical or different substituents selected from the group consisting of halogen, alkyl, haloalkyl, alkoxy, haloalkoxy, cyano, amino, hydroxy and hydroxyalkyl;
R^{G0c}, R^{T}, R^{d}, R^{e}, R^{f}, R^{g}, R^{h}, Rⁱ, R^{j}, R^{k}, R^{j1}, R^{k1} and R^{j2} are identical or different and are each independently selected from the group consisting of a hydrogen atom, alkyl, alkenyl, alkynyl, haloalkyl, hydroxyalkyl, cycloalkyl, heterocyclyl, aryl and heteroaryl; u, v, w, w1 and w2 are identical or different and are each independently selected from the group consisting of 0, 1, 2 and 3;
z1 is 0 or 1;
z2 is 0 or 1;
r is 0, 1, 2 or 3;
p is 0, 1, 2, 3, 4 or 5;
q is 0, 1, 2, 3, 4 or 5; and
t is 0, 1, 2, 3, 4 or 5.

The present disclosure provides a compound of general formula (I) or a pharmaceutically acceptable salt thereof: 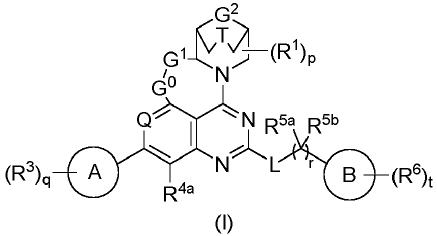 wherein:
G⁰ is selected from the group consisting of O, S, S(O), S(O)₂, CR^{G0a}R^{G0b} and NR^{G0c};
G¹ is selected from the group consisting of CR^{G1a}R^{G1b}, CR^{G1a}R^{G1b}CR^{G1c}R^{G1d}, C=O and C(O)CR^{G1a}R^{G1b};
G² is NR^{d};
T is a chemical bond or is selected from the group consisting of CR^{a}R^{b}, NR^{T} and O;
Q is N or CR^{2a};
ring A is aryl or heteroaryl;
ring B is selected from the group consisting of cycloalkyl, heterocyclyl, aryl and heteroaryl;
L is selected from the group consisting of a single bond, O and NR^{e};
R^{a}, R^{b}, R^{G0a}, R^{G0b}, R^{G1a}, R^{G1b}, R^{G1c} and R^{G1d} are identical or different and are each independently selected from the group consisting of a hydrogen atom, halogen, alkyl, alkoxy, haloalkyl, haloalkoxy, cyano, amino, nitro, hydroxy, hydroxyalkyl, cycloalkyl and heterocyclyl; or, R^{G1a} and R^{G1b}, together with the carbon atom to which they are attached, form cycloalkyl; or, R^{G1c} and R^{G1d}, together with the carbon atom to which they are attached, form cycloalkyl;
each R¹ is identical or different and is independently selected from the group consisting of a hydrogen atom, halogen, alkyl, alkenyl, alkynyl, alkoxy, haloalkyl, haloalkoxy, cyano, amino, -(CH₂)ᵤ-NR^{f}R^{g}, hydroxy and hydroxyalkyl;
R^{2a} and R^{4a} are identical or different and are each independently selected from the group consisting of a hydrogen atom, halogen, alkyl, alkenyl, alkynyl, alkoxy, haloalkyl, haloalkoxy, cyano, amino, -(CH₂)ᵥ-NR^{h}Rⁱ, hydroxy, hydroxyalkyl and cycloalkyl;
each R³ and R⁶ are identical or different and are each independently selected from the group consisting of a hydrogen atom, halogen, alkyl, alkenyl, alkynyl, alkoxy, haloalkyl, haloalkoxy, cyano, amino, -(CH₂)_{w}-NR^{j}R^{k}, nitro, hydroxy, hydroxyalkyl, cycloalkyl, heterocyclyl, aryl and heteroaryl;
R^{5a} and R^{5b} are identical or different and are each independently selected from the group consisting of a hydrogen atom, halogen, alkyl, haloalkyl, cyano, hydroxy and hydroxyalkyl;
R^{G0c}, R^{T}, R^{d}, R^{e}, R^{f}, R^{g}, R^{h}, Rⁱ, R^{j}, R^{k} and R^{j} are identical or different and are each independently selected from the group consisting of a hydrogen atom, alkyl, alkenyl, alkynyl, haloalkyl, hydroxyalkyl, cycloalkyl, heterocyclyl, aryl and heteroaryl;
u, v and w are identical or different and are each independently selected from the group consisting of 0, 1, 2 and 3;
r is 0, 1 or 2;
p is 0, 1, 2, 3, 4 or 5;
q is 0, 1, 2, 3, 4 or 5; and
t is 0, 1, 2, 3, 4 or 5.

In some embodiments of the present disclosure, the compound of general formula (I) or the pharmaceutically acceptable salt thereof is a compound of general formula (I') or a pharmaceutically acceptable salt thereof: 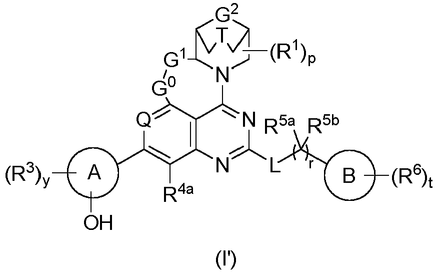 wherein,
y is 0, 1, 2, 3 or 4;
ring A, ring B, G⁰, G¹, T, G², Q, L, R¹, R³, R^{4a}, R^{5a}, R^{5b}, R⁶, p, r and t are as defined in general formula (I).

In some embodiments of the present disclosure, provided is the compound of general formula (I) or (I') or the pharmaceutically acceptable salt thereof, wherein 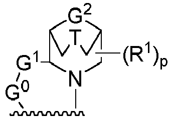 is selected from the group consisting of 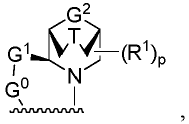 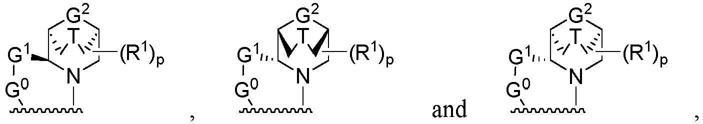 is preferably 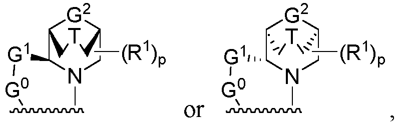 and is further preferably

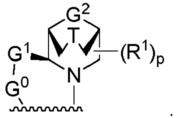

In some embodiments of the present disclosure, provided is the compound of general formula (I) or (I') or the pharmaceutically acceptable salt thereof, wherein G⁰ is selected from the group consisting of O, CR^{G0a}R^{G0b} and NR^{G0c}; R^{G0a} and R^{G0b} are identical or different and are each independently selected from the group consisting of a hydrogen atom, halogen, C₁₋₆ alkyl and C₁₋₆ haloalkyl, and R^{G0c} is a hydrogen atom or C₁₋₆ alkyl; preferably, G⁰ is selected from the group consisting of O, CH₂ and NH; further preferably, G⁰ is O.

In some embodiments of the present disclosure, provided is the compound of general formula (I) or (I') or the pharmaceutically acceptable salt thereof, wherein G¹ is CR^{G1a}R^{G1b}, CR^{G1a}R^{G1b}CR^{G1c}R^{G1d} or C=O, R^{G1a}, R^{G1b}, R^{G1c} and R^{G1d} are identical or different and are each independently selected from the group consisting of a hydrogen atom, halogen, C₁₋₆ alkyl and C₁₋₆ haloalkyl; preferably, G¹ is CH₂ or C=O; further preferably, G¹ is CH₂.

In some embodiments of the present disclosure, provided is the compound of general formula (I) or (I') or the pharmaceutically acceptable salt thereof, wherein -G⁰-G¹- is selected from the group consisting of -O-CH₂-, -NH-C(O)-, -NH-CH₂-, -CH₂-CH₂- and -O-CH₂-CH₂-, is preferably -O-CH₂- or -NH-C(O)-, and is further preferably -O-CH₂-. In some embodiments of the present disclosure, provided is the compound of general formula (I) or (I') or the pharmaceutically acceptable salt thereof, wherein T is a chemical bond.

In some embodiments of the present disclosure, the compound of general formula (I) or the pharmaceutically acceptable salt thereof is a compound of general formula (II) or a pharmaceutically acceptable salt thereof: 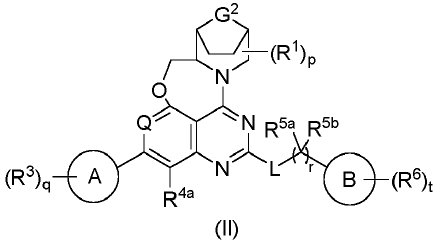 wherein,
ring A, ring B, G², Q, L, R¹, R³, R^{4a}, R^{5a}, R^{5b}, R⁶, p, q, r and t are as defined in general formula (I).

In some embodiments of the present disclosure, provided is the compound of general formula (II) or the pharmaceutically acceptable salt thereof, wherein 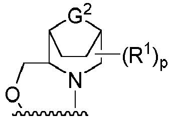 is selected from the group consisting of 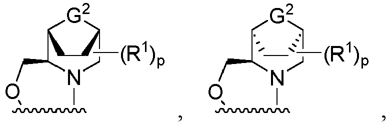 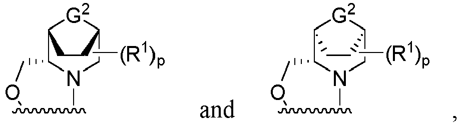 is preferably 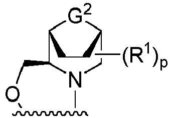 or 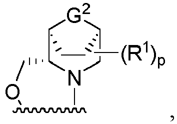 and is further preferably

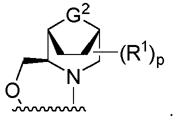

In some embodiments of the present disclosure, provided is the compound of general formula (I), (I') or (II) or the pharmaceutically acceptable salt thereof, wherein Q is N or CH, and preferably N.

In some embodiments of the present disclosure, provided is the compound of general formula (I), (I') or (II) or the pharmaceutically acceptable salt thereof, wherein R^{2a} is selected from the group consisting of a hydrogen atom, halogen, C₁₋₆ alkyl and C₁₋₆ haloalkyl; preferably, R^{2a} is a hydrogen atom or C₁₋₆ alkyl; more preferably, R^{2a} is a hydrogen atom.

In some embodiments of the present disclosure, provided is the compound of general formula (I), (I') or (II) or the pharmaceutically acceptable salt thereof, wherein ring A is 6-10 membered aryl or 5-10 membered heteroaryl; preferably, ring A is phenyl or naphthyl; further preferably, ring A is naphthyl.

In some embodiments of the present disclosure, provided is the compound of general formula (I) or (II) or the pharmaceutically acceptable salt thereof, wherein 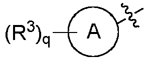 is 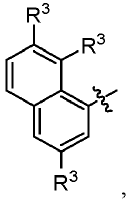 and R³ is as defined in formula (I); preferably, each R³ is identical or different and is independently selected from the group consisting of a hydrogen atom, halogen, C₁₋₆ alkyl, C₂₋₆ alkynyl, C₁₋₆ haloalkyl, hydroxy, C₁₋₆ hydroxyalkyl and 3-8 membered cycloalkyl; further preferably, each R³ is identical or different and is independently selected from the group consisting of a hydrogen atom, halogen, C₁₋₆ alkyl, C₂₋₆ alkynyl, C₁₋₆ haloalkyl, hydroxy and cyclopropyl; still further preferably, each R³ is identical or different and is independently selected from the group consisting of a hydrogen atom, halogen, C₁₋₆ alkyl and hydroxy.

In some embodiments of the present disclosure, provided is the compound of general formula (I) or (II) or the pharmaceutically acceptable salt thereof, wherein 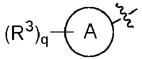 is 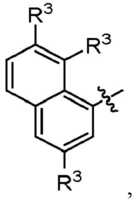 and R³ is as defined in formula (I); preferably, each R³ is identical or different and is independently selected from the group consisting of a hydrogen atom, halogen, C₁₋₆ alkyl, C₂₋₆ alkynyl, C₁₋₆ haloalkyl, hydroxy and 3-8 membered cycloalkyl; further preferably, each R³ is identical or different and is independently selected from the group consisting of a hydrogen atom, F, ethyl, ethynyl and hydroxy.

In some embodiments of the present disclosure, provided is the compound of general formula (I) or (II) or the pharmaceutically acceptable salt thereof, wherein 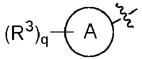 is 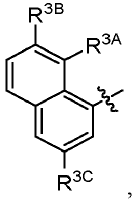 and R^{3A}, R^{3B} and R^{3C} are identical or different and are each independently selected from the group consisting of a hydrogen atom, halogen, C₁₋₆ alkyl, C₂₋₆ alkynyl, C₁₋₆ haloalkyl and hydroxy; preferably, R^{3A} is selected from the group consisting of halogen, C₁₋₆ alkyl and C₂₋₆ alkynyl; R^{3B} is a hydrogen atom or halogen; R^{3C} is hydroxy; more preferably, R^{3A} is selected from the group consisting of F, ethyl and ethynyl; R^{3B} is a hydrogen atom or F; R^{3C} is hydroxy.

In some embodiments of the present disclosure, provided is the compound of general formula (I') or the pharmaceutically acceptable salt thereof, wherein 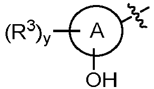 is 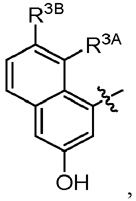 and R^{3A} and R^{3B} are identical or different and are each independently selected from the group consisting of a hydrogen atom, halogen, C₁₋₆ alkyl, C₂₋₆ alkynyl and C₁₋₆ haloalkyl; preferably, R^{3A} is selected from the group consisting of halogen, C₁₋₆ alkyl and C₂₋₆ alkynyl; R^{3B} is a hydrogen atom or halogen; more preferably, R^{3A} is selected from the group consisting of F, ethyl and ethynyl; R^{3B} is selected from the group consisting of a hydrogen atom and F.

In some embodiments of the present disclosure, provided is the compound of general formula (I) or (II) or the pharmaceutically acceptable salt thereof, wherein 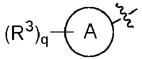 is 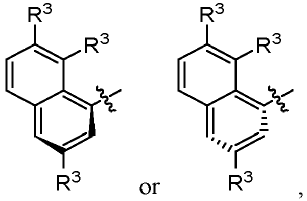 and R³ is as defined in formula (I); preferably, each R³ is identical or different and is independently selected from the group consisting of a hydrogen atom, halogen, C₁₋₆ alkyl, C₂₋₆ alkynyl, C₁₋₆ haloalkyl, hydroxy, C₁₋₆ hydroxyalkyl and 3-8 membered cycloalkyl; further preferably, each R³ is identical or different and is independently selected from the group consisting of a hydrogen atom, halogen, C₁₋₆ alkyl, C₂₋₆ alkynyl, C₁₋₆ haloalkyl, hydroxy and cyclopropyl; still further preferably, each R³ is identical or different and is independently selected from the group consisting of a hydrogen atom, halogen, C₁₋₆ alkyl and hydroxy.

In some embodiments of the present disclosure, provided is the compound of general formula (I) or (II) or the pharmaceutically acceptable salt thereof, wherein 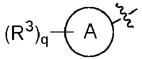 is 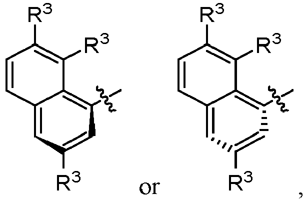 and R³ is as defined in formula (I); preferably, each R³ is identical or different and is independently selected from the group consisting of a hydrogen atom, halogen, C₁₋₆ alkyl, C₂₋₆ alkynyl, C₁₋₆ haloalkyl, hydroxy and 3-8 membered cycloalkyl; further preferably, each R³ is identical or different and is each independently selected from the group consisting of a hydrogen atom, F, ethyl, ethynyl and hydroxy.

In some embodiments of the present disclosure, provided is the compound of general formula (I) or (II) or the pharmaceutically acceptable salt thereof, wherein 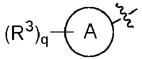 is 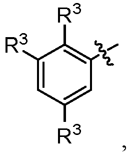 and R³ is as defined in formula (I); preferably, each R³ is identical or different and is independently selected from the group consisting of a hydrogen atom, halogen, C₁₋₆ alkyl, C₂₋₆ alkynyl, C₁₋₆ haloalkyl, hydroxy, C₁₋₆ hydroxyalkyl and 3-8 membered cycloalkyl; further preferably, each R³ is identical or different and is independently selected from the group consisting of a hydrogen atom, halogen, C₁₋₆ alkyl, C₂₋₆ alkynyl, C₁₋₆ haloalkyl, hydroxy and cyclopropyl; still further preferably, each R³ is identical or different and is independently selected from the group consisting of a hydrogen atom, halogen, cyclopropyl and hydroxy.

In some embodiments of the present disclosure, provided is the compound of general formula (I) or (II) or the pharmaceutically acceptable salt thereof, wherein 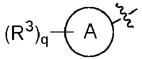 is 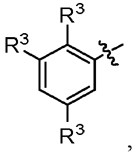 and R³ is as defined in formula (I); preferably, each R³ is identical or different and is independently selected from the group consisting of a hydrogen atom, halogen, C₁₋₆ alkyl, C₂₋₆ alkynyl, C₁₋₆ haloalkyl, hydroxy and 3-8 membered cycloalkyl; further preferably, each R³ is identical or different and is independently selected from the group consisting of a hydrogen atom, halogen, C₁₋₆ alkyl, C₁₋₆ haloalkyl, hydroxy and 3-8 membered cycloalkyl; more preferably, each R³ is identical or different and is independently selected from the group consisting of Cl, hydroxy, CF₃ and cyclopropyl.

In some embodiments of the present disclosure, provided is the compound of general formula (I) or (II) or the pharmaceutically acceptable salt thereof, wherein 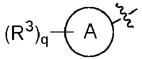 is 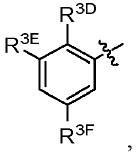 and R^{3D}, R^{3E} and R^{3F} are identical or different and are each independently selected from the group consisting of halogen, C₁₋₆ haloalkyl, hydroxy, and 3-8 membered cycloalkyl; preferably, R^{3D} is C₁₋₆ haloalkyl or 3-8 membered cycloalkyl; R^{3E} is halogen; R^{3F} is hydroxy; more preferably, R^{3D} is CF₃ or cyclopropyl; R^{3E} is Cl; R^{3F} is hydroxy.

In some embodiments of the present disclosure, provided is the compound of general formula (I') or the pharmaceutically acceptable salt thereof, wherein 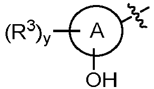 is 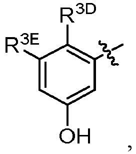 and R^{3D} and R^{3E} are identical or different and are each independently selected from the group consisting of halogen, C₁₋₆ haloalkyl and 3-8 membered cycloalkyl; preferably, R^{3D} is C₁₋₆ haloalkyl or 3-8 membered cycloalkyl; R^{3E} is halogen; more preferably, R^{3D} is CF₃ or cyclopropyl; R^{3E} is Cl.

In some embodiments of the present disclosure, provided is the compound of general formula (I), (I') or (II) or the pharmaceutically acceptable salt thereof, wherein ring B is 7-10 membered fused heterocyclyl, R⁶ can substitute at any position of ring B; preferably, ring B is 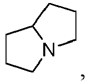 and R⁶ can substitute at any position of the ring B.

In some embodiments of the present disclosure, provided is the compound of general formula (I), (I') or (II) or the pharmaceutically acceptable salt thereof, wherein ring B is 3-8 membered heterocyclyl.

In some embodiments of the present disclosure, provided is the compound of general formula (I), (I') or (II) or the pharmaceutically acceptable salt thereof, wherein 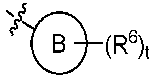 is 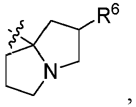 and R⁶ is as defined in formula (I); preferably, 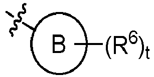 is 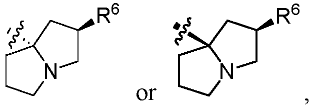 and R⁶ is halogen; further preferably, 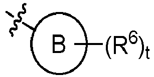 is 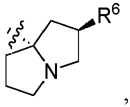 and R⁶ is halogen; R⁶ is further preferably F.

In some embodiments of the present disclosure, provided is the compound of general formula (I), (I') or (II) or the pharmaceutically acceptable salt thereof, wherein 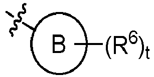 is 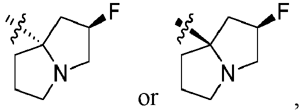 and preferably

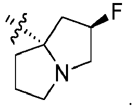

In some embodiments of the present disclosure, provided is the compound of general formula (I), (I') or (II) or the pharmaceutically acceptable salt thereof, wherein 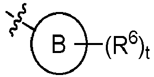 is selected from the group consisting of 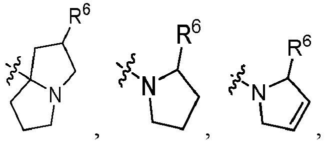 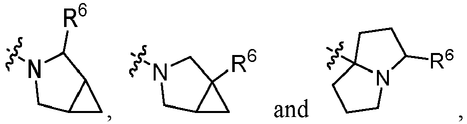 and R⁶ is as defined in general formula (I); preferably, 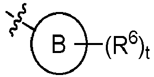 is selected from the group consisting of 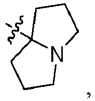 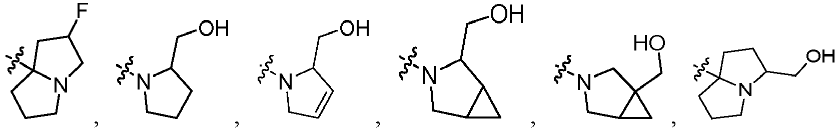 and 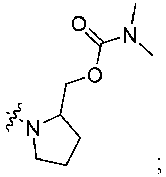 more preferably, 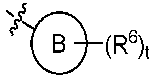 is selected from the group consisting of 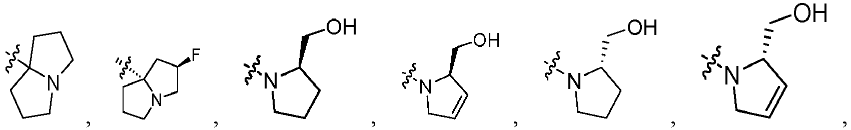 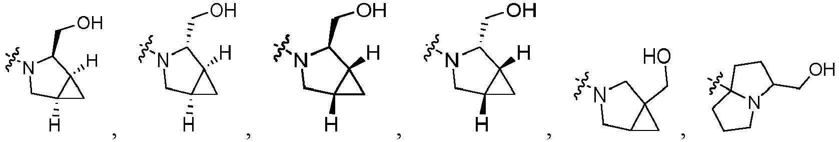 and

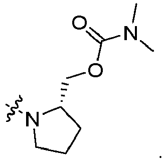

In some embodiments of the present disclosure, provided is the compound of general formula (I), (I') or (II) or the pharmaceutically acceptable salt thereof, wherein R^{4a} is selected from the group consisting of a hydrogen atom, halogen, C₁₋₆ alkyl and C₁₋₆ haloalkyl, is preferably a hydrogen atom or halogen, and is more preferably a hydrogen atom or F.

In some embodiments of the present disclosure, provided is the compound of general formula (I), (I') or (II) or the pharmaceutically acceptable salt thereof, wherein R^{d} is a hydrogen atom or C₁₋₆ alkyl; preferably, R^{d} is a hydrogen atom.

In some embodiments of the present disclosure, provided is the compound of general formula (I), (I') or (II) or the pharmaceutically acceptable salt thereof, wherein G² is NH.

In some embodiments of the present disclosure, provided is the compound of general formula (I), (I') or (II) or the pharmaceutically acceptable salt thereof, wherein L is selected from the group consisting of CH₂, NH and O, and is preferably O.

In some embodiments of the present disclosure, provided is the compound of general formula (I), (I') or (II) or the pharmaceutically acceptable salt thereof, wherein R^{e} is a hydrogen atom or C₁₋₆ alkyl; preferably, R^{e} is a hydrogen atom.

In some embodiments of the present disclosure, provided is the compound of general formula (I), (I') or (II) or the pharmaceutically acceptable salt thereof, wherein each R¹ is identical or different and is independently selected from the group consisting of a hydrogen atom, halogen, C₁₋₆ alkyl, C₁₋₆ haloalkyl, cyano, amino, -(CH₂)ᵤ-NR^{f}R^{g}, hydroxy and C₁₋₆ hydroxyalkyl, R^{f} and R^{g} are identical or different and are each independently a hydrogen atom or C₁₋₆ alkyl, and u is 0 or 1; preferably, each R¹ is identical or different and is independently selected from the group consisting of a hydrogen atom, halogen, C₁₋₆ alkyl and C₁₋₆ haloalkyl; further preferably, R¹ is a hydrogen atom.

In some embodiments of the present disclosure, provided is the compound of general formula (I), (I') or (II) or the pharmaceutically acceptable salt thereof, wherein each R³ is identical or different and is independently selected from the group consisting of a hydrogen atom, halogen, C₁₋₆ alkyl, C₂₋₆ alkenyl, C₂₋₆ alkynyl, C₁₋₆ alkoxy, C₁₋₆ haloalkyl, C₁₋₆ haloalkoxy, cyano, amino, -(CH₂)_{w}-NR^{j}R^{k}, hydroxy and C₁₋₆ hydroxyalkyl, R^{j} and R^{k} are identical or different and are each independently a hydrogen atom or C₁₋₆ alkyl, and w is 0 or 1; preferably, each R³ is identical or different and is independently selected from the group consisting of a hydrogen atom, halogen, C₁₋₆ alkyl, C₂₋₆ alkynyl, C₁₋₆ haloalkyl, hydroxy, C₁₋₆ hydroxyalkyl and 3-8 membered cycloalkyl; further preferably, each R³ is identical or different and is independently selected from the group consisting of a hydrogen atom, halogen, C₁₋₆ alkyl, C₂₋₆ alkynyl, C₁₋₆ haloalkyl, hydroxy and cyclopropyl; still further preferably, each R³ is identical or different and is independently selected from the group consisting of a hydrogen atom, halogen, C₁₋₆ alkyl and hydroxy.

In some embodiments of the present disclosure, provided is the compound of general formula (I), (I') or (II) or the pharmaceutically acceptable salt thereof, wherein each R³ is identical or different and is independently selected from the group consisting of a hydrogen atom, halogen, C₁₋₆ alkyl, C₂₋₆ alkynyl, C₁₋₆ haloalkyl, hydroxy and 3-8 membered cycloalkyl; further preferably, each R³ is identical or different and is independently selected from the group consisting of a hydrogen atom, F, ethyl, ethynyl and hydroxy, or selected from the group consisting of Cl, hydroxyl, CF₃, and cyclopropyl.

In some embodiments of the present disclosure, provided is the compound of general formula (I), (I') or (II) or the pharmaceutically acceptable salt thereof, wherein R^{5a} and R^{5b} are identical or different and are each independently selected from the group consisting of a hydrogen atom, halogen, C₁₋₆ alkyl, C₁₋₆ haloalkyl, hydroxy and C₁₋₆ hydroxyalkyl; preferably, R^{5a} and R^{5b} are identical or different and are each independently selected from the group consisting of a hydrogen atom, C₁₋₆ alkyl, hydroxy and C₁₋₆ hydroxyalkyl; further preferably, R^{5a} and R^{5b} are hydrogen atoms.

In some embodiments of the present disclosure, provided is the compound of general formula (I), (I') or (II) or the pharmaceutically acceptable salt thereof, wherein R^{5a} and R^{5b} are hydrogen atoms; or R^{5a} and R^{5b}, together with the carbon atom to which they are attached, form 3-6 membered cycloalkyl; preferably, R^{5a} and R^{5b} are hydrogen atoms; or R^{5a} and R^{5b}, together with the carbon atom to which they are attached, form cyclopropyl.

In some embodiments of the present disclosure, provided is the compound of general formula (I), (I') or (II) or the pharmaceutically acceptable salt thereof, wherein each R⁶ is identical or different and is independently selected from the group consisting of a hydrogen atom, halogen, C₁₋₆ alkyl, C₁₋₆ alkoxy, C₁₋₆ haloalkyl, C₁₋₆ haloalkoxy, cyano, amino, -(CH₂)_{w}-NR^{j}R^{k}, hydroxy and C₁₋₆ hydroxyalkyl, R^{j} and R^{k} are identical or different and are each independently a hydrogen atom or C₁₋₆ alkyl, and w is 0 or 1; preferably, each R⁶ is identical or different and is independently selected from the group consisting of a hydrogen atom, halogen, C₁₋₆ alkyl and C₁₋₆ haloalkyl, is further preferably hydrogen or halogen, and is more preferably F.

In some embodiments of the present disclosure, provided is the compound of general formula (I), (I') or (II) or the pharmaceutically acceptable salt thereof, wherein each R⁶ is identical or different and is independently selected from the group consisting of a hydrogen atom, halogen, C₁₋₆ hydroxyalkyl and -CH₂-O-C(O)NR^{j1}R^{k1}, and R^{j1} and R^{k1} are identical or different and are each independently a hydrogen atom or C₁₋₆ alkyl; preferably, each R⁶ is identical or different and is independently selected from the group consisting of a hydrogen atom, F, hydroxymethyl and-CH₂-O-C(O)N(CH₃)₂.

In some embodiments of the present disclosure, provided is the compound of general formula (I), (I') or (II) or the pharmaceutically acceptable salt thereof, wherein R^{j1} is C₁₋₆ alkyl, and preferably methyl.

In some embodiments of the present disclosure, provided is the compound of general formula (I), (I') or (II) or the pharmaceutically acceptable salt thereof, wherein R^{k1} is C₁₋₆ alkyl, and preferably methyl.

In some embodiments of the present disclosure, provided is the compound of general formula (I), (I') or (II) or the pharmaceutically acceptable salt thereof, wherein r is 0 or 1, and preferably 1.

In some embodiments of the present disclosure, provided is the compound of general formula (I), (I') or (II) or the pharmaceutically acceptable salt thereof, wherein r is 1 or 3.

In some embodiments of the present disclosure, provided is the compound of general formula (I), (I') or (II) or the pharmaceutically acceptable salt thereof, wherein p is 0 or 1, and preferably 1. In some embodiments of the present disclosure, provided is the compound of general formula (I), (I') or (II) or the pharmaceutically acceptable salt thereof, wherein (R¹)ₚ is absent.

In some embodiments of the present disclosure, provided is the compound of general formula (I) or (II) or the pharmaceutically acceptable salt thereof, wherein q is 2 or 3, and preferably 2.

In some embodiments of the present disclosure, provided is the compound of general formula (I') or the pharmaceutically acceptable salt thereof, wherein y is 1 or 2, and preferably 1.

In some embodiments of the present disclosure, provided is the compound of general formula (I), (I') or (II) or the pharmaceutically acceptable salt thereof, wherein t is 1 or 2, and preferably 1.

In some embodiments of the present disclosure, provided is the compound of general formula (I), (I') or (II) or the pharmaceutically acceptable salt thereof, wherein u is 0 or 1.

In some embodiments of the present disclosure, provided is the compound of general formula (I), (I') or (II) or the pharmaceutically acceptable salt thereof, wherein v is 0 or 1.

In some embodiments of the present disclosure, provided is the compound of general formula (I), (I') or (II) or the pharmaceutically acceptable salt thereof, wherein w is 0 or 1.

In some embodiments of the present disclosure, provided is the compound of general formula (I), (I') or (II) or the pharmaceutically acceptable salt thereof, wherein w1 is 0 or 1.

In some embodiments of the present disclosure, provided is the compound of general formula (I), (I') or (II) or the pharmaceutically acceptable salt thereof, wherein w2 is 0 or 1.

In some embodiments of the present disclosure, provided is the compound of general formula (II) or the pharmaceutically acceptable salt thereof, wherein ring A is 6-10 membered aryl or 5-10 membered heteroaryl; ring B is 7-10 membered fused heterocyclyl, and R⁶ can substitute at any position of the ring B; G² is NH; Q is N or CH; L is O; p is 1; R¹ is selected from the group consisting of a hydrogen atom, halogen, C₁₋₆ alkyl and C₁₋₆ haloalkyl; q is 2 or 3; each R³ is identical or different and is independently selected from the group consisting of a hydrogen atom, halogen, C₁₋₆ alkyl, C₂₋₆ alkynyl, C₁₋₆ haloalkyl, hydroxyl, C₁₋₆ hydroxyalkyl and 3-8 membered cycloalkyl; R^{4a} is selected from the group consisting of a hydrogen atom, halogen, C₁₋₆ alkyl and C₁₋₆ haloalkyl; r is 0 or 1; R^{5a} and R^{5b} are identical or different and are each independently selected from the group consisting of a hydrogen atom, C₁₋₆ alkyl, a hydroxy and C₁₋₆ hydroxyalkyl; t is 1; R⁶ is selected from the group consisting of a hydrogen atom, halogen, C₁₋₆ alkyl and C₁₋₆ haloalkyl.

In some embodiments of the present disclosure, provided is the compound of general formula (II) or the pharmaceutically acceptable salt thereof, wherein ring A is 6-10 membered aryl or 5-10 membered heteroaryl; ring B is 3-8 membered heterocyclyl; G² is NH; Q is N or CH; L is O; (R¹)ₚ is absent; q is 2 or 3; each R³ is identical or different and is independently selected from the group consisting of a hydrogen atom, halogen, C₁₋₆ alkyl, C₂₋₆ alkynyl, C₁₋₆ haloalkyl, hydroxy, C₁₋₆ hydroxyalkyl and 3-8 membered cycloalkyl; R^{4a} is a hydrogen atom or halogen; r is 1 or 3; R^{5a} and R^{5b} are hydrogen atoms; or R^{5a} and R^{5b}, together with the carbon atom to which they are attached, form cyclopropyl; t is 1; R⁶ is selected from the group consisting of a hydrogen atom, halogen, C₁₋₆ hydroxyalkyl and -CH₂-O-C(O)NR^{j1}R^{k1}, and R^{j1} and R^{k1} are identical or different and are each independently a hydrogen atom or C₁₋₆ alkyl.

In some embodiments of the present disclosure, provided is the compound of general formula (II) or the pharmaceutically acceptable salt thereof, wherein 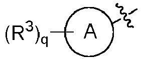 is 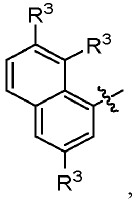 and each R³ is identical or different and is independently selected from the group consisting of a hydrogen atom, halogen, C₁₋₆ alkyl, C₂₋₆ alkynyl, C₁₋₆ haloalkyl, hydroxy and 3-8 membered cycloalkyl; 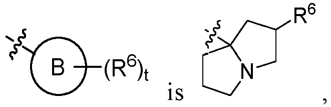 and R⁶ is halogen; G² is NH; Q is N or CH; L is O; (R¹)ₚ is absent; R^{4a} is a hydrogen atom or halogen; r is 0 or 1; R^{5a} and R^{5b} are hydrogen atoms.

In some embodiments of the present disclosure, provided is the compound of general formula (II) or the pharmaceutically acceptable salt thereof, wherein 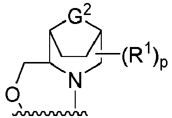 is 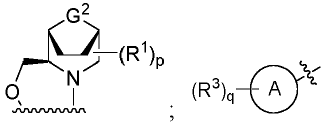 is 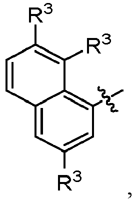 and each R³ is identical or different and is independently selected from the group consisting of a hydrogen atom, halogen, C₁₋₆ alkyl, C₂₋₆ alkynyl, C₁₋₆ haloalkyl, hydroxy and 3-8 membered cycloalkyl; 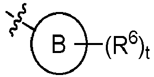 is 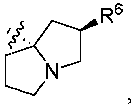 and R⁶ is halogen; G² is NH; Q is N or CH; L is O; (R¹)ₚ is absent; R^{4a} is a hydrogen atom or halogen; r is 0 or 1; R^{5a} and R^{5b} are hydrogen atoms.

In some embodiments of the present disclosure, provided is the compound of general formula (II) or the pharmaceutically acceptable salt thereof, wherein 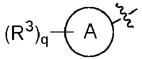 is 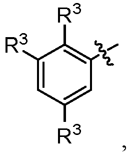 and each R³ is identical or different and is independently selected from the group consisting of a hydrogen atom, halogen, C₁₋₆ alkyl, C₁₋₆ haloalkyl, hydroxy and 3-8 membered cycloalkyl; 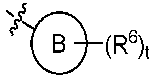 is 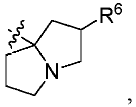 and R⁶ is halogen; G² is NH; Q is N or CH; L is O; (R¹)ₚ is absent; R^{4a} is a hydrogen atom or halogen; r is 0 or 1; R^{5a} and R^{5b} are hydrogen atoms.

In some embodiments of the present disclosure, provided is the compound of general formula (II) or the pharmaceutically acceptable salt thereof, wherein 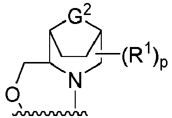 is 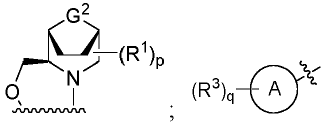 is 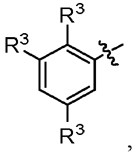 and each R³ is identical or different and is independently selected from the group consisting of a hydrogen atom, halogen, C₁₋₆ alkyl, C₁₋₆ haloalkyl, hydroxy and 3-8 membered cycloalkyl; 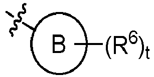 is 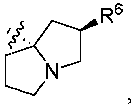 and R⁶ is halogen; G² is NH; Q is N or CH; L is O; (R¹)ₚ is absent; R^{4a} is a hydrogen atom or halogen; r is 0 or 1; R^{5a} and R^{5b} are hydrogen atoms.

**Table A. Typical compounds disclosed herein include, but are not limited to:**

| Compound No. | Compound structure | Name |
|---|---|---|
| | 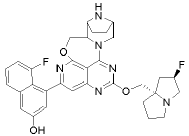 | 5-Fluoro-4-(12-(((2*R*,7*aS*)-2-fluorotetrahydro-11 *H*-pyrrolizin-7a(5*H*)-yl)methoxy)-5a,6,7,8,9,10-hexahydro-5*H*-4-oxa-3,10*a*,11,13,14-pentaaza-6 ,9-methanonaphtho[1,8-*ab*]heptalen-2-yl)naphth alen-2-ol |
| | 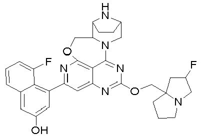 | 5-Fluoro-4-(12-((2-fluorotetrahydro-1*H*-pyrroliz in-7a(5*H*)-yl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)naphthalen-2-ol |
| 1-p2 | 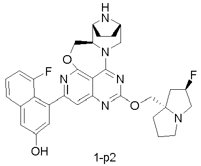 | 5-Fluoro-4-((5*aR*,6*R*,9*S*)-12-(((2*R*,7*aS*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho [1,8-*ab*]heptale n-2-yl)naphthalen-2-ol **1-p2** |
| | 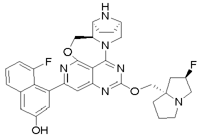 | 5-Fluoro-4-((5*aR*,6*S*,9*R*)-12-(((2*R*,7*aS*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho [1,8-*ab*]heptale n-2-yl)naphthalen-2-ol |
| | 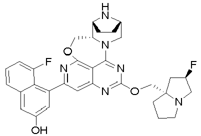 | 5-Fluoro-4-((5*aS*,6*R*,9*S*)-12-(((2*R*,7*aS*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho [1,8-*ab*]heptale n-2-yl)naphthalen-2-ol |
| 1-pl | 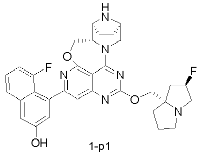 | 5-Fluoro-4-((5*aS*,6*S*,9*R*)-12-(((2*R*,7*aS*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho [1,8-*ab*]heptale n-2-yl)naphthalen-2-ol **1-p1** |
| | 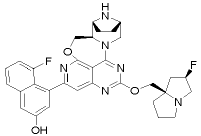 | 5-Fluoro-4-((5*aR*,6*R*,9*S*)-12-(((2*R*,7*aR*)-2-fluor otetrahydro-1*H*-pyrrolizin-7*a*(5*H*)-yl)methoxy)-5*a*,6,7,8,9,10-hexahydro-5*H*-4-oxa-3,10*a*,11,13, 14-pentaaza-6, 9-methanonaphtho [1,8-*ab*]heptal en-2-yl)naphthalen-2-ol |
| | 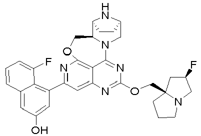 | 5-Fluoro-4-((5*aR*,6*S*,9*R*)-12-(((2*R*,7*aR*)-2-fluor otetrahydro-1*H*-pyrrolizin-7a(5*H*)-yl)methoxy)-5*a*,6,7,8,9,10-hexahydro-5*H*-4-oxa-3,10*a*,11,13, 14-pentaaza-6, 9-methanonaphtho [1,8-*ab*]heptal en-2-yl)naphthalen-2-ol |
| | 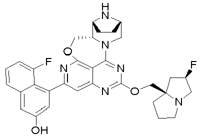 | 5-Fluoro-4-((5*aS*,6*R*,9*S*)-12-(((2*R*,7*aR*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho [1,8-*ab*]heptale n-2-yl)naphthalen-2-ol |
| | 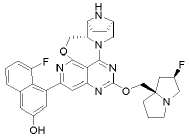 | 5-Fluoro-4-((5*aS*,6*S*,9*R*)-12-(((2*R*,7*aR*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho [1,8-*ab*]heptale n-2-yl)naphthalen-2-ol |
| | 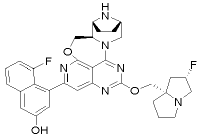 | 5-Fluoro-4-((5*aR*,6*R*,9*S*)-12-(((2*S*,7*aS*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho [1,8-*ab*]heptalen-2-yl)naphthalen-2-ol |
| | 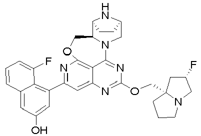 | 5-Fluoro-4-((5*aR*,6*S*,9*R*)-12-(((2*S*,7*aS*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho [1,8-*ab*]heptale n-2-yl)naphthalen-2-ol |
| | 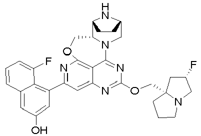 | 5-Fluoro-4-((5*aS*,6*R*,9*S*)-12-(((2*S*,7*aS*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho[1,8-*ab*]heptale n-2-yl)naphthalen-2-ol |
| | 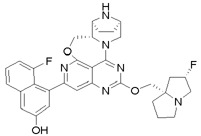 | 5 -Fluoro-4-((5*aS*,6*S*,9*R*)-12-(((2*S*,7*aS*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho[1,8-*ab*]heptale n-2-yl)naphthalen-2-ol |
| | 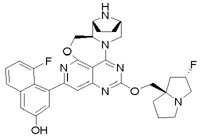 | 5-Fluoro-4-((5*aR*,6*R*,9*S*)-12-(((2*S*,7*aR*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho[1,8-*ab*]heptale n-2-yl)naphthalen-2-ol |
| | 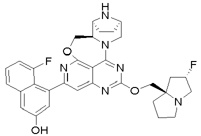 | 5-Fluoro-4-((5*aR*,6*S*,9*R*)-12-(((2*S*,7*aR*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| | 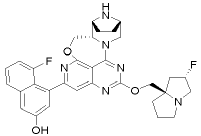 | 5-Fluoro-4-((5*aS*,6*R*,9*S*)-12-(((2*S*,7*aR*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho[1,8-*ab*]heptale n-2-yl)naphthalen-2-ol |
| | 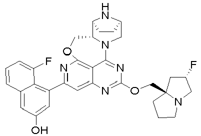 | 5-Fluoro-4-((5*aS*,6*S*,9*S*)-12-(((2*S*,7*aS*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho[1,8-*ab*]heptale n-2-yl)naphthalen-2-ol |
| | 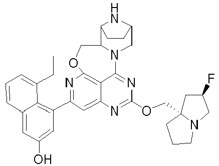 | 5-Ethyl-4-(12-(((2*R*,7*aS*)-2-fluorotetrahydro-1*H* -pyrrolizin-7*a*(5*H*)-yl)methoxy)-5*a*,6,7,8,9,10-h exahydro-5*H*-4-oxa-3,10*a*,11,13,14-pentaaza-6, 9-methanonaphtho[1,8-*ab*]heptalen-2-yl)naphth alen-2-ol |
| | 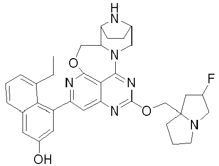 | 5-Ethyl-4-(12-((2-fluorotetrahydro-1*H*-pyrrolizi n-7*a*(5*H*)-yl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)naphthalen-2-ol |
| 2-p2 | 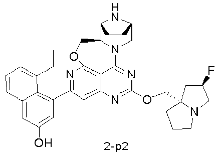 | 5-Ethyl-4-((5*aR*,6*R*,9*S*)-12-(((2*R*,7*aS*)-2-fluorot etrahydro-1*H*-pyrrolizin-7*a*(5*H*)-yl)methoxy)-5 a,6,7,8,9,10-hexahydro-SH-4-oxa-3,10a,11,13,1 4-pentaaza-6,9-methanonaphtho[1,8-*ab*]heptale n-2-yl)naphthalen-2-ol **2-p2** |
| | 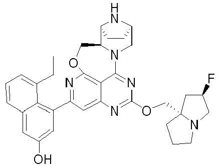 | 5-Ethyl-4-((5*aR*,6*S*,9*R*)-12-(((2*R*,7*aS*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| | 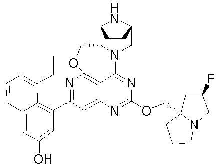 | 5-Ethyl-4-((5*aS*,6*R*,9*S*)-12-(((2*R*,7*aS*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| 2-p1 | 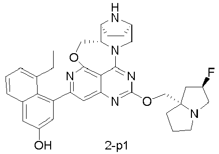 | 5-Ethyl-4-((5*aS*,6*S*,9*R*)-12-(((2*R*,7*aS*)-2-fluorot etrahydro-1*H*-pyrrolizin-7*a*(5*H*)-yl)methoxy)-5 a,6,7,8,9,10-hexahydro-SH-4-oxa-3,10a,11,13,1 4-pentaaza-6,9-methanonaphtho[1,8-*ab*]heptale n-2-yl)naphthalen-2-ol **2-p1** |
| | 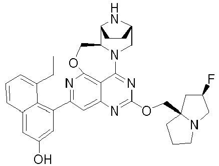 | 5-Ethyl-4-((5*aR*,6*R*,9*S*)-12-(((2*R*,7*aR*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| | 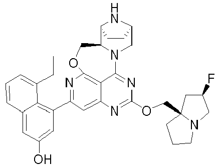 | 5-Ethyl-4-((5*aR*,6*S*,9*R*)-12-(((2*R*,7*aR*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| | 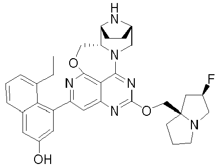 | 5-Ethyl-4-((5*aS*,6*R*,9*S*)-12-(((2*R*,7*aR*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6, 9-methanonaphtho[1,8-*ab*]heptale n-2-yl)naphthalen-2-ol |
| | 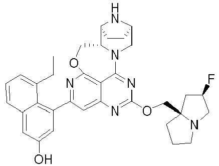 | 5-Ethyl-4-((5*aS*,6*S*,9*R*)-12-(((2*R*,7*aS*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| | 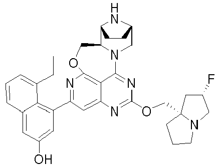 | 5-Ethyl-4-((5*aR*,6*R*,9*S*)-12-(((2*S*,7*aS*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| | 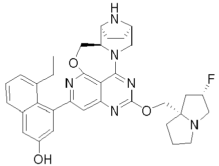 | 5-Ethyl-4-((5*aR*,6*S*,9*R*)-12-(((2*S*,7*aS*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| | 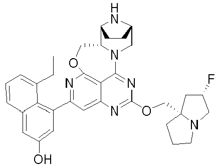 | 5-Ethyl-4-((5*aS*,6*R*,9*S*)-12-(((2*S*,7*aS*)-2-fluorote trahydro-1*H*-pyrrolizin-7*a*(5*H*)-yl)methoxy)-5*a*, 6,7,8,9,10-hexahydro-5*H*-4-oxa-3,10*a*,11,13,14-pentaaza-6,9-methanonaphtho[1,8-*ab*]heptalen-2-yl)naphthalen-2-ol |
| | 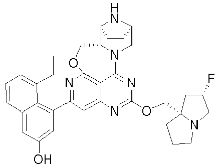 | 5-Ethyl-4-((5*aS*,6*S*,9*R*)-12-(((2*S*,7*aS*)-2-fluorote trahydro-1*H*-pyrrolizin-7*a*(5*H*)-yl)methoxy)-5*a*, 6,7,8,9,10-hexahydro-5*H*-4-oxa-3,10*a*,11,13,14-pentaaza-6,9-methanonaphtho[1,8-*ab*]heptalen-2-yl)naphthalen-2-ol |
| | 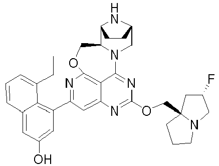 | 5-Ethyl-4-((5*aR*,6*R*,9*S*)-12-(((2*S*,7*aS*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| | 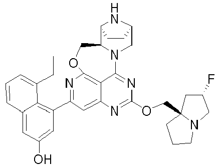 | 5-Ethyl-4-((5*aR*,6*S*,9*R*)-12-(((2*S*,7*aR*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| | 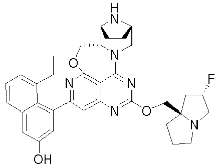 | 5-Ethyl-4-((5*aS*,6*R*,9*S*)-12-(((2*S*,7*aR*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| | 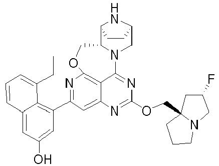 | 5-Ethyl-4-((5*aS*,6*S*,9*R*)-12-(((2*S*,7*aR*)-2-fluorot etrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| | 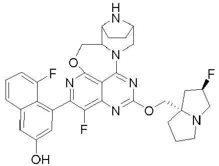 | 5-Fluoro-4-(1-fluoro-12-(((2*R*,7*aS*)-2-fluorotetr ahydro-1*H*-pyrrolizin-7*a*(5*H*)-yl)methoxy)-5*a*,6, 7,8,9,10-hexahydro-5*H*-4-oxa-3,10*a*,11,13,14-p entaaza-6,9-methanonaphtho[1,8-*ab*]heptalen-2-yl)naphthalen-2-ol |
| | 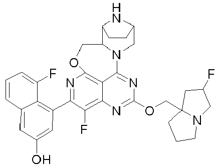 | 5-Fluoro-4-(1-fluoro-12-((2-fluorotetrahydro-1 *H*-pyrrolizin-7*a*(5*H*)-yl)methoxy)-5*a*,6,7,8,9,10-hexahydro-5*H*-4-oxa-3,10*a*,11,13,14-pentaaza-6 ,9-methanonaphtho[1,8-*ab*]heptalen-2-yl)naphth alen-2-ol |
| 3-p2 | 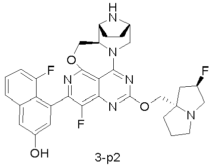 | 5-Fluoro-4-((5*aR*,6*R*,9*S*)-1-fluoro-12-(((2*R*,7*aS*) -2-fluorotetrahydro-1*H*-pyrrolizin-7*a*(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol **3-p2** |
| | 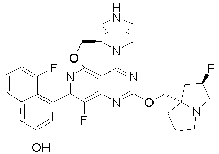 | 5-Fluoro-4-((5*aR*,6*S*,9*R*)-1-fluoro-12-(((2*R*,7*aS*) -2-fluorotetrahydro-1*H*-pyrrolizin-7*a*(5*H*)-yl)me thoxy)-5*a*,6,7,8,9,10-hexahydro-5*H*-4-oxa-3,10 *a*,11,13,14-pentaaza-6,9-methanonaphtho[1,8-*ab*]heptalen-2-yl)naphthalen-2-ol |
| | 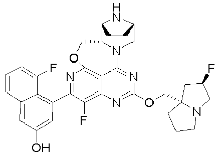 | 5-Fluoro-4-((5*aS*,6*R*,9*S*)-1-fluoro-12-(((2*R*,7*aS*) -2-fluorotetrahydro-1*H*-pyrrolizin-7*a*(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| 3-p 1 | 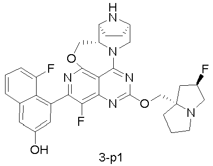 | 5-Fluoro-4-((5*aS*,6*S*,9*R*)-1-fluoro-12-(((2*R*,7*aS*) -2-fluorotetrahydro-1*H*-pyrrolizin-7*a*(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol **3-p1** |
| | 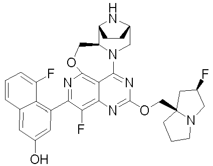 | 5-Fluoro-4-((5*aR*,6*R*,9*S*)-1-fluoro-12-(((2*R*,7*aR* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13, 14-pentaaza-6,9-methanonaphtho[1,8-*a b*]heptalen-2-yl)naphthalen-2-ol |
| | 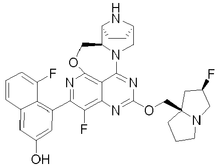 | 5-Fluoro-4-((5*aR*,6*S*,9*R*)-1-fluoro-12-(((2*R*,7*aR* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| | 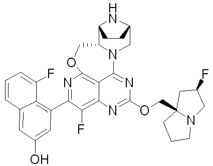 | 5-Fluoro-4-((5*aS*,6*R*,9*S*)-1-fluoro-12-(((2*R*,7*aR*) -2-fluorotetrahydro-1*H*-pyrrolizin-7*a*(5*H*)-yl)me thoxy)-5*a*,6,7,8,9,10-hexahydro-5*H*-4-oxa-3,10 *a*, 11,13,14-pentaaza-6,9-methanonaphtho[1,8-*a b*]heptalen-2-yl)naphthalen-2-ol |
| | 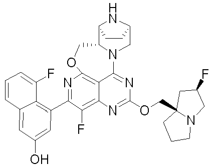 | 5-Fluoro-4-((5*aS*,6*S*,9*R*)-1-fluoro-12-(((2*R*,7*aR*) -2-fluorotetrahydro-1*H*-pyrrolizin-7*a*(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| | 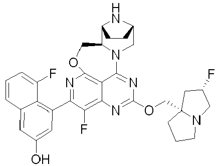 | 5-Fluoro-4-((5*aR*,6*R*,9*S*)-1-fluoro-12-(((2*S*,7*aS*) -2-fluorotetrahydro-1*H*-pyrrolizin-7*a*(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| | 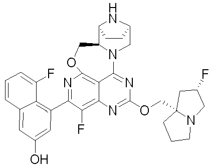 | 5-Fluoro-4-((5*aR*,6*S*,9*R*)-1-fluoro-12-(((2*S*,7*aS*) -2-fluorotetrahydro-1*H*-pyrrolizin-7*a*(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| | 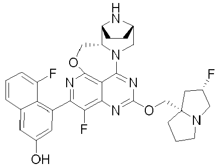 | 5-Fluoro-4-((5*aS*,6*R*,9*S*)-1-fluoro-12-(((2*S*,7*aS*) -2-fluorotetrahydro-1H-pyrrolizin-7*a*(5*H*)-yl)me thoxy)-5a,6,7,8,9,10-hexahydro-5H-4-oxa-3,10 *a*,11,13,14-pentaaza-6,9-methanonaphtho[1,8-*a b*]heptalen-2-yl)naphthalen-2-ol |
| | 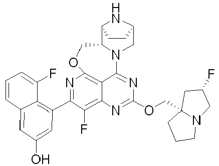 | 5-Fluoro-4-((5*aS*,6*S*,9*R*)-1-fluoro-12-(((2*S*,7*aS*) -2-fluorotetrahydro-1H-pyrrolizin-7*a*(5*H*)-yl)me thoxy)-Sa,6,7,8,9,10-hexahydro-SH-4-oxa-3,10 *a*,11,13,14-pentaaza-6,9-methanonaphtho[1,8-*a b*]heptalen-2-yl)naphthalen-2-ol |
| | 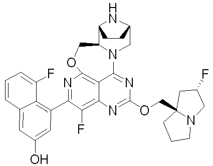 | 5-Fluoro-4-((5*aR*,6*R*,9*S*)-1-fluoro-12-(((2*S*,7*aR*) -2-fluorotetrahydro-1H-pyrrolizin-7*a*(5*H*)-yl)me thoxy)-5a,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| | 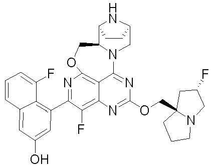 | 5-Fluoro-4-((5*aR*,6*S*,9*R*)-1-fluoro-12-(((2*S*,7*aR*) -2-fluorotetrahydro-1H-pyrrolizin-7*a*(5*H*)-yl)me thoxy)-5a,6,7,8,9,10-hexahydro-5H-4-oxa-3, 10 *a*, 11,13,14-pentaaza-6,9-methanonaphtho[1,8-*a b*]heptalen-2-yl)naphthalen-2-ol |
| | 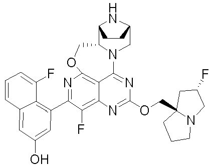 | 5-Fluoro-4-((5*aS*,6*R*,9*S*)-1-fluoro-12-(((2*S*,7*aS*) -2-fluorotetrahydro-1H-pyrrolizin-7*a*(5*H*)-yl)me thoxy)-5a,6,7,8,9,10-hexahydro-5H-4-oxa-3,10 *a*, 11,13,14-pentaaza-6,9-methanonaphtho[1,8-*a b*]heptalen-2-yl)naphthalen-2-ol |
| | 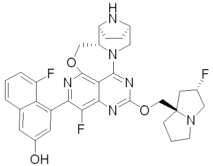 | 5-Fluoro-4-((5*aS*,6*S*,9*R*)-1-fluoro-12-(((2*S*,7*aR*) -2-fluorotetrahydro-1H-pyrrolizin-7*a*(5*H*)-yl)me thoxy)-5a,6,7,8,9,10-hexahydro-5H-4-oxa-3,10 *a*,11,13,14-pentaaza-6,9-methanonaphtho[1,8-*a b*]heptalen-2-yl)naphthalen-2-ol |
| | 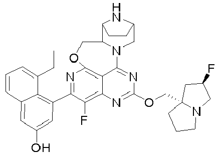 | 5-Ethyl-4-(1-fluoro-12-(((2*R*,7*aS*)-2-fluorotetra hydro-1*H*-pyrrolizin-7*a*(5*H*)-yl)methoxy)-5*a*,6, 7,8,9,10-hexahydro-5*H*-4-oxa-3,10*a*,11,13,14-p entaaza-6,9-methanonaphtho[1,8-*ab*]heptalen-2-yl)naphthalen-2-ol |
| | 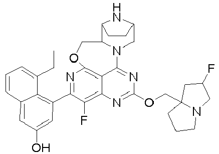 | 5-Ethyl-4-(1-fluoro-12-((2-fluorotetrahydro-1*H-*pyrrolizin-7a(5*H*)-yl)methoxy)-5*a*,6,7,8,9,10-he xahydro-5*H*-4-oxa-3,10*a*,11,13,14-pentaaza-6,9 -methanonaphtho[1,8-*ab*]heptalen-2-yl)naphthal en-2-ol |
| 4-p2 | 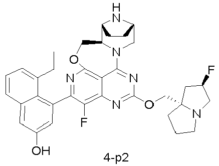 | 5-Ethyl-4-((5*aR*,6*R*,9*S*)-1-fluoro-12-(((2*R*,7*aS*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol **4-p2** |
| 12-p1 | 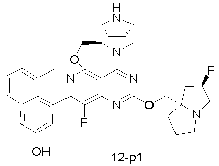 | 5-Ethyl-4-((5*aR*,6*S*,9*R*)-1-fluoro-12-(((2*R*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol **12-p1** |
| 12-p2 | 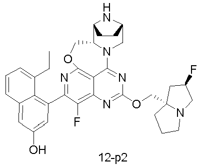 | 5-Ethyl-4-((5*aS*,6*R*,9*S*)-1-fluoro-12-(((2*R*,7*aS*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol **12-p2** |
| 4-p1 | 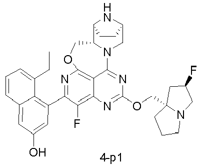 | S-Ethyl-4-((SaS,6S,9R)-I-fluoro-12-(((2R,7aS)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8-*ab*] heptalen-2-yl)naphthalen-2-ol **4-p1** |
| | 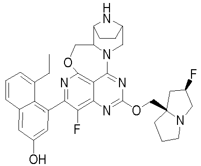 | 5-Ethyl-4-(1-fluoro-12-(((2*R*,7*aR*)-2-fluorotetra hydro-1*H*-pyrrolizin-7*a*(5*H*)-yl)methoxy)-5*a*,6, 7,8,9,10-hexahydro-5*H*-4-oxa-3,10*a*,11,13,14-p entaaza-6, 9-methanonaphtho[1,8-*ab*]heptalen-2-yl)naphthalen-2-ol |
| 5-p2 | 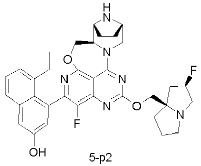 | 5-Ethyl-4-((5*aR*,6*R*,9*S*)-1-fluoro-12-(((2*R*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol **5-p2** |
| | 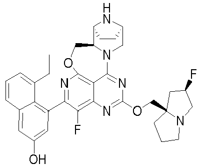 | 5-Ethyl-4-((5*aR*,6*S*,9*R*)-1-fluoro-12-(((2*R*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol |
| | 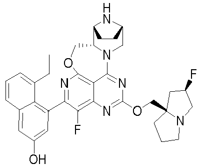 | 5-Ethyl-4-((5*aS*,6*R*,9*S*)-1-fluoro-12-(((2*R*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol |
| 5-p 1 | 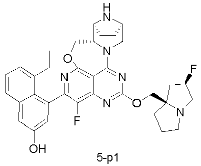 | 5-Ethyl-4-((5*aS*,6*S*,9*R*)-1-fluoro-12-(((2*R*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol **5-p1** |
| | 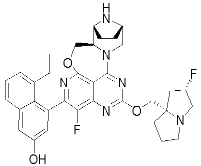 | 5-Ethyl-4-((5*aR*,6*R*,9*S*)-1-fluoro-12-(((2*S*,7*aS*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol |
| | 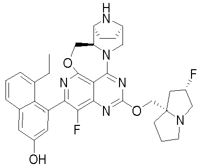 | 5-Ethyl-4-((5*aR*,6*S*,9*R*)-1-fluoro-12-(((2*S*,7*aS*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol |
| | 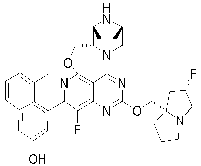 | 5-Ethyl-4-((5*aS*,6*R*,9*S*)-1-fluoro-12-(((2*S*,7*aS*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol |
| | 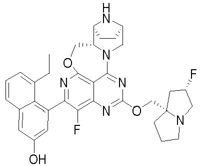 | 5-Ethyl-4-((5*aS*,6*S*,9*R*)-1-fluoro-12-(((2*S*,7*aS*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol |
| | 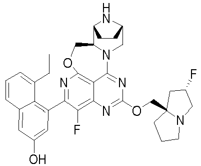 | 5-Ethyl-4-((5*aS*,6*R,*9*S*)-1-fluoro-12-(((2*S*,7*aS*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol |
| | 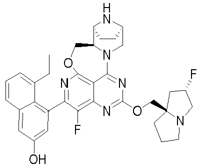 | 5-Ethyl-4-((5*aR*,6*S*,9*R*)-1-fluoro-12-(((2*S*,7*aS*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol |
| | 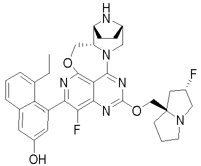 | 5-Ethyl-4-((5*aS*,6*R*,9*S*)-1-fluoro-12-(((2*S*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol |
| | 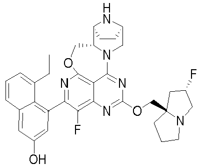 | 5-Ethyl-4-((5*aS*,6*S*,9*R*)-1-fluoro-12-(((2*S*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10*a* ,11,13,14-pentaaza-6,9-methanonaphtho[1,8*-ab*] heptalen-2-yl)naphthalen-2-ol |
| | 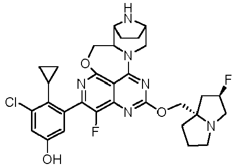 | 3-Chloro-4-cyclopropyl-5-(1-fluoro-12-(((2*R*,7*a S*)-2-fluorotetrahydro-1*H*-pyrrolizin-7*a*(5*H*)-yl) methoxy)-5*a*,6,7,8,9,10-hexahydro-5*H*-4-oxa-3, 10*a*,11,13,14-pentaaza-6,9-methanonaphtho[1,8 -*ab*]heptalen-2-yl)phenol |
| | 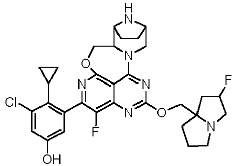 | 3 -Chloro-4-cyclopropyl-5 -(1 -fluoro-12-(2-fluor otetrahydro-1*H*-pyrrolizin-7*a*(5*H*)-yl)methoxy)-5*a*,6,7,8,9,10-hexahydro-5*H*-4-oxa-3,10*a*,11,13, 14-pentaaza-6,9-methanonaphtho[1,8-*ab*]heptal en-2-yl)phenol |
| 6-p2 | 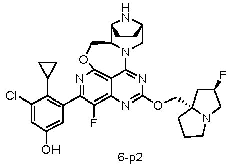 | 3-Chloro-4-cyclopropyl-5-((5*aR*,6*R*,9*S*)-1-fluor o-12-(((2*R*,7aS)-2-fluorotetrahydro-1*H*-pyrrolizi n-7*a*(5*H*)-yl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)phenol **6-p2** |
| | 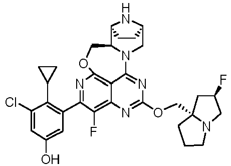 | 3-Chloro-4-cyclopropyl-5-((SaR,6S,9R)-1-fluor o-12-(((2*R*,7*aS*)-2-fluorotetrahydro-1*H*-pyrrolizi n-7*a*(5*H*)-yl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)phenol |
| | 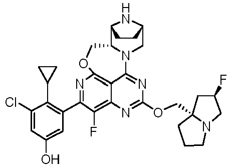 | *3*-Chloro-4-cyclopropyl-5-((5*aS*,6*R*,9*S*)-1-fluoro -12-(((2*R*,7aS)-2-fluorotetrahydro-1*H*-pyrrolizin -7*a*(5*H*)-yl)methoxy)-5*a*,6,7,8,9,10-hexahydro-5*H*-4-oxa-3,10*a*,11,13,14-pentaaza-6,9-methano naphtho[1,8-*ab*]heptalen-2-yl)phenol |
| 6-p1 | 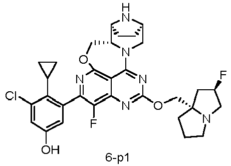 | 3-Chloro-4-cyclopropyl-5-((SaS,6S,9R)-1-fluoro -12-(((2*R*,7*aS*)-2-fluorotetrahydro-1*H*-pyrrolizin -7*a*(5*H*)-yl)methoxy)-5a,6,7,8,9,10-hexahydro-5*H*-4-oxa-3,10a,11,13,14-pentaaza-6,9-methano naphtho[1,8-*ab*]heptalen-2-yl)phenol **6-p1** |
| | 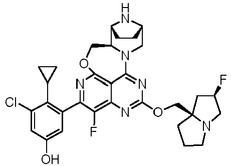 | 3-Chloro-4-cyclopropyl-5-((5*aR*,6*R*,9*S*)-1-fluor o-12-(((2*R*,7*aR*)-2-fluorotetrahydro-1*H*-pyrroliz in-7*a*(5*H*)-yl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)phenol |
| | 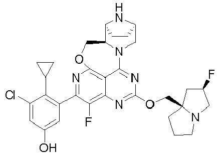 | 3-Chloro-4-cyclopropyl-5-((SaR,6S,9R)-1-fluor o-12-(((2*R*,7*aR*)-2-fluorotetrahydro-1*H*-pyrroliz in-7*a*(5*H*)-yl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)phenol |
| | 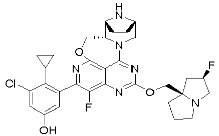 | 3-Chloro-4-cyclopropyl-5-((5*aS*,6*R*,9*S*)-1-fluoro -12-(((2*R*,7*aR*)-2-fluorotetrahydro-1*H*-pyrrolizi n-7*a*(5*H*)-yl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)phenol |
| | 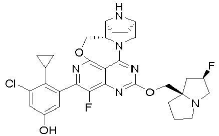 | 3-Chloro-4-cyclopropyl-5-((SaS,6S,9R)-1-fluoro -12-(((2*R*,7*aR*)-2-fluorotetrahydro-1*H*-pyrrolizi n-7*a*(5*H*)-yl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)phenol |
| | 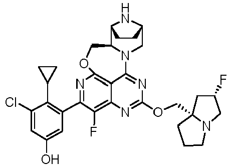 | *3-*Chloro-4-cyclopropyl-5*-*((5*aR*,6*R*,9*S*)*-*1-fluor o-12-(((2*S*,7*aS*)-2-fluorotetrahydro-1*H*-pyrrolizi n-7*a*(5*H*)-yl)methoxy)-5*a*,6,7,8,9,10-hexahydro *-*5*H*-4-oxa-3,10*a*,11,13,14-pentaaza-6,9-methan onaphtho[1,8-*ab*]heptalen-2-yl)phenol |
| | 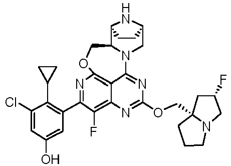 | 3-Chloro-4-cyclopropyl-5-((5*aR*,6*S*,9*R*)-1-fluor o-12-(((2*S*,7*aS*)-2-fluorotetrahydro-1*H*-pyrrolizi n-7*a*(5*H*)-yl)methoxy)-5*a*,6,7,8,9,10-hexahydro *-*5*H-*4-oxa-3,10*a*,11,13,14-pentaaza-6,9-methan onaphtho[1,8-*ab*]heptalen-2-yl)phenol |
| | 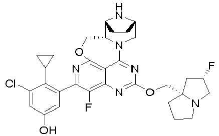 | 3-Chloro-4-cyclopropyl-5-((5*aS*,6*R*,9*S*)-1-fluoro -12-(((2*S*,7*aS*)-2-fluorotetrahydro-1*H*-pyrrolizin -7a(5*H*)-yl)methoxy)-5*a*,6,7,8,9,10-hexahydro-5*H*-4-oxa-3,10*a*,11,13,14-pentaaza-6,9-methano naphtho[1,8-*ab*]heptalen-2-yl)phenol |
| | 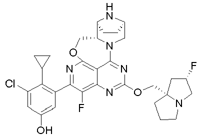 | 3-Chloro-4-cyclopropyl-5-((5*aS*,6*S*,9*R*)-1-fluoro -12-(((2*S*,7*aS*)-2-fluorotetrahydro-1*H*-pyrrolizin -7*a*(5*H*)-yl)methoxy)-5*a*,6,7,8,9,10-hexahydro-5*H*-4-oxa-3,10*a*,11,13,14-pentaaza-6,9-methano naphtho[1,8-*ab*]heptalen-2-yl)phenol |
| | 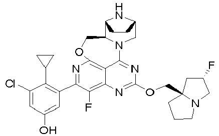 | 3-Chloro-4-cyclopropyl-5-((5*aR*,6*R*,9*S*)-1-fluor o-12-(((2*S*,7*aR*)-2-fluorotetrahydro-1*H*-pyrrolizi n-7*a*(5*H*)-yl)methoxy)-5*a*,6,7,8,9,10-hexahydro *-*5*H*-4-oxa-3,10*a*,11,13,14-pentaaza-6,9-methan onaphtho[1,8-*ab*]heptalen-2-yl)phenol |
| | 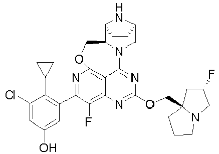 | 3-Chloro-4-cyclopropyl-5-((5*aR*,6*S*,9*R*)-1-fluor o-12-(((2*S*,7*aR*)-2-fluorotetrahydro-1*H*-pyrrolizi n-7*a*(5*H*)-yl)methoxy)-5*a*,6,7,8,9,10-hexahydro *-*5*H*-4-oxa-3,10*a*,11,13,14-pentaaza-6,9-methan onaphtho[1,8-*ab*]heptalen-2-yl)phenol |
| | 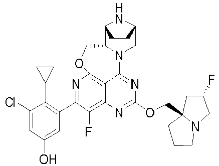 | 3-Chloro-4-cyclopropyl-5-((5*aS*,6*R*,9*S*)-1-fluoro -12-(((2*S*,7*aR*)-2-fluorotetrahydro-1*H*-pyrrolizin -7*a*(5*H*)-yl)methoxy)-5*a*,6,7,8,9,10-hexahydro-5*H*-4-oxa-3,10*a*,11,13,14-pentaaza-6,9-methano naphtho[1,8-*ab*]heptalen-2-yl)phenol |
| | 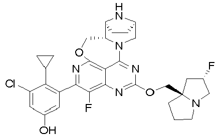 | 3-Chloro-4-cyclopropyl-5-((5*aS*,6*S*,9*R*)-1-fluoro -12-(((2*S*,7*aR*)-2-fluorotetrahydro-1*H*-pyrrolizin -7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)phenol |
| | 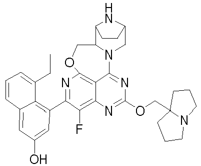 | 5-Ethyl-4-(1-fluoro-12-((tetrahydro-1*H*-pyrroliz in-7*a*(5*H*)-yl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)naphthalen-2-ol |
| 7-p2 | 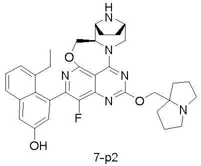 | 5-Ethyl-4-((5*aR*,6*R*,9*S*)-1-fluoro-12-((tetrahydro -1*H*-pyrrolizin-7*a*(5*H*)-yl)methoxy)-5*a*,6,7,8,9,1 0-hexahydro-5*H*-4-oxa-3,10*a*,11,13,14-pentaaza -6,9-methanonaphtho[1,8-*ab*]heptalen-2-yl)naph thalen-2-ol **7-p2** |
| | 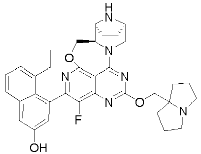 | 5-Ethyl-4-((5*aR*,6*S*,9*R*)-1-fluoro-12-((tetrahydro -1*H*-pyrrolizin-7*a*(5*H*)-yl)methoxy)-5*a*,6,7,8,9,1 0-hexahydro-5*H*-4-oxa-3,10*a*,11,13,14-pentaaza -6,9-methanonaphtho[1,8-*ab*]heptalen-2-yl)naph thalen-2-ol |
| | 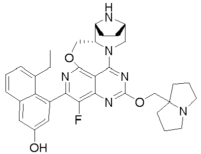 | 5-Ethyl-4-((5*aS*,6*R*,9*S*)-1-fluoro-12-((tetrahydro -1*H*-pyrrolizin-7*a*(5*H*)-yl)methoxy)-5*a*,6,7,8,9,1 0-hexahydro-5*H*-4-oxa-3,10*a*,11,13,14-pentaaza -6,9-methanonaphtho[1,8-*ab*]heptalen-2-yl)naph thalen-2-ol |
| 7-p1 | 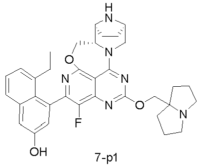 | 5-Ethyl-4-((5*aS*,6*S*,9*R*)-1-fluoro-12-((tetrahydro -1*H*-pyrrolizin-7*a*(5*H*)-yl)methoxy)-5*a*,6,7,8,9,1 0-hexahydro-5*H*-4-oxa-3,10*a*,11,13,14-pentaaza -6,9-methanonaphtho[1,8-*ab*]heptalen-2-yl)naph thalen-2-ol **7-p1** |
| | 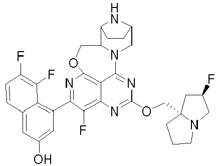 | 5,6-Difluoro-4-(1-fluoro-12-(((2*R*,7*aS*)-2-fluoro tetrahydro-1*H*-pyrrolizin-7*a*(5*H*)-yl)methoxy)-5 *a*,6,7,8,9,10-hexahydro-5*H*-4-oxa-3,10*a*,11,13,1 4-pentaaza-6,9-methanonaphtho[1,8-*ab*]heptale n-2-yl)naphthalen-2-ol |
| | 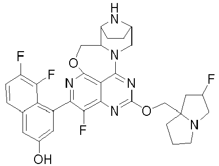 | 5,6-Difluoro-4-(1-fluoro-12-((2-fluorotetrahydr o-1*H*-pyrrolizin-7*a*(5*H*)-yl)methoxy)-5*a*,6,7,8,9, 10-hexahydro-5*H*-4-oxa-3,10*a*,11,13,14-pentaaz a-6,9-methanonaphtho[1,8-*ab*]heptalen-2-yl)nap hthalen-2-ol |
| 8-p2 | 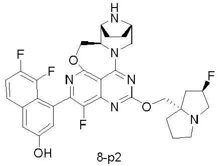 | 5,6-Difluoro-4-((5*aR*,6*R*,9*S*)-1-fluoro-12-(((2*R*, 7*aS*)-2-fluorotetrahydro-1*H*-pyrrolizin-7*a*(5*H*)-y l)methoxy)-5*a*,6,7,8,9,10-hexahydro-5*H*-4-oxa-3,10*a*,11,13,14-pentaaza-6,9-methanonaphtho[1 ,8-*ab*]heptalen-2-yl)naphthalen-2-ol **8-p2** |
| | 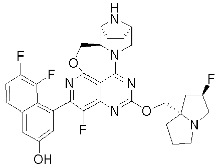 | 5,6-Difluoro-4-((5*aR*,6*S*,9*R*)-1-fluoro-12-(((2*R*, 7*aS*)-2-fluorotetrahydro-1*H*-pyrrolizin-7*a*(5*H*)-y l)methoxy)-5*a*,6,7,8,9,10-hexahydro-5*H*-4-oxa-3,10*a*,11,13,14-pentaaza-6,9-methanonaphtho[1 ,8-*ab*]heptalen-2-yl)naphthalen-2-ol |
| | 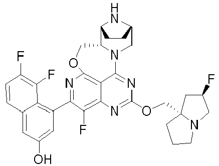 | 5,6-Difluoro-4-((5*aS*,6*R*,9*S*)-1-fluoro-12-(((2*R*,7 *aS*)-2-fluorotetrahydro-1*H*-pyrrolizin-7*a*(5*H*)-yl )methoxy)-5*a*,6,7,8,9,10-hexahydro-5*H*-4-oxa-3 ,10*a*,11,13,14-pentaaza-6,9-methanonaphtho[1, 8-*ab*]heptalen-2-yl)naphthalen-2-ol |
| 8-p1 | 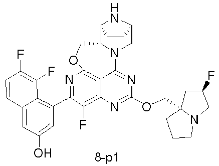 | 5,6-Difluoro-4-((5*aS*,6*S*,9*R*)-1-fluoro-12-(((2*R*,7 *aS*)-2-fluorotetrahydro-1*H*-pyrrolizin-7*a*(5*H*)-yl )methoxy)-5*a*,6,7,8,9,10-hexahydro-5*H*-4-oxa-3 ,10*a*,11,13,14-pentaaza-6,9-methanonaphtho[1, 8-*ab*]heptalen-2-yl)naphthalen-2-ol **8-p1** |
| | 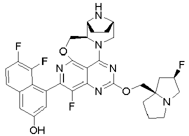 | 5,6-Difluoro-4-((5*aR*,6*R*,9*S*)-1-fluoro-12-(((2*R*, 7*aR*)-2-fluorotetrahydro-1*H*-pyrrolizin-7*a*(5*H*)-yl)methoxy)-5*a*,6,7,8,9,10-hexahydro-5*H*-4-oxa -3,10*a*,11,13,14-pentaaza-6,9-methanonaphtho[ 1,8-*ab*]heptalen-2-yl)naphthalen-2-ol |
| | 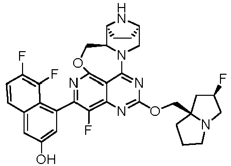 | 5,6-Difluoro-4-((5*aR*,6*S*,9*R*)-1-fluoro-12-(((2*R*, 7*aR*)-2-fluorotetrahydro-1*H*-pyrrolizin-7*a*(5*H*)-yl)methoxy)-5*a*,6,7,8,9,10-hexahydro-5*H*-4-oxa -3,10*a*,11,13,14-pentaaza-6,9-methanonaphtho[ 1,8-*ab*]heptalen-2-yl)naphthalen-2-ol |
| | 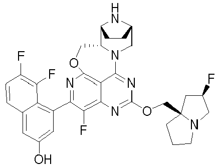 | 5,6-Difluoro-4-((5*aS*,6*R*,9*S*)-1-fluoro-12-(((2*R*,7 *aR*)-2-fluorotetrahydro-1*H*-pyrrolizin-7*a*(5*H*)-yl )methoxy)-5*a*,6,7,8,9,10-hexahydro-5*H*-4-oxa-3 ,10*a*,11,13,14-pentaaza-6,9-methanonaphtho[1, 8-*ab*]heptalen-2-yl)naphthalen-2-ol |
| | 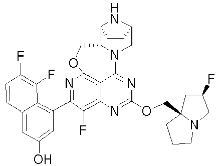 | 5,6-Difluoro-4-((5*aS*,6*S*,9*R*)-1-fluoro-1-(((2*R*,7 *aR*)-2-fluorotetrahydro-1*H*-pyrrolizin-7*a*(5*H*)-yl )methoxy)-5*a*,6,7,8,9,10-hexahydro-5*H*-4-oxa-3 ,10*a*,11,13,14-pentaaza-6,9-methanonaphtho[1, 8-*ab*]heptalen-2-yl)naphthalen-2-ol |
| | 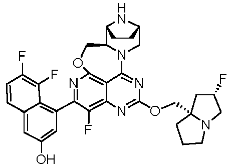 | 5,6-Difluoro-4-((5*aR*,6*R*,9*S*)-1-fluoro-12-(((2*S*,7 *aS*)-2-fluorotetrahydro-1*H*-pyrrolizin-7*a*(5*H*)-yl )methoxy)-5*a*,6,7,8,9,10-hexahydro-5*H*-4-oxa-3 ,10*a*,11,13,14-pentaaza-6,9-methanonaphtho[1, 8-*ab*]heptalen-2-yl)naphthalen-2-ol |
| | 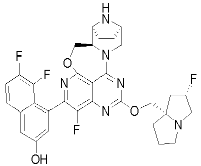 | 5,6-Difluoro-4-((5*aR*,6*S*,9*R*)-1-fluoro-12-(((2*S*,7 *aS*)-2-fluorotetrahydro-1*H*-pyrrolizin-7*a*(5*H*)-yl )methoxy)-5*a*,6,7,8,9,10-hexahydro-5*H*-4-oxa-3 ,10*a*,11,13,14-pentaaza-6,9-methanonaphtho[1, 8-*ab*]heptalen-2-yl)naphthalen-2-ol |
| | 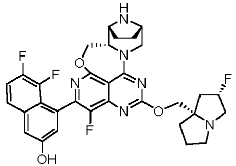 | 5,6-Difluoro-4-((5*aS*,6*R*,9*S*)-1-fluoro-12-(((2*S*,7 *aS*)-2-fluorotetrahydro-1*H*-pyrrolizin-7*a*(5*H*)-yl )methoxy)-5*a*,6,7,8,9,10-hexahydro-5*H*-4-oxa-3 ,10*a*,11,13,14-pentaaza-6,9-methanonaphtho[1, 8-*ab*]heptalen-2-yl)naphthalen-2-ol |
| | 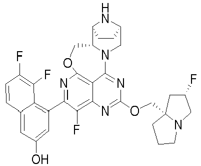 | 5,6-Difluoro-4-((5*aS*,6*S*,9*R*)-1-fluoro-12-(((2*R*,7 *aS*)-2-fluorotetrahydro-1*H*-pyrrolizin-7*a*(5*H*)-yl )methoxy)-5*a*,6,7,8,9,10-hexahydro-5*H*-4-oxa-3 ,10*a*,11,13,14-pentaaza-6,9-methanonaphtho[1, 8-*ab*]heptalen-2-yl)naphthalen-2-ol |
| | 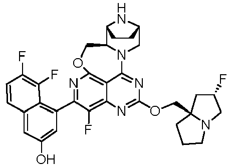 | 5,6-Difluoro-4-((5*aR*,6*R*,9*S*)-1-fluoro-12-(((2*S*,7 *aR*)-2-fluorotetrahydro-1*H*-pyrrolizin-7*a*(5*H*)-yl )methoxy)-5*a*,6,7,8,9,10-hexahydro-5*H*-4-oxa-3 ,10*a*,11,13,14-pentaaza-6,9-methanonaphtho[1, 8-*ab*]heptalen-2-yl)naphthalen-2-ol |
| | 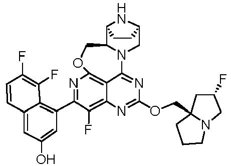 | 5,6-Difluoro-4-((5*aR*,6*S*,9*R*)-1-fluoro-12-(((2*S*,7 *aR*)-2-fluorotetrahydro-1*H*-pyrrolizin-7*a*(5*H*)-yl )methoxy)-5*a*,6,7,8,9,10-hexahydro-5*H*-4-oxa-3 ,10*a*,11,13,14-pentaaza-6,9-methanonaphtho[1, 8-*ab*]heptalen-2-yl)naphthalen-2-ol |
| | 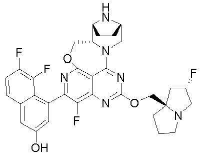 | 5,6-Difluoro-4-((5*aS*,6*R*,9*S*)-1-fluoro-12-(((2*S*,7 *aR*)-2-fluorotetrahydro-1*H*-pyrrolizin-7*a*(5*H*)-yl )methoxy)-5*a*,6,7,8,9,10-hexahydro-5*H*-4-oxa-3 ,10*a*,11,13,14-pentaaza-6,9-methanonaphtho[1, 8-*ab*]heptalen-2-yl)naphthalen-2-ol |
| | 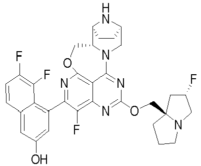 | 5,6-Difluoro-4-((5*aS*,6*S*,9*R*)-1-fluoro-12-(((2*S*,7 *aR*)-2-fluorotetrahydro-1*H*-pyrrolizin-7*a*(5*H*)-yl )methoxy)-5*a*,6,7,8,9,10-hexahydro-5*H*-4-oxa-3 ,10*a*,11,13,14-pentaaza-6,9-methanonaphtho[1, 8-*ab*]heptalen-2-yl)naphthalen-2-ol |
| | 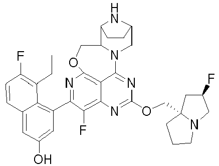 | 5-Ethyl-6-fluoro-4-(1-fluoro-12-(((2*R*,7*aS*)-2-fl uorotetrahydro-1*H*-pyrrolizin-7*a*(5*H*)-yl)methox y)-5*a*,6,7,8,9,10-hexahydro-5*H*-4-oxa-3,10*a*,11, 13,14-pentaaza-6,9-methanonaphtho[1,8-*ab*]hep talen-2-yl)naphthalen-2-ol |
| | 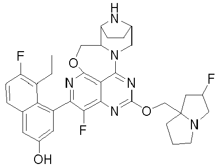 | 5-Ethyl-6-fluoro-4-(1-fluoro-12-((2-fluorotetrah ydro-1*H*-pyrrolizin-7*a*(5*H*)-yl)methoxy)-5*a*,6,7, 8,9,10-hexahydro-5*H*-4-oxa-3,10*a*,11,13,14-pen taaza-6,9-methanonaphtho[1,8-*ab*]heptalen-2-yl )naphthalen-2-ol |
| 9-p2 | 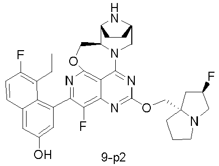 | 5-Ethyl-6-fluoro-4-((5*aR*,6*R*,9*S*)-1-fluoro-12-((( 2*R*,7*aS*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol **9-p2** |
| | 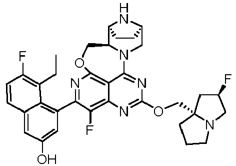 | 5-Ethyl-6-fluoro-4-((5*aR*,6*S*,9*R*)-1-fluoro-12-((( 2*R*,7*aS*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| | 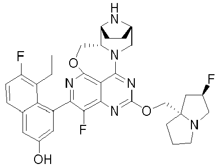 | 5-Ethyl-6-fluoro-4-((5*aS*,6*R*,9*S*)-1-fluoro-12-((( 2*R*,7*aS*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| 9-p1 | 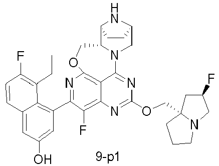 | 5-Ethyl-6-fluoro-4-((5*aS*,6*S*,9*R*)-1-fluoro-12-((( 2*R*,7*aS*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol **9-p1** |
| | 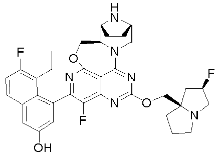 | 5-Ethyl-6-fluoro-4-((5*aR*,6*R*,9*S*)-1-fluoro-12-((( 2*R*,7*aR*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| | 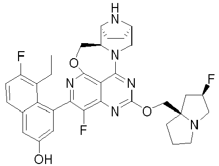 | 5-Ethyl-6-fluoro-4-((5*aR*,6*S*,9*R*)-1-fluoro-12-((( 2*R*,7*aR*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| | 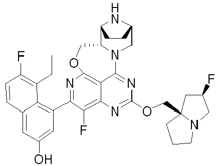 | 5-Ethyl-6-fluoro-4-((5*aS*,6*R*,9*S*)-1-fluoro-12-((( 2*R*,7*aR*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| | 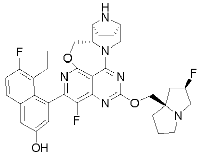 | 5-Ethyl-6-fluoro-4-((5*aS*,6*S*,9*R*)-1-fluoro-12-((( 2*R*,7*aR*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| | 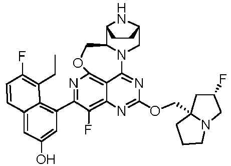 | 5-Ethyl-6-fluoro-4-((5*aR*,6*R*,9*S*)-1-fluoro-12-((( 2*S*,7*aS*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| | 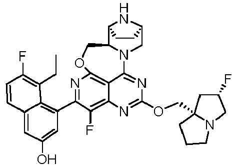 | 5-Ethyl-6-fluoro-4-((5*aR*,6*S*,9*R*)-1-fluoro-12-((( 2*S*,7*aS*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| | 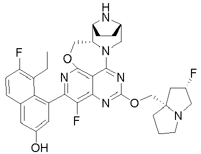 | 5-Ethyl-6-fluoro-4-((5*aS*,6*R*,9*S*)-1-fluoro-12-((( 2*S*,7*aS*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| | 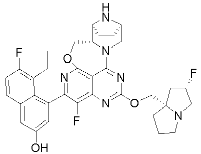 | 5-Ethyl-6-fluoro-4-((5*aS*,6*S*,9*R*)-1-fluoro-12-((( 2*S*,7*aS*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol |
| | 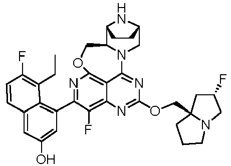 | 5-Ethyl-6-fluoro-4-((5*aR*,6*R*,9*S*)-1-fluoro-12-((( 2*S*,7*aR*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| | 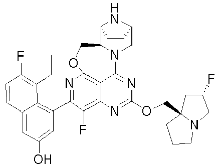 | 5-Ethyl-6-fluoro-4-((5*aR*,6*S*,9*R*)-1-fluoro-12-((( 2*S*,7*aR*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| | 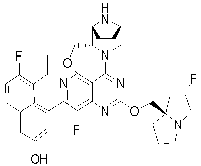 | 5-Ethyl-6-fluoro-4-((5*aS*,6*R*,9*S*)-1-fluoro-12-((( 2*S*,7*aR*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| | 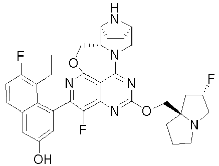 | 5-Ethyl-6-fluoro-4-((5*aS*,6*S*,9*R*)-1-fluoro-12-((( 2*S*,7*aR*)-2-fluorotetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol |
| | 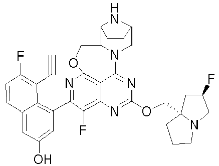 | 5-Ethynyl-6-fluoro-4-(1-fluoro-12-(((2*R*,7*aS*)-2-fluorotetrahydro-1*H*-pyrrolizin-7*a*(5*H*)-yl)meth oxy)-5*a*,6,7,8,9,10-hexahydro-5*H*-4-oxa-3,10*a*, 11,13,14-pentaaza-6,9-methanonaphtho[1,8-*ab*] heptalen-2-yl)naphthalen-2-ol |
| | 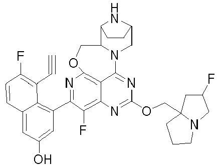 | 5-Ethynyl-6-fluoro-4-(1-fluoro-12-((2-fluorotetr ahydro-1*H*-pyrrolizin-7*a*(5*H*)-yl)methoxy)-5*a*,6, 7,8,9,10-hexahydro-5*H*-4-oxa-3,10*a*,11,13,14-p entaaza-6,9-methanonaphtho[1,8-*ab*]heptalen-2-yl)naphthalen-2-ol |
| 10-p2 | 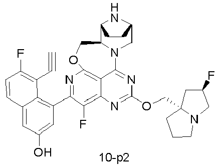 | 5-Ethynyl-6-fluoro-4-((5*aR*,6*R*,9*S*)-1-fluoro-12-(((2*R*,7*aS*)-2-fluorotetrahydro-1*H*-pyrrolizin-7*a*( 5*H*)-yl)methoxy)-5*a*,6,7,8,9,10-hexahydro-5*H*-4 -oxa-3,10*a*,11,13,14-pentaaza-6,9-methanonaph tho[1,8-*ab*]heptalen-2-yl)naphthalen-2-ol **10-p2** |
| | 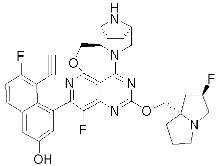 | 5-Ethynyl-6-fluoro-4-((5*aR*,6*S*,9*R*)-1-fluoro-12-(((2*R*,7*a*S)-2-fluorotetrahydro-1*H*-pyrrolizin-7*a*( 5*H*)-yl)methoxy)-5*a*,6,7,8,9,10-hexahydro-5*H*-4 -oxa-3,10*a*,11,13,14-pentaaza-6,9-methanonaph tho[1,8-*ab*]heptalen-2-yl)naphthalen-2-ol |
| | 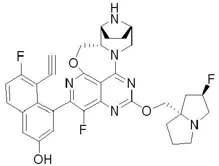 | 5-Ethynyl-6-fluoro-4-((5*aS*,6*R*,9*S*)-1-fluoro-12-(((2*R*,7*aS*)-2-fluorotetrahydro-1*H*-pyrrolizin-7*a*( 5*H*)-yl)methoxy)-5*a*,6,7,8,9,10-hexahydro-5*H*-4 -oxa-3,10*a*,11,13,14-pentaaza-6,9-methanonaph tho[1,8-*ab*]heptalen-2-yl)naphthalen-2-ol |
| 10-p1 | 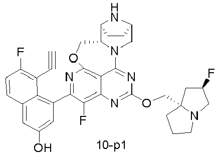 | 5-Ethynyl-6-fluoro-4-((5*aS*,6*S*,9*R*)-1-fluoro-12-(((2*R*,7*aS*)-2-fluorotetrahydro-1*H*-pyrrolizin-7*a*( 5*H*)-yl)methoxy)-5*a*,6,7,8,9,10-hexahydro-5*H*-4 -oxa-3,10*a*,11,13,14-pentaaza-6,9-methanonaph tho[1,8-*ab*]heptalen-2-yl)naphthalen-2-ol **10-p1** |
| | 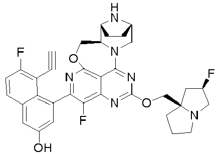 | 5-Ethynyl-6-fluoro-4-((5*aR*,6R,9*S*)-1-fluoro-12-(((2*R*,7*aR*)-2-fluorotetrahydro-1*H*-pyrrolizin-7*a* (5*H*)-yl)methoxy)-5*a*,6,7,8,9,10-hexahydro-5*H-*4-oxa-3,10*a*,11,13,14-pentaaza-6,9-methanonap htho[1,8-*ab*]heptalen-2-yl)naphthalen-2-ol |
| | 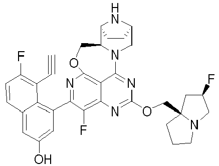 | 5-Ethynyl-6-fluoro-4-((5*aR*,6*S*,9*R*)-1-fluoro-12-(((2*R*,7*aR*)-2-fluorotetrahydro-1*H*-pyrrolizin-7*a* (5*H*)-yl)methoxy)-5*a*,6,7,8,9,10-hexahydro-5*H-*4-oxa-3,10*a*,11,13,14-pentaaza-6,9-methanonap htho[1,8-*ab*]heptalen-2-yl)naphthalen-2-ol |
| | 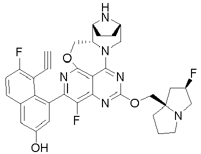 | 5-Ethynyl-6-fluoro-4-((5*aS*,6*R*,9*S*)-1-fluoro-12-(((2*R*,7*aR*)-2-fluorotetrahydro-1*H*-pyrrolizin-7*a* (5*H*)-yl)methoxy)-5*a*,6,7,8,9,10-hexahydro-5*H-*4-oxa-3,10*a*,11,13,14-pentaaza-6,9-methanonap htho[1,8-*ab*]heptalen-2-yl)naphthalen-2-ol |
| | 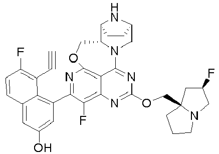 | 5-Ethynyl-6-fluoro-4-((5*aS*,6*S*,9*R*)-1-fluoro-12-(((2*R*,7*aR*)-2-fluorotetrahydro-1*H*-pyrrolizin-7*a* (5*H*)-yl)methoxy)-5*a*,6,7,8,9,10-hexahydro-5*H-*4-oxa-3,10*a*,11,13,14-pentaaza-6,9-methanonap htho[1,8-*ab*]heptalen-2-yl)naphthalen-2-ol |
| | 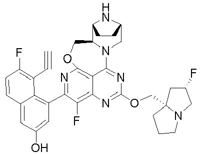 | 5-Ethynyl-6-fluoro-4-((5*aR*,6*R*,9*S*)-1-fluoro-12-(((2*S*,7*aS*)-2-fluorotetrahydro-1*H*-pyrrolizin-7*a*( 5*H*)-yl)methoxy)-5*a*,6,7,8,9,10-hexahydro-5*H*-4 -oxa-3,10*a*,11,13,14-pentaaza-6,9-methanonaphtho[1,8-*ab*]heptalen-2-yl)naphthalen-2-ol |
| | 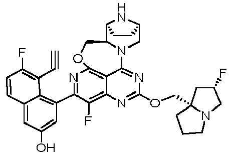 | 5-Ethynyl-6-fluoro-4-((5*aR*,6*S*,9*R*)-1-fluoro-12-(((2*S*,7*aS*)-2-fluorotetrahydro-1*H*-pyrrolizin-7*a*( 5*H*)-yl)methoxy)-5*a*,6,7,8,9,10-hexahydro-5*H*-4 -oxa-3,10*a*,11,13,14-pentaaza-6,9-methanonaph tho[1,8-*ab*]heptalen-2-yl)naphthalen-2-ol |
| | 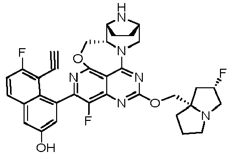 | 5-Ethynyl-6-fluoro-4-((5*aS*,6*R*,9*S*)-1-fluoro-12-(((2*S*,7*aS*)-2-fluorotetrahydro-1*H*-pyrrolizin-7*a*( 5*H*)-yl)methoxy)-5*a*,6,7,8,9,10-hexahydro-5*H*-4 -oxa-3,10*a*,11,13,14-pentaaza-6,9-methanonaph tho[1,8-*ab*]heptalen-2-yl)naphthalen-2-ol |
| | 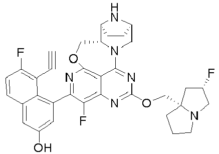 | 5-Ethynyl-6-fluoro-4-((5*aS*,6*S*,9*R*)-1-fluoro-12-(((2*S*,7*aS*)-2-fluorotetrahydro-1*H*-pyrrolizin-7*a*( 5*H*)-yl)methoxy)-5*a*,6,7,8,9,10-hexahydro-5*H*-4 -oxa-3,10*a*,11,13,14-pentaaza-6,9-methanonaph tho[1,8-*ab*]heptalen-2-yl)naphthalen-2-ol |
| | 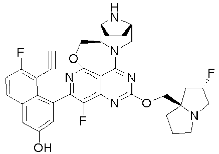 | 5-Ethynyl-6-fluoro-4-((5*aR*,6*R*,9*S*)-1-fluoro-12-(((2*S*,7*aR*)-2-fluorotetrahydro-1*H*-pyrrolizin-7*a*( 5*H*)-yl)methoxy)-5*a*,6,7,8,9,10-hexahydro-5*H*-4 -oxa-3,10*a*,11,13,14-pentaaza-6,9-methanonaph tho[1,8-*ab*]heptalen-2-yl)naphthalen-2-ol |
| | 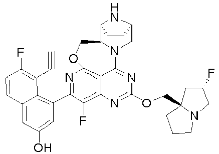 | 5-Ethynyl-6-fluoro-4-((5*aR*,6*S*,9*R*)-1-fluoro-12-(((2*S*,7*aR*)-2-fluorotetrahydro-1*H*-pyrrolizin-7*a*( 5*H*)-yl)methoxy)-5*a*,6,7,8,9,10-hexahydro-5*H*-4 -oxa-3,10*a*,11,13,14-pentaaza-6,9-methanonaph tho[1,8-*ab*]heptalen-2-yl)naphthalen-2-ol |
| | 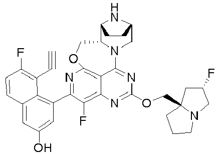 | 5-Ethynyl-6-fluoro-4-((5*aS*,6*R*,9*S*)-1-fluoro-12-(((2*S*,7*aR*)-2-fluorotetrahydro-1*H*-pyrrolizin-7*a*( 5*H*)-yl)methoxy)-5*a*,6,7,8,9,10-hexahydro-5*H*-4 -oxa-3,10*a*,11,13,14-pentaaza-6,9-methanonaph tho[1,8-*ab*]heptalen-2-yl)naphthalen-2-ol |
| | 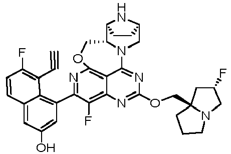 | 5-Ethynyl-6-fluoro-4-((5*aS*,6*S*,9*R*)-1-fluoro-12-(((2*S*,7*aR*)-2-fluorotetrahydro-1*H*-pyrrolizin-7*a*( 5*H*)-yl)methoxy)-5*a*,6,7,8,9,10-hexahydro-5*H*-4 -oxa-3,10*a*,11,13,14-pentaaza-6,9-methanonaph tho[1,8-*ab*]heptalen-2-yl)naphthalen-2-ol |
| | 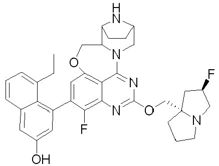 | 5-Ethyl-4-(1-fluoro-13-(((2*R*,7*aS*)-2-fluorotetra hydro-1*H*-pyrrolizin-7*a*(5*H*)-yl)methoxy)-5*a*,6, 7,8,9,10-hexahydro-5*H*-6,9-epiminoazepino[2',1 ':3,4][1,4]oxazepino[5,6,7-*de*]quinazolin-2-yl)na phthalen-2-ol |
| | 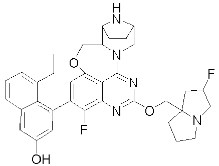 | 5-Ethyl-4-(1-fluoro-13-((2-fluorotetrahydro-1*H-*pyrrolizin-7*a(*5*H*)-yl)methoxy)-5*a*,6,7,8,9,10-he xahydro-5*H*-6,9-epiminoazepino[2',1':3,4][1,4]o xazepino[5,6,7-*de*]quinazolin-2-yl)naphthalen-2 -ol |
| 11-p2 | 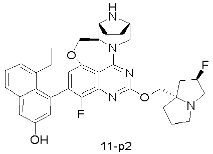 | 5-Ethyl-4-((5*aR*,6*R*,9*S*)-1-fluoro-13-(((2*R*,7*aS*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol **11-p2** |
| | 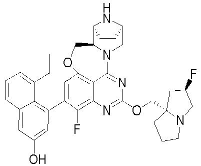 | 5-Ethyl-4-((5*aR*,6*S*,9*R*)-1-fluoro-13-(((2*R*,7*aS*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 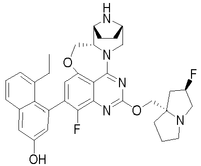 | 5-Ethyl-4-((5*aS*,6*R*,9*S*)-1-fluoro-13-(((2*R*,7*aS*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| 11-p1 | 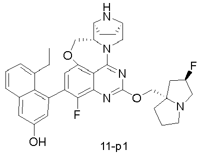 | 5-Ethyl-4-((5*aS*,6*S*,9*R*)-1-fluoro-13-(((2*R*,7*aS*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol **11-p1** |
| | 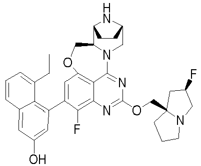 | 5-Ethyl-4-((5*aR*,6*R*,9*S*)-1-fluoro-13-(((2*R*,7*aR*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 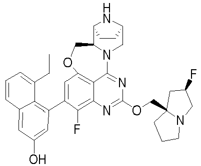 | 5-Ethyl-4-((5*aR*,6*S*,9*R*)-1-fluoro-13-(((2*R*,7*aR*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 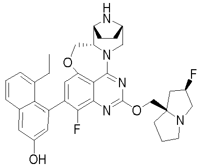 | 5-Ethyl-4-((5*aS*,6*R*,9*S*)-1-fluoro-13-(((2*R*,7*aR*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 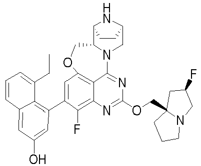 | 5-Ethyl-4-((5*aS*,6*S*,9*R*)-1-fluoro-13-(((2*R*,7*aR*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 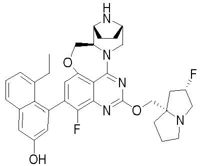 | 5-Ethyl-4-((5*aR*,6*R*,9*S*)-1-fluoro-13-(((2*S*,7*aS*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 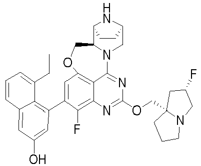 | 5-Ethyl-4-((5*aR*,6*S*,9*R*)-1-fluoro-13-(((2*S*,7*aS*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 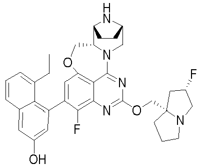 | 5-Ethyl-4-((5*aS*,6*R*,9*S*)-1-fluoro-13-(((2*S*,7*aS*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 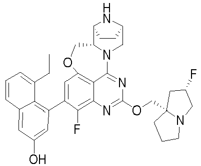 | 5-Ethyl-4-((5*aS*,6*S*,9*R*)-1-fluoro-13-(((2*S*,7*aS*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 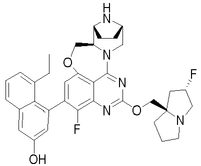 | 5-Ethyl-4-((5*aR*,6*R*,9*S*)-1-fluoro-13-(((2*S*,7*aR*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 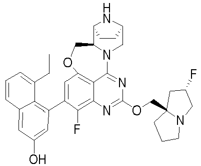 | 5-Ethyl-4-((5*aR*,6*S*,9*R*)-1-fluoro-13-(((2*S*,7*aR*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 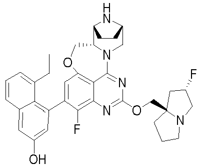 | 5-Ethyl-4-((5*aS*,6*R*,9*S*)-1-fluoro-13-(((2*S*,7*aR*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 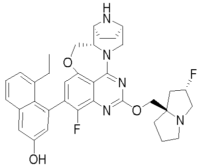 | 5-Ethyl-4-((5*aS*,6*S*,9*R*)-1-fluoro-13-(((2*S*,7*aR*)-2-fluorotetrahydro-1*H*-pyrrolizin-7*a*(5*H*)-yl)met hoxy)-5*a*,6,7,8,9,10-hexahydro-5*H*-6,9-epimino azepino[2',1':3,4][1,4]oxazepino[5,6,7-*de*]quina zolin-2-yl)naphthalen-2-ol |
| | 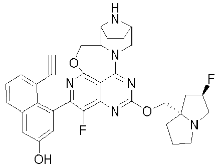 | 5-Ethynyl-4-(1-fluoro-12-(((2*R*,7*aS*)-2-fluorotet rahydro-1*H*pyrrolizin-7*a*(5*H*)-yl)methoxy)-5*a*, 6,7,8,9,10-hexahydro-5*H*-4-oxa-3,10*a*,11,13,14-pentaaza-6,9-methanonaphtho[1,8-*ab*]heptalen-2-yl)naphthalen-2-ol |
| | 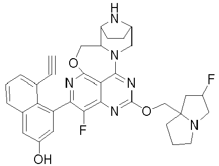 | 5-Ethynyl-4-(1-fluoro-12-((2-fluorotetrahydro-1 *H*-pyrrolizin-7*a*(5*H*)-yl)methoxy)-5*a*,6,7,8,9,10-hexahydro-5*H*-4-oxa-3,10*a*,11,13,14-pentaaza-6 ,9-methanonaphtho[1,8-*ab*]heptalen-2-yl)naphth alen-2-ol |
| 13-p2 | 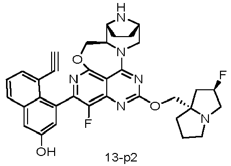 | 5-Ethynyl-4-((5*aR*,6*R*,9*S*)-1-fluoro-12-(((2*R*,7*a S*)-2-fluorotetrahydro-1*H*-pyrrolizin-7*a*(5*H*)-yl) methoxy)-5*a*,6,7,8,9,10-hexahydro-*5*H-4-oxa-3, 10*a*,11,13,14-pentaaza-6,9-methanonaphtho[1,8 -*ab*]heptalen-2-yl)naphthalen-2-ol **13-p2** |
| | 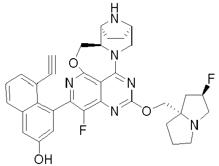 | 5-Ethynyl-4-((5*aR*,6*S*,9*R*)-1-fluoro-12-(((2*R*,7*a S*)-2-fluorotetrahydro-1*H*-pyrrolizin-7*a*(5*H*)-yl) methoxy)-5*a*,6,7,8,9,10-hexahydro-*5*H-4-oxa-3, 10*a*,11,13,14-pentaaza-6,9-methanonaphtho[1,8 -*ab*]heptalen-2-yl)naphthalen-2-ol |
| | 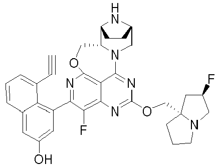 | 5-Ethynyl-4-((5*aS*,6*R*,9*S*)-1-fluoro-12-(((2*R*,7*aS* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| 13-p1 | 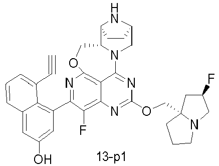 | 5-Ethynyl-4-((5*aS*,6*S*,9*R*)-1-fluoro-12-(((2*R*,7*aS* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol **13-p1** |
| | 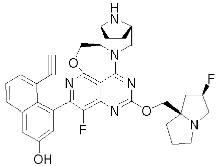 | 5-Ethynyl-4-((5*aR*,6*R*,9*S*)-1-fluoro-12-(((2*R*,7*a R*)-2-fluorotetrahydro-1*H*-pyrrolizin-7*a*(5*H*)-yl) methoxy)-5*a*,6,7,8,9,10-hexahydro-*5*H-4-oxa-3, 10*a*,11,13,14-pentaaza-6,9-methanonaphtho[1,8 -*ab*]heptalen-2-yl)naphthalen-2-ol |
| | 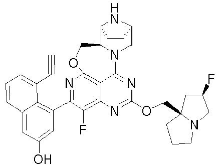 | 5-Ethynyl-4-((5*aR*,6*S*,9*R*)-1-fluoro-12-(((2*R*,7*a R*)-2-fluorotetrahydro-1*H*-pyrrolizin-7*a*(5*H*)-yl) methoxy)-5*a*,6,7,8,9,10-hexahydro-*5*H-4-oxa-3, 10*a*,11,13,14-pentaaza-6,9-methanonaphtho[1,8 -*ab*]heptalen-2-yl)naphthalen-2-ol |
| | 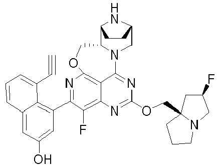 | 5-Ethynyl-4-((5*aS*,6*R*,9*S*)-1-fluoro-12-(((2*R*,7*a R*)-2-fluorotetrahydro-1*H*-pyrrolizin-7*a*(5*H*)-yl) methoxy)-5*a*,6,7,8,9,10-hexahydro-*5*H-4-oxa-3, 10*a*,11,13,14-pentaaza-6,9-methanonaphtho[1,8 -*ab*]heptalen-2-yl)naphthalen-2-ol |
| | 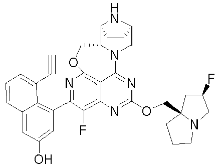 | 5-Ethynyl-4-((5*aS*,6*S*,9*R*)-1-fluoro-12-(((2*R*,7*a R*)-2-fluorotetrahydro-1*H*-pyrrolizin-7*a*(5*H*)-yl) methoxy)-5*a*,6,7,8,9,10-hexahydro-5*H*-4-oxa-3, 10*a*,11,13,14-pentaaza-6,9-methanonaphtho[1,8 -*ab*]heptalen-2-yl)naphthalen-2-ol |
| | 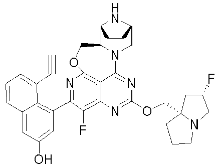 | 5-Ethynyl-4-((5*aR*,6*R*,9*S*)-1-fluoro-12-(((2*S*,7*aS* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| | 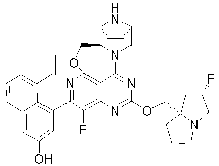 | 5-Ethynyl-4-((5*aR*,6*S*,9*R*)-1-fluoro-12-(((2*S*,7*aS* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| | 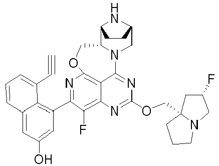 | 5-Ethynyl-4-((5*aS*,6*R*,9*S*)-1-fluoro-12-(((2*S*,7*aS* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| | 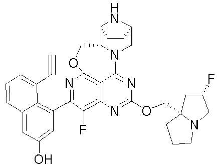 | 5-Ethynyl-4-((5*aS*,6*S*,9*R*)-1-fluoro-12-(((2*S*,7*aS* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| | 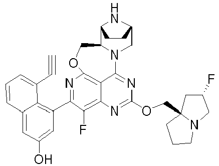 | 5-Ethynyl-4-((5*aR*,6*R*,9*S*)-1-fluoro-12-(((2*S*,7*a R*)-2-fluorotetrahydro-1*H*-pyrrolizin-7*a*(5*H*)-yl) methoxy)-5*a*,6,7,8,9,10-hexahydro-5*H*-4-oxa-3, *10a*,11,13,14-pentaaza-6,9-methanonaphtho[1,8 -*ab*]heptalen-2-yl)naphthalen-2-ol |
| | 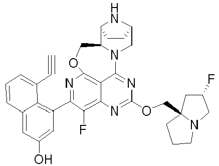 | 5-Ethynyl-4-((5*aR*,6*S*,9*R*)-1-fluoro-12-(((2*S*,7*a R*)-2-fluorotetrahydro-1*H*-pyrrolizin-7*a*(5*H*)-yl) methoxy)-5*a*,6,7,8,9,10-hexahydro-5*H*-4-oxa-3, *10a*,11,13,14-pentaaza-6,9-methanonaphtho[1,8 -*ab*]heptalen-2-yl)naphthalen-2-ol |
| | 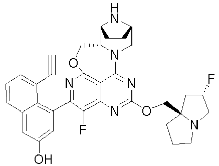 | 5-Ethynyl-4-((5*aS*,6*R*,9*S*)-1-fluoro-12-(((2*S*,7*aR* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| | 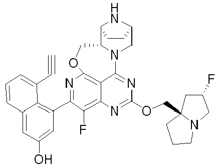 | 5-Ethynyl-4-((5*aS*,6*S*,9*R*)-1-fluoro-12-(((2*S*,7*aR* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| | 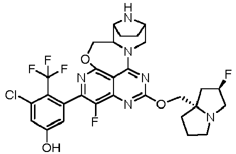 | 3-Chloro-5-(1-fluoro-12-(((2*R*,7a*S*)-2-fluorotetr ahydro-1*H*-pyrrolizin-7*a*(5*H*)-yl)methoxy)-5*a*,6, 7,8,9,10-hexahydro-5*H*-4-oxa-3,10*a*,11,13,14-p entaaza-6,9-methanonaphtho[1,8-*ab*]heptalen-2-yl)-4-(trifluoromethyl)phenol |
| | 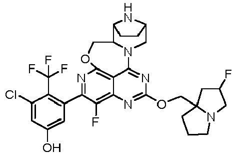 | 3-Chloro-5-(1-fluoro-12-((2-fluorotetrahydro-1 *H*-pyrrolizin-7*a*(5*H*)-yl)methoxy)-5*a*,6,7,8,9,10-hexahydro-5*H*-4-oxa-3,10*a*,11,13,14-pentaaza-6 ,9-methanonaphtho[1,8-*ab*]heptalen-2-yl)-4-(trif luoromethyl)phenol |
| 14-p2 | 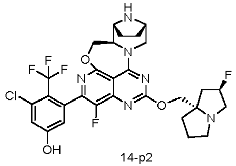 | 3-Chloro-5-((5*aR*,6*R*,9*S*)-1-fluoro-12-(((2*R*,7*aS* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol **14-p2** |
| | 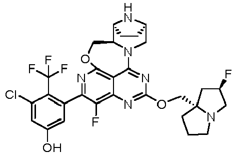 | 3-Chloro-5-((5*aR*,6*S*,9*R*)-1-fluoro-12-(((2*R*,7*aS* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 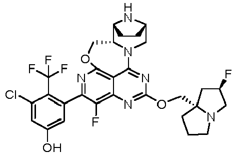 | 3-Chloro-5-((5*aS*,6*R*,9*S*)-1-fluoro-12-(((2*R*,7*aS*) -2-fluorotetrahydro-1*H*-pyrrolizin-7*a*(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| 14-p1 | 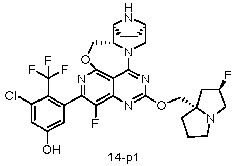 | 3-Chloro-5-((5*aS*,6*S*,9*R*)-1-fluoro-12-(((2*R*,7*aS*) -2-fluorotetrahydro-1*H*-pyrrolizin-7a(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol **14-p1** |
| | 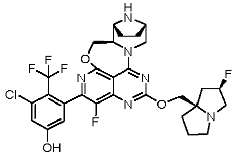 | 3-Chloro-5-((5*aR*,6*R*,9*S*)-1-fluoro-12-(((2*R*,7*aR* )-2-fluorotetrahydro-1*H-*pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 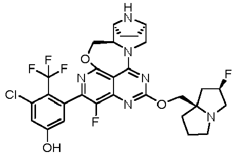 | 3-Chloro-5-((5*aR*,6*S*,9*R*)-1-fluoro-12-(((2*R*,7*aR* )-2-fluorotetrahydro-1*H-*pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 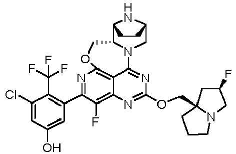 | 3-Chloro-5-((5*aR*,6*R*,9*S*)-1-fluoro-12-(((2*R*,7*aR* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 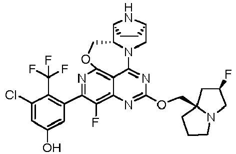 | 3-Chloro-5-((5*aR*,6*R*,9*S*)-1-fluoro-12-(((2*R*,7*aR* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 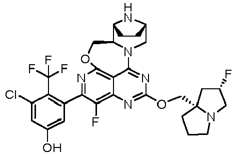 | 3-Chloro-5-((5*aR*,6*R*,9*S*)-1-fluoro-12-(((2*S*,7*aS*) -2-fluorotetrahydro-1*H*-pyrrolizin-7*a*(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 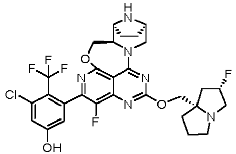 | 3-Chloro-5-((5*aR*,6*S*,9*R*)-1-fluoro-12-(((2*S*,7*aS*) -2-fluorotetrahydro-1*H*-pyrrolizin-7*a*(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 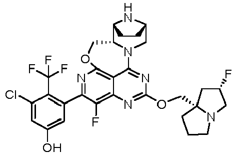 | 3-Chloro-5-((5*aR*,6*R*,9*S*)-1-fluoro-12-(((2*S*,7*aS*) -2-fluorotetrahydro-1*H*-pyrrolizin-7*a*(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 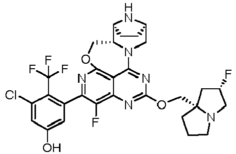 | 3-Chloro-5-((5*aR*,6*R*,9*S*)-1-fluoro-12-(((2*S*,7*aS*) -2-fluorotetrahydro-1*H*-pyrrolizin-7*a*(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 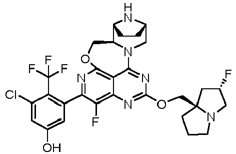 | 3-Chloro-5-((5*aR*,6*R*,9*S*)-1-fluoro-12-(((2*S*,7*aR* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 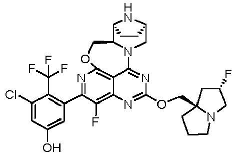 | 3-Chloro-5-((5*aR*,6*S*,9*R*)-1-fluoro-12-(((2*S*,7*aR* )-2-fluorotetrahydro-1*H*-pyrrolizin-7*a*(5*H*)-yl)m ethoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 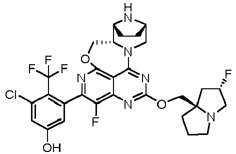 | 3-Chloro-5-((5*aS*,6*R*,9*S*)-1-fluoro-12-(((2*S*,7*aR*) -2-fluorotetrahydro-1*H*-pyrrolizin-7a(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 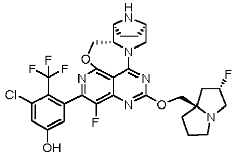 | 3-Chloro-5-((5*aS*,6*S*,9*R*)-1-fluoro-12-(((2*S*,7*aR*) -2-fluorotetrahydro-1*H*-pyrrolizin-7a(5*H*)-yl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)-4-(trifluoromethyl)phenol |
| | 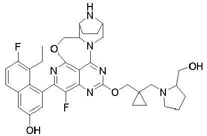 | 5-Ethyl-6-fluoro-4-(1-fluoro-12-((1-((2-(hydrox ymethyl)pyrrolidin-1-yl)methyl)cyclopropyl)me thoxy)-5*a*,6,7,8,9,10-hexahydro-5*H*-4-oxa-3,10 *a*,11,13,14-pentaaza-6,9-methanonaphtho[1,8-*a b*]heptalen-2-yl)naphthalen-2-ol |
| | 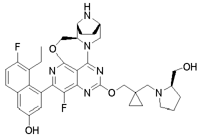 | 5-Ethyl-6-fluoro-4-((5*aR*,6*R*,9*S*)-1-fluoro-12-((1 -(((*R*)-2-(hydroxymethyl)pyrrolidin-1-yl)methyl )cyclopropyl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)naphthalen-2-ol |
| | 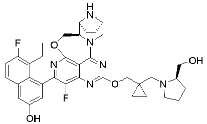 | 5-Ethyl-6-fluoro-4-((5*aR*,6*S*,9*R*)-1-fluoro-12-((1 -(((*R*)-2-(hydroxymethyl)pyrrolidin-1-yl)methyl )cyclopropyl)methoxy)-5*a*,6,7,8,9,10-hexahydro *-*5*H*-4-oxa-3,10*a*,11,13,14-pentaaza-6,9-methan onaphtho[1,8-*ab*]heptalen-2-yl)naphthalen-2-ol |
| | 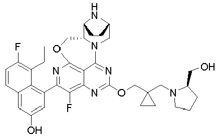 | 5-Ethyl-6-fluoro-4-((5*aS*,6*R*,9*S*)-1-fluoro-12-((1 -(((*R*)-2-(hydroxymethyl)pyrrolidin-1-yl)methyl )cyclopropyl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)naphthalen-2-ol |
| 15 | 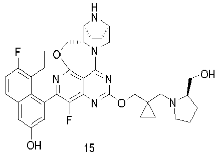 | 5-Ethyl-6-fluoro-4-((5*aS*,6*S*,9*R*)-1-fluoro-12-((1 -(((*R*)-2-(hydroxymethyl)pyrrolidin-1-yl)methyl )cyclopropyl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)naphthalen-2-ol **15** |
| | 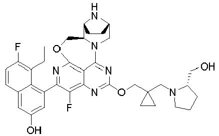 | 5-Ethyl-6-fluoro-4-((5*aR*,6*R*,9*S*)-1-fluoro-12-((1 -(((*S*)-2-(hydroxymethyl)pyrrolidin-1-yl)methyl )cyclopropyl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)naphthalen-2-ol |
| | 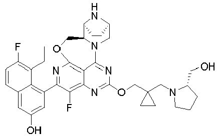 | 5-Ethyl-6-fluoro-4-((5*aR*,6*S*,9*R*)-1-fluoro-12-((1 -(((*S*)-2-(hydroxymethyl)pyrrolidin-1-yl)methyl )cyclopropyl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)naphthalen-2-ol |
| | 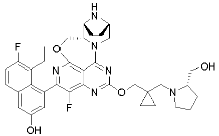 | 5-Ethyl-6-fluoro-4-((5*aS*,6*R*,9*S*)-1-fluoro-12-((1 -(((*S*)-2-(hydroxymethyl)pyrrolidin-1-yl)methyl )cyclopropyl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)naphthalen-2-ol |
| 17 | 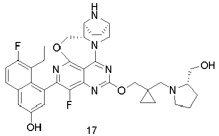 | 5-Ethyl-6-fluoro-4-((5*aS*,6*S*,9*R*)-1-fluoro-12-((1 -(((*S*)-2-(hydroxymethyl)pyrrolidin-1-yl)methyl )cyclopropyl)methoxy)-5*a*,6,7,8,9,10-hexahydro -5*H*-4-oxa-3,10*a*,11,13,14-pentaaza-6,9-methan onaphtho[1,8-*ab*]heptalen-2-yl)naphthalen-2-ol **17** |
| | 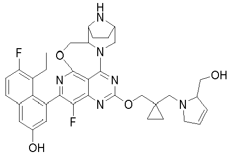 | 5-Ethyl-6-fluoro-4-(1-fluoro-12-((1-((2-(hydrox ymethyl)-2,5-dihydro-1*H*-pyrrol-1-yl)methyl)cy clopropyl)methoxy)-5*a*,6,7,8,9,10-hexahydro-5 *H*-4-oxa-3,10*a*,11,13,14-pentaaza-6,9-methanon aphtho[1,8-*ab*]heptalen-2-yl)naphthalen-2-ol |
| | 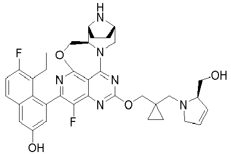 | 5-Ethyl-6-fluoro-4-((5*aR*,6*R*,9*S*)-1-fluoro-12-((1 -(((*R*)-2-(hydroxymethyl)-2, 5-dihydro-1*H*-pyrro l-1-yl)methyl)cyclopropyl)methoxy)-5*a*,6,7,8,9, 10-hexahydro-5*H*-4-oxa-3,10*a*,11,13,14-pentaaz a-6,9-methanonaphtho[1,8-*ab*]heptalen-2-yl)nap hthalen-2-ol |
| | 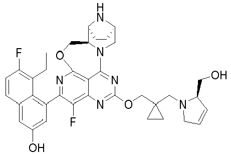 | 5-Ethyl-6-fluoro-4-((5*aR*,6*S*,9*R*)-1-fluoro-12-((1 -(((*R*)-2-(hydroxymethyl)-2,5-dihydro-1*H*-pyrro l-1-yl)methyl)cyclopropyl)methoxy)-5*a*,6,7,8,9, 10-hexahydro-5*H*-4-oxa-3,10*a*,11,13,14-pentaaz a-6,9-methanonaphtho[1,8-*ab*]heptalen-2-yl)nap hthalen-2-ol |
| | 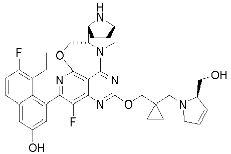 | 5-Ethyl-6-fluoro-4-((5a*S*,6*R*,9*S*)-1-fluoro-12-((1 -(((*R*)-2-(hydroxymethyl)-2,5-dihydro-1*H*-pyrro l-1-yl)methyl)cyclopropyl)methoxy)-5*a*,6,7,8,9, 10-hexahydro-5*H*-4-oxa-3,10*a*,11,13,14-pentaaz a-6,9-methanonaphtho[1,8-*ab*]heptalen-2-yl)nap hthalen-2-ol |
| 16 | 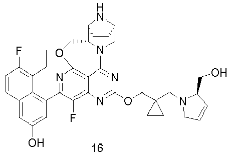 | 5-Ethyl-6-fluoro-4-((5*aS*,6*S*,9*R*)-1-fluoro-12-((1 -(((*R*)-2-(hydroxymethyl)-2,5-dihydro-1*H*-pyrro l-1-yl)methyl)cyclopropyl)methoxy)-5*a*,6,7,8,9, 10-hexahydro-5*H*-4-oxa-3,10*a*,11,13,14-pentaaz a-6,9-methanonaphtho[1,8-*ab*]heptalen-2-yl)nap hthalen-2-ol **16** |
| | 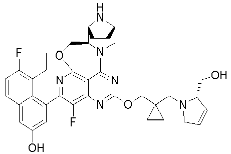 | 5-Ethyl-6-fluoro-4-((5*aR*,6*R*,9*S*)-1-fluoro-12-((1 -(((*S*)-2-(hydroxymethyl)-2,5-dihydro-1*H*-pyrro l-1-yl)methyl)cyclopropyl)methoxy)-5*a*,6,7,8,9, 10-hexahydro-5*H*-4-oxa-3,10*a*,11,13,14-pentaaz a-6,9-methanonaphtho[1,8-*ab*]heptalen-2-yl)nap hthalen-2-ol |
| | 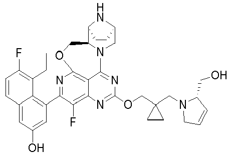 | 5-Ethyl-6-fluoro-4-((5*aR*,6*S*,9*R*)-1-fluoro-12-((1 -(((*S*)-2-(hydroxymethyl)-2,5-dihydro-1*H*-pyrro l-1-yl)methyl)cyclopropyl)methoxy)-5a,6,7,8,9, 10-hexahydro-5*H*-4-oxa-3,10*a*,11,13,14-pentaaz a-6,9-methanonaphtho[1,8-*ab*]heptalen-2-yl)nap hthalen-2-ol |
| | 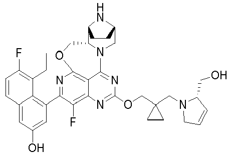 | 5-Ethyl-6-fluoro-4-((5*aS*,6*R*,9*S*)-1-fluoro-12-((1 -(((*S*)-2-(hydroxymethyl)-2,5-dihydro-1*H*-pyrro l-1-yl)methyl)cyclopropyl)methoxy)-5*a*,6,7,8,9, 10-hexahydro-5*H*-4-oxa-3,10*a*,11,13,14-pentaaz a-6,9-methanonaphtho[1,8-*ab*]heptalen-2-yl)nap hthalen-2-ol |
| 18 | 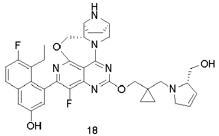 | 5-Ethyl-6-fluoro-4-((5*aS*,6*S*,9*R*)-1-fluoro-12-((1 -(((*S*)-2-(hydroxymethyl)-2,5-dihydro-1*H*-pyrro l-1-yl)methyl)cyclopropyl)methoxy)-5*a*,6,7,8,9, 10-hexahydro-5*H*-4-oxa-3,10*a*,11,13,14-pentaaz a-6,9-methanonaphtho[1,8-*ab*]heptalen-2-yl)nap hthalen-2-ol **18** |
| | 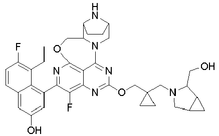 | 5-Ethyl-6-fluoro-4-(1-fluoro-12-((1-((2-(hydrox ymethyl)-3-azabicyclo[3.1.0]hex-3-yl)methyl)c yclopropyl)methoxy)-5*a*,6,7,8,9,10-hexahydro-5*H*-4-oxa-3,10*a*,11,13,14-pentaaza-6,9-methano naphtho[1,8-*ab*]heptalen-2-yl)naphthalen-2-ol |
| | 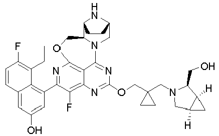 | 5-Ethyl-6-fluoro-4-((5*aR*,6*R*,9*S*)-1-fluoro-12-((1 -(((1*S*,2*R*,5*R*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol |
| | 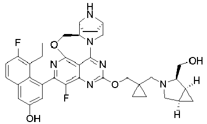 | 5-Ethyl-6-fluoro-4-((5*aR*,6*S*,9*R*)-1-fluoro-12-((1 -(((1*S*,2*R*,5*R*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol |
| | 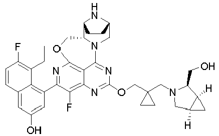 | 5-Ethyl-6-fluoro-4-((5*aS*,6*R*,9*S*)-1-fluoro-12-((1 -(((1*S*,2*R*,5*R*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6, 9-methanonaphtho[1,8-*ab*]heptalen-2-yl)naphthalen-2-ol |
| 19 | 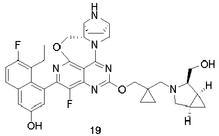 | 5-Ethyl-6-fluoro-4-((5*aS*,6*S*,9*R*)-1-fluoro-12-((1 -(((1*S*,2*R*,5*R*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14-pentaaza-6,9-methanonaphtho[1,8-*ab*]heptalen-2-yl)naphthalen-2-ol **19** |
| | 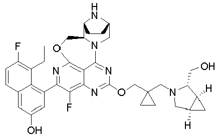 | 5-Ethyl-6-fluoro-4-((5*aR*,6*R*,9*S*)-1-fluoro-12-((1 -(((1*S*,2*S*,5*R*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol |
| | 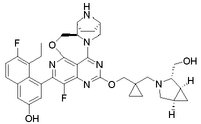 | 5-Ethyl-6-fluoro-4-((5*aR*,6*S*,9*R*)-1-fluoro-12-((1 -(((1*S*,2*S*,5*R*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol |
| | 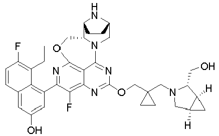 | 5-Ethyl-6-fluoro-4-((5*aS*,6*R*,9*S*)-1-fluoro-12-((1 -(((1*S*,2*S*,5*R*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol |
| 20 | 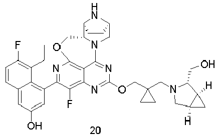 | 5-Ethyl-6-fluoro-4-((5*aS*,6*S*,9*R*)-1-fluoro-12-((1 -(((1*S*,2*S*,5*R*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol **20** |
| | 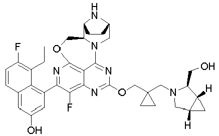 | 5-Ethyl-6-fluoro-4-((5*aR*,6*R*,9*S*)-1-fluoro-12-((1 -(((1*R*,2*R*,5*S*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol |
| | 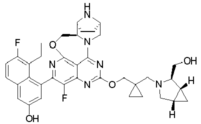 | 5-Ethyl-6-fluoro-4-((5*aR*,6*S*,9*R*)-1-fluoro-12-((1 -(((1*R*,2*R*,5*S*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol |
| | 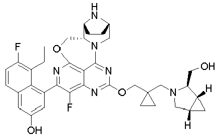 | 5-Ethyl-6-fluoro-4-((5*aS*,6*R*,9*S*)-1-fluoro-12-((1 -(((1*R*,2*R*,5*S*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol |
| 21 | 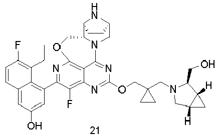 | 5-Ethyl-6-fluoro-4-((5*aS*,6*S*,9*R*)-1-fluoro-12-((1 -(((1*R*,2*R*,5*S*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol **21** |
| | 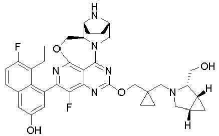 | 5-Ethyl-6-fluoro-4-((5*aR*,6*R*,9*S*)-1-fluoro-12-((1 -(((1*R*,2*S*,5*S*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho [1,8-*ab*]heptalen-2-yl)naphthalen-2-ol |
| | 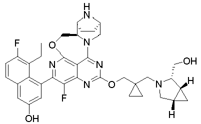 | 5-Ethyl-6-fluoro-4-((5*aR*,6*S*,9*R*)-1-fluoro-12-((1 -(((1*R*,2*S*,5*S*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol |
| | 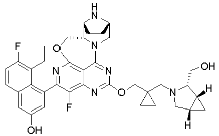 | 5-Ethyl-6-fluoro-4-((5*aS*,6*R*,9*S*)-1-fluoro-12-((1 -(((1*R*,2*S*,5*S*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol |
| 22 | 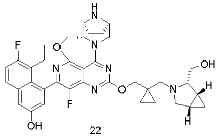 | 5-Ethyl-6-fluoro-4-((5*aS*,6*S*,9*R*)-1-fluoro-12-((1 -(((1*R*,2*S*,5*S*)-2-(hydroxymethyl)-3-azabicyclo[ 3.1.0]hex-3-yl)methyl)cyclopropyl)methoxy)-5*a* ,6,7,8,9,10-hexahydro-5*H*-4-oxa-3,10*a*,11,13,14 -pentaaza-6,9-methanonaphtho[1,8-*ab*]heptalen-2-yl)naphthalen-2-ol **22** |
| | 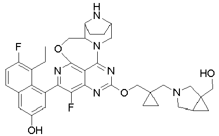 | 5-Ethyl-6-fluoro-4-(1-fluoro-12-((1-((1-(hydrox ymethyl)-3-azabicyclo[3.1.0]hex-3-yl)methyl)c yclopropyl)methoxy)-5*a*,6,7,8,9,10-hexahydro-5*H*-4-oxa-3,10*a*,11,13,14-pentaaza-6,9-methano naphtho[1,8-*ab*]heptalen-2-yl)naphthalen-2-ol |
| 23 | 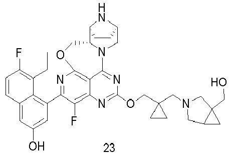 | 5-Ethyl-6-fluoro-4-((5*aS*,6*S*,9*R*)-1-fluoro-12-((1 -((1-(hydroxymethyl)-3-azabicyclo[3.1.0]hex-3-yl)methyl)cyclopropyl)methoxy)-5*a*,6,7,8,9,10-hexahydro-5*H*-4-oxa-3,10*a*,11,13,14-pentaaza-6 ,9-methanonaphtho[1,8-*ab*]heptalen-2-yl)naphth alen-2-ol **23** |
| | 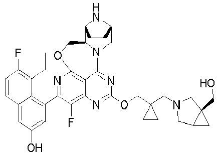 | 5-Ethyl-6-fluoro-4-((5*aR*,6*R*,9*S*)-1-fluoro-12-((1 -(((1*S*)-1-(hydroxymethyl)-3-azabicyclo[3.1.0]h ex-3-yl)methyl)cyclopropyl)methoxy)-5*a*,6,7,8, 9,10-hexahydro-5*H*-4-oxa-3,10*a*,11,13,14-penta aza-6,9-methanonaphtho[1,8-*ab*]heptalen-2-yl)n aphthalen-2-ol |
| | 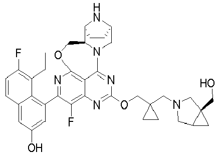 | 5-Ethyl-6-fluoro-4-((5*aR*,6*S*,9*R*)-1-fluoro-12-((1 -(((1*S*)-1-(hydroxymethyl)-3-azabicyclo[3.1.0]h ex-3-yl)methyl)cyclopropyl)methoxy)-5*a*,6,7,8, 9,10-hexahydro-5*H*-4-oxa-3,10*a*,11,13,14-penta aza-6,9-methanonaphtho[1,8-*ab*]heptalen-2-yl)n aphthalen-2-ol |
| | 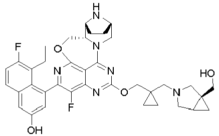 | 5-Ethyl-6-fluoro-4-((5*aS*,6*R*,9*S*)-1-fluoro-12-((1 -(((1*S*)-1-(hydroxymethyl)-3-azabicyclo[3.1.0]h ex-3-yl)methyl)cyclopropyl)methoxy)-5*a*,6,7,8, 9,10-hexahydro-5*H*-4-oxa-3,10*a*,11,13,14-penta aza-6,9-methanonaphtho[1,8-*ab*]heptalen-2-yl)n aphthalen-2-ol |
| | 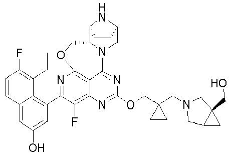 | 5-Ethyl-6-fluoro-4-((5*aS*,6*S*,9*R*)-1-fluoro-12-((1 -(((1*S*)-1-(hydroxymethyl)-3-azabicyclo[3.1.0]h ex-3-yl)methyl)cyclopropyl)methoxy)-5*a*,6,7,8, 9,10-hexahydro-5*H*-4-oxa-3,10*a*,11,13,14-penta aza-6,9-methanonaphtho[1,8-*ab*]heptalen-2-yl)n aphthalen-2-ol |
| | 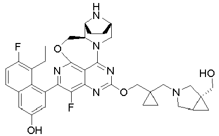 | 5-Ethyl-6-fluoro-4-((5*aR*,6*R*,9*S*)-1-fluoro-12-((1 -(((1*R*)-1-(hydroxymethyl)-3-azabicyclo[3.1.0]h ex-3-yl)methyl)cyclopropyl)methoxy)-5*a*,6,7,8, 9,10-hexahydro-5*H*-4-oxa-3,10*a*,11,13,14-penta aza-6,9-methanonaphtho[1,8-*ab*]heptalen-2-yl)n aphthalen-2-ol |
| | 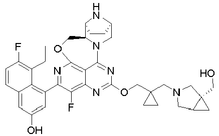 | 5-Ethyl-6-fluoro-4-((5*aR*,6*S*,9*R*)-1-fluoro-12-((1 -(((1*R*)-1-(hydroxymethyl)-3-azabicyclo[3.1.0]h ex-3-yl)methyl)cyclopropyl)methoxy)-5*a*,6,7,8, 9,10-hexahydro-5*H*-4-oxa-3,10*a*,11,13,14-penta aza-6,9-methanonaphtho[1,8-*ab*]heptalen-2-yl)n aphthalen-2-ol |
| | 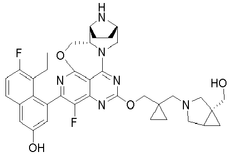 | 5-Ethyl-6-fluoro-4-((5*aS*,6*R*,9*S*)-1-fluoro-12-((1 -(((1R)-1-(hydroxymethyl)-3-azabicyclo[3.1.0]h ex-3-yl)methyl)cyclopropyl)methoxy)-5*a*,6,7,8, 9,10-hexahydro-5*H*-4-oxa-3,10*a*,11,13,14-penta aza-6,9-methanonaphtho[1,8-*ab*]heptalen-2-yl)n aphthalen-2-ol |
| | 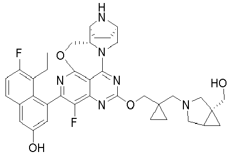 | 5-Ethyl-6-fluoro-4-((5*aS*,6*S*,9*R*)-1-fluoro-12-((1 -(((1*R*)-1-(hydroxymethyl)-3-azabicyclo[3.1.0]h ex-3-yl)methyl)cyclopropyl)methoxy)-5*a*,6,7,8, 9,10-hexahydro-5*H*-4-oxa-3,10*a*,11,13,14-penta aza-6,9-methanonaphtho[1,8-*ab*]heptalen-2-yl)n aphthalen-2-ol |
| | 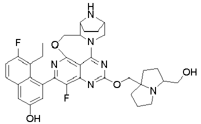 | 5-Ethyl-6-fluoro-4-(1-fluoro-12-((3-(hydroxyme thyl)tetrahydro-1*H*-pyrrolizin-7*a*(5*H*)-yl)metho xy)-5*a*,6,7,8,9,10-hexahydro-5*H*-4-oxa-3,10*a*,1 1,13,14-pentaaza-6,9-methanonaphtho[1,8-*ab*]h eptalen-2-yl)naphthalen-2-ol |
| 24 | 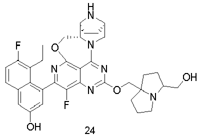 | 5-Ethyl-6-fluoro-4-((5*aS*,6*S*,9*R*)-1-fluoro-12-((3 -(hydroxymethyl)tetrahydro-1*H*-pyrrolizin-7*a*(5 *H*)-yl)methoxy)-5*a*,6,7,8,9,10-hexahydro-5*H*-4-oxa-3,10*a*,11,13,14-pentaaza-6,9-methanonapht ho[1,8-*ab*]heptalen-2-yl)naphthalen-2-ol **24** |
| | 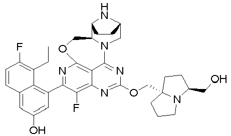 | 5-Ethyl-6-fluoro-4-((5*aR*,6*R*,9*S*)-1-fluoro-12-((( 3*S*,7*aS*)-3-(hydroxymethyl)tetrahydro-1*H*-pyrro lizin-7*a*(5*H*)-yl)methoxy)-5*a*,6,7,8,9,10-hexahy dro-5*H*-4-oxa-3,10*a*,11,13,14-pentaaza-6,9-met hanonaphtho[1,8-*ab*]heptalen-2-yl)naphthalen-2 -ol |
| | 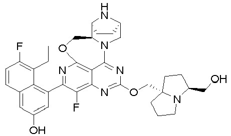 | 5-Ethyl-6-fluoro-4-((5*aR*,6*S*,9*R*)-1-fluoro-12-((( 3*S*,7*aS*)-3-(hydroxymethyl)tetrahydro-1*H*-pyrro lizin-7*a*(5*H*)-yl)methoxy)-5*a*,6,7,8,9,10-hexahy dro-5*H*-4-oxa-3,10*a,*11,13,14-pentaaza-6,9-met hanonaphtho[1,8-*ab*]heptalen-2-yl)naphthalen-2 -ol |
| | 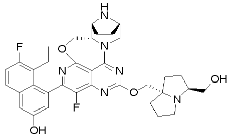 | 5-Ethyl-6-fluoro-4-((5*aS*,6*R,*9*S*)-1-fluoro-12-((( 3*S*,7*aS*)-3-(hydroxymethyl)tetrahydro-1*H*-pyrro lizin-7*a*(5*H*)-yl)methoxy)-5*a*,6,7,8,9,10-hexahy dro-5*H*-4-oxa-3,10*a,* 11,13,14-pentaaza-6,9-met hanonaphtho[1,8-*ab*]heptalen-2-yl)naphthalen-2 -ol |
| | 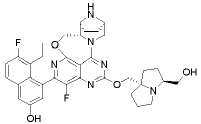 | 5-Ethyl-6-fluoro-4-((5*aS*,6*S*,9*R*)-1-fluoro-12-((( 3*S*,7*aS*)-3-(hydroxymethyl)tetrahydro-1*H*-pyrro lizin-7*a*(5*H*)-yl)methoxy)-5*a*,6,7,8,9,10-hexahy dro-5*H*-4-oxa-3,10*a*,11,13,14-pentaaza-6,9-met hanonaphtho[1,8-*ab*]heptalen-2-yl)naphthalen-2 -ol |
| | 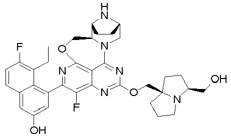 | *5*-Ethyl-6-fluoro-4-((5*aR*,6*R*,9*S*)-1-fluoro-12-((( 3*S*,7*aR*)-3-(hydroxymethyl)tetrahydro-1*H*-pyrro lizin-7*a*(5*H*)-yl)methoxy)-5*a*,6,7,8,9,10-hexahy dro-5*H*-4-oxa-3,10*a,* 11,13,14-pentaaza-6,9-met hanonaphtho[1,8-*ab*]heptalen-2-yl)naphthalen-2 -ol |
| | 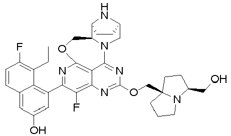 | 5-Ethyl-6-fluoro-4-((5*aR*,6*S*,9*R*)-1-fluoro-12-((( 3*S*,7*aR*)-3-(hydroxymethyl)tetrahydro-1*H*-pyrro lizin-7*a*(5*H*)-yl)methoxy)-5*a*,6,7,8,9,10-hexahy dro-5*H*-4-oxa-3,10*a*,11,13,14-pentaaza-6,9-met hanonaphtho[1,8-*ab*]heptalen-2-yl)naphthalen-2 -ol |
| | 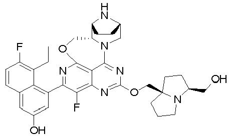 | 5-Ethyl-6-fluoro-4-((5*aS,*6*R*,9*S*)-1-fluoro-12-((( 3*S*,7*aR*)-3-(hydroxymethyl)tetrahydro-1*H*-pyrro lizin-7*a*(5*H*)-yl)methoxy)-5*a*,6,7,8,9,10-hexahy dro-5H-4-oxa-3,*10a,* 11,13,14-pentaaza-6,9-met hanonaphtho[1,8-*ab*]heptalen-2-yl)naphthalen-2 -ol |
| | 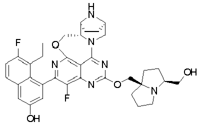 | 5-Ethyl-6-fluoro-4-((5*aS*,6*S*,9*R*)-1-fluoro-12-((( 3*S*,7*aR*)-3-(hydroxymethyl)tetrahydro-1*H*-pyrro lizin-7*a*(5*H*)-yl)methoxy)-5*a*,6,7,8,9,10-hexahydro-5*H*-4-oxa-3,10*a,*11,13,14-pentaaza-6,9-met hanonaphtho[1,8-*ab*]heptalen-2-yl)naphthalen-2 -ol |
| | 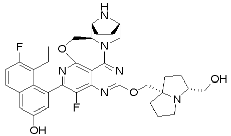 | 5-Ethyl-6-fluoro-4-((5*aR*,6*R*,9*S*)-1-fluoro-12-((( 3*R*,7*aS*)-3-(hydroxymethyl)tetrahydro-1*H*-pyrro lizin-7*a*(5*H*)-yl)methoxy)-5*a*,6,7,8,9,10-hexahy dro-5*H*-4-oxa-3,10*a,* 11,13,14-pentaaza-6,9-met hanonaphtho[1,8-*ab*]heptalen-2-yl)naphthalen-2 -ol |
| | 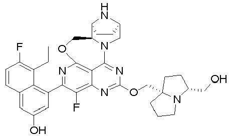 | 5-Ethyl-6-fluoro-4-((5*aR*,6*S*,9*R*)-1-fluoro-12-((( 3*R*,7*aS*)-3-(hydroxymethyl)tetrahydro-1*H*-pyrro lizin-7*a*(5*H*)-yl)methoxy)-5*a*,6,7,8,9,10-hexahy dro-5*H*-4-oxa-3,10*a,*11,13,14-pentaaza-6,9-met hanonaphtho[1,8-*ab*]heptalen-2-yl)naphthalen-2 -ol |
| | 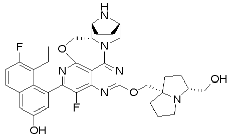 | 5-Ethyl-6-fluoro-4-((5*aS*,6*R*,9*S*)-1-fluoro-12-((( 3*R*,7*aS*)-3-(hydroxymethyl)tetrahydro-1*H*-pyrro lizin-7*a*(5*H*)-yl)methoxy)-5*a*,6,7,8,9,10-hexahy dro-5*H*-4-oxa-3*,*10*a,*11,13,14-pentaaza-6,9-met hanonaphtho[1,8-*ab*]heptalen-2-yl)naphthalen-2 -ol |
| | 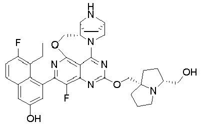 | 5-Ethyl-6-fluoro-4-((5*aS*,6*S*,9*R*)-1-fluoro-12-((( 3*R*,7*aS*)-3-(hydroxymethyl)tetrahydro-1*H*-pyrro lizin-7a(5H)-yl)methoxy)-5a,6,7,8,9,10-hexahy dro-5*H*-4-oxa-3*,*10*a,*11,13,14-pentaaza-6,9-met hanonaphtho[1,8-*ab*]heptalen-2-yl)naphthalen-2 -ol |
| | 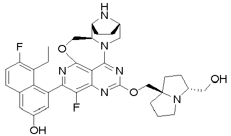 | 5-Ethyl-6-fluoro-4-((5*aR*,6*R*,9*S*)-1-fluoro-12-((( 3*R*,7*aR*)-3-(hydroxymethyl)tetrahydro-1*H*-pyrro lizin-7*a*(5*H*)-yl)methoxy)-5*a*,6,7,8,9,10-hexahy dro-5*H*-4-oxa-3*,*10*a,* 11,13,14-pentaaza-6,9-met hanonaphtho[1,8-*ab*]heptalen-2-yl)naphthalen-2 -ol |
| | 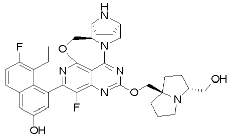 | 5-Ethyl-6-fluoro-4-((5*aR*,6*S*,9*R*)-1-fluoro-12-((( 3*R*,7*aR*)-3-(hydroxymethyl)tetrahydro-1*H*-pyrro lizin-7*a*(5*H*)-yl)methoxy)-5*a*,6,7,8,9,10-hexahy dro-5*H*-4-oxa-3,10*a,*11,13,14-pentaaza-6,9-met hanonaphtho[1,8-*ab*]heptalen-2-yl)naphthalen-2 -ol |
| | 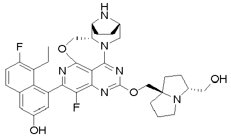 | 5-Ethyl-6-fluoro-4-((5*aS*,6*R*,9*S*)-1-fluoro-12-((( 3*R*,7*aR*)-3-(hydroxymethyl)tetrahydro-1*H*-pyrro lizin-7*a*(5*H*)-yl)methoxy)-5*a*,6,7,8,9,10-hexahy dro-5*H*-4-oxa-3,10*a,*11,13,14-pentaaza-6,9-met hanonaphtho[1,8-*ab*]heptalen-2-yl)naphthalen-2 -ol |
| | 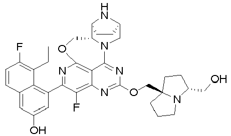 | 5-Ethyl-6-fluoro-4-((5*aS*,6*S*,9*R*)-1-fluoro-12-((( 3*R*,7*aR*)-3-(hydroxymethyl)tetrahydro-1*H*-pyrro lizin-7*a*(5*H*)-yl)methoxy)-5*a*,6,7,8,9,10-hexahy dro-5*H*-4-oxa-3,10*a,* 11,13,14-pentaaza-6,9-met hanonaphtho[1,8-*ab*]heptalen-2-yl)naphthalen-2 -ol |
| | 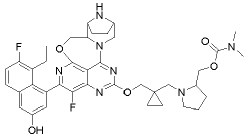 | (1-((1-(((2-(8-ethyl-7-fluoro-3-hydroxynaphthal en-1-yl)-1-fluoro-5*a*,6,7,8,9,10-hexahydro-5*H*-4 -oxa-3,10*a*,11,13,14-pentaaza-6,9-methanonaph tho[1,8-*ab*]heptalen-12-yl)oxy)methyl)cyclopro pyl)methyl)pyrrolidin-2-yl)methyl dimethylcarbamate |
| | 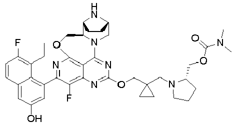 | ((*S*)-1-((1-((((5*aR*,6*R*,9*S*)-2-(8-ethyl-7-fluoro-3-hydroxynaphthalen-1-yl)-1-fluoro-5*a*,6,7,8,9,10 -hexahydro-5*H*-4-oxa-3,10*a,*11,13,14-pentaaza-6,9-methanonaphtho[1,8-*ab*]heptalen-12-yl)oxy )methyl)cyclopropyl)methyl)pyrrolidin-2-yl)met hyl dimethylcarbamate |
| | 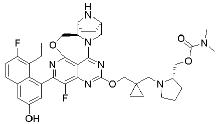 | ((*S*)-1-((1-((((5*aR*,6*S*,9*R*)-2-(8-ethyl-7-fluoro-3-hydroxynaphthalen-1-yl)-1-fluoro-5*a*,6,7,8,9,10 -hexahydro-5*H*-4-oxa-3,10*a,*11,13,14-pentaaza-6,9-methanonaphtho[1,8-*ab*]heptalen-12-yl)oxy )methyl)cyclopropyl)methyl)pyrrolidin-2-yl)met hyl dimethylcarbamate |
| | 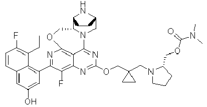 | ((*S*)*-*1-((1-((((5*aS*,6*R*,9*S*)-2-(8-ethyl-7-fluoro-3-hydroxynaphthalen-1-yl)-1-fluoro-5*a*,6,7,8,9,10 -hexahydro-5*H*-4-oxa-3,10*a,*11,13,14-pentaaza-6,9-methanonaphtho[1,8-*ab*]heptalen-12-yl)oxy )methyl)cyclopropyl)methyl)pyrrolidin-2-yl)met hyl dimethylcarbamate |
| 25 | 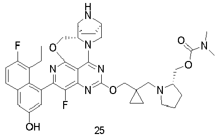 | ((*S*)-1-((1-((((5*aS*,6*S*,9*R*)-2-(8-ethyl-7-fluoro-3-hydroxynaphthalen-1-yl)-1-fluoro-5*a*,6,7,8,9,10 -hexahydro-5*H*-4-oxa-3,10*a,*11,13,14-pentaaza-6,9-methanonaphtho[1,8-*ab*]heptalen-12-yl)oxy )methyl)cyclopropyl)methyl)pyrrolidin-2-yl)met hyl dimethylcarbamate **25** |
| | 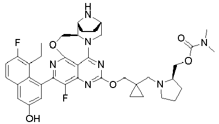 | ((*R*)-1-((1-((((5*aR*,6*R*,9*S*)-2-(8-ethyl-7-fluoro-3-hydroxynaphthalen-1-yl)-1-fluoro-5*a*,6,7,8,9,10 -hexahydro-5*H*-4-oxa-3,10*a*,11,13,14-pentaaza-6,9-methanonaphtho[1,8-*ab*]heptalen-12-yl)oxy )methyl)cyclopropyl)methyl)pyrrolidin-2-yl)met hyl dimethylcarbamate |
| | 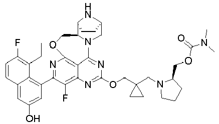 | ((*R*)-1-((1-((((5*aR*,6*S*,9*R*)-2-(8-ethyl-7-fluoro-3-hydroxynaphthalen-1-yl)-1-fluoro-5*a*,6,7,8,9,10 -hexahydro-5*H*-4-oxa-3,10*a*,11,13,14-pentaaza-6,9-methanonaphtho[1,8-ab]heptalen-12-yl)oxy )methyl)cyclopropyl)methyl)pyrrolidin-2-yl)met hyl dimethylcarbamate |
| | 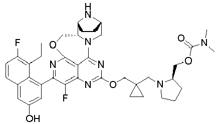 | ((*R*)-1-((1-((((5*aS*,6*R*,9*S*)-2-(8-ethyl-7-fluoro-3-hydroxynaphthalen-1-yl)-1-fluoro-5*a*,6,7,8,9,10 -hexahydro-5*H*-4-oxa-3,10*a*,11,13,14-pentaaza-6,9-methanonaphtho[1,8-*ab*]heptalen-12-yl)oxy )methyl)cyclopropyl)methyl)pyrrolidin-2-yl)met hyl dimethylcarbamate |
| | 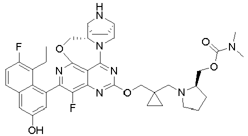 | ((*R*)-1-((1-((((5*aS*,6*S*,9*R*)-2-(8-ethyl-7-fluoro-3-hydroxynaphthalen-1-yl)-1-fluoro-5*a*,6,7,8,9,10 -hexahydro-5*H*-4-oxa-3,10*a,*11,13,14-pentaaza-6,9-methanonaphtho[1,8-*ab*]heptalen-12-yl)oxy )methyl)cyclopropyl)methyl)pyrrolidin-2-yl)met hyl dimethylcarbamate |

**Table B. Typical compounds disclosed herein include, but are not limited to:**

| Structure and name of compound |
|---|
| 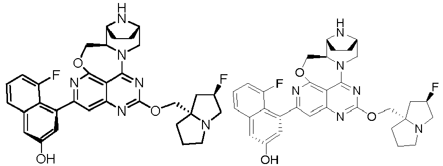 |
| 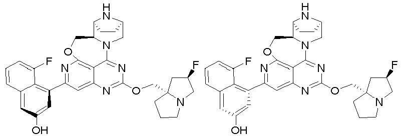 |
| 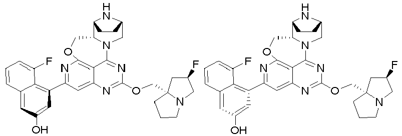 |
| 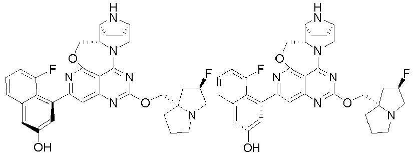 |
| 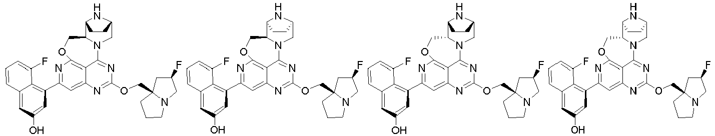 |
| 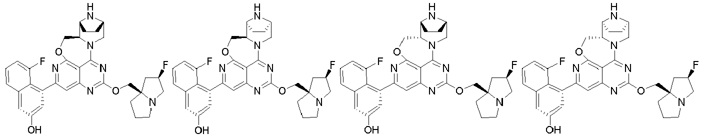 |
| 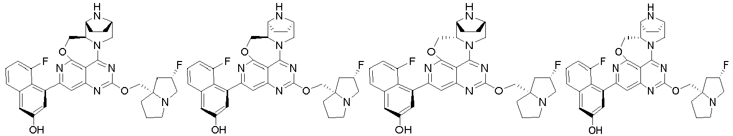 |
| 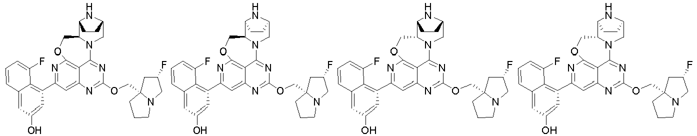 |
| 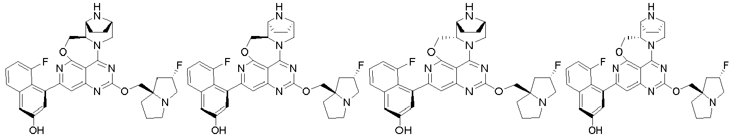 |
| 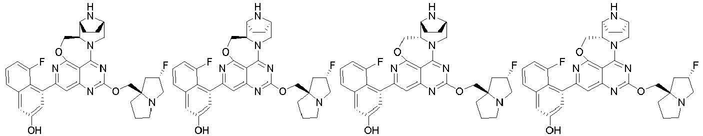 |
| 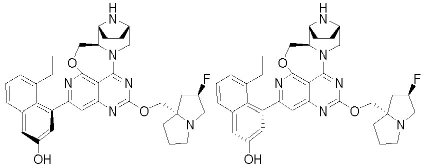 |
| 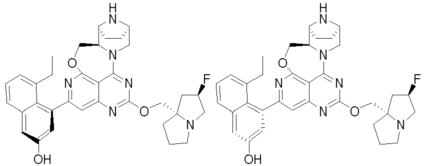 |
| 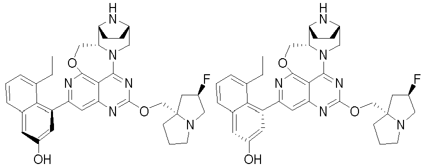 |
| 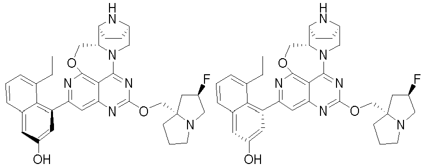 |
| 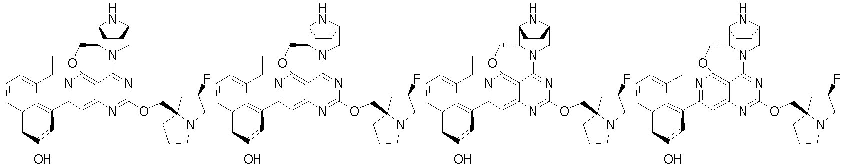 |
| 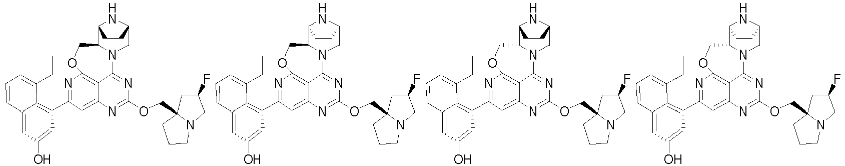 |
| 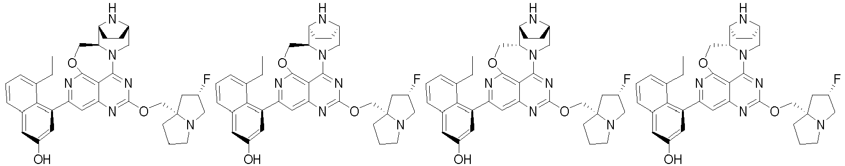 |
| 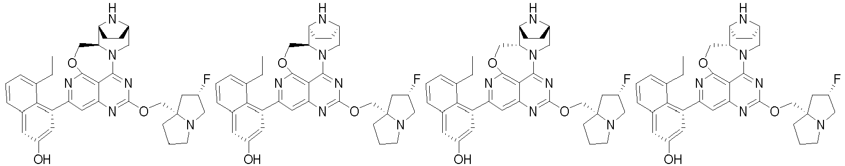 |
| 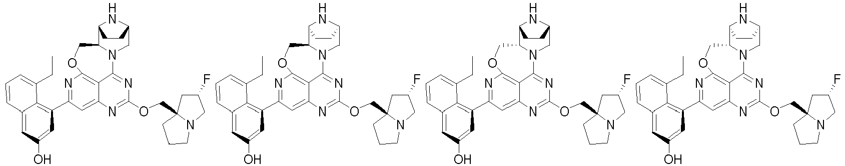 |
| 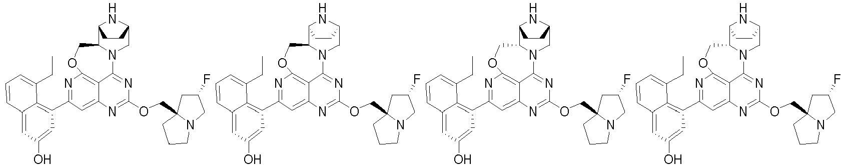 |
| 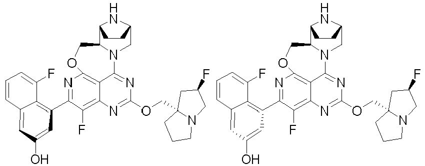 |
| 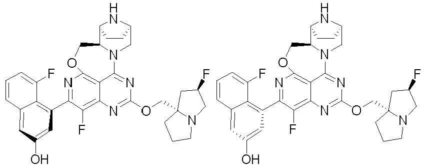 |
| 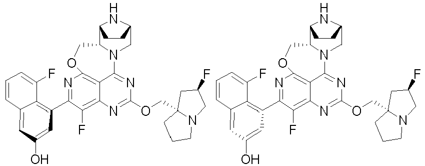 |
| 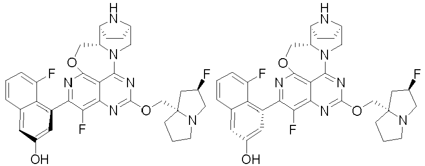 |
| 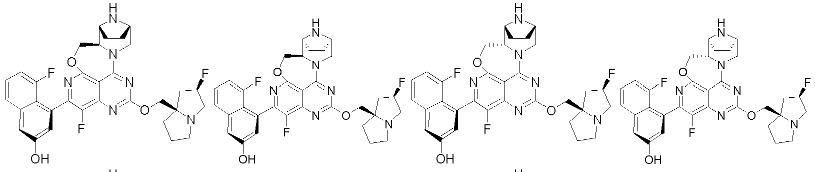 |
| 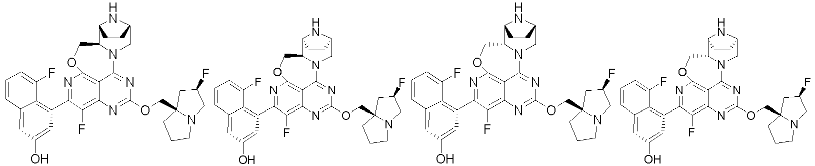 |
| 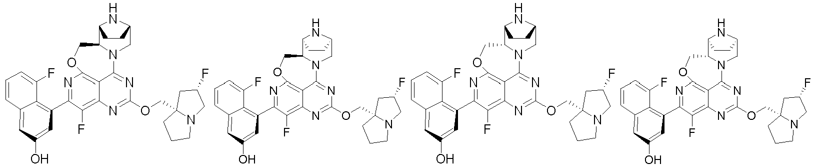 |
| 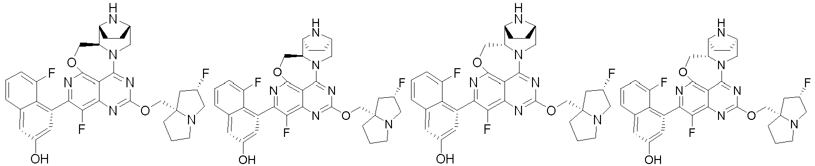 |
| 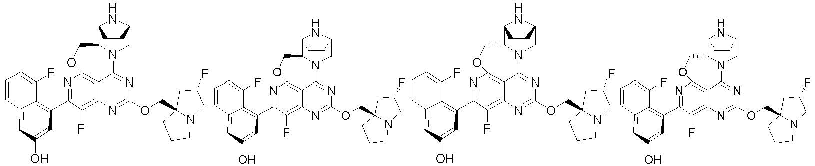 |
| 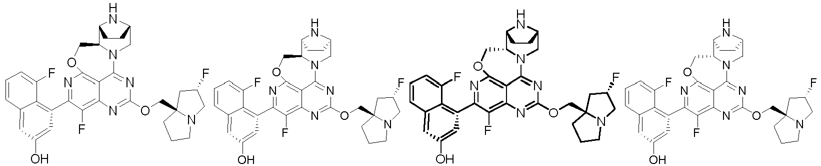 |
| 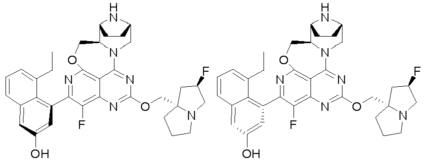 |
| 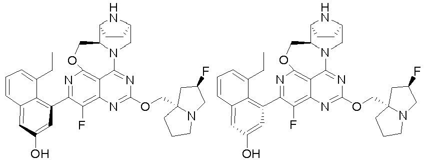 |
| 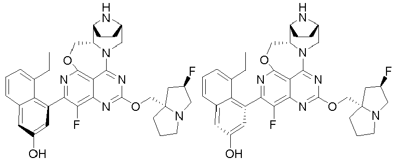 |
| 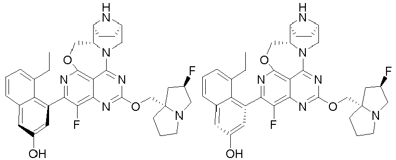 |
| 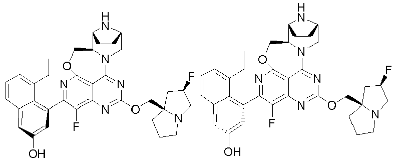 |
| 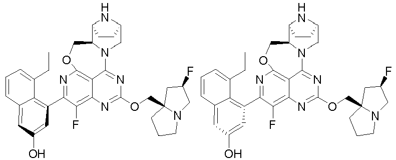 |
| 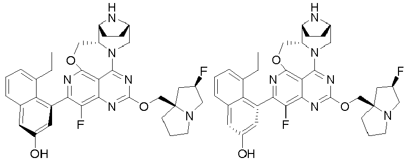 |
| 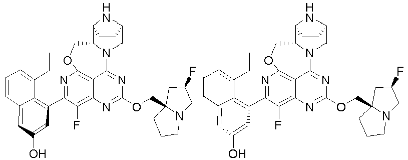 |
| 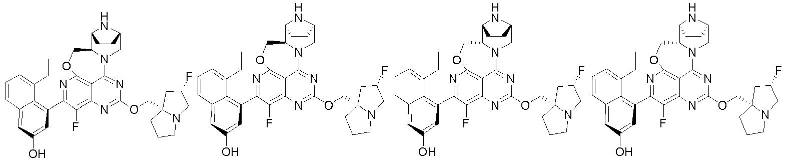 |
| 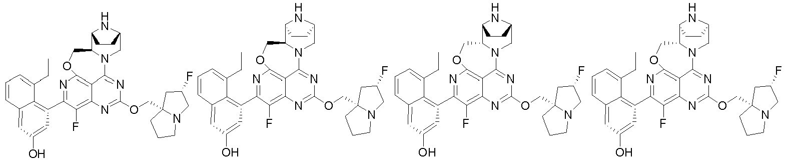 |
| 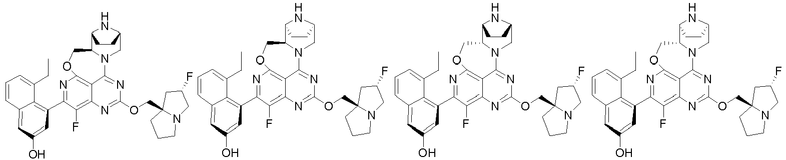 |
| 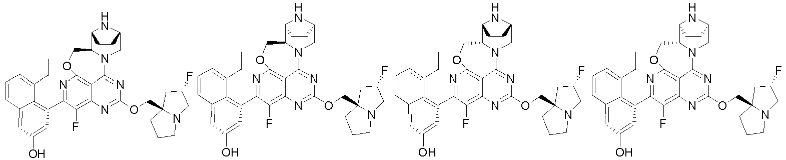 |
| 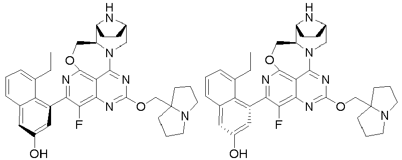 |
| 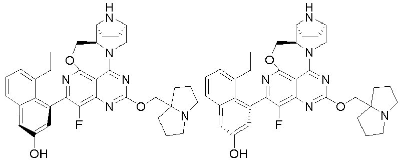 |
| 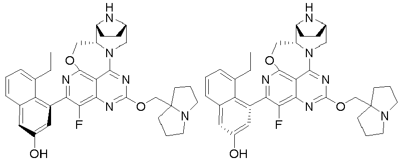 |
| 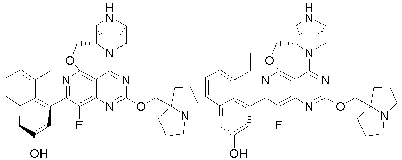 |
| 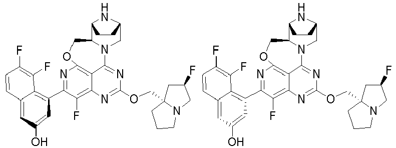 |
| 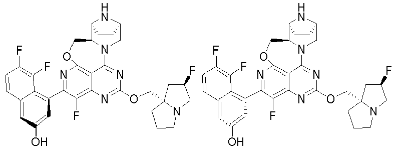 |
| 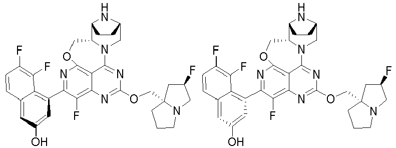 |
| 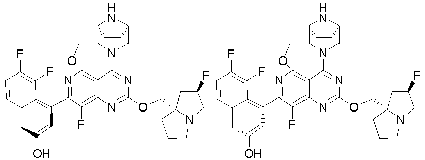 |
| 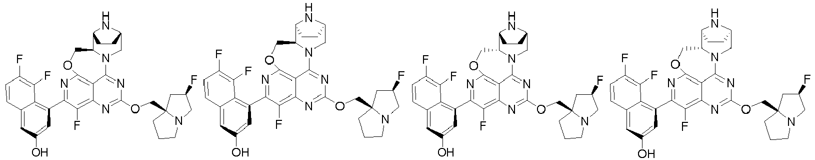 |
| 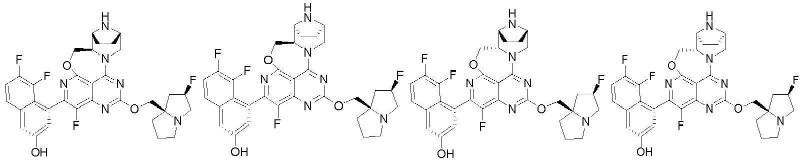 |
| 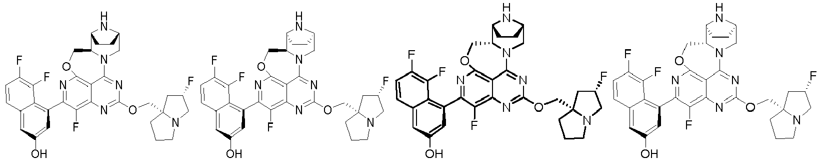 |
| 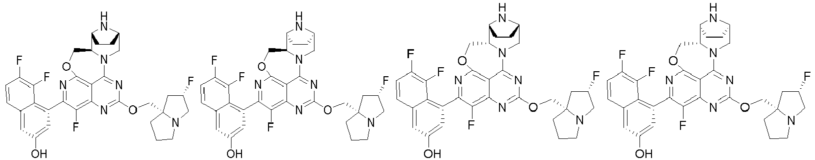 |
| 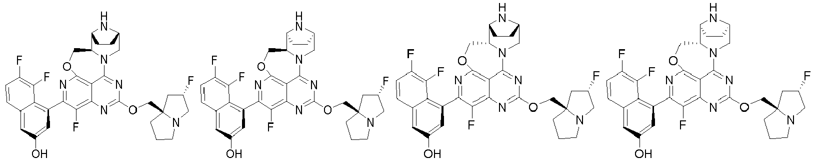 |
| 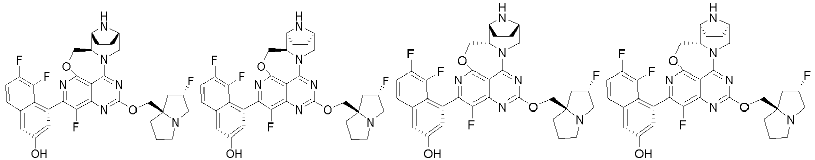 |
| 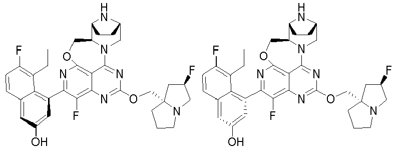 |
| 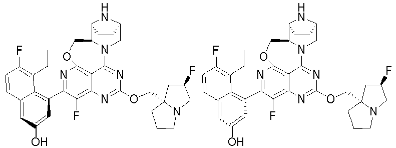 |
| 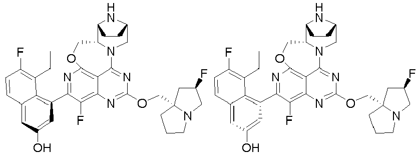 |
| 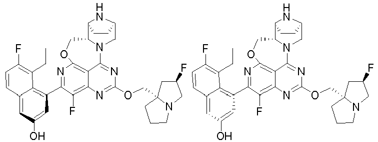 |
| 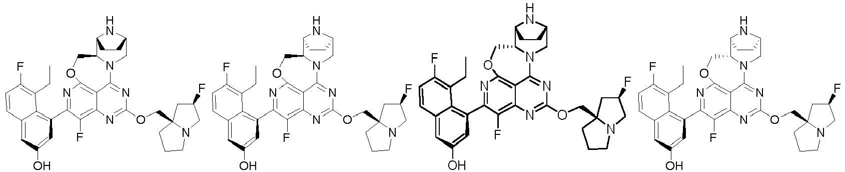 |
| 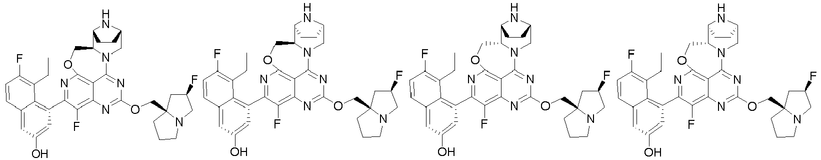 |
| 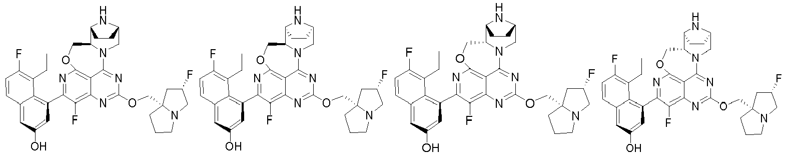 |
| 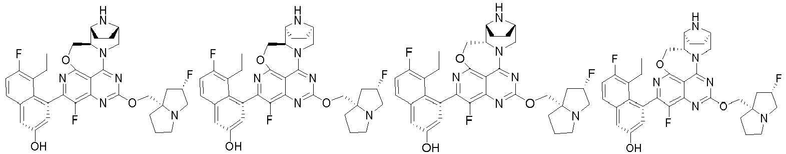 |
| 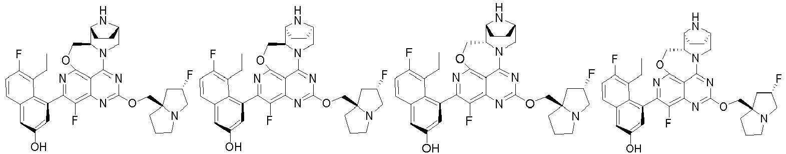 |
| 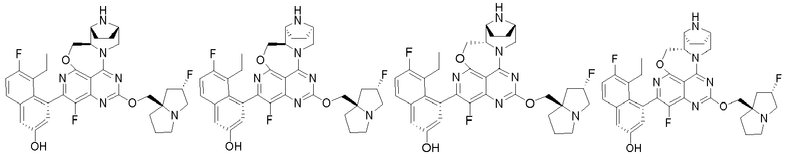 |
| 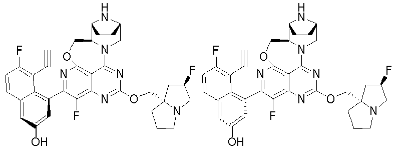 |
| 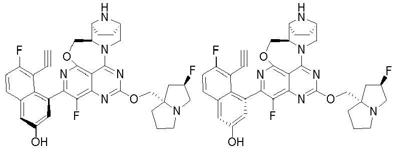 |
| 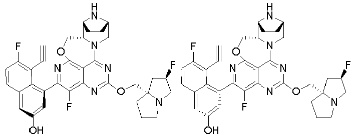 |
| 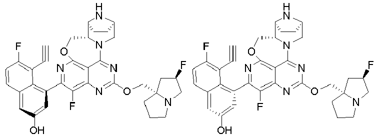 |
| 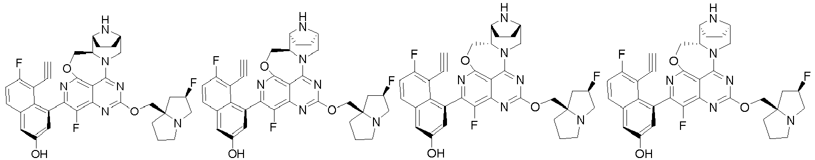 |
| 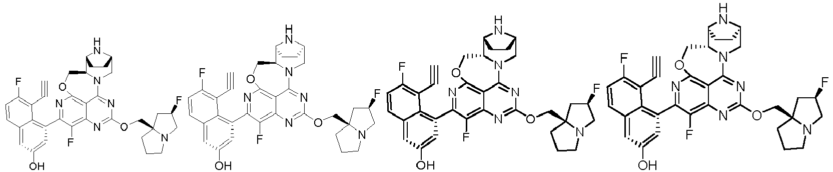 |
| 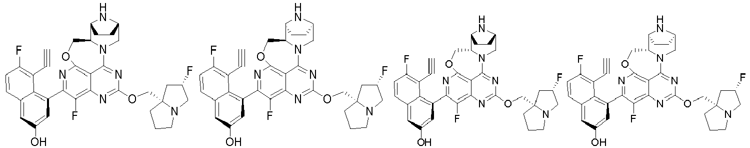 |
| 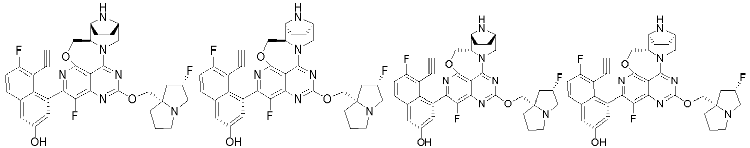 |
| 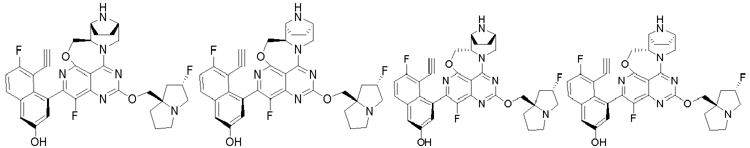 |
| 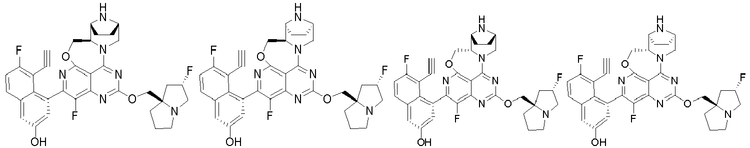 |
| 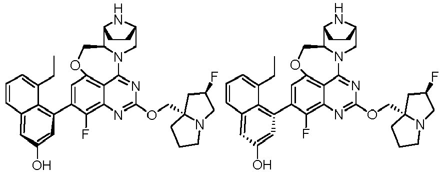 |
| 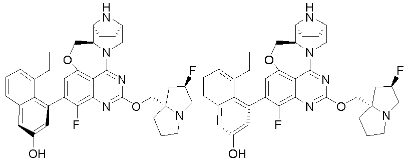 |
| 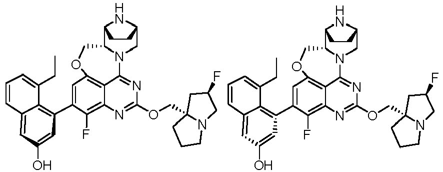 |
| 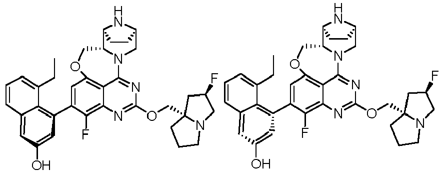 |
| 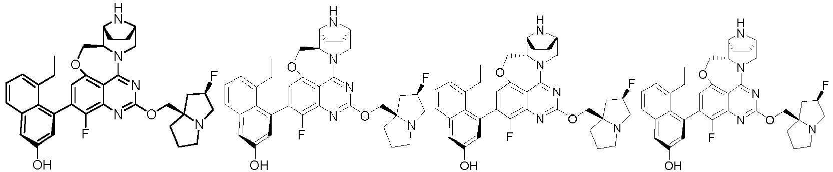 |
| 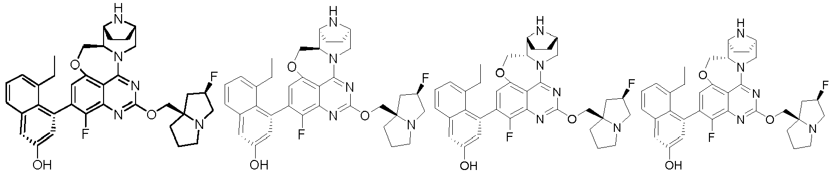 |
| 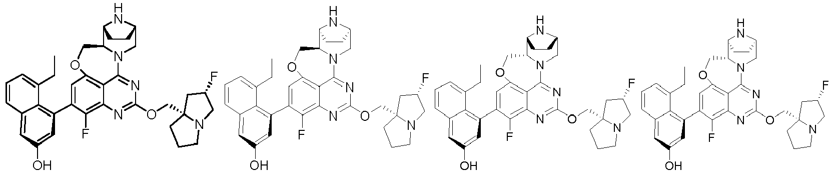 |
| 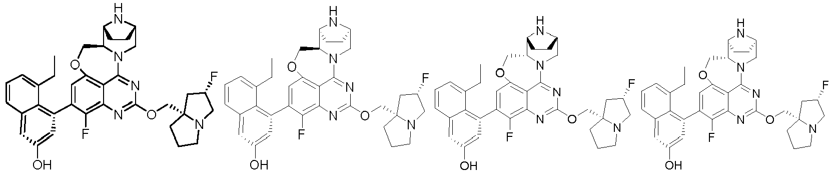 |
| 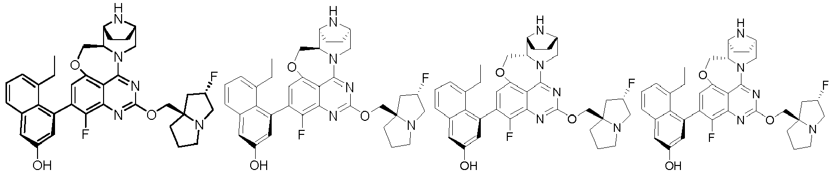 |
| 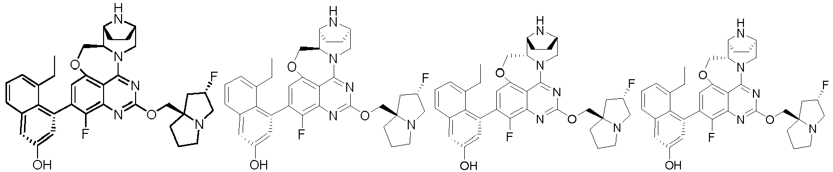 |
| 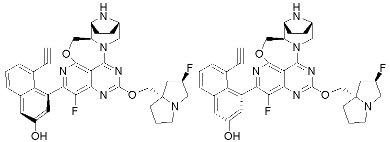 |
| 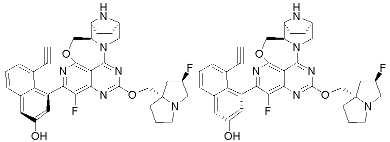 |
| 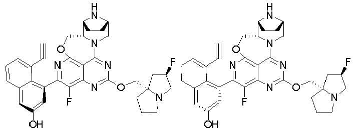 |
| 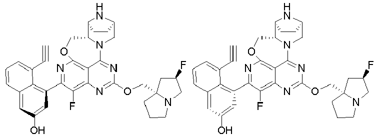 |
| 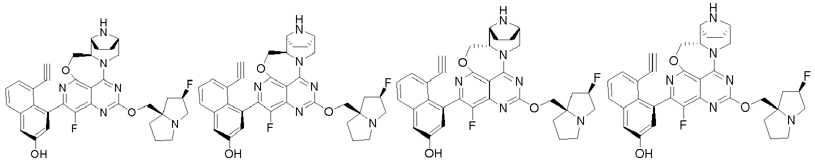 |
| 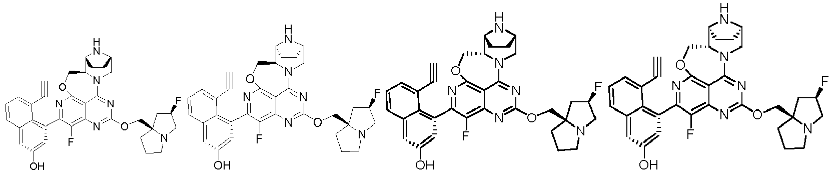 |
| 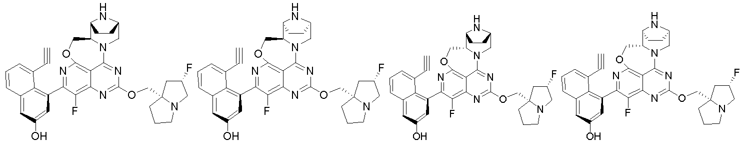 |
| 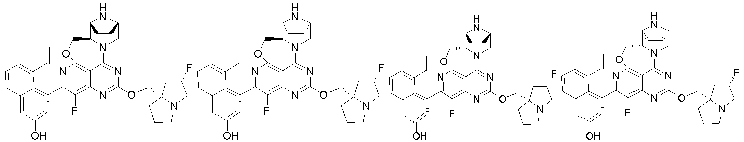 |
| 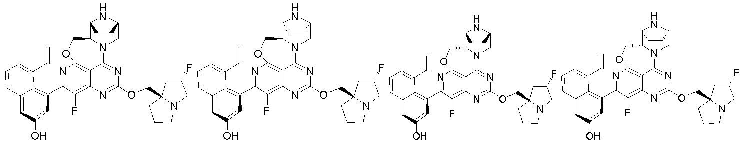 |
| 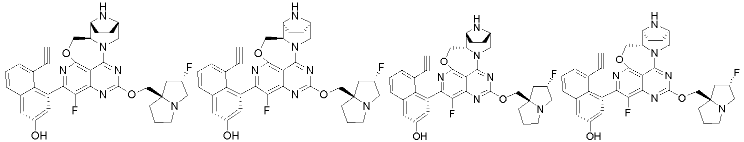 |

Another aspect of the present disclosure relates to a compound of general formula (IA) or a salt thereof, 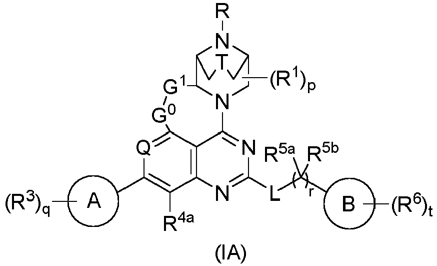 wherein,
R is an amino protecting group, and preferably Boc;
G⁰, G¹, T, ring A, ring B, Q, L, R¹, R³, R^{4a}, R^{5a}, R^{5b}, R⁶, p, q, r and t are as defined in general formula (I).

Another aspect of the present disclosure relates to a compound of general formula (I'A) or a salt thereof, 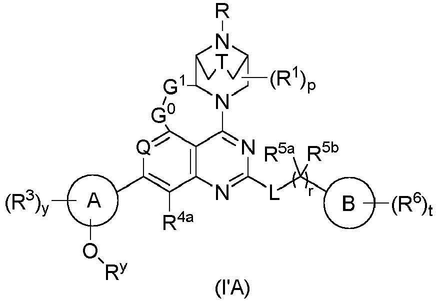 wherein,
R is an amino protecting group, and preferably Boc;
R^{y} is a hydroxy protecting group, and preferably MOM;
y is 0, 1, 2, 3 or 4;
G⁰, G¹, T, ring A, ring B, Q, L, R¹, R³, R^{4a}, R^{5a}, R^{5b}, R⁶, p, r and t are as defined in general formula (I').

Another aspect of the present disclosure relates to a compound of general formula (IIA) or a salt thereof, 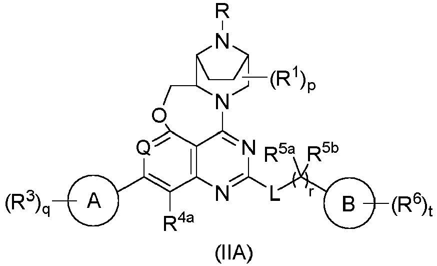 wherein,
R is an amino protecting group, and preferably Boc;
ring A, ring B, Q, L, R¹, R³, R^{4a}, R^{5a}, R^{5b}, R⁶, p, q, r and t are as defined in general formula (II).

**Table C. Typical intermediate compounds disclosed herein include, but are not limited to:**

| Compound No. | Compound structure | Name |
|---|---|---|
| | 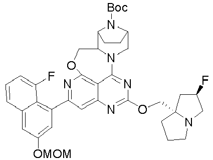 | *Tert*-butyl 2-(8-fluoro-3-(methoxymethoxy)naphthalen-1-yl)-12-(((2*R*,7*aS*)-2-fluorotetrahydro-1*H*-pyrro lizin-7*a*(5*H*)-yl)methoxy)-5*a*,6,7,8,9,10-hexah ydro-5*H*-4-oxa-3,10*a*,11,13,14-pentaaza-6,9-m ethanonaphtho[1,8-*ab*]heptalen-14-carboxylate |
| | 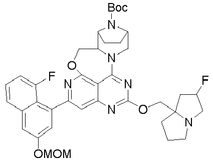 | *Tert*-butyl 2-(8-fluoro-3-(methoxymethoxy)naphthalen-1-yl)-12-((2-fluorotetrahydro-1*H*-pyrrolizin-7a(5 *H*)-yl)methoxy)-5*a*,6,7,8,9,10-hexahydro-5*H*-4 -oxa-3,10*a*,11,13,14-pentaaza-6,9-methanonap htho[1,8-*ab*]heptalen-14-carboxylate |
| 1p-2 | 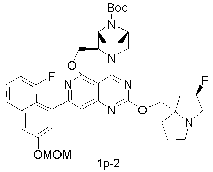 | *Tert*-butyl (5*aR*,6*R*,9*S*)-2-(8-fluoro-3-(methoxymethoxy) naphthalen-1-yl)-12-(((2*R*,7*aS*)-2-fluorotetrah ydro-1*H*-pyrrolizin-7*a*(5*H*)-yl)methoxy)-5*a*,6, 7,8,9,10-hexahydro-5*H*-4-oxa-3,10*a*,11,13,14-pentaaza-6,9-methanonaphtho[1,8-*ab*]heptalen -14-carboxylate **1p-2** |
| | 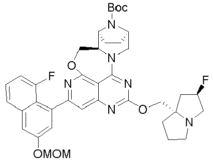 | *Tert*-butyl (5*aR*,6*S*,9*R*)-2-(8-fluoro-3-(methoxymethoxy) naphthalen-1-yl)-12-(((2*R*,7*aS*)-2-fluorotetrah ydro-1*H*-pyrrolizin-7*a*(5*H*)-yl)methoxy)-5a,6, 7,8,9,10-hexahydro-5*H*-4-oxa-3,10*a*,11,13,14-pentaaza-6,9-methanonaphtho[1,8-*ab*]heptalen -14-carboxylate |
| | 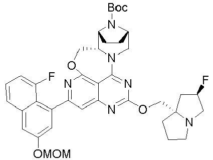 | *Tert*-butyl (5*aS*,6*R*,9*S*)-2-(8-fluoro-3-(methoxymethoxy) naphthalen-1-yl)-12-(((2*R*,7*aS*)-2-fluorotetrah ydro-1*H*-pyrrolizin-7a(5*H*)-yl)methoxy)-5a,6, 7,8,9,10-hexahydro-5*H*-4-oxa-3,10*a*,11,13,14-pentaaza-6,9-methanonaphtho[1,8-*ab*]heptalen -14-carboxylate |
| 1p-1 | 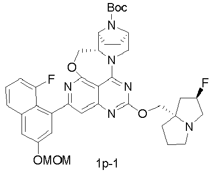 | *Tert*-butyl (5*aS*,6*S*,9*R*)-2-(8-fluoro-3-(methoxymethoxy) naphthalen-1-yl)-12-(((2*R*,7*aS*)-2-fluorotetrah ydro-1*H*-pyrrolizin-7*a*(5*H*)-yl)methoxy)-5*a*,6, 7,8,9,10-hexahydro-5*H*-4-oxa-3,10*a*,11,13,14-pentaaza-6,9-methanonaphtho[1,8-*ab*]heptalen -14-carboxylate **1p-1** |
| | 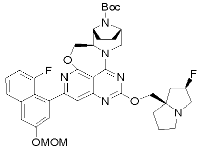 | *Tert*-butyl (5*aR*,6*R*,9*S*)-2-(8-fluoro-3-(methoxymethoxy) naphthalen-1-yl)-12-(((2*R*,7*aR*)-2-fluorotetrah ydro-1*H*-pyrrolizin-7*a*(5*H*)-yl)methoxy)-5*a*,6, 7,8,9,10-hexahydro-5*H*-4-oxa-3,10*a*,11,13,14-pentaaza-6,9-methanonaphtho[1,8-*ab*]heptalen -14-carboxylate |
| | 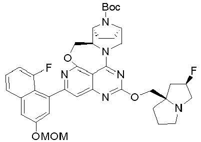 | *Tert*-butyl (5*aR*,6*S*,9*R*)-2-(8-fluoro-3 -(methoxymethoxy) naphthalen-1-yl)-12-(((2*R*,7*aR*)-2-fluorotetrah ydro-1*H*-pyrrolizin-7*a*(5*H*)-yl)methoxy)-5*a*,6, 7,8,9,10-hexahydro-5*H*-4-oxa-3,10*a*,11,13,14-pentaaza-6,9-methanonaphtho[1,8-*ab*]heptalen -14-carboxylate |
| | 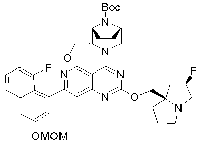 | *Tert*-butyl (5*aS*,6*R*,9*S*)-2-(8-fluoro-3-(methoxymethoxy) naphthalen-1-yl)-12-(((2*R*,7*aR*)-2-fluorotetrah ydro-1*H*-pyrrolizin-7*a*(5*H*)-yl)methoxy)-5*a*,6, 7,8,9,10-hexahydro-5*H*-4-oxa-3,10*a*,11,13,14-pentaaza-6,9-methanonaphtho[1,8-*ab*]heptalen -14-carboxylate |
| | 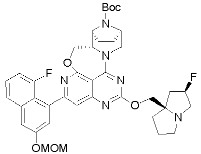 | *Tert*-butyl (5*aS*,6*S*,9*R*)-2-(8-fluoro-3-(methoxymethoxy) naphthalen-1-yl)-12-(((2*R*,7*aR*)-2-fluorotetrah ydro-1*H*-pyrrolizin-7*a*(5*H*)-yl)methoxy)-5*a*,6, 7,8,9,10-hexahydro-5*H*-4-oxa-3,10*a*,11,13,14-pentaaza-6,9-methanonaphtho[1,8-*ab*]heptalen -14-carboxylate |
| | 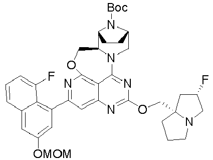 | *Tert*-butyl (5*aR*,6*R*,9*S*)-2-(8-fluoro-3-(methoxymethoxy) naphthalen-1-yl)-12-(((2*S*,7*aS*)-2-fluorotetrahy dro-1*H*-pyrrolizin-7a(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 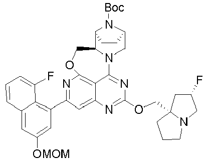 | *Tert*-butyl (5*aR*,6*S*,9*R*)-2-(8-fluoro-3 -(methoxymethoxy) naphthalen-1-yl)-12-(((2*S*,7*aS*)-2-fluorotetrahy dro-1*H*-pyrrolizin-7*a*(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 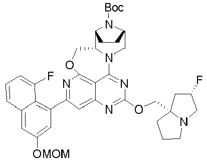 | *Tert*-butyl (5*aS*,6*R*,9*S*)-2-(8-fluoro-3-(methoxymethoxy) naphthalen-1-yl)-12-(((2*S*,7*aS*)-2-fluorotetrahy dro-1*H*-pyrrolizin-7a(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 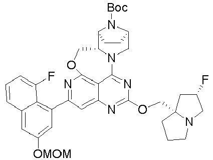 | *Tert*-butyl (5*aS*,6*S*,9*R*)-2-(8-fluoro-3-(methoxymethoxy) naphthalen-1-yl)-12-(((2*S*,7*aS*)-2-fluorotetrahy dro-1*H*-pyrrolizin-7a(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 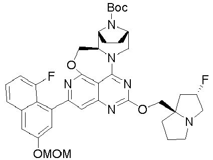 | *Tert*-butyl (5*aR*,6*R*,9*S*)-2-(8-fluoro-3-(methoxymethoxy) naphthalen-1-yl)-12-(((2*S*,7*aR*)-2-fluorotetrah ydro-1*H*-pyrrolizin-7*a*(5*H*)-yl)methoxy)-5*a*,6, 7,8,9,10-hexahydro-5*H*-4-oxa-3,10*a*,11,13,14-pentaaza-6,9-methanonaphtho[1,8-*ab*]heptalen -14-carboxylate |
| | 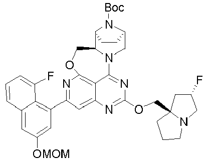 | *Tert*-butyl (5*aR*,6*S*,9*R*)-2-(8-fluoro-3 -(methoxymethoxy) naphthalen-1-yl)-12-(((2*S*,7*aR*)-2-fluorotetrah ydro-1*H*-pyrrolizin-7*a*(5*H*)-yl)methoxy)-5*a*,6, 7,8,9,10-hexahydro-5*H*-4-oxa-3,10*a*,11,13,14-pentaaza-6, 9-methanonaphtho[1,8-*ab*]heptalen -14-carboxylate |
| | 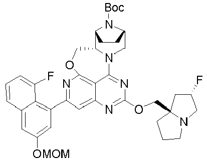 | *Tert*-butyl (5*aS*,6*R*,9*S*)-2-(8-fluoro-3-(methoxymethoxy) naphthalen-1-yl)-12-(((2*S*,7*aR*)-2-fluorotetrah ydro-1*H*-pyrrolizin-7*a*(5*H*)-yl)methoxy)-5*a*,6, 7,8,9,10-hexahydro-5*H*-4-oxa-3,10*a*,11,13,14-pentaaza-6,9-methanonaphtho[1,8-*ab*]heptalen -14-carboxylate |
| | 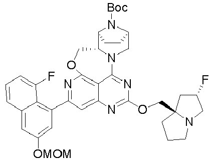 | *Tert*-butyl (5*aS*,6*S*,9*R*)-2-(8-fluoro-3-(methoxymethoxy) naphthalen-1-yl)-12-(((2*S*,7*aR*)-2-fluorotetrah ydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen -14-carboxylate |
| | 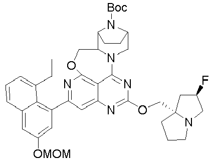 | *Tert*-butyl 2-(8-ethyl-3-(methoxymethoxy)naphthalen-1-yl)-12-(((2*R*,7*aS*)-2-fluorotetrahydro-1*H*-pyrro lizin-7*a*(5*H*)-yl)methoxy)-5*a*,6,7,8,9,10-hexah ydro-5*H*-4-oxa-3,10*a*,11,13,14-pentaaza-6,9-m ethanonaphtho[1,8-*ab*]heptalen-14-carboxylate |
| | 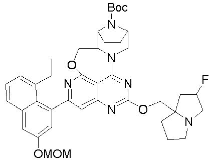 | *Tert*-butyl 2-(8-ethyl-3-(methoxymethoxy)naphthalen-1-yl)-12-((2-fluorotetrahydro-1*H*-pyrrolizin-7*a*(5 *H*)-yl)methoxy)-5*a*,6,7,8,9,10-hexahydro-5*H*-4 -oxa-3,10*a*,11,13,14-pentaaza-6,9-methanonap htho[1,8-*ab*]heptalen-14-carboxylate |
| | 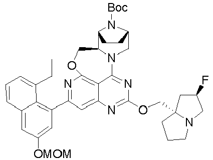 | *Tert*-butyl (5*aR*,6*R*,9*S*)-2-(8-ethyl-3-(methoxymethoxy)n aphthalen-1-yl)-12-(((2*R*,7*aS*)-2-fluorotetrahy dro-1*H*-pyrrolizin-7a(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 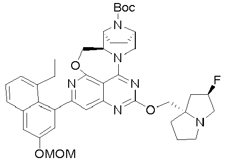 | *Tert*-butyl (5*aR,*6*S*,9*R*)-2-(8-ethyl-3-(methoxymethoxy)n aphthalen-1-yl)-12-(((2*R*,7*aS*)-2-fluorotetrahy dro-1*H*-pyrrolizin-7a(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 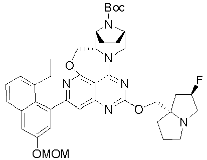 | *Tert*-butyl (5*aS,*6*R*,9*S*)-2-(8-ethyl-3-(methoxymethoxy)n aphthalen-1-yl)-12-(((2*R*,7*aS*)-2-fluorotetrahy dro-1*H*-pyrrolizin-7*a*(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 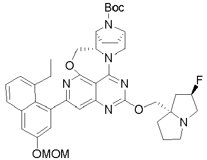 | *Ter*t-butyl (5*aS,*6*S*,9*R*)-2-(8-ethyl-3 -(methoxymethoxy)n aphthalen-1-yl)-12-(((2*R*,7*a*S)-2-fluorotetrahy dro-1*H*-pyrrolizin-7*a*(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 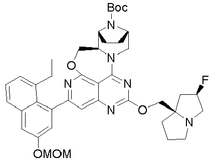 | *Tert*-butyl (5*aR*,6*R*,9*S*)-2-(8-ethyl-3-(methoxymethoxy)n aphthalen-1-yl)-12-(((2*R*,7*aR*)-2-fluorotetrahy dro-1*H*-pyrrolizin-7*a*(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 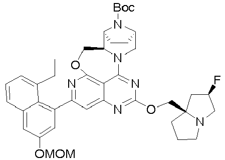 | *Tert*-butyl (5*aR*,6*S,*9*R*)-2-(8-ethyl-3-(methoxymethoxy)n aphthalen-1-yl)-12-(((2*R*,7*aR*)-2-fluorotetrahy dro-1*H*-pyrrolizin-7*a*(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 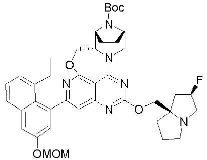 | *Tert*-butyl (5*aS,*6*R*,9*S*)-2-(8-ethyl-3-(methoxymethoxy)n aphthalen-1-yl)-12-(((2*R*,7*aR*)-2-fluorotetrahy dro-1*H*-pyrrolizin-7a(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 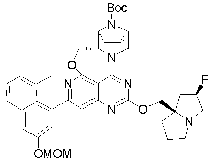 | *Tert*-butyl (5*aS,*6*S*,9*R*)-2-(8-ethyl-3-(methoxymethoxy)n aphthalen-1-yl)-12-(((2*R*,7*aR*)-2-fluorotetrahy dro-1*H*-pyrrolizin-7*a*(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 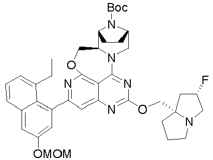 | *Tert*-butyl (5*aR*,6*R*,9*S*)-2-(8-ethyl-3-(methoxymethoxy)n aphthalen-1-yl)-12-(((2*S*,7*aS*)-2-fluorotetrahyd ro-1*H*-pyrrolizin-7*a*(5*H*)-yl)methoxy)-5*a*,6,7,8,9,10-hexahydro-5*H-*4-oxa-3,10*a*,11,13,14-pen taaza-6,9-methanonaphtho[1,8-*ab*]heptalen-14 -carboxylate |
| | 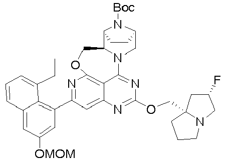 | *Tert-*butyl (5*aR,*6*S,*9*R*)-2-(8-ethyl-3-(methoxymethoxy)n aphthalen-1-yl)-12-(((2*S*,7*aS*)-2-fluorotetrahyd ro-1*H*-pyrrolizin-7*a*(5*H*)-yl)methoxy)-5*a*,6,7,8 ,9,10-hexahydro-5*H*-4-oxa-3,10*a*,11,13,14-pen taaza-6,9-methanonaphtho[1,8-*ab*]heptalen-14 -carboxylate |
| | 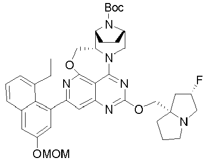 | *Tert*-butyl (5*aS*,6*R*,9*S*)-2-(8-ethyl-3-(methoxymethoxy)n aphthalen-1-yl)-12-(((2*S*,7*aS*)-2-fluorotetrahyd ro-1*H*-pyrrolizin-7a(5*H*)-yl)methoxy)-5*a*,6,7,8 ,9,10-hexahydro-5*H*-4-oxa-3,10*a,*11,13,14-pen taaza-6,9-methanonaphtho[1,8-*ab*]heptalen-14 -carboxylate |
| | 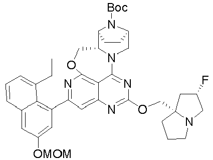 | *Tert*-butyl (5*aS*,6*S*,9*R*)-2-(8-ethyl-3-(methoxymethoxy)n aphthalen-1-yl)-12-(((2*S*,7*aS*)-2-fluorotetrahyd ro-1*H*-pyrrolizin-7*a*(5*H*)-yl)methoxy)-5*a*,6,7,8 ,9,10-hexahydro-5*H*-4-oxa-3,10*a*,11,13,14-pen taaza-6,9-methanonaphtho[1,8-*ab*]heptalen-14 -carboxylate |
| | 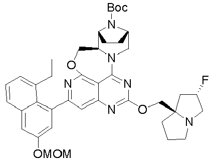 | *Tert*-butyl (5*aR*,6*R*,9*S*)-2-(8-ethyl-3-(methoxymethoxy)n aphthalen-1-yl)-12-(((2*S*,7*aR*)-2-fluorotetrahy dro-1*H*-pyrrolizin-7*a*(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 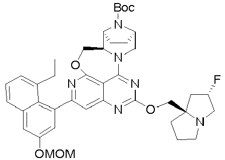 | *Tert*-butyl (5*aR*,6*S*,9*R*)-2-(8-ethyl-3-(methoxymethoxy)n aphthalen-1-yl)-12-(((2*S*,7*aR*)-2-fluorotetrahy dro-1*H*-pyrrolizin-7*a*(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 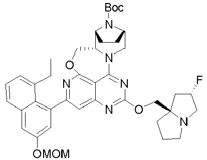 | *Tert*-butyl (5*aS*,6*R*,9*S*)-2-(8-ethyl-3-(methoxymethoxy)n aphthalen-1-yl)-12-(((2*S*,7*aR*)-2-fluorotetrahy dro-1*H*-pyrrolizin-7a(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 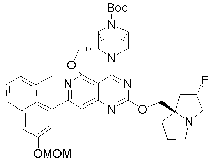 | *Tert*-butyl (5*aS*,6*S*,9*R*)-2-(8-ethyl-3-(methoxymethoxy)n aphthalen-1-yl)-12-(((2*S*,7*aR*)-2-fluorotetrahy dro-1*H*-pyrrolizin-7*a*(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 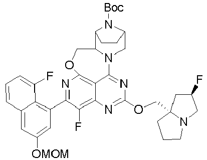 | *Tert*-butyl 1-fluoro-2-(8-fluoro-3 -(methoxymethoxy)naph thalen-1-yl)-12-(((2*R*,7*aS*)-2-fluorotetrahydro-1*H*-pyrrolizin-7*a*(5*H*)-yl)methoxy)-5*a*,6,7,8,9, 10-hexahydro-5*H*-4-oxa-3,10*a*,11,13,14-penta aza-6,9-methanonaphtho[1,8-*ab*]heptalen-14-c arboxylate |
| | 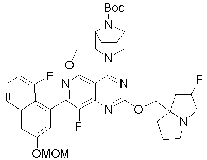 | *Tert*-butyl 1-fluoro-2-(8-fluoro-3 -(methoxymethoxy)naph thalen-1-yl)-12-((2-fluorotetrahydro-1*H*-pyrrol izin-7*a*(5*H*)-yl)methoxy)-5*a*,6,7,8,9,10-hexahy dro-5*H*-4-oxa-3,10*a*,11,13,14-pentaaza-6,9-me thanonaphtho[1,8-*ab*]heptalen-14-carboxylate |
| | 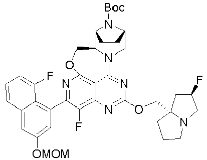 | *Tert*-butyl (5*aR*,6*R*,9*S*)-1-fluoro-2-(8-fluoro-3-(methoxy methoxy)naphthalen-1-yl)-12-(((2*R*,7*aS*)-2-flu orotetrahydro-1*H*-pyrrolizin-7*a*(5*H*)-yl)metho xy)-5a,6,7,8,9,10-hexahydro-5*H*-4-oxa-3,10*a*, 11,13,14-pentaaza-6,9-methanonaphtho[1,8-*ab* ]heptalen-14-carboxylate |
| | 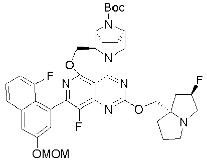 | *Tert*-butyl (5*aR*,6*S,*9*R)*-1-fluoro-2-(8-fluoro-3-(methoxy methoxy)naphthalen-1-yl)-12-(((2*R*,7*aS*)-2-flu orotetrahydro-1*H*-pyrrolizin-7*a*(5*H*)-yl)metho xy)-5*a*,6,7,8,9,10-hexahydro-5*H*-4-oxa-3,10*a*,11,13,14-pentaaza-6,9-methanonaphtho[1,8-*ab* ]heptalen-14-carboxylate |
| | 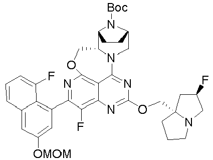 | *Tert*-butyl (5*aS*,6*R*,9*S*)-1-fluoro-2-(8-fluoro-3-(methoxy methoxy)naphthalen-1-yl)-12-(((2*R*,7*aS*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho [1,8-*ab* ]heptalen-14-carboxylate |
| | 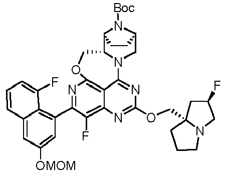 | *Tert*-butyl (5*aS*,6*S*,9*R*)-1-fluoro-2-(8-fluoro-3-(methoxy methoxy)naphthalen-1-yl)-12-(((2*R*,7*aS*)-2-flu orotetrahydro-1*H*-pyrrolizin-7*a*(5*H*)-yl)metho xy)-5a,6,7,8,9,10-hexahydro-5*H*-4-oxa-3,10*a*, 11,13,14-pentaaza-6,9-methanonaphtho [1,8-*ab* ]heptalen-14-carboxylate |
| | 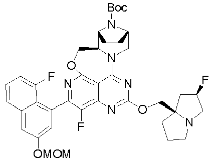 | *Tert*-butyl (5*aR*,6*R*,9*S*)-1-fluoro-2-(8-fluoro-3-(methoxy methoxy)naphthalen-1-yl)-12-(((2*R*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho [1,8-*ab* ]heptalen-14-carboxylate |
| | 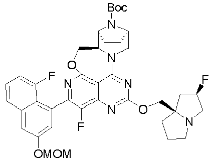 | *Tert*-butyl (5*aR*,6*S*,9*R*)-1-fluoro-2-(8-fluoro-3-(methoxy methoxy)naphthalen-1-yl)-12-(((2*R*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho [1,8-*ab* ]heptalen-14-carboxylate |
| | 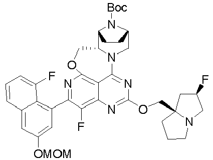 | *Tert*-butyl (5*aS*,6*R*,9*S*)-1-fluoro-2-(8-fluoro-3-(methoxy methoxy)naphthalen-1-yl)-12-(((2*R*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 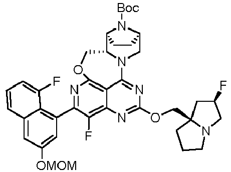 | *Tert*-butyl (5*aS*,6*S*,9*R*)-1-fluoro-2-(8-fluoro-3-(methoxy methoxy)naphthalen-1-yl)-12-(((2*R*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho [1,8-*ab* ]heptalen-14-carboxylate |
| | 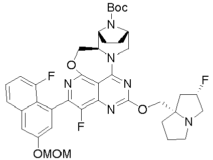 | *Tert*-butyl (5*aR*,6*R*,9*S*)-1-fluoro-2-(8-fluoro-3-(methoxy methoxy)naphthalen-1-yl)-12-(((2*S*,7*aS*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho [1,8-*ab* ]heptalen-14-carboxylate |
| | 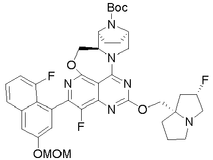 | *Tert*-butyl (5*aR*,6*S*,9*R*)-1-fluoro-2-(8-fluoro-3-(methoxy methoxy)naphthalen-1-yl)-12-(((2*S*,7*aS*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho [1,8-*ab* ]heptalen-14-carboxylate |
| | 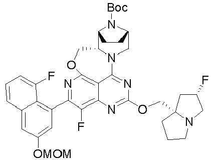 | *Tert*-butyl (5*aS*,6*R*,9*S*)-1-fluoro-2-(8-fluoro-3-(methoxy methoxy)naphthalen-1-yl)-12-(((2*S*,7*aS*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho [1,8-*ab* ]heptalen-14-carboxylate |
| | 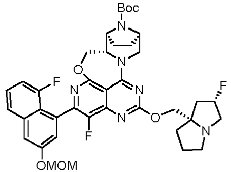 | *Tert*-butyl (5*aS*,6*S*,9*R*)-1-fluoro-2-(8-fluoro-3-(methoxy methoxy)naphthalen-1-yl)-12-(((2*S*,7*aS*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho [1,8-*ab* ]heptalen-14-carboxylate |
| | 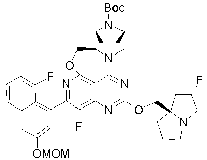 | *Tert*-butyl (5*aR*,6*R*,9*S*)-1-fluoro-2-(8-fluoro-3-(methoxy methoxy)naphthalen-1-yl)-12-(((2*S*,7*aR*)-2-flu orotetrahydro-1*H*-pyrrolizin-7*a*(5*H*)-yl)methoxy)-5*a*,6,7,8,9,10-hexahydro-5*H*-4-oxa-3,10*a*, 11,13,14-pentaaza-6,9-methanonaphtho [1,8-*ab* ]heptalen-14-carboxylate |
| | 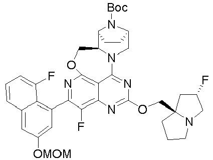 | *Tert*-butyl (5*aR*,6*S*,9*R*)-1-fluoro-2-(8-fluoro-3-(methoxy methoxy)naphthalen-1-yl)-12-(((2*S*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho [1,8-*ab* ]heptalen-14-carboxylate |
| | 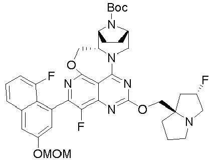 | *Tert*-butyl (5*aS*,6*R*,9*S*)-1-fluoro-2-(8-fluoro-3-(methoxy methoxy)naphthalen-1-yl)-12-(((2*S*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho [1,8-*ab* ]heptalen-14-carboxylate |
| | 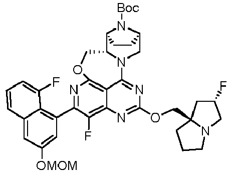 | *Tert*-butyl (5*aS*,6*S*,9*R*)-1-fluoro-2-(8-fluoro-3-(methoxy methoxy)naphthalen-1-yl)-12-(((2*S*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho [1,8-*ab* ]heptalen-14-carboxylate |
| | 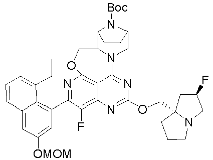 | *Tert*-butyl 2-(8-ethyl-3-(methoxymethoxy)naphthalen-1-yl)-1-fluoro-12-(((2*R*,7*aS*)-2-fluorotetrahydro-1*H*-pyrrolizin-7*a*(5*H*)-yl)methoxy)-5*a*,6,7,8,9, 10-hexahydro-5*H*-4-oxa-3,10*a*,11,13,14-penta aza-6,9-methanonaphtho[1,8-*ab*]heptalen-14-c arboxylate |
| | 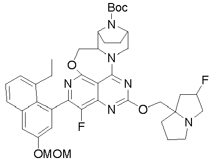 | *Tert*-butyl 2-(8-ethyl-3-(methoxymethoxy)naphthalen-1-yl)-1-fluoro-12-((2-fluorotetrahydro-1*H*-pyrrol izin-7*a*(5*H*)-yl)methoxy)-5*a*,6,7,8,9,10-hexahy dro-5*H*-4-oxa-3,10*a*,11,13,14-pentaaza-6,9-me thanonaphtho[1,8-*ab*]heptalen-14-carboxylate |
| 4j-2 | 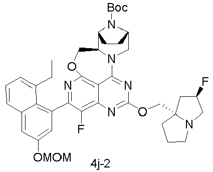 | *Tert*-butyl (5*aR*,6*R*,9*S*)-2-(8-ethyl-3-(methoxymethoxy)n aphthalen-1-yl)-1-fluoro-12-(((2*R*,7*aS*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate **4j-2** |
| | 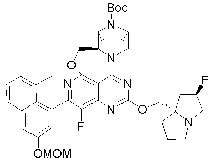 | *Tert*-butyl (5a*R,*6*S*,9*R*)-2-(8-ethyl-3-(methoxymethoxy)n aphthalen-1-yl)-1-fluoro-12-(((2*R*,7*aS*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 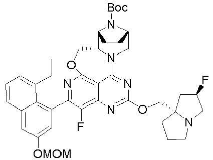 | *Tert*-butyl (5*aS,*6*R*,9*S*)-2-(8-ethyl-3-(methoxymethoxy)n aphthalen-1-yl)-1-fluoro-12-(((2*R*,7*a5*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| 4j-1 | 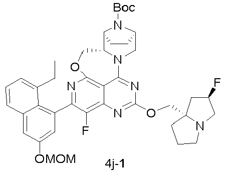 | *Tert*-butyl (5*aS*,6*S*,9*R*)-2-(8-ethyl-3-(methoxymethoxy)n aphthalen-1-yl)-1-fluoro-12-(((2*R*,7*aS*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate **4j-1** |
| | 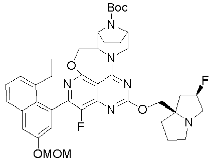 | *Tert*-butyl 2-(8-ethyl-3-(methoxymethoxy)naphthalen-1-yl)-1-fluoro-12-(((2*R*,7*aR*)-2-fluorotetrahydro-1*H*-pyrrolizin-7*a*(5*H*)-yl)methoxy)-5*a*,6,7,8,9, 10-hexahydro-5*H*-4-oxa-3,10*a*,11,13,14-penta aza-6,9-methanonaphtho[1,8-*ab*]heptalen-14-c arboxylate |
| | 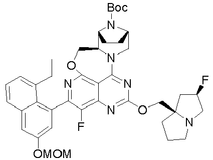 | *Tert*-butyl (5*aR*,6*R*,9*S*)-2-(8-ethyl-3-(methoxymethoxy)n aphthalen-1-yl)-1-fluoro-12-(((2*R*,7*aR*)-2-fluor otetrahydro-1*H*-pyrrolizin-7*a*(5*H*)-yl)methoxy)-5*a*,6,7,8,9,10-hexahydro-5*H*-4-oxa-3,10*a,*11, 13,14-pentaaza-6,9-methanonaphtho[1,8-*ab*]he ptalen-14-carboxylate |
| | 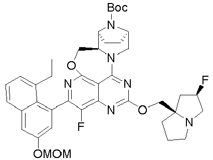 | *Tert*-butyl (5*aR*,6*S,*9*R*)-2-(8-ethyl-3-(methoxymethoxy)n aphthalen-1-yl)-1-fluoro-12-(((2*R*,7*aR*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 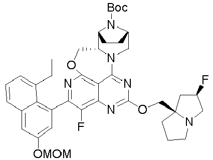 | *Tert*-butyl (5*aS,*6*R*,9*S*)-2-(8-ethyl-3-(methoxymethoxy)n aphthalen-1-yl)-1-fluoro-12-(((2*R*,7*aR*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 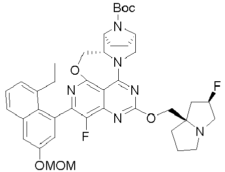 | *Tert*-butyl (5*aS*,6*S*,9*R*)-2-(8-ethyl-3-(methoxymethoxy)n aphthalen-1-yl)-1-fluoro-12-(((2*R*,7*aR*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 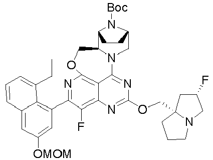 | *Tert*-butyl (5*aR*,6*R*,9*S*)-2-(8-ethyl-3-(methoxymethoxy)n aphthalen-1-yl)-1-fluoro-12-(((2*S*,7*aS*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 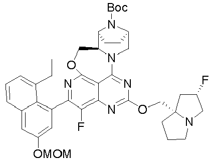 | *Tert*-butyl (5*aR,*6*S,*9*R*)-2-(8-ethyl-3-(methoxymethoxy)n aphthalen-1-yl)-1-fluoro-12-(((2*S*,7*aS*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 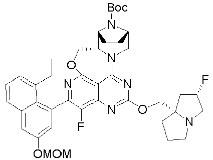 | *Tert*-butyl (5*aS*,6*R*,9*S*)-2-(8-ethyl-3-(methoxymethoxy)n aphthalen-1-yl)-1-fluoro-12-(((2*S*,7*aS*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 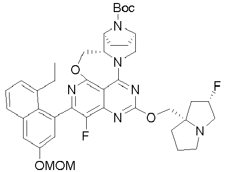 | *Tert*-butyl (5*aS*,6*S*,9*R*)-2-(8-ethyl-3-(methoxymethoxy)n aphthalen-1-yl)-1-fluoro-12-(((25,7a5)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 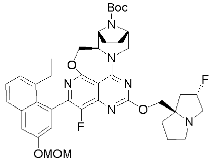 | *Tert*-butyl (5*aR*,6*R*,9*S*)-2-(8-ethyl-3-(methoxymethoxy)n aphthalen-1-yl)-1-fluoro-12-(((2*S*,7*aR*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 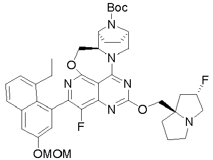 | *Tert*-butyl (5*aR,*6*S,*9*R*)-2-(8-ethyl-3-(methoxymethoxy)n aphthalen-1-yl)-1-fluoro-12-(((2*S*,7*aR*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 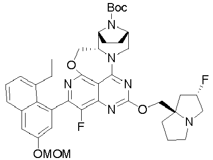 | *Tert*-butyl (5*aS,*6*R*,9*S*)-2-(8-ethyl-3-(methoxymethoxy)n aphthalen-1-yl)-1-fluoro-12-(((2*S*,7*aR*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 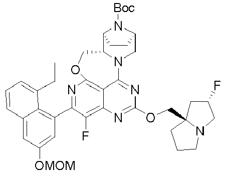 | *Tert*-butyl (5*aS,6*S,9*R*)-2-(8-ethyl-3-(methoxymethoxy)n aphthalen-1-yl)-1-fluoro-12-(((2*S*,7*aR*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 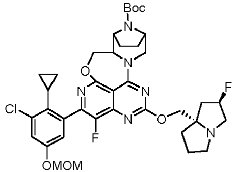 | *Tert*-butyl 2-(3-chloro-2-cyclopropyl-5-(methoxymethox y)phenyl)-1-fluoro-12-(((2*S*,7*aS*)-2-fluorotetra hydro-1*H*-pyrrolizin-7*a*(5*H*)-yl)methoxy)-5*a*,6 ,7,8,9,10-hexahydro-5*H*-4-oxa-3,10*a*,11,13,14 -pentaaza-6,9-methanonaphtho[1,8-*ab*]heptale n-14-carboxylate |
| | 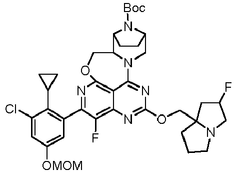 | *Tert*-butyl 2-(3-chloro-2-cyclopropyl-5-(methoxymethox y)phenyl)-1-fluoro-12-((2-fluorotetrahydro-1*H* -pyrrolizin-7*a*(5*H*)-yl)methoxy)-5*a*,6,7,8,9,10-hexahydro-5*H*-4-oxa-3,10*a*,11,13,14-pentaaza -6,9-methanonaphtho[1,8-*ab*]heptalen-14-carb oxylate |
| 6a-2 | 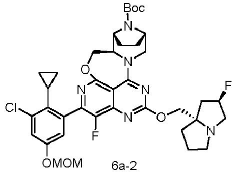 | *Tert*-butyl (5*aR*,6*R*,9*S*)-2-(3-chloro-2-cyclopropyl-5-(met hoxymethoxy)phenyl)-1-fluoro-12-(((2*R*,7*aS*)-2-fluorotetrahydro-1*H*-pyrrolizin-7*a*(5*H*)-y1)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate **6a-2** |
| | 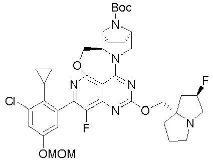 | *Tert*-butyl (5*aR*,6*S*,9*R*)-2-(3-chloro-2-cyclopropyl-5-(met hoxymethoxy)phenyl)-1-fluoro-12-(((2*R*,7*aS*)-2-fluorotetrahydro-1*H*-pyrrolizin-7*a*(5*H*)-yl)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| | 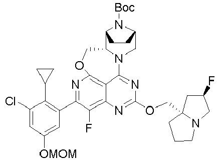 | *Tert*-butyl (5*aS*,6*R*,9*S*)-2-(3-chloro-2-cyclopropyl-5-(met hoxymethoxy)phenyl)-1-fluoro-12-(((2*R*,7*aS*)-2-fluorotetrahydro-1*H*-pyrrolizin-7*a*(5*H*)-yl)methoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| 6a-1 | 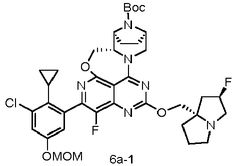 | *Ter*t-butyl (5*aS*,6*S*,9*R*)-2-(3-chloro-2-cyclopropyl-5-(met hoxymethoxy)phenyl)-1-fluoro-12-(((2*R*,7*aS*)-2-fluorotetrahydro-1*H*-pyrrolizin-7*a*(5*H*)-yl)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate **6a-1** |
| | 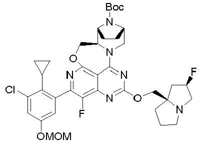 | *Tert*-butyl (5*aR*,6*R*,9*S*)-2-(3-chloro-2-cyclopropyl-5-(met hoxymethoxy)phenyl)-1-fluoro-12-(((2*R*,7*aR*)-2-fluorotetrahydro-1*H*-pyrrolizin-7*a*(5*H*)-yl)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| | 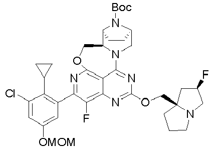 | *Tert*-butyl (5*aR*,6*S*,9*R*)-2-(3-chloro-2-cyclopropyl-5-(met hoxymethoxy)phenyl)-1-fluoro-12-(((2*R*,7*aR*)-2-fluorotetrahydro-1*H*-pyrrolizin-7*a*(5*H*)-yl)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| | 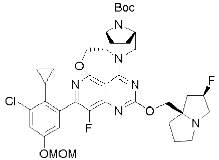 | *Tert*-butyl (5*aS*,6*R*,9*S*)-2-(3-chloro-2-cyclopropyl-5-(met hoxymethoxy)phenyl)-1-fluoro-12-(((2*R*,7*aR*)-2-fluorotetrahydro-1*H*-pyrrolizin-7*a*(5*H*)-yl)m ethoxy)-5*a*,6,7,8,9,10-hexahydro-5H-4-oxa-3, 10*a,*11,13,14-pentaaza-6,9-methanonaphtho[1, 8-*ab*]heptalen-14-carboxylate |
| | 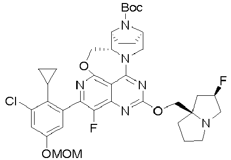 | *Tert*-butyl (5*aS*,6*S*,9*R*)-2-(3-chloro-2-cyclopropyl-5-(met hoxymethoxy)phenyl)-1-fluoro-12-(((2*R*,7*aR*)-2-fluorotetrahydro-1*H*-pyrrolizin-7*a*(5*H*)-yl)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| | 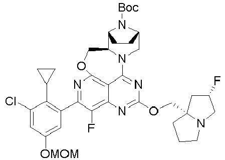 | *Tert*-butyl (5*aR*,6*R*,9*S*)-2-(3-chloro-2-cyclopropyl-5-(met hoxymethoxy)phenyl)-1-fluoro-12-(((2*S*,7*aS*)-2-fluorotetrahydro-1*H*-pyrrolizin-7*a*(5*H*)-yl)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| | 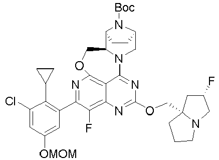 | *Tert*-butyl (5*aR*,6*S*,9*R*)-2-(3-chloro-2-cyclopropyl-5-(met hoxymethoxy)phenyl)-1-fluoro-12-(((2*S*,7*aS*)-2-fluorotetrahydro-1*H*-pyrrolizin-7*a*(5*H*)-yl)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| | 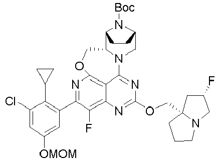 | *Tert*-butyl (5*aS*,6*R*,9*S*)-2-(3-chloro-2-cyclopropyl-5-(met hoxymethoxy)phenyl)-1-fluoro-12-(((2*S*,7*aS*)-2-fluorotetrahydro-1*H*-pyrrolizin-7*a*(5*H*)-yl)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| | 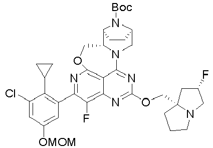 | *Tert*-butyl (5*aS*,6*S*,9*R*)-2-(3-chloro-2-cyclopropyl-5-(met hoxymethoxy)phenyl)-1-fluoro-12-(((2*S*,7*aS*)-2-fluorotetrahydro-1*H*-pyrrolizin-7*a*(5*H*)-yl)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| | 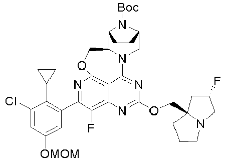 | *Tert*-butyl (5*aR*,6*R*,9*S*)-2-(3-chloro-2-cyclopropyl-5-(met hoxymethoxy)phenyl)-1-fluoro-12-(((2*S*,7*aR*)-2-fluorotetrahydro-1*H*-pyrrolizin-7*a*(5*H*)-yl)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| | 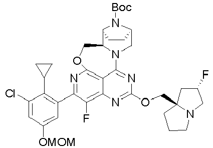 | *Tert*-butyl (5*aR*,6*S*,9*R*)-2-(3-chloro-2-cyclopropyl-5-(met hoxymethoxy)phenyl)-1-fluoro-12-(((2*S*,7*aR*)-2-fluorotetrahydro-1*H*-pyrrolizin-7*a*(5*H*)-yl)methoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| | 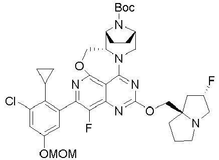 | *Tert*-butyl (5*aS*,6*R*,9*S*)-2-(3-chloro-2-cyclopropyl-5-(met hoxymethoxy)phenyl)-1-fluoro-12-(((2*S*,7*aR*)-2-fluorotetrahydro-1*H*-pyrrolizin-7*a*(5*H*)-yl)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| | 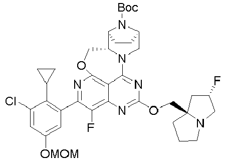 | *Tert*-butyl (5*aS*,6*S*,9*R*)-2-(3-chloro-2-cyclopropyl-5-(met hoxymethoxy)phenyl)-1-fluoro-12-(((2*S*,7*aR*)-2-fluorotetrahydro-1*H*-pyrrolizin-7*a*(5*H*)-yl)m ethoxy)-5*a*,6,7,8,9,10-hexahydro-5*H*-4-oxa-3, 10*a,*11,13,14-pentaaza-6,9-methanonaphtho[1, 8-*ab*]heptalen-14-carboxylate |
| | 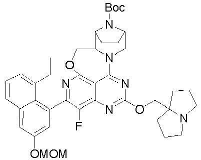 | *Tert*-butyl 2-(8-ethyl-3-(methoxymethoxy)naphthalen-1-yl)-1-fluoro-12-((tetrahydro-1*H*-pyrrolizin-7*a*( 5*H*)-yl)methoxy)-5*a*,6,7,8,9,10-hexahydro-5*H-*4-oxa-3,10*a,*11,13,14-pentaaza-6,9-methanona phtho[1,8-*ab*]heptalen-14-carboxylate |
| | 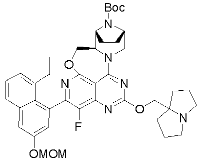 | *Tert*-butyl (5*aR*,6*R*,9*S*)-2-(8-ethyl-3-(methoxymethoxy)n aphthalen-1-yl)-1-fluoro-12-((tetrahydro-1*H*-p yrrolizin-7*a*(5*H*)-yl)methoxy)-5*a*,6,7,8,9,10-he xahydro-5*H*-4-oxa-3,10*a*,11,13,14-pentaaza-6, 9-methanonaphtho[1,8-*ab*]heptalen-14-carbox ylate |
| | 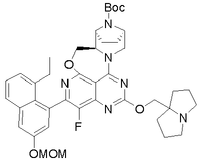 | *Tert*-butyl (5*aR*,6*S,*9*R*)-2-(8-ethyl-3-(methoxymethoxy)n aphthalen-1-yl)-1-fluoro-12-((tetrahydro-1*H*-p yrrolizin-7*a*(5*H*)-yl)methoxy)-5*a*,6,7,8,9,10-he xahydro-5*H*-4-oxa-3,10*a*,11,13,14-pentaaza-6, 9-methanonaphtho[1,8-*ab*]heptalen-14-carbox ylate |
| | 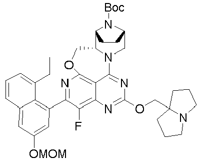 | *Tert*-butyl (5*aS,*6*R*,9*S*)-2-(8-ethyl-3-(methoxymethoxy)n aphthalen-1-yl)-1-fluoro-12-((tetrahydro-1*H*-p yrrolizin-7*a*(5*H*)-yl)methoxy)-5*a*,6,7,8,9,10-he xahydro-5*H*-4-oxa-3,10*a*,11,13,14-pentaaza-6, 9-methanonaphtho[1,8-*ab*]heptalen-14-carbox ylate |
| | 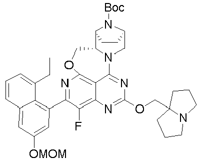 | *Tert*-butyl (5*aS,*6*S*,9*R*)-2-(8-ethyl-3-(methoxymethoxy)n aphthalen-1-yl)-1-fluoro-12-((tetrahydro-1*H*-p yrrolizin-7*a*(5*H*)-yl)methoxy)-5*a*,6,7,8,9,10-he xahydro-5*H*-4-oxa-3,10*a*,11,13,14-pentaaza-6, 9-methanonaphtho[1,8-*ab*]heptalen-14-carbox ylate |
| | 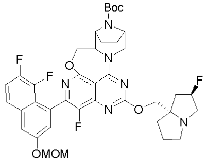 | *Tert*-butyl 2-(7,8-difluoro-3-(methoxymethoxy)naphthale n-1-yl)-1-fluoro-12-(((2*R*,7*aS*)-2-fluorotetrahy dro-1*H*-pyrrolizin-7*a*(5*H*)-yl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 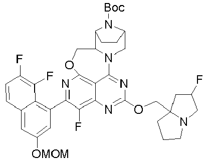 | *Tert*-butyl 2-(7,8-difluoro-3-(methoxymethoxy)naphthale n-1-yl)-1-fluoro-12-((2-fluorotetrahydro-1*H*-p yrrolizin-7*a*(5*H*)-yl)methoxy)-5*a*,6,7,8,9,10-he xahydro-5*H*-4-oxa-3,10*a*,11,13,14-pentaaza-6, 9-methanonaphtho[1,8-*ab*]heptalen-14-carbox ylate |
| | 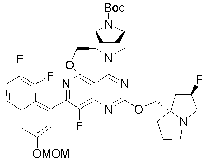 | *Tert*-butyl (5*aR*,6*R*,9*S*)-2-(7,8-difluoro-3-(methoxymetho xy)naphthalen-1-yl)-1-fluoro-12-(((2*R*,7*aS*)-2-fluorotetrahydro-1*H*-pyrrolizin-7*a*(5*H*)-yl)met hoxy)-5*a*,6,7,8,9,10-hexahydro-5*H*-4-oxa-3,10 *a*,11,13,14-pentaaza-6,9-methanonaphtho[1,8-*ab*]heptalen-14-carboxylate |
| | 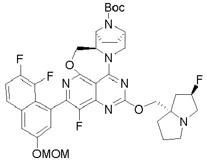 | *Tert*-butyl (5*aR*,6*S*,9*R*)-2-(7,8-difluoro-3-(methoxymetho xy)naphthalen-1-yl)-1-fluoro-12-(((2*R*,7*aS*)-2-fluorotetrahydro-1*H*-pyrrolizin-7*a*(5*H*)-yl)methoxy)-5*a*,6,7,8,9,10-hexahydro-5*H*-4-oxa-3,10 *a*,11,13,14-pentaaza-6,9-methanonaphtho[1,8-*ab*]heptalen-14-carboxylate |
| | 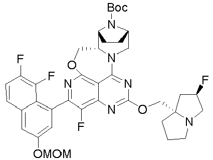 | *Tert*-butyl (5*aS*,6*R*,9*S*)-2-(7,8-difluoro-3-(methoxymetho xy)naphthalen-1-yl)-1-fluoro-12-(((2*R*,7*aS*)-2-fluorotetrahydro-1*H*-pyrrolizin-7*a*(5*H*)-yl)met hoxy)-5*a*,6,7,8,9,10-hexahydro-5*H*-4-oxa-3,10 *a*,11,13,14-pentaaza-6,9-methanonaphtho[1,8-*ab*]heptalen-14-carboxylate |
| | 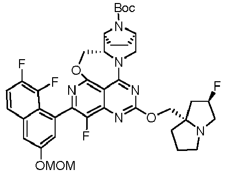 | *Tert*-butyl (5*aS*,6*S*,9*R*)-2-(7,8-difluoro-3-(methoxymetho xy)naphthalen-1-yl)-1-fluoro-12-(((2*R*,7*aS*)-2-fluorotetrahydro-1*H*-pyrrolizin-7*a*(5*H*)-yl)met hoxy)-5*a*,6,7,8,9,10-hexahydro-5*H*-4-oxa-3,10 *a*,11,13,14-pentaaza-6,9-methanonaphtho[1,8-*ab*]heptalen-14-carboxylate |
| | 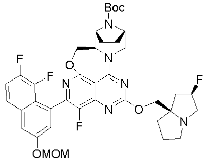 | *Tert*-butyl (5*aR*,6*R*,9*S*)-2-(7,8-difluoro-3-(methoxymetho xy)naphthalen-1-yl)-1-fluoro-12-(((2*R*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10 *a*,11,13,14-pentaaza-6,9-methanonaphtho[1,8-*ab*]heptalen-14-carboxylate |
| | 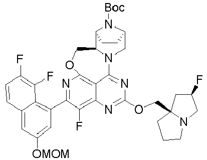 | *Tert*-butyl (5*aR*,6*S*,9*R*)-2-(7,8-difluoro-3-(methoxymetho xy)naphthalen-1-yl)-1-fluoro-12-(((2*R*,7*aR*)-2-fluorotetrahydro-1*H*-pyrrolizin-7*a*(5*H*)-yl)met hoxy)-5a,6,7,8,9,10-hexahydro-5*H*-4-oxa-3,10 *a*,11,13,14-pentaaza-6,9-methanonaphtho[1,8-*ab*]heptalen-14-carboxylate |
| | 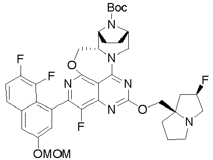 | *Tert*-butyl (5*aS*,6*R*,9*S*)-2-(7,8-difluoro-3-(methoxymetho xy)naphthalen-1-yl)-1-fluoro-12-(((2*R*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10 *a*,11,13,14-pentaaza-6,9-methanonaphtho[1,8-*ab*]heptalen-14-carboxylate |
| | 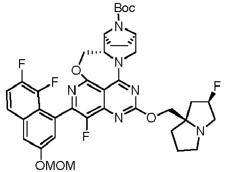 | *Tert*-butyl (5*aS*,6*S*,9*R*)-2-(7,8-difluoro-3-(methoxymetho xy)naphthalen-1-yl)-1-fluoro-12-(((2*R*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10 *a*,11,13,14-pentaaza-6,9-methanonaphtho[1,8-*ab*]heptalen-14-carboxylate |
| | 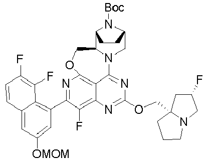 | *Tert*-butyl (5*aR*,6*R*,9*S*)-2-(7,8-difluoro-3-(methoxymetho xy)naphthalen-1-yl)-1-fluoro-12-(((2*S*,7*aS*)-2-f luorotetrahydro-1*H*-pyrrolizin-7*a*(5*H*)-yl)meth oxy)-5*a*,6,7,8,9,10-hexahydro-5*H*-4-oxa-3,10*a* ,11,13,14-pentaaza-6,9-methanonaphtho[1,8-*a b*]heptalen-14-carboxylate |
| | 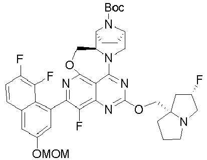 | *Tert*-butyl (5*aR*,6*S*,9*R*)-2-(7,8-difluoro-3-(methoxymetho xy)naphthalen-1-yl)-1-fluoro-12-(((2*S*,7*aS*)-2-f luorotetrahydro-1*H*-pyrrolizin-7*a*(5*H*)-yl)meth oxy)-5*a*,6,7,8,9,10-hexahydro-5*H*-4-oxa-3,10*a* ,11,13,14-pentaaza-6,9-methanonaphtho[1,8-*a b*]heptalen-14-carboxylate |
| | 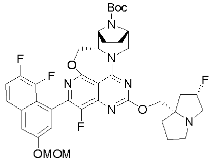 | *Tert*-butyl (5*aS*,6*R*,9*S*)-2-(7,8-difluoro-3-(methoxymetho xy)naphthalen-1-yl)-1-fluoro-12-(((2*S*,7*aS*)-2-f luorotetrahydro-1*H*-pyrrolizin-7*a*(5*H*)-yl)meth oxy)-5*a*,6,7,8,9,10-hexahydro-5*H*-4-oxa-3,10*a* ,11,13,14-pentaaza-6,9-methanonaphtho[1,8-*a b*]heptalen-14-carboxylate |
| | 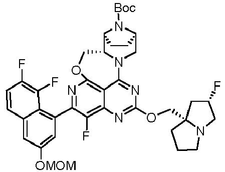 | *Tert*-butyl (5*aS*,6*S*,9*R*)-2-(7,8-difluoro-3-(methoxymetho xy)naphthalen-1-yl)-1-fluoro-12-(((2*S*,7*aS*)-2-f luorotetrahydro-1*H*-pyrrolizin-7*a*(5*H*)-yl)meth oxy)-5*a*,6,7,8,9,10-hexahydro-5H-4-oxa-3,10*a* ,11,13,14-pentaaza-6,9-methanonaphtho[1,8-*a b*]heptalen-14-carboxylate |
| | 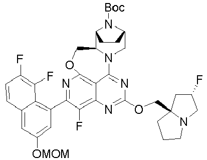 | *Tert*-butyl (5*aR*,6*R*,9*S*)-2-(7,8-difluoro-3-(methoxymetho xy)naphthalen-1-yl)-1-fluoro-12-(((2*S*,7*aR*)-2-fluorotetrahydro-1*H*-pyrrolizin-7*a*(5*H*)-yl)methoxy)-5*a*,6,7,8,9,10-hexahydro-5*H*-4-oxa-3,10 *a*,11,13,14-pentaaza-6,9-methanonaphtho[1, 8-*ab*]heptalen-14-carboxylate |
| | 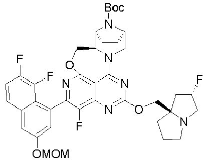 | *Tert*-butyl (5*aR,*6*S*,9*R*)-2-(7,8-difluoro-3-(methoxymetho xy)naphthalen-1-yl)-1-fluoro-12-(((2*S*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10 *a*,11,13,14-pentaaza-6,9-methanonaphtho[1,8-*ab*]heptalen-14-carboxylate |
| | 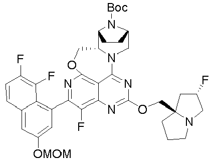 | *Tert*-butyl (5*aS*,6*R*,9*S*)-2-(7,8-difluoro-3-(methoxymetho xy)naphthalen-1-yl)-1-fluoro-12-(((2*S*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10 a,11,13,14-pentaaza-6,9-methanonaphtho[1,8-*ab*]heptalen-14-carboxylate |
| | 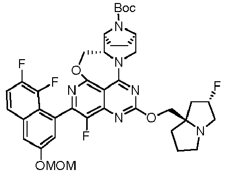 | *Tert*-butyl (5*aS*,6*S*,9*R*)-2-(7,8-difluoro-3-(methoxymetho xy)naphthalen-1-yl)-1-fluoro-12-(((2*S*,7*aR*)-2-fluorotetrahydro-1*H*-pyrrolizin-7*a*(5*H*)-yl)met hoxy)-5*a*,6,7,8,9,10-hexahydro-5*H*-4-oxa-3,10 *a*,11,13,14-pentaaza-6,9-methanonaphtho[1,8-*ab*]heptalen-14-carboxylate |
| | 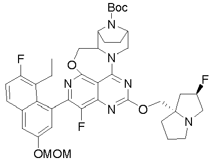 | *Tert*-butyl 2-(8-ethyl-7-fluoro-3-(methoxymethoxy)napht halen-1-yl)-1-fluoro-12-(((2*R*,7*aS*)-2-fluorotetr ahydro-1*H*-pyrrolizin-7*a*(5*H*)-yl)methoxy)-5*a*, 6,7,8,9,10-hexahydro-5*H*-4-oxa-3,*10a,*11,13,1 4-pentaaza-6,9-methanonaphtho[1,8-*ab*]heptal en-14-carboxylate |
| | 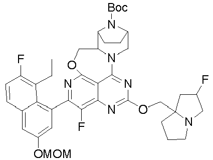 | *Tert*-butyl 2-(8-ethyl-7-fluoro-3-(methoxymethoxy)napht halen-1-yl)-1-fluoro-12-((2-fluorotetrahydro-1 *H*-pyrrolizin-7*a*(5*H*)-yl)methoxy)-5a,6,7,8,9,1 0-hexahydro-5*H*-4-oxa-3,10*a*,11,13,14-pentaa za-6,9-methanonaphtho[1,8-*ab*]heptalen-14-ca rboxylate |
| | 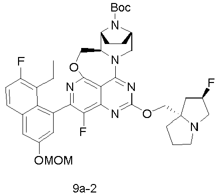 | *Tert*-butyl (5*aR*,6*R*,9*S*)-2-(8-ethyl-7-fluoro-3-(methoxym ethoxy)naphthalen-1-yl)-1-fluoro-12-(((2*R*,7*aS* )-2-fluorotetrahydro-1*H*-pyrrolizin-7*a*(5*H*)-yl) methoxy)-5*a*,6,7,8,9,10-hexahydro-5*H*-4-oxa-3,10*a,*11,13,14-pentaaza-6,9-methanonaphtho[ 1,8-*ab*]heptalen-14-carboxylate |
| | 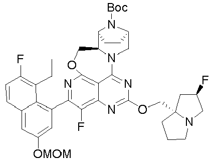 | *Tert*-butyl (5*aR*,6*S,*9*R*)-2-(8-ethyl-7-fluoro-3-(methoxym ethoxy)naphthalen-1-yl)-1-fluoro-12-(((2*R*,7*aS* )-2-fluorotetrahydro-1*H*-pyrrolizin-7*a*(5*H*)-yl) methoxy)-5*a*,6,7,8,9,10-hexahydro-5*H*-4-oxa-3,10*a,*11,13,14-pentaaza-6,9-methanonaphtho[ 1,8-*ab*]heptalen-14-carboxylate |
| | 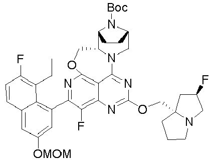 | *Tert*-butyl (5*aS*,6*R*,9*S*)-2-(8-ethyl-7-fluoro-3-(methoxym ethoxy)naphthalen-1-yl)-1-fluoro-12-(((2*R*,7*aS* )-2-fluorotetrahydro-1*H*-pyrrolizin-7*a*(5*H*)-yl) methoxy)-5*a*,6,7,8,9,10-hexahydro-5*H*-4-oxa-3,10*a,*11,13,14-pentaaza-6,9-methanonaphtho[ 1,8-*ab*]heptalen-14-carboxylate |
| | 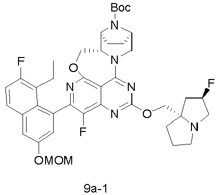 | *Tert*-butyl (5*aS*,6*S*,9*R*)-2-(8-ethyl-7-fluoro-3-(methoxym ethoxy)naphthalen-1-yl)-1-fluoro-12-(((2*R*,7*aS* )-2-fluorotetrahydro-1*H*-pyrrolizin-7*a*(5*H*)-yl) methoxy)-5*a*,6,7,8,9,10-hexahydro-5*H*-4-oxa-3,10*a,*11,13,14-pentaaza-6,9-methanonaphtho[ 1,8-*ab*]heptalen-14-carboxylate |
| | 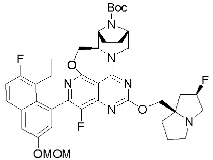 | *Tert*-butyl (5*aR*,6*R*,9*S*)-2-(8-ethyl -7-fluoro-3-(methoxym ethoxy)naphthalen-1-yl)-1-fluoro-12-(((2*R*,7*a R*)-2-fluorotetrahydro-1*H*-pyrrolizin-7*a*(5*H*)-yl )methoxy)-5*a*,6,7,8,9,10-hexahydro-5*H*-4-oxa-3,10*a,*11,13,14-pentaaza-6,9-methanonaphtho[ 1,8-*ab*]heptalen-14-carboxylate |
| | 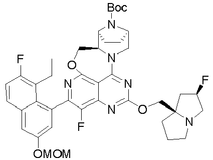 | *Tert*-butyl (5*aR*,6*S*,9*R*)-2-(8-ethyl-7-fluoro-3-(methoxym ethoxy)naphthalen-1-yl)-1-fluoro-12-(((2*R*,7*a R*)-2-fluorotetrahydro-1*H*-pyrrolizin-7*a*(5*H*)-yl)methoxy)-5*a*,6,7,8,9,10-hexahydro-5*H*-4-oxa-3,10*a,*11,13,14-pentaaza-6,9-methanonaphtho[ 1,8-*ab*]heptalen-14-carboxylate |
| | 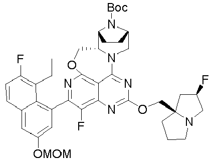 | *Tert*-butyl (5*aS*,6*R*,9*S*)-2-(8-ethyl-7-fluoro-3-(methoxym ethoxy)naphthalen-1-yl)-1-fluoro-12-(((2*R*,7*a R*)-2-fluorotetrahydro-1*H*-pyrrolizin-7*a*(5*H*)-yl )methoxy)-5*a*,6,7,8,9,10-hexahydro-5*H*-4-oxa-3,10*a,*11,13,14-pentaaza-6,9-methanonaphtho[ 1,8-*ab*]heptalen-14-carboxylate |
| | 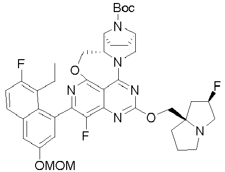 | *Tert*-butyl (5*aS*,6*S*,9*R*)-2-(8-ethyl-7-fluoro-3-(methoxym ethoxy)naphthalen-1-yl)-1-fluoro-12-(((2*R*,7*a R*)-2-fluorotetrahydro-1*H*-pyrrolizin-7*a*(5*H*)-yl )methoxy)-5*a*,6,7,8,9,10-hexahydro-5*H*-4-oxa-3,10*a,*11,13,14-pentaaza-6,9-methanonaphtho[ 1,8-*ab*]heptalen-14-carboxylate |
| | 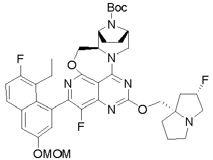 | *Tert*-butyl (5*aR*,6*R*,9*S*)-2-(8-ethyl-7-fluoro-3-(methoxym ethoxy)naphthalen-1-yl)-1-fluoro-12-(((2*S*,7*aS* )-2-fluorotetrahydro-1*H*-pyrrolizin-7*a*(5*H*)-yl) methoxy)-5*a*,6,7,8,9,10-hexahydro-5*H*-4-oxa-3,10*a,*11,13,14-pentaaza-6,9-methanonaphtho[ 1,8-*ab*]heptalen-14-carboxylate |
| | 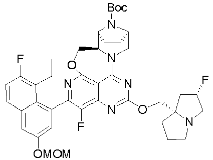 | *Tert*-butyl (5*aR*,6*S*,9*R*)-2-(8-ethyl-7-fluoro-3-(methoxym ethoxy)naphthalen-1-yl)-1-fluoro-12-(((2*S*,7*aS* )-2-fluorotetrahydro-1*H*-pyrrolizin-7*a*(5*H*)-yl) methoxy)-5*a*,6,7,8,9,10-hexahydro-5*H*-4-oxa-3,10*a,*11,13,14-pentaaza-6,9-methanonaphtho[ 1,8-*ab*]heptalen-14-carboxylate |
| | 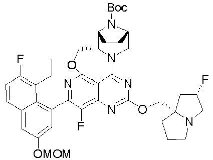 | *Tert*-butyl (5*aS*,6*R*,9*S*)-2-(8-ethyl-7-fluoro-3-(methoxym ethoxy)naphthalen-1-yl)-1-fluoro-12-(((2*S*,7*aS* )-2-fluorotetrahydro-1*H*-pyrrolizin-7*a*(5*H*)-yl) methoxy)-5*a*,6,7,8,9,10-hexahydro-5*H*-4-oxa-3,10*a,*11,13,14-pentaaza-6,9-methanonaphtho[ 1,8-*ab*]heptalen-14-carboxylate |
| | 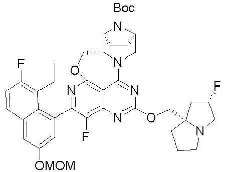 | *Tert*-butyl (5*aS*,6*S*,9*R*)-2-(8-ethyl-7-fluoro-3-(methoxym ethoxy)naphthalen-1-yl)-1-fluoro-12-(((2*S*,7*aS* )-2-fluorotetrahydro-1*H*-pyrrolizin-7*a*(5*H*)-yl) methoxy)-5*a*,6,7,8,9,10-hexahydro-5*H*-4-oxa-3,10*a*,11,13,14-pentaaza-6,9-methanonaphtho[ 1,8-*ab*]heptalen-14-carboxylate |
| | 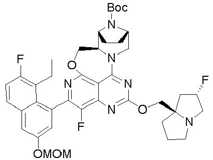 | *Tert*-butyl (5*aR*,6*R*,9*S*)-2-(8-ethyl-7-fluoro-3-(methoxym ethoxy)naphthalen-1-yl)-1-fluoro-12-(((2*S*,7*aR* )-2-fluorotetrahydro-1*H*-pyrrolizin-7*a*(5*H*)-yl) methoxy)-5*a*,6,7,8,9,10-hexahydro-5*H*-4-oxa-3,10*a*,11,13,14-pentaaza-6,9-methanonaphtho[ 1,8-*ab*]heptalen-14-carboxylate |
| | 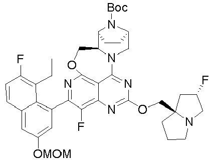 | *Tert*-butyl (5*aR*,6*S*, 9*R*)-2-(8-ethyl-7-fluoro-3-(methoxym ethoxy)naphthalen-1-yl)-1-fluoro-12-(((2*S*,7*aR* )-2-fluorotetrahydro-1*H*-pyrrolizin-7*a*(5*H*)-yl) methoxy)-5*a*,6,7,8,9,10-hexahydro-5*H*-4-oxa-3,10*a*,11,13,14-pentaaza-6,9-methanonaphtho[ 1,8-*ab*]heptalen-14-carboxylate |
| | 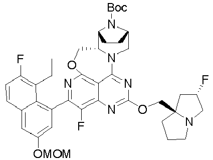 | *Tert*-butyl (5*aS*,6*R*,9*S*)-2-(8-ethyl-7-fluoro-3-(methoxym ethoxy)naphthalen-1-yl)-1-fluoro-12-(((2*S*,7*aR* )-2-fluorotetrahydro-1*H*-pyrrolizin-7*a*(5*H*)-yl) methoxy)-5*a*,6,7,8,9,10-hexahydro-5*H*-4-oxa-3,10*a*,11,13,14-pentaaza-6,9-methanonaphtho[ 1,8-*ab*]heptalen-14-carboxylate |
| | 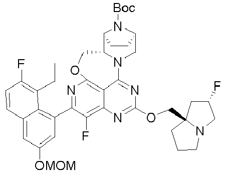 | *Tert*-butyl (5*aS*,6*S*,9*R*)-2-(8-ethyl-7-fluoro-3-(methoxym ethoxy)naphthalen-1-yl)-1-fluoro-12-(((2*S*,7*aR* )-2-fluorotetrahydro-1*H*-pyrrolizin-7*a*(5*H*)-yl) methoxy)-5*a*,6,7,8,9,10-hexahydro-5*H*-4-oxa-3,10*a*,11,13,14-pentaaza-6,9-methanonaphtho[ 1,8-*ab*]heptalen-14-carboxylate |
| | 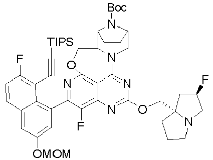 | *Tert*-butyl 1-fluoro-2-(7-fluoro-3-(methoxymethoxy)-8-(( triisopropylsilyl)ethynyl)-naphthalen-1-yl)-12-(((2*R*,7*aS*)-2-fluorotetrahydro-1*H*-pyrrolizin-7a(5H)-yl)methoxy)-5a,6,7,8,9,10-hexahydro-5 *H*-4-oxa-3,10*a*,11,13,14-pentaaza-6,9-methano naphtho[1,8-*ab*]heptalen-14-carboxylate |
| | 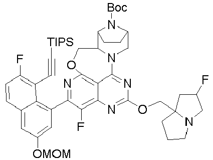 | *Tert*-butyl 1-fluoro-2-(7-fluoro-3-(methoxymethoxy)-8-(( triisopropylsilyl)ethynyl)-naphthalen-1-yl)-12-((2-fluorotetrahydro-1*H*-pyrrolizin-7a(5H)-yl) methoxy)-5a,6,7,8,9,10-hexahydro-5*H*-4-oxa-3,10a,11,13,14-pentaaza-6,9-methanonaphtho[ 1,8-*ab*]heptalen-14-carboxylate |
| | 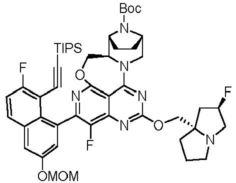 | *Tert*-butyl (5*aR*,6*R*,9*S*)-1-fluoro-2-(7-fluoro-3-(methoxy methoxy)-8-((triisopropylsilyl)ethynyl)-naphth alen-1-yl)-12-(((2*R*,7*aS*)-2-fluorotetrahydro-1 H-pyrrolizin-7a(5H)-yl)methoxy)-5a,6,7,8,9,1 0-hexahydro-5*H*-4-oxa-3,10*a*,11,13,14-pentaa za-6,9-methanonaphtho[1,8-*ab*]heptalen-14-ca rboxylate |
| | 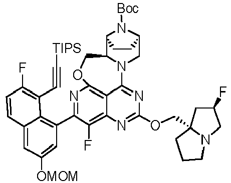 | *Tert*-butyl (5*aR*,6*S*,9*R*)-1-fluoro-2-(7-fluoro-3-(methoxy methoxy)-8-((triisopropylsilyl)ethynyl)-naphth alen-1-yl)-12-(((2*R*,7*aS*)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-*ab*]heptalen-14-ca rboxylate |
| | 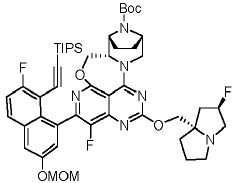 | *Tert*-butyl (5*aS*,6*R*,9*S*)-1-fluoro-2-(7-fluoro-3-(methoxy methoxy)-8-((triisopropylsilyl)ethynyl)-naphth alen-1-yl)-12-(((2R,7aS)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-*ab*]heptalen-14-ca rboxylate |
| | 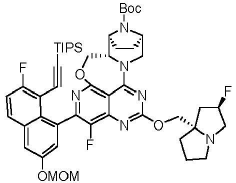 | *Tert*-butyl (5*aS*,6*S*,9R)-1-fluoro-2-(7-fluoro-3-(methoxy methoxy)-8-((triisopropylsilyl)ethynyl)-naphth alen-1-yl)-12-(((2*R*,7*aS*)-2-fluorotetrahydro-1 *H*-pyrrolizin-7*a*(5*H*)-yl)methoxy)-5*a*,6,7,8,9,10-hexahydro-5*H*-4-oxa-3,10*a,*11,13,14-pentaa za-6,9-methanonaphtho[1,8-*ab*]heptalen-14-ca rboxylate |
| | 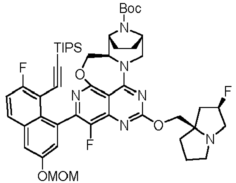 | *Tert*-butyl (5*aR*,6*R*,9*S*)-1-fluoro-2-(7-fluoro-3-(methoxy methoxy)-8-((triisopropylsilyl)ethynyl)-naphth alen-1-yl)-12-(((2*R*,7*aR*)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-ab]heptalen-14-ca rboxylate |
| | 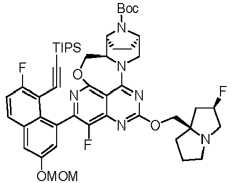 | Tert-butyl (5*aR*,6*S*,9*R*)-1-fluoro-2-(7-fluoro-3-(methoxy methoxy)-8-((triisopropylsilyl)ethynyl)-naphth alen-1-yl)-12-(((2*R*,7*aR*)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-ab]heptalen-14-ca rboxylate |
| | 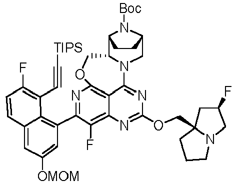 | Tert-butyl (5*aS*,6*R*,9*S*)-1-fluoro-2-(7-fluoro-3-(methoxy methoxy)-8-((triisopropylsilyl)ethynyl)-naphth alen-1-yl)-12-(((2*R*,7*aR*)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-ab]heptalen-14-ca rboxylate |
| | 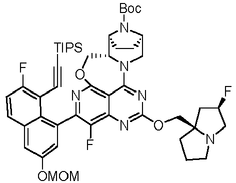 | *Tert*-butyl (5*aS*,6*S*,9*R*)-1-fluoro-2-(7-fluoro-3-(methoxy methoxy)-8-((triisopropylsilyl)ethynyl)-naphth alen-1-yl)-12-(((2*R*,7*aR*)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-ab]heptalen-14-ca rboxylate |
| | 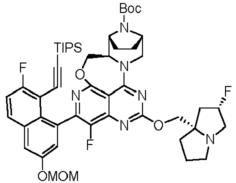 | *Tert*-butyl (5*aR*,6*R*,9*S*)-1-fluoro-2-(7-fluoro-3-(methoxy methoxy)-8-((triisopropylsilyl)ethynyl)-naphth alen-1-yl)-12-(((2*S*,7*aS*)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-*ab*]heptalen-14-ca rboxylate |
| | 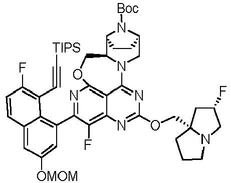 | *Tert*-butyl (5*aR*,6*S*,9*R*)-1-fluoro-2-(7-fluoro-3-(methoxy methoxy)-8-((triisopropylsilyl)ethynyl)-naphth alen-1-yl)-12-(((2*S*,7*aS*)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-*ab*]heptalen-14-ca rboxylate |
| | 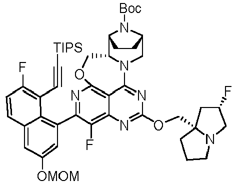 | *Tert*-butyl (5*aS,*6*R*,9*S*)-1-fluoro-2-(7-fluoro-3-(methoxy methoxy)-8-((triisopropylsilyl)ethynyl)-naphth alen-1-yl)-12-(((2*S*,7*aS*)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-*ab*]heptalen-14-ca rboxylate |
| | 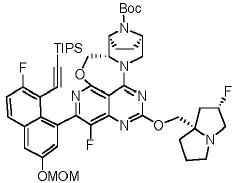 | *Tert*-butyl (5*aS*,6*S*,9*R*)-1-fluoro-2-(7-fluoro-3-(methoxy methoxy)-8-((triisopropylsilyl)ethynyl)-naphth alen-1-yl)-12-(((2*S*,7*aS*)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-*ab*]heptalen-14-ca rboxylate |
| | 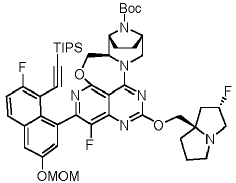 | *Tert*-butyl (5*aR*,6*R*,9*S*)-1-fluoro-2-(7-fluoro-3-(methoxy methoxy)-8-((triisopropylsilyl)ethynyl)-naphth alen-1-yl)-12-(((2*S*,7*aR*)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-*ab*]heptalen-14-ca rboxylate |
| | 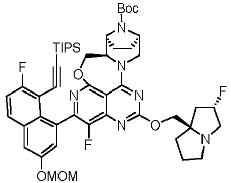 | *Tert*-butyl (5*aR*,6*S*,9*R*)-1-fluoro-2-(7-fluoro-3-(methoxy methoxy)-8-((triisopropylsilyl)ethynyl)-naphth alen-1-yl)-12-(((2*S*,7*aR*)-2-fluorotetrahydro-1 *H*-pyrrolizin-7a(5*H*)-yl)methoxy)-5*a*,6,7,8,9,1 0-hexahydro-5*H*-4-oxa-3,10a,11,13,14-pentaa za-6,9-methanonaphtho[1,8-*ab*]heptalen-14-ca rboxylate |
| | 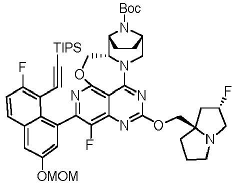 | *Tert*-butyl (5*aS*,6*R*,9*S*)-1-fluoro-2-(7-fluoro-3-(methoxy methoxy)-8-((triisopropylsilyl)ethynyl)-naphth alen-1-yl)-12-(((2*S*,7*aR*)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-*ab*]heptalen-14-ca rboxylate |
| | 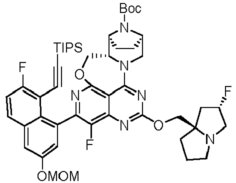 | *Tert*-butyl (5*aS*,6*S*,9*R*)-1-fluoro-2-(7-fluoro-3-(methoxy methoxy)-8-((triisopropylsilyl)ethynyl)-naphth alen-1-yl)-12-(((2*S*,7*aR*)-2-fluorotetrahydro-1 *H*-pyrrolizin-7*a*(5*H*)-yl)methoxy)-5*a*,6,7,8,9,1 0-hexahydro-5*H*-4-oxa-3,10*a*,11,13,14-pentaa za-6,9-methanonaphtho[1,8-*ab*]heptalen-14-ca rboxylate |
| | 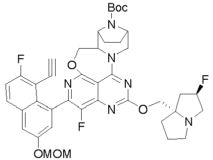 | *Tert*-butyl 2-(8-ethynyl-7-fluoro-3-(methoxymethoxy)na phthalen-1-yl)-1-fluoro-12-(((2*R*,7*aS*)-2-fluoro tetrahydro-1*H*-pyrrolizin-7*a*(5*H*)-yl)methoxy)-5*a*,6,7,8,9,10-hexahydro-5*H*-4-oxa-3,10*a*,11,1 3,14-pentaaza-6,9-methanonaphtho[1,8-*ab*]hep talen-14-carboxylate |
| | 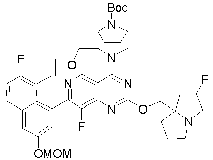 | *Tert*-butyl 2-(8-ethynyl-7-fluoro-3-(methoxymethoxy)na phthalen-1-yl)-1-fluoro-12-((2-fluorotetrahy dr o-1*H*-pyrrolizin-7*a*(5*H*)-yl)methoxy)-5*a*,6,7,8, 9,10-hexahydro-5*H*-4-oxa-3,10*a,*11,13,14-pent aaza-6,9-methanonaphtho[1,8-*ab*]heptalen- 14-carboxylate |
| | 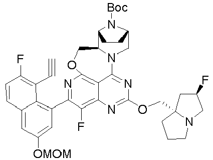 | *Tert*-butyl (5*aR*,6*R*,9*S*)-2-(8-ethynyl-7-fluoro-3-(methoxy methoxy)naphthalen-1-yl)-1-fluoro-12-(((2*R*,7 *aS*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 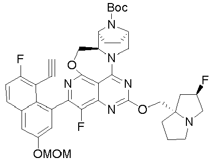 | *Tert*-butyl (5*aR*,6*S*,9*R*)-2-(8-ethynyl-7-fluoro-3-(methoxy methoxy)naphthalen-1-yl)-1-fluoro-12-(((2*R*,7 *aS*)-2-fluorotetrahydro-1*H*-pyrrolizin-7*a*(5*H*)-yl)methoxy)-*5*a,6,7,8,9,10-hexahydro-5*H*-4-ox a-3,10*a,*11,13,14-pentaaza-6,9-methanonaphth o[1,8-*ab*]heptalen-14-carboxylate |
| | 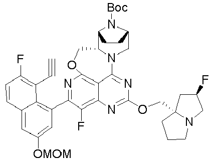 | *Tert*-butyl (5*aS*,6*R*,9*S*-2-(8-ethynyl-7-fluoro-3-(methoxy methoxy)naphthalen-1-yl)-1-fluoro-12-(((2*R*,7 *aS*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 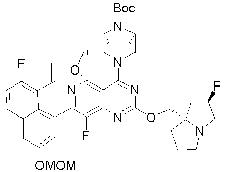 | *Ter*t-butyl (5*aS*,6*S*,9*R*)-2-(8-ethynyl-7-fluoro-3-(methoxy methoxy)naphthalen-1-yl)-1-fluoro-12-(((2*R*,7 *aS*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 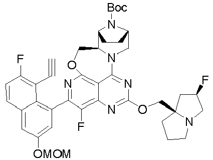 | *Tert*-butyl (5*aR*,6*R*,9*S*)-2-(8-ethynyl-7-fluoro-3-(methoxy methoxy)naphthalen-1-yl)-1-fluoro-12-(((2*R*,7 *aR*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 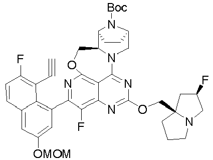 | *Tert*-butyl (5*aR*,6*S*,9*R*)-2-(8-ethynyl-7-fluoro-3-(methoxy methoxy)naphthalen-1-yl)-1-fluoro-12-(((2*R*,7 *aR*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 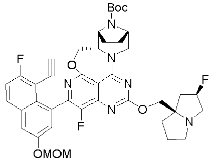 | *Tert*-butyl (*5a*S,6*R,*9*S*-2-(8-ethynyl-7-fluoro-3-(methoxy methoxy)naphthalen-1-yl)-1-fluoro-12-(((2*R*,7 *aR*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 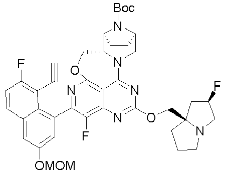 | *Tert*-butyl (5*aS*,6*S*,9*R*)-2-(8-ethynyl-7-fluoro-3-(methoxy methoxy)naphthalen-1-yl)-1-fluoro-12-(((2*R*,7 *aR*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 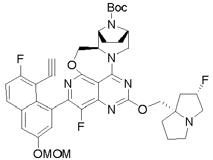 | *Tert*-butyl (5*aR*,6*R*,9*S*)-2-(8-ethynyl-7-fluoro-3-(methoxy methoxy)naphthalen-1-yl)-1-fluoro-12-(((2*S*,7 *aS*)-2-fluorotetrahydro-1*H*-pyrrolizin-7*a*(5*H*)-yl)methoxy)-Sa, 6,7,8,9,10-hexahydro-SH-4-ox a-3,10*a,*11,13,14-pentaaza-6,9-methanonaphth o[1,8-*ab*]heptalen-14-carboxylate |
| | 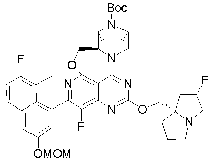 | *Tert*-butyl (5*aR*,6*S*,9*R*)-2-(8-ethynyl-7-fluoro-3-(methoxy methoxy)naphthalen-1-yl)-1-fluoro-12-(((2*S*,7 *aS*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 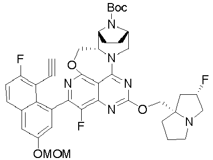 | *Tert*-butyl (5*aS*,6*R*,9*S*-2-(8-ethynyl-7-fluoro-3-(methoxy methoxy)naphthalen-1-yl)-1-fluoro-12-(((2*S*,7 *aS*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 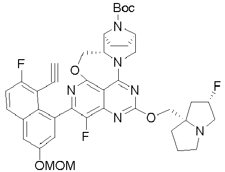 | *Tert*-butyl (5*aS*,6*S*,9*R*)-2-(8-ethynyl-7-fluoro-3-(methoxy methoxy)naphthalen-1-yl)-1-fluoro-12-(((2*S*,7 *aS*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 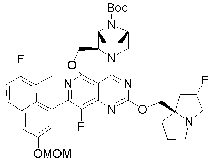 | *Tert*-butyl (5*aR*,6*R,*9*S*)-2-(8-ethynyl-7-fluoro-3-(methoxy methoxy)naphthalen-1-yl)-1-fluoro-12-(((2*S*,7 *aR*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 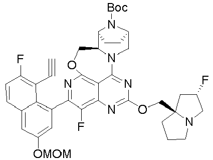 | *Tert*-butyl (5*aR*,6*S*,9*R*)-2-(8-ethynyl-7-fluoro-3-(methoxy methoxy)naphthalen-1-yl)-1-fluoro-12-(((2*S*,7 *aR*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 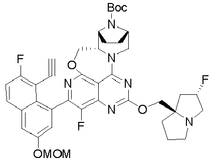 | *Tert*-butyl (5*aS* 6*R*,9*S*-2-(8-ethynyl-7-fluoro-3-(methoxy methoxy)naphthalen-1-yl)-1-fluoro-12-(((2*S*,7 *aR*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 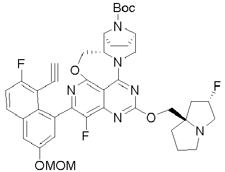 | *Tert*-butyl (5*aS*,6*S*,9*R*)-2-(8-ethynyl-7-fluoro-3-(methoxy methoxy)naphthalen-1-yl)-1-fluoro-12-(((2*S*,7 *aR*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 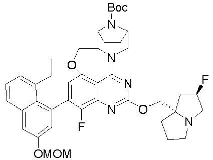 | *Tert*-butyl 2-(8-ethyl-3-(methoxymethoxy)naphthalen-1-yl)-1-fluoro-13-(((2*R*,7*aS*)-2-fluorotetrahydro-1*H*-pyrrolizin-7*a*(5*H*-yl)methoxy)-5*a*,6,7,8,9,10-hexahydro-5*H*-6,9-epiminoazepino[2',1':3, 4][1,4]oxaazepino[5,6,7-*de*]quinazoline-15-car boxylate |
| | 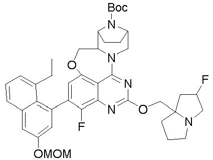 | *Tert*-butyl 2-(8-ethyl-3-(methoxymethoxy)naphthalen-1-yl)-1-fluoro-13-((2-fluorotetrahydro-dro-1H-pyrrol izin-7*a*(5*H*)-yl)methoxy)-5*a*,6,7,8,9,10-hexahy dro-5*H*-6,9-epiminoazepino[2',1':3,4][1,4] oxaa zepino[5,6,7-*de*]quinazoline-15-carboxylate |
| | 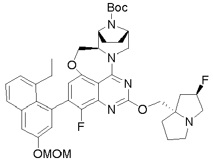 | *Tert*-butyl (5*aR*,6*R*,9*S*)-2-(8-ethyl-3-(methoxymethoxy)n aphthalen-1-yl)-1-fluoro-13-(((2*R*,7*aS*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-*de*]quinaz oline-15-carboxylate |
| | 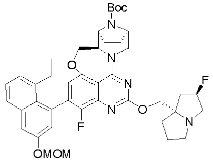 | *Tert*-butyl (5*aR,*6*S,*9*R*)-2-(8-ethyl-3-(methoxymethoxy)n aphthalen-1-yl)-1-fluoro-13-(((2*R*,7*aS*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-*de*]quinaz oline-15-carboxylate |
| | 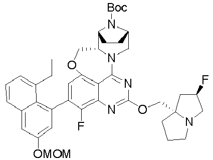 | *Tert*-butyl (5*aS,*6*R*,9S)-2-(8-ethyl-3-(methoxymethoxy)n aphthalen-1-yl)-1-fluoro-13-(((2*R*,7*aS*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-*de*]quinaz oline-15-carboxylate |
| | 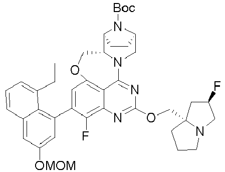 | *Tert-*butyl (5*aS,*6*S*,9*R*)-2-(8-ethyl-3-(methoxymethoxy)n aphthalen-1-yl)-1-fluoro-13-(((2*R*,7*aS*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-*de*]quinaz oline-15-carboxylate |
| | 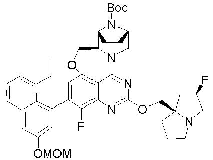 | *Tert*-butyl (5*aR*,6*R*,9*S*)-2-(8-ethyl-3-(methoxymethoxy)n aphthalen-1-yl)-1-fluoro-13-(((2*R*,7*aR*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-*de*]quinaz oline-15-carboxylate |
| | 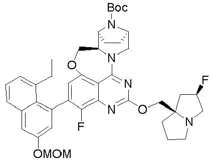 | *Tert*-butyl (*5aR,*6*S,*9*R*)-2-(8-ethyl-3-(methoxymethoxy)n aphthalen-1-yl)-1-fluoro-13-(((2*R*,7*aR*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-*de*]quinaz oline-15-carboxylate |
| | 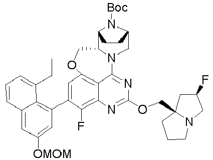 | *Tert*-butyl (5*aS,*6*R*,9*S*)-2-(8-ethyl-3-(methoxymethoxy)n aphthalen-1-yl)-1-fluoro-13-(((2*R*,7*aR*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-*de*]quinaz oline-15-carboxylate |
| | 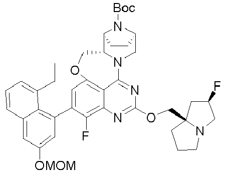 | *Tert*-butyl (*5aS,*6*S*,9*R*)-2-(8-ethyl-3-(methoxymethoxy)n aphthalen-1-yl)-1-fluoro-13-(((2*R*,7*aR*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-*de*]quinaz oline-15-carboxylate |
| | 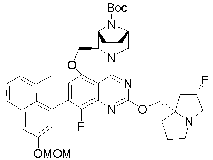 | *Tert*-butyl (5*aR*,6*R*,9*S*)-2-(8-ethyl-3-(methoxymethoxy)n aphthalen-1-yl)-1-fluoro-13-(((2*S*,7*aS*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4] [1,4]oxaazepino[5,6,7-de]quinaz oline-15-carboxylate |
| | 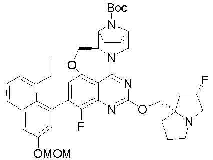 | *Tert*-butyl (5*aR,*6*S,*9*R*)-2-(8-ethyl-3-(methoxymethoxy)n aphthalen-1-yl)-1-fluoro-13-(((2*S*,7*aS*)-2-fluor otetrahydro-1*H*-pyrrolizin-7*a*(5*H*)-yl)methoxy)-5*a*,6,7,8,9,10-hexahydro-5*H*-6,9-epiminoaze pino[2',1':3,4][1,4]oxaazepino[5,6,7-*de*]quinaz oline-15-carboxylate |
| | 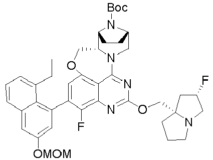 | *Tert*-butyl (5*aS,*6*R*,9*S*)-2-(8-ethyl-3-(methoxymethoxy)n aphthalen-1-yl)-1-fluoro-13-(((2*S*,7*aS*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-*de*]quinaz oline-15-carboxylate |
| | 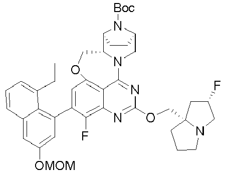 | *Tert*-butyl (5*aS,*6*S*,9*R*)-2-(8-ethyl-3-(methoxymethoxy)n aphthalen-1-yl)-1-fluoro-13-(((2*S*,7*aS*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-de]quinaz oline-15-carboxylate |
| | 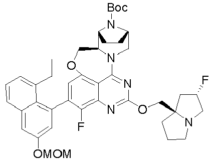 | *Tert*-butyl (5*aR*,6*R*,9*S*)-2-(8-ethyl-3-(methoxymethoxy)n aphthalen-1-yl)-1-fluoro-13-(((2*S*,7*aR*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-de]quinaz oline-15-carboxylate |
| | 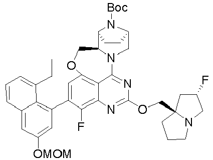 | *Tert*-butyl (5*aR,*6*S*,9*R*)-2-(8-ethyl-3-(methoxymethoxy)n aphthalen-1-yl)-1-fluoro-13-(((2*S*,7*aR*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-*de*]quinaz oline-15-carboxylate |
| | 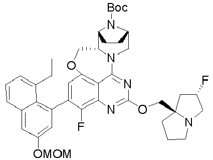 | *Tert*-butyl (5*aS,*6*R*,9*S*)-2-(8-ethyl-3-(methoxymethoxy)n aphthalen-1-yl)-1-fluoro-13-(((2*S*,7*aR*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-de]quinaz oline-15-carboxylate |
| | 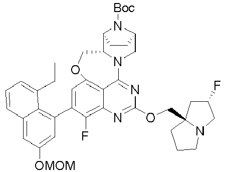 | *Tert*-butyl (5*aS,*6*S*,9*R*)-2-(8-ethyl-3-(methoxymethoxy)n aphthalen-1-yl)-1-fluoro-13-(((2*S*,7*aR*)-2-fluor otetrahydro-1*H*-pyrrolizin-7*a*(5*H*)-yl)methoxy )-5*a*,6,7,8,9,10-hexahydro-5*H*-6,9-epiminoaze pino[2',1':3,4][1,4]oxaazepino[5,6,7-*de*]quinaz oline-15-carboxylate |
| | 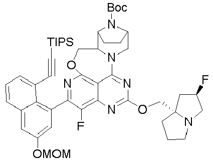 | *Tert*-butyl 1-fluoro-12-(((2*R*,7*aS*)-2-fluorotetrahydro-1H-pyrrolizin-7*a*(5*H*)-yl)methoxy)-2-(3-(methoxy methoxy)-8-((triisopropylsilyl)ethynyl)-naphth alen-1-yl)-5*a*,6,7,8,9,10-hexahydro-5*H*-4-oxa-3,10*a*,11,13,14-pentaaza-6,9-methanonaphtho[ 1,8-*ab*]heptalen-14-carboxylate |
| | 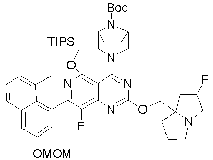 | *Tert*-butyl 1-fluoro-12-((2-fluorotetrahydro-1*H*-pyrrolizin -7*a*(5*H*)-yl)methoxy)-2-(3-(methoxymethoxy)-8-((triisopropylsilyl)ethynyl)-naphthalen-1-yl) -5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 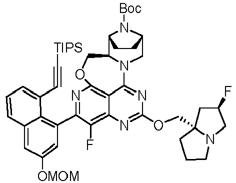 | *Tert*-butyl (5*aR*,6*R*,9*S*)-1-fluoro-12-(((2*R*,7*aS*(-2-fluorote trahydro-1*H*-pyrrolizin-7*a*(5*H*)-yl)methoxy)-2 -(3-(methoxymethoxy)-8-((triisopropylsilyl)et hynyl)-naphthalen-1-yl)-5*a*,6,7,8,9,10-hexahy dro-5*H*-4-oxa-3,10*a*,11,13,14-pentaaza-6,9-me thanonaphtho[1,8-*ab*]heptalen-14-carboxylate |
| | 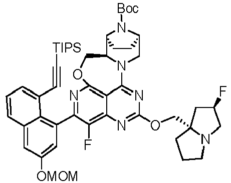 | *Tert*-butyl (5*aR*,6*S*,9*R*)-1-fluoro-12-(((2*R*,7*aS*)-2-fluorote trahydro-1*H*-pyrrolizin-7*a*(5*H*)-yl)methoxy)-2 -(3-(methoxymethoxy)-8-((triisopropylsilyl)et hynyl)-naphthalen-1-yl)-5*a*,6,7,8,9,10-hexahy dro-5*H*-4-oxa-3,10*a*,11,13,14-pentaaza-6,9-me thanonaphtho[1,8-*ab*]heptalen-14-carboxylate |
| | 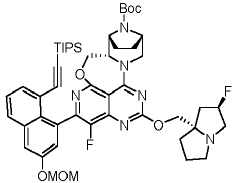 | *Tert*-butyl (5*aS*,6*R*,9*S*)-1-fluoro-12-(((2*R*,7*aS*)-2-fluorotet rahydro-1*H*-pyrrolizin-7*a*(5*H*)-yl)methoxy)-2-(3-(methoxymethoxy)-8-((triisopropylsilyl)eth ynyl)-naphthalen-1-yl)-5*a*,6,7,8,9,10-hexahydr o-5*H*-4-oxa-3,10*a*,11,13,14-pentaaza-6,9-meth anonaphtho[1,8-*ab*]heptalen-14-carboxylate |
| | 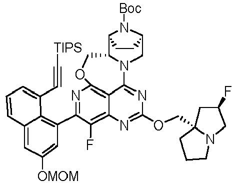 | *Tert*-butyl (5*aS*,6*S*,9*R*)-1-fluoro-12-(((2*R*,7*aS*)-2-fluorotet rahydro-1*H*-pyrrolizin-7*a*(5*H*)-yl)methoxy)-2-(3-(methoxymethoxy)-8-((triisopropylsilyl)eth ynyl)-naphthalen-1-yl)-5*a*,6,7,8,9,10-hexahydr o-5*H*-4-oxa-3,10*a*,11,13,14-pentaaza-6,9-meth anonaphtho[1,8-*ab*]heptalen-14-carboxylate |
| | 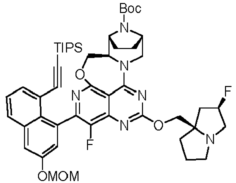 | *Tert*-butyl (5*aR*,6*R*,9*S*)-1-fluoro-12-(((2*R*,7*aR*)-2-fluorote trahydro-1*H*-pyrrolizin-7*a*(5*H*)-yl)methoxy)-2 -(3-(methoxymethoxy)-8-((triisopropylsilyl)et hynyl)-naphthalen-1-yl)-5*a*,6,7,8,9,10-hexahy dro-5*H*-4-oxa-3,10*a*,11,13,14-pentaaza-6,9-me thanonaphtho[1,8-*ab*]heptalen-14-carboxylate |
| | 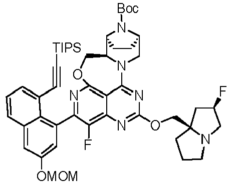 | *Tert*-butyl (5*aR*,6*S*,9*R*)-1-fluoro-12-(((2*R*,7*aR*)-2-fluorote trahydro-1*H*-pyrrolizin-7*a*(5*H*)-yl)methoxy)-2 -(3-(methoxymethoxy)-8-((triisopropylsilyl)et hynyl)-naphthalen-1-yl)-5*a*,6,7,8,9,10-hexahy dro-5*H*-4-oxa-3,10*a*,11,13,14-pentaaza-6,9-me thanonaphtho[1,8-*ab*]heptalen-14-carboxylate |
| | 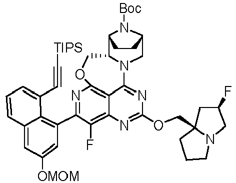 | *Tert*-butyl (5*aS*,6*R*,9*S*)-1-fluoro-12-(((2*R*,7*aR*)-2-fluorote trahydro-1*H*-pyrrolizin-7*a*(5*H*)-yl)methoxy)-2 -(3-(methoxymethoxy)-8-((triisopropylsilyl)et hynyl)-naphthalen-1-yl)-5*a*,6,7,8,9,10-hexahy dro-5*H*-4-oxa-3,10*a*,11,13,14-pentaaza-6,9-me thanonaphtho[1,8-*ab*]heptalen-14-carboxylate |
| | 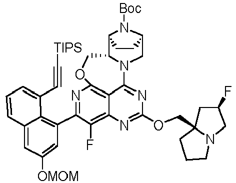 | *Tert*-butyl (5*aS*,6*S*,9*R*)-1-fluoro-12-(((2*R*,7*aR*)-2-fluorote trahydro-1*H*-pyrrolizin-7*a*(5*H*)-yl)methoxy)-2 -(3-(methoxymethoxy)-8-((triisopropylsilyl)et hynyl)-naphthalen-1-yl)-5*a*,6,7,8,9,10-hexahy dro-5*H*-4-oxa-3,10*a*,11,13,14-pentaaza-6,9-me thanonaphtho[1,8-*ab*]heptalen-14-carboxylate |
| | 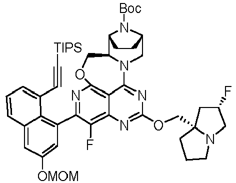 | *Tert*-butyl (5*aR*,6*R*,9*S*)-1-fluoro-12-(((2*S*,7*aS*)-2-fluorotet rahydro-1*H*-pyrrolizin-7*a*(5*H*)-yl)methoxy)-2-(3-(methoxymethoxy)-8-((triisopropylsilyl)eth ynyl)-naphthalen-1-yl)-5*a*,6,7,8,9,10-hexahydr o-5*H*-4-oxa-3,10*a*,11,13,14-pentaaza-6,9-meth anonaphtho[1,8-ab]heptalen-14-carboxylate |
| | 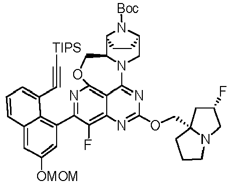 | *Tert*-butyl (5*aR*,6*S*,9*R*)-1-fluoro-12-(((2*S*,7*aS*)-2-fluorotet rahydro-1*H*-pyrrolizin-7*a*(5*H*)-yl)methoxy)-2-(3-(methoxymethoxy)-8-((triisopropylsilyl)eth ynyl)-naphthalen-1-yl)-5*a*,6,7,8,9,10-hexahydr o-5*H*-4-oxa-3,10*a*,11,13,14-pentaaza-6,9-meth anonaphtho[1,8-ab]heptalen-14-carboxylate |
| | 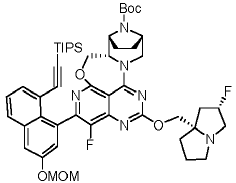 | *Tert*-butyl (5*aS*,6*R*,9*S*)-1-fluoro-12-(((2*S*,7*aS*)-2-fluorotet rahydro-1*H*-pyrrolizin-7*a*(5*H*)-yl)methoxy)-2-(3-(methoxymethoxy)-8-((triisopropylsilyl)eth ynyl)-naphthalen-1-yl)-5*a*,6,7,8,9,10-hexahydr o-5*H*-4-oxa-3,10*a*,11,13,14-pentaaza-6,9-meth anonaphtho[1,8-*ab*]heptalen-14-carboxylate |
| | 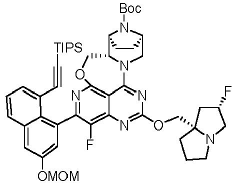 | *Tert*-butyl (5*aS*,6*S*,9*R*)-1-fluoro-12-(((2*S*,7*aS*)-2-fluorotet rahydro-1*H*-pyrrolizin-7*a*(5*H*)-yl)methoxy)-2-(3-(methoxymethoxy)-8-((triisopropylsilyl)eth ynyl)-naphthalen-1-yl)-5*a*,6,7,8,9,10-hexahydr o-5*H*-4-oxa-3,10*a*,11,13,14-pentaaza-6,9-meth anonaphtho[1,8-*ab*]heptalen-14-carboxylate |
| | 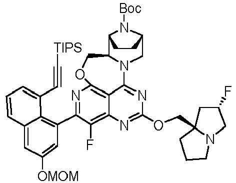 | *Tert*-butyl (5*aR*,6*R*,9*S*)-1-fluoro-12-(((2*S*,7*aR*)-2-fluorote trahydro-1*H*-pyrrolizin-7*a*(5*H*)-yl)methoxy)-2 -(3-(methoxymethoxy)-8-((triisopropylsilyl)et hynyl)-naphthalen-1-yl)-5*a*,6,7,8,9,10-hexahy dro-5*H*-4-oxa-3,10*a*,11,13,14-pentaaza-6,9-me thanonaphtho[1,8-*ab*]heptalen-14-carboxylate |
| | 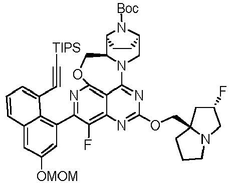 | *Tert*-butyl (5*aR*,6*S*,9*R*)-1-fluoro-12-(((2*S*,7*aR*)-2-fluorote trahydro-1*H*-pyrrolizin-7*a*(5*H*)-yl)methoxy)-2 -(3-(methoxymethoxy)-8-((triisopropylsilyl)et hynyl)-naphthalen-1-yl)-5*a*,6,7,8,9,10-hexahy dro-5*H*-4-oxa-3,10*a*,11,13,14-pentaaza-6,9-me thanonaphtho[1,8-*ab*]heptalen-14-carboxylate |
| | 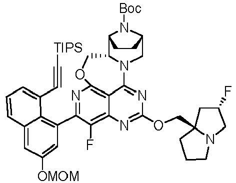 | *Tert*-butyl (5*aS*,6*R*,9*S*)-1-fluoro-12-(((2*S*,7*aR*)-2-fluorotet rahydro-1*H*-pyrrolizin-7*a*(5*H*)-yl)methoxy)-2-(3-(methoxymethoxy)-8-((triisopropylsilyl)eth ynyl)-naphthalen-1-yl)-5*a*,6,7,8,9,10-hexahydr o-5*H*-4-oxa-3,10*a*,11,13,14-pentaaza-6,9-meth anonaphtho[1,8-*ab*]heptalen-14-carboxylate |
| | 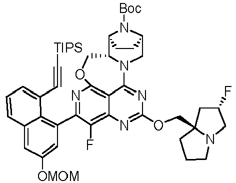 | *Tert*-butyl (5*aS*,6*S*,9*R*)-1-fluoro-12-(((2*S*,7*aR*)-2-fluorotet rahydro-1*H*-pyrrolizin-7*a*(5*H*-yl)methoxy)-2-(3-(methoxymethoxy)-8-((triisopropylsilyl)eth ynyl)-naphthalen-1-yl)-5*a*,6,7,8,9,10-hexahydr o-5*H*-4-oxa-3,10*a*,11,13,14-pentaaza-6,9-meth anonaphtho[1,8-*ab*]heptalen-14-carboxylate |
| | 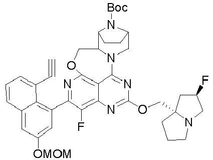 | *Tert*-butyl 2-(8-ethynyl-3-(methoxymethoxy)naphthalen-1-yl)-1-fluoro-12-(((2*R*,7*aS*)-2-fluorotetrahydr o-1*H*-pyrrolizin-7*a*(5*H*)-yl)methoxy)-5*a*,6,7,8, 9,10-hexahydro-5*H*-4-oxa-3,10*a,*11,13,14-pent aaza-6,9-methanonaphtho[1,8-*ab*]heptalen-14-carboxylate |
| | 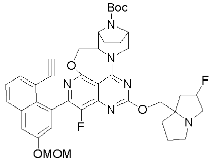 | *Tert*-butyl 2-(8-ethynyl-3-(methoxymethoxy)naphthalen-1-yl)-1-fluoro-12-((2-fluorotetrahydro-1*H*-pyrr olizin-7*a*(5*H*)-yl)methoxy)-5*a*,6,7,8,9,10-hexahydro-5*H*-4-oxa-3,10*a,*11,13,14-pentaaza-6,9-methanonaphtho[1,8-*ab*]heptalen-14-carboxyl ate |
| | 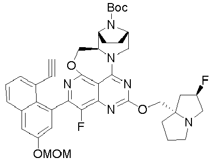 | *Tert*-butyl (5*aR*,6*R*,9*S*)-2-(8-ethynyl-3-(methoxymethoxy )naphthalen-1-yl)-1-fluoro-12-(((2*R*,7*aS*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 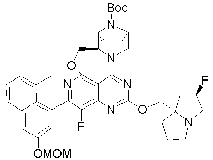 | *Tert*-butyl (5*aR,*6*S,*9*R*)-2-(8-ethynyl-3-(methoxymethoxy )naphthalen-1-yl)-1-fluoro-12-(((2*R*,7*aS*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 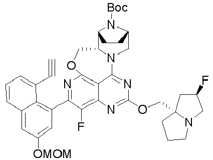 | *Tert*-butyl (5*aS,*6*R*,9*S*)-2-(8-ethynyl-3-(methoxymethoxy )naphthalen-1-yl)-1-fluoro-12-(((2*R*,7*aS*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 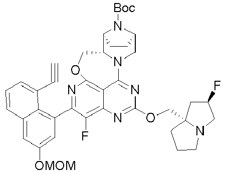 | *Tert*-butyl (5*aS,*6*S*,9*R*)-2-(8-ethynyl-3-(methoxymethoxy )naphthalen-1-yl)-1-fluoro-12-(((2*R*,7*aS*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 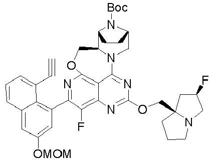 | *Tert*-butyl (5*aR*,6*R*,9*S*)-2-(8-ethynyl-3-(methoxymethoxy )naphthalen-1-yl)-1-fluoro-12-(((2*R*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 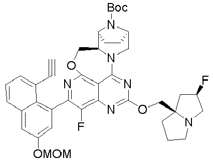 | *Tert*-butyl (5*aR,*6*S,*9*R*)-2-(8-ethynyl-3-(methoxymethoxy )naphthalen-1-yl)-1-fluoro-12-(((2*R*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 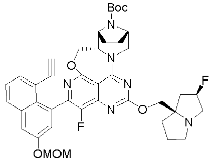 | *Tert*-butyl (*5*aS*,*6*R,*9*S*)-2-(8-ethynyl-3-(methoxymethoxy )naphthalen-1-yl)-1-fluoro-12-(((2*R*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 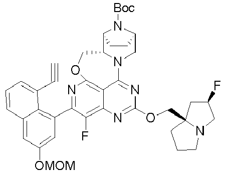 | *Tert*-butyl (*5*aS*,*6*S,*9*R*)-2-(8-ethynyl-3-(methoxymethoxy )naphthalen-1-yl)-1-fluoro-12-(((2*R*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 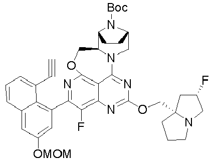 | *Tert*-butyl (5*aR*,6*R*,9*S*)-2-(8-ethynyl-3-(methoxymethoxy )naphthalen-1-yl)-1-fluoro-12-(((2*S*,7*aS*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 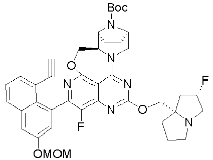 | *Tert*-butyl (5*aR*,6*S*,9*R*)-2-(8-ethynyl-3-(methoxymethoxy )naphthalen-1-yl)-1-fluoro-12-(((2*S*,7*aS*)-2-flu orotetrahydro-1*H*-pyrrolizin-7a(5*H*)-yl)metho xy)-5a,6,7,8,9,10-hexahydro-5H-4-oxa-3,10a, 11,13,14-pentaaza-6,9-methanonaphtho[1,8-*ab* ]heptalen-14-carboxylate |
| | 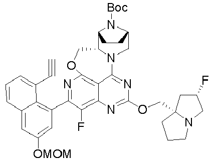 | *Tert*-butyl (5*aS,*6*R*,9*S*)-2-(8-ethynyl-3-(methoxymethoxy )naphthalen-1-yl)-1-fluoro-12-(((2*S*,7*aS*)-2-flu orotetrahydro-1*H*-pyrrolizin-7*a*(5*H*)-yl)methoxy)-5*a*,6,7,8,9,10-hexahydro-5*H*-4-oxa-3,10a, 11,13,14-pentaaza-6,9-methanonaphtho[1,8-*ab* ]heptalen-14-carboxylate |
| | 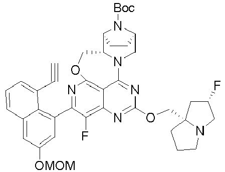 | *Tert*-butyl (5*aS,*6*S*,9*R*)-2-(8-ethynyl-3-(methoxymethoxy )naphthalen-1-yl)-1-fluoro-12-(((2*S*,7*aS*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 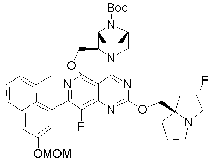 | *Tert*-butyl (5*aR*,6*R*,9*S*)-2-(8-ethynyl-3-(methoxymethoxy )naphthalen-1-yl)-1-fluoro-12-(((2*S*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 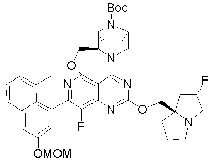 | *Tert*-butyl (5*aR,*6*S,*9*R*)-2-(8-ethynyl-3-(methoxymethoxy )naphthalen-1-yl)-1-fluoro-12-(((2*S*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 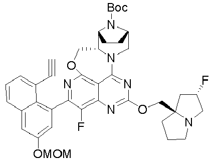 | *Tert*-butyl (5*aS,*6*R,*9*S*)-2-(8-ethynyl-3-(methoxymethoxy )naphthalen-1-yl)-1-fluoro-12-(((2*S*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 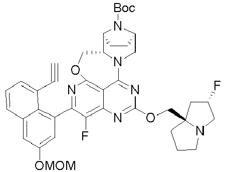 | *Tert*-butyl (5*aS*,6*S,*9*R*)-2-(8-ethynyl-3-(methoxymethoxy )naphthalen-1-yl)-1-fluoro-12-(((2*S*,7*aR*)-2-flu orotetrahydro-1*H*-pyrrolizin-7*a*(5*H*)-yl)metho xy)-5*a*,6,7,8,9,10-hexahydro-5*H*-4-oxa-3,10*a*, 11,13,14-pentaaza-6,9-methanonaphtho[1,8-*ab* ]heptalen-14-carboxylate |
| | 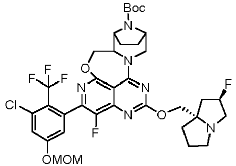 | *Tert*-butyl 2-(3-chloro-5-(methoxymethoxy)-2-(trifluoro methyl)phenyl)-1-fluoro-12-(((2*R*,7*aS*)-2-fluor otetrahydro-1*H*-pyrrolizin-7*a*(5*H*)-yl)methoxy )-5*a*,6,7,8,9,10-hexahydro-5*H*-4-oxa-3,10*a*,11, 13,14-pentaaza-6,9-methanonaphtho[1,8-*ab*]he ptalen-14-carboxylate |
| | 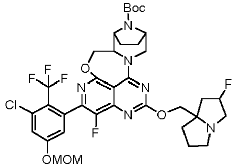 | *Tert*-butyl 2-(3-chloro-5-(methoxymethoxy)-2-(trifluoro methyl)phenyl)-1-fluoro-12-((2-fluorotetrahyd ro-1*H*-pyrrolizin-7*a*(5*H*)-yl)methoxy)-5*a*,6,7,8 *,*9,10-hexahydro-5*H*-4-oxa-3,10*a*,11,13,14-pen taaza-6,9-methanonaphtho[1,8-*ab*]heptalen-14 -carboxylate |
| 14a-2 | 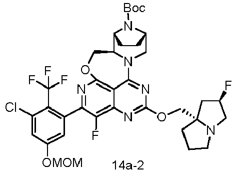 | *Tert*-butyl (5*aR*,6*R*,9*S*)-2-(3-chloro-5-(methoxymethoxy) -2-(trifluoromethyl)phenyl)-1 -fluoro-12-(((2R, 7*aS*)-2-fluorotetrahydro-1*H*-pyrrolizin-7*a*(5*H*) -yl)methoxy)-5*a*,6,7,8,9,10-hexahydro-5*H*-4-o xa-3,10*a,*11,13,14-pentaaza-6,9-methanonapht ho[1,8-*ab*]heptalen-14-carboxylate **14a-2** |
| | 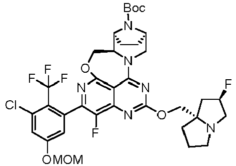 | Tert-butyl (5*aR*,6*S*,9*R*)-2-(3-chloro-5-(methoxymethoxy) -2-(trifluoromethyl)phenyl)-1-fluoro-12-(((2*R*, 7*aS*)-2-fluorotetrahydro-1*H*-pyrrolizin-7*a*(5*H*) -yl)methoxy)-5*a*,6,7,8,9,10-hexahydro-5*H*-4-o xa-3,10*a,*11,13,14-pentaaza-6,9-methanonapht ho[1,8-*ab*]heptalen-14-carboxylate |
| | 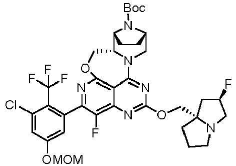 | *Tert*-butyl (5*aS*,6*R*,9*S*)-2-(3-chloro-5-(methoxymethoxy)-2-(trifluoromethyl)phenyl)-1-fluoro-12-(((2*R*,7 *aS*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| 14a-1 | 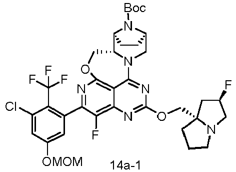 | *Tert*-butyl (5*aS*,6*S*,9*R*)-2-(3-chloro-5-(methoxymethoxy)-2-(trifluoromethyl)phenyl)-1-fluoro-12-(((2R,7 *aS*)-2-fluorotetrahydro-1*H*-pyrrolizin-7*a*(5*H*)-yl)methoxy)-5a,6,7,8,9,10-hexahydro-5*H*-4-ox a-3,10a,11,13,14-pentaaza-6,9-methanonaphth o[1,8-*ab*]heptalen-14-carboxylate **14a-1** |
| | 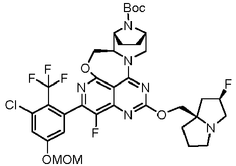 | *Tert*-butyl (5*aR*,6*R*,9*S*)-2-(3-chloro-5-(methoxymethoxy) -2-(trifluoromethyl)phenyl)-1-fluoro-12-(((2*R*, 7*aR*)-2-fluorotetrahydro-1*H*-pyrrolizin-7*a*(5*H*) -yl)methoxy)-5*a*,6,7,8,9,10-hexahydro-5*H*-4-o xa-3,10*a,*11,13,14-pentaaza-6,9-methanonapht ho[1,8-*ab*]heptalen-14-carboxylate |
| | 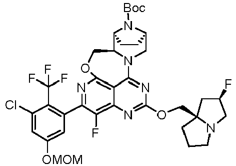 | *Tert*-butyl (5*aR*,6*S*,9*R*)-2-(3-chloro-5-(methoxymethoxy) -2-(trifluoromethyl)phenyl)-1-fluoro-12-(((2*R*, 7*aR*)-2-fluorotetrahydro-1*H*-pyrrolizin-7*a*(5*H*) -yl)methoxy)-5*a*,6,7,8,9,10-hexahydro-5*H*-4-o xa-3,10*a,*11,13,14-pentaaza-6,9-methanonapht ho[1,8-*ab*]heptalen-14-carboxylate |
| | 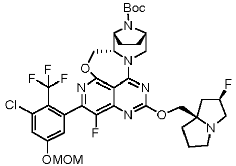 | *Tert*-butyl (5*aS*,6*R*,9*S*)-2-(3-chloro-5-(methoxymethoxy)-2-(trifluoromethyl)phenyl)-1-fluoro-12-(((2*R*,7 *aR*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 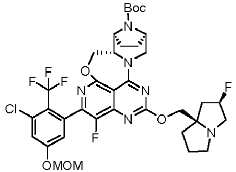 | *Tert*-butyl (5*aS*,6*S*,9*R*)-2-(3-chloro-5-(methoxymethoxy)-2-(trifluoromethyl)phenyl)-1-fluoro-12-(((2*R*,7 *aR*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 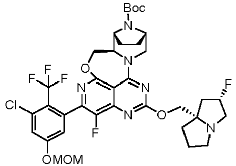 | *Tert*-butyl (5*aR*,6*R*,9*S*)-2-(3-chloro-5-(methoxymethoxy) -2-(trifluoromethyl)phenyl)-1-fluoro-12-(((2*S*, 7*aS*)-2-fluorotetrahydro-1*H*-pyrrolizin-7*a*(5*H*)-yl)methoxy)-5*a*,6,7,8,9,10-hexahydro-5*H*-4-o xa-3,10*a,*11,13,14-pentaaza-6,9-methanonapht ho[1,8-*ab*]heptalen-14-carboxylate |
| | 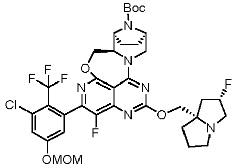 | *Tert*-butyl (5*aR*,6*S*,9*R*)-2-(3-chloro-5-(methoxymethoxy) -2-(trifluoromethyl)phenyl)-1-fluoro-12-(((2*S*, 7*aS*)-2-fluorotetrahydro-1*H*-pyrrolizin-7*a*(5*H*) -yl)methoxy)-5*a*,6,7,8,9,10-hexahydro-5*H*-4-o xa-3,10*a,*11,13,14-pentaaza-6,9-methanonapht ho[1,8-ab]heptalen-14-carboxylate |
| | 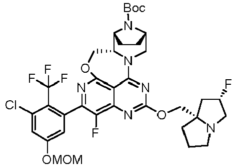 | *Tert*-butyl (5*aS*,6*R*,9*S*)-2-(3-chloro-5-(methoxymethoxy)-2-(trifluoromethyl)phenyl)-1-fluoro-12-(((2*S*,7 *aS*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-ab]heptalen-14-carboxylate |
| | 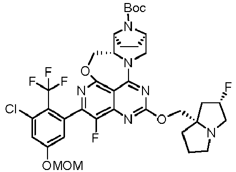 | *Tert*-butyl (5*aS*,6*S*,9*R*)-2-(3-chloro-5-(methoxymethoxy)-2-(trifluoromethyl)phenyl)-1-fluoro-12-(((2*S*,7 *aS*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 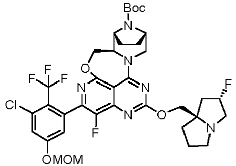 | Tert-butyl (5*aR*,6*R*,9*S*)-2-(3-chloro-5-(methoxymethoxy) -2-(trifluoromethyl)phenyl)-1-fluoro-12-(((2*S*, 7*aR*)-2-fluorotetrahydro-1*H*-pyrrolizin-7*a*(5*H*) -yl)methoxy)-5*a*,6,7,8,9,10-hexahydro-5*H*-4-o xa-3,10*a,*11,13,14-pentaaza-6,9-methanonapht ho[1,8-*ab*]heptalen-14-carboxylate |
| | 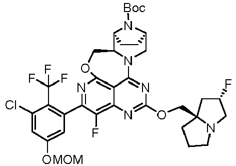 | *Tert*-butyl (5*aR*,6*S*,9*R*)-2-(3-chloro-5-(methoxymethoxy) -2-(trifluoromethyl)phenyl)-1-fluoro-12-(((2*S*, 7*aR*)-2-fluorotetrahydro-1*H*-pyrrolizin-7*a*(5*H*) -yl)methoxy)-Sa,6,7,8,9,10-hexahydro-SH-4-o xa-3,10*a,*11,13,14-pentaaza-6,9-methanonapht ho[1,8-*ab*]heptalen-14-carboxylate |
| | 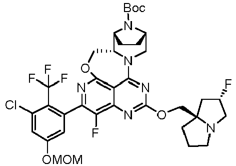 | *Tert*-butyl (5*aS*,6*R*,9*S*)-2-(3-chloro-5-(methoxymethoxy)-2-(trifluoromethyl)phenyl)-1-fluoro-12-(((2*S*,7 *aR*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 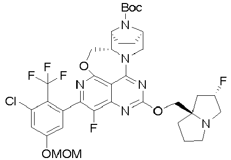 | *Tert*-butyl (5*aS*,6*S*,9*R*)-2-(3-chloro-5-(methoxymethoxy)-2-(trifluoromethyl)phenyl)-1-fluoro-12-(((2*S*,7 *aR*)-2-fluorotetrahydro-1*H*-pyrrolizin-7*a*(5*H*)-yl)methoxy)-5*a*,6,7,8,9,10-hexahydro-5*H*-4-ox a-3,10*a,*11,13,14-pentaaza-6,9-methanonaphth o[1,8-*ab*]heptalen-14-carboxylate |
| | 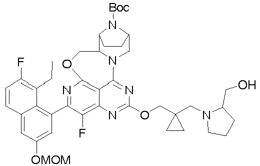 | *Tert*-butyl 2-(8-ethyl-7-fluoro-3-(methoxymethoxy)napht halen-1-yl)-1-fluoro-12-(1-((2-(hydroxymethyl )pyrrolidin-1-yl)methyl)cyclopropyl)methoxy) -5*a*,6,7,8,9,10-hexahydro-5*H*-4-oxa-3,10*a*,11, 13,14-pentaaza-6,9-methanonaphtho[1,8-*ab*]he ptalen-14-carboxylate |
| 15m | 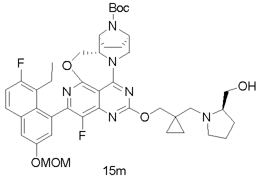 | *Tert*-butyl (5*aS*,6*S*,9*R*)-2-(8-ethyl-7-fluoro-3-(methoxym ethoxy)naphthalen-1-yl)-1-fluoro-12-(1-(((R)-2-(hydroxymethyl)pyrrolidin-1-yl)methyl)cycl opropyl)methoxy)-5a,6,7,8,9,10-hexahydro-5 *H*-4-oxa-3,10*a*,11,13,14-pentaaza-6,9-methano naphtho[1,8-*ab*]heptalen-14-carboxylate 15m |
| | 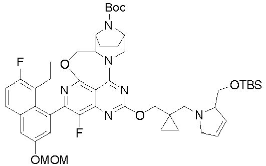 | Tert-butyl 12-((1-((2-(((*tert*-butyldimethylsilyl)oxy)meth yl)-2,5-dihydro-1*H*-pyrrol-1-yl)methyl)cyclopr opyl)methoxy)-2-(8-ethyl-7-fluoro-3-(methox ymethoxy)naphthalen-1-yl)-1-fluoro-5*a*,6,7,8, 9,10-hexahydro-5*H*-4-oxa-3,10*a,*11,13,14-pent aaza-6,9-methanonaphtho[1,8-*ab*]heptalen-14-carboxylate |
| 16e | 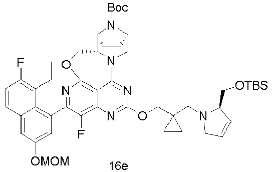 | *Tert*-butyl (5*aS*,6*S*,9*R*)-12-((1-(((*R*)-2-(((*tert*-butyldimeth ylsilyl)oxy)methyl)-2,5-dihydro-1*H*-pyrrol-1-y l)methyl)cyclopropyl)methoxy)-2-(8-ethyl-7-fl uoro-3-(methoxymethoxy)naphthalen-1-yl)-1-f luoro-5*a*,6,7,8,9,10-hexahydro-5*H*-4-oxa-3,10 *a*,11,13,14-pentaaza-6,9-methanonaphtho[1,8-*ab*]heptalen-14-carboxylate **16e** |
| 25a | 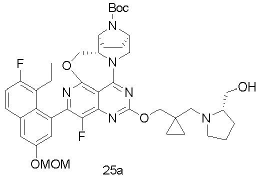 | *Tert-*butyl (5*aS*,6*S*,9*R*)-2-(8-ethyl-7-fluoro-3-(methoxym ethoxy)naphthalen-1-yl)-1-fluoro-12-(1-(((*S*)-2 -(hydroxymethyl)pyrrolidin-1-yl)methyl)cyclo propyl)methoxy)-5*a*,6,7,8,9,10-hexahydro-5*H-*4-oxa-3,10*a*,11,13,14-pentaaza-6,9-methanona phtho[1,8-*ab*]heptalen-14-carboxylate **25a** |
| | 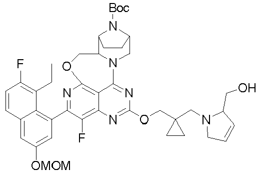 | *Tert*-butyl 2-(8-ethyl-7-fluoro-3-(methoxymethoxy)napht halen-1-yl)-1-fluoro-12-((1-((2-(hydroxymeth yl)-2,5-dihydro-1*H*-pyrrol-1-yl)methyl)cyclopr opyl)methoxy)-5*a*,6,7,8,9,10-hexahydro-5*H*-4-oxa-3,10*a*,11,13,14-pentaaza-6,9-methanonap htho[1,8-*ab*]heptalen-14-carboxylate |
| | 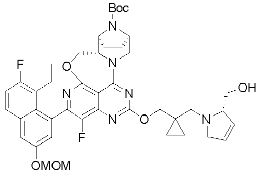 | *Tert*-butyl (5a*S*,6*S*,9*R*)-2-(8-ethyl-7-fluoro-3-(methoxym ethoxy)naphthalen-1-yl)-1-fluoro-12-((1-(((*S*)-2-(hydroxymethyl)-2,5-dihydro-1*H*-pyrrol-1-y l)methyl)cyclopropyl)methoxy)-5*a*,6,7,8,9,10-hexahydro-5*H*-4-oxa-3,10*a*,11,13,14-pentaaza -6,9-methanonaphtho[1,8-*ab*]heptalen-14-carb oxylate |
| | 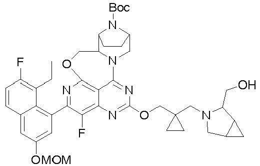 | *Tert*-butyl 2-(8-ethyl-7-fluoro-3-(methoxymethoxy)napht halen-1-yl)-1-fluoro-12-(1-((2-(hydroxymethyl )-3-azabicyclo[3.1,0]hex-3-yl)methyl)cyclopro pyl)methoxy)-5*a*,6,7,8,9,10-hexahydro-5*H*-4-o xa-3,10*a,*11,13,14-pentaaza-6,9-methanonapht ho[1,8-*ab*]heptalen-14-carboxylate |
| | 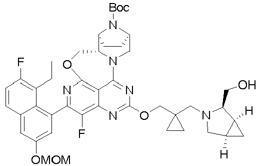 | *Tert*-butyl (5aS,6S,9R)-2-(8-ethyl-7-fluoro-3-(methoxym ethoxy)naphthalen-1-yl)-1-fluoro-12-(1-(((1S, 2*R*,5*R*)-2-(hydroxymethyl)-3-azabicyclo[3.1.0] hex-3-yl)methyl)cyclopropyl)methoxy)-5a,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 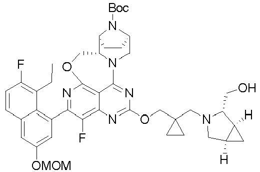 | *Tert*-butyl (5*aS*,6*S*,9*R*)-2-(8-ethyl-7-fluoro-3-(methoxym ethoxy)naphthalen-1-yl)-1-fluoro-12-(1-(((1*S*, 2*S*,5*R*)-2-(hydroxymethyl)-3-azabicyclo[3.1.0] hex-3-yl)methyl)cyclopropyl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 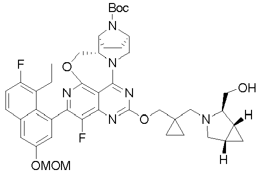 | *Tert*-butyl (5aS,6S,9R)-2-(8-ethyl-7-fluoro-3-(methoxym ethoxy)naphthalen-1-yl)-1-fluoro-12-(1-(((1*R*, 2*R*,5*S*)-2-(hydroxymethyl)-3-azabicyclo[3.1.0] hex-3-yl)methyl)cyclopropyl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 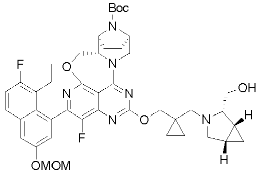 | *Tert*-butyl (5*aS*,6*S*,9*R*)-2-(8-ethyl-7-fluoro-3-(methoxym ethoxy)naphthalen-1-yl)-1-fluoro-12-(1-(((1R, 2*S*,5*S*)-2-(hydroxymethyl)-3-azabicyclo[3.1.0] hex-3-yl)methyl)cyclopropyl)methoxy)-5*a*,6,7, 8,9,10-hexahydro-5*H*-4-oxa-3,10*a*,11,13,14-pe ntaaza-6,9-methanonaphtho[1,8-*ab*]heptalen-1 4-carboxylate |
| | 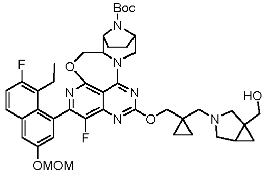 | *Tert*-butyl 2-(8-ethyl-7-fluoro-3-(methoxymethoxy)napht halen-1-yl)-1-fluoro-12-(1-((1-(hydroxymethyl )-3-azabicyclo[3.1.0]hex-3-yl)methyl)cyclopro pyl)methoxy)-5*a*,6,7,8,9,10-hexahydro-5*H*-4-o xa-3,10*a,*11,13,14-pentaaza-6,9-methanonapht ho[1,8-*ab*]heptalen-14-carboxylate |
| | 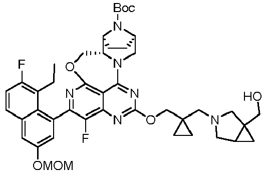 | *Tert*-butyl (5*aS*,6*S*,9*R*)-2-(8-ethyl-7-fluoro-3-(methoxym ethoxy)naphthalen-1-yl)-1-fluoro-12-(1-((1-(h ydroxymethyl)-3-azabicyclo[3.1.0]hex-3-yl)m ethyl)cyclopropyl)methoxy)-5*a*,6,7,8,9,10-hex ahydro-5*H*-4-oxa-3,10*a*,11,13,14-pentaaza-6,9 -methanonaphtho[1,8-*ab*]heptalen-14-carboxyl ate |
| | 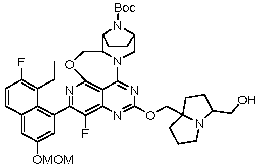 | *Tert*-butyl 2-(8-ethyl-7-fluoro-3-(methoxymethoxy)napht halen-1-yl)-1-fluoro-12-((3-hydroxymethyl)-te trahydro-1*H*-pyrrolizin-7*a*(5*H*)-yl)methoxy)-5 *a*,6,7,8,9,10-hexahydro-5*H*-4-oxa-3,10*a*,11,13, 14-pentaaza-6,9-methanonaphtho[1,8-*ab*]hepta len-14-carboxylate |
| | 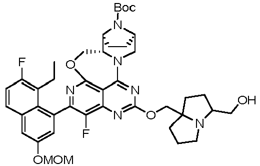 | *Tert*-butyl (5*aS*,6*S*,9*R*)-2-(8-ethyl-7-fluoro-3-(methoxym ethoxy)naphthalen-1-yl)-1-fluoro-12-((3-hydro xymethyl)-tetrahydro-1*H*-pyrrolizin-7*a*(5*H*)-yl )methoxy)-5*a*,6,7,8,9,10-hexahydro-5*H*-4-oxa-3,10*a*,11,13,14-pentaaza-6,9-methanonaphtho[ 1,8-*ab*]heptalen-14-carboxylate |
| | 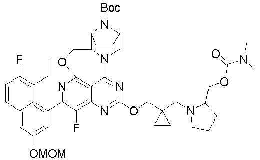 | *Tert*-butyl 12-((1-((2-(((dimethylaminocarbonyl)oxy)met hyl)pyrrolidin-1-yl)methyl)cyclopropyl)metho xy)-2-(8-ethyl-7-fluoro-3-(methoxymethoxy)n aphthalen-1-yl)-1-fluoro-5*a*,6,7,8,9,10-hexahy dro-5*H*-4-oxa-3,10*a*,11,13,14-pentaaza-6,9-me thanonaphtho[1,8-*ab*]heptalen-14-carboxylate |
| 25b | 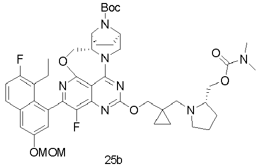 | *Tert*-butyl (5*aS*,6*S*,9*R*)-12-((1-(((*S*)-2-(((dimethylaminoc arbonyl)oxy)methyl)pyrrolidin-1-yl)methyl)cy clopropyl)methoxy)-2-(8-ethyl-7-fluoro-3-(me thoxymethoxy)naphthalen-1-yl)-1-fluoro-5*a*,6, 7,8,9,10-hexahydro-5*H*-4-oxa-3,10*a*,11,13,14-pentaaza-6,9-methanonaphtho[1,8-*ab*]heptalen -14-carboxylate **25b** |

Another aspect of the present disclosure relates to a method for preparing a compound of general formula (I) or a pharmaceutically acceptable salt thereof, which comprises: 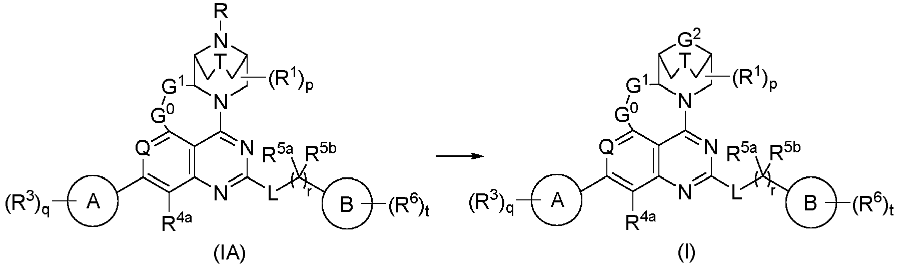 performing a deprotection reaction on a compound of general formula (IA) or a salt thereof to obtain the compound of general formula (I) or the pharmaceutically acceptable salt thereof, wherein optionally, when R³ and/or R⁶ groups contain a protecting group, a step of removing the protecting group on the R³ and/or R⁶ groups is also included before, simultaneously with or after the deprotection reaction;
wherein,
R is an amino protecting group, and preferably Boc;
G² is NH;
G⁰, G¹, T, ring A, ring B, Q, L, R¹, R³, R^{4a}, R^{5a}, R^{5b}, R⁶, p, q, r and t are as defined in general formula (I).

Another aspect of the present disclosure relates to a method for preparing a compound of general formula (I') or a pharmaceutically acceptable salt thereof, which comprises: 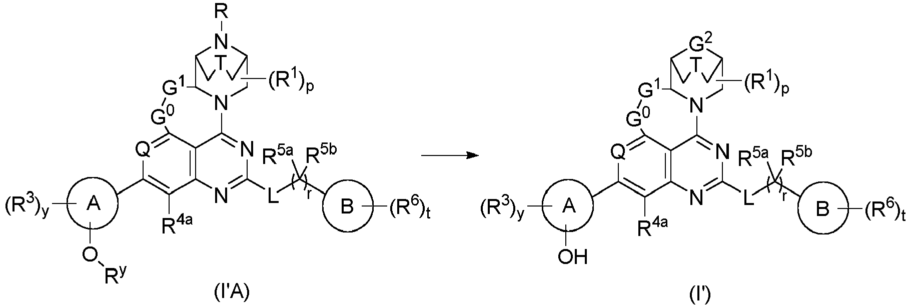 performing a deprotection reaction on a compound of general formula (I'A) or a salt thereof to obtain the compound of general formula (I') or the pharmaceutically acceptable salt thereof, wherein optionally, when R³ and/or R⁶ groups contain a protecting group, a step of removing the protecting group on the R³ and/or R⁶ groups is also included before, simultaneously with or after the deprotection reaction;
wherein,
R is an amino protecting group, and preferably Boc;
R^{y} is a hydroxy protecting group, and preferably MOM;
y is 0, 1, 2, 3 or 4;
G² is NH;
G⁰, G¹, T, ring A, ring B, Q, L, R¹, R³, R^{4a}, R^{5a}, R^{5b}, R⁶, p, r and t are as defined in general formula (I').

Another aspect of the present disclosure relates to a method for preparing a compound of general formula (II) or a pharmaceutically acceptable salt thereof, which comprises: 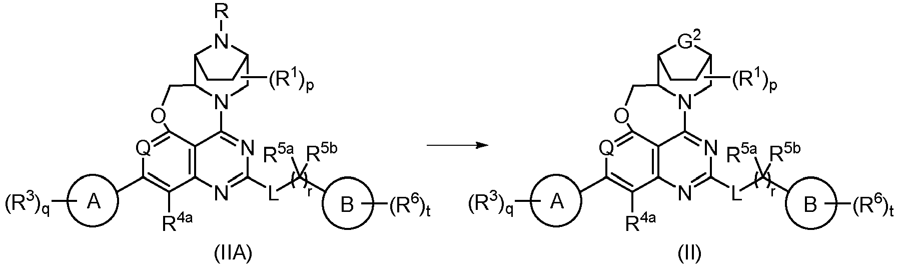 performing a deprotection reaction on a compound of general formula (IIA) or a salt thereof to obtain the compound of general formula (II) or the pharmaceutically acceptable salt thereof, wherein optionally, when R³ and/or R⁶ groups contain a protecting group, a step of removing the protecting group on the R³ and/or R⁶ groups is also included before, simultaneously with or after the deprotection reaction;
wherein R is an amino protecting group, and preferably Boc;
G² is NH;
ring A, ring B, Q, L, R¹, R³, R^{4a}, R^{5a}, R^{5b}, R⁶, p, q, r and t are as defined in general formula (II).

Another aspect of the present disclosure relates to a pharmaceutical composition comprising a compound of general formula (I), (I'), (II), Table A or Table B, of the present disclosure or a pharmaceutically acceptable salt thereof, and one or more pharmaceutically acceptable carriers, diluents or excipients.

The present disclosure further relates to use of a compound of general formula (I), (I'), (II), Table A or Table B, or a pharmaceutically acceptable salt thereof, or a pharmaceutical composition comprising the same, in the preparation of a medicament for inhibiting KRAS G12D.

The present disclosure further relates to use of a compound of general formula (I), (I'), (II), Table A or Table B, or a pharmaceutically acceptable salt thereof, or a pharmaceutical composition comprising the same, in the preparation of a medicament for treating and/or preventing a disease or condition, wherein the disease or condition is cancer, is preferably selected from the group consisting of brain cancer, thyroid cancer, head and neck cancer, nasopharyngeal cancer, laryngeal cancer, oral cancer, salivary gland cancer, esophageal cancer, gastric cancer, lung cancer, liver cancer, kidney cancer, pleural cancer, peritoneal cancer, pancreatic cancer, gall bladder cancer, bile duct cancer, colorectal cancer, small intestine cancer, gastrointestinal stromal tumors, urothelial cancer, urinary tract cancer, bladder cancer, anal cancer, joint cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, melanoma, lipoma, bone cancer, soft tissue sarcoma, neurofibroma, neuroglioma, neuroblastoma and glioblastoma, and further preferably is selected from the group consisting of pancreatic cancer, colorectal cancer and non-small cell lung cancer.

The present disclosure further relates to a method for inhibiting KRAS G12D comprising administering to a patient in need thereof a therapeutically effective amount of a compound of general formula (I), (I'), (II), Table A or Table B, or a pharmaceutically acceptable salt thereof, or a pharmaceutical composition comprising the same.

The present disclosure further relates to a method for treating and/or preventing a disease or condition comprising administering to a patient in need thereof a therapeutically effective amount of a compound of general formula (I), (I'), (II), Table A or Table B, or a pharmaceutically acceptable salt thereof, or a pharmaceutical composition comprising the same, wherein the disease or condition is cancer; is preferably selected from the group consisting of brain cancer, thyroid cancer, head and neck cancer, nasopharyngeal cancer, laryngeal cancer, oral cancer, salivary gland cancer, esophageal cancer, gastric cancer, lung cancer, liver cancer, kidney cancer, pleural cancer, peritoneal cancer, pancreatic cancer, gall bladder cancer, bile duct cancer, colorectal cancer, small intestine cancer, gastrointestinal stromal tumors, urothelial cancer, urinary tract cancer, bladder cancer, anal cancer, joint cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, melanoma, lipoma, bone cancer, soft tissue sarcoma, neurofibroma, neuroglioma, neuroblastoma and glioblastoma, and further preferably is selected from the group consisting of pancreatic cancer, colorectal cancer and non-small cell lung cancer.

The present disclosure further relates to a compound of general formula (I), (I'), (II), Table A or Table B, or a pharmaceutically acceptable salt thereof, or a pharmaceutical composition comprising the same, for use as a medicament.

The present disclosure further relates to a compound of general formula (I), (I'), (II), Table A or Table B, or a pharmaceutically acceptable salt thereof, or a pharmaceutical composition comprising the same, for use as a medicament for inhibiting KRAS G12D. The present disclosure further relates to a compound of general formula (I), (I'), (II), Table A or Table B, or a pharmaceutically acceptable salt thereof, or a pharmaceutical composition comprising the same, for use as a medicament for treating and/or preventing a disease or condition, wherein the disease or condition is cancer, is preferably selected from the group consisting of brain cancer, thyroid cancer, head and neck cancer, nasopharyngeal cancer, laryngeal cancer, oral cancer, salivary gland cancer, esophageal cancer, gastric cancer, lung cancer, liver cancer, kidney cancer, pleural cancer, peritoneal cancer, pancreatic cancer, gall bladder cancer, bile duct cancer, colorectal cancer, small intestine cancer, gastrointestinal stromal tumors, urothelial cancer, urinary tract cancer, bladder cancer, anal cancer, joint cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, melanoma, lipoma, bone cancer, soft tissue sarcoma, neurofibroma, neuroglioma, neuroblastoma and glioblastoma, and further preferably is selected from the group consisting of pancreatic cancer, colorectal cancer and non-small cell lung cancer.

The disease or condition described in the present disclosure is one that is treated and/or prevented by inhibiting KRAS G12D.

The colorectal cancer described in the present disclosure is preferably colon cancer or rectal cancer.

Preferably, the brain cancer described in the present disclosure is selected from the group consisting of glioblastoma multiforme and neuroblastoma; the soft tissue cancer is selected from the group consisting of fibrosarcoma, gastrointestinal sarcoma, rhabdomyoma, leiomyosarcoma, dedifferentiated liposarcoma, polymorphic liposarcoma, malignant fibrous histiocytoma, round cell sarcoma and synovial sarcoma; the lymphoma is selected from the group consisting of Hodgkin's disease and non-Hodgkin's lymphoma (e.g., mantle cell lymphoma, diffuse large B cell lymphoma, follicular center lymphoma, marginal zone B cell lymphoma, lymphoplasmacytic lymphoma and peripheral T cell lymphoma); the liver cancer is preferably hepatocellular carcinoma; the lung cancer (also known as bronchogenic lung cancer) is selected from the group consisting of non-small cell lung cancer (NSCLC), small cell lung cancer (SCLC) and squamous cell carcinoma; the kidney cancer is selected from the group consisting of renal cell carcinoma, clear cell and renal eosinophilic tumor; the leukemia is selected from the group consisting of chronic lymphocytic leukemia (CLL), chronic myelogenous leukemia, acute lymphoblastic leukemia (ALL), T-cell acute lymphoblastic leukemia (T-ALL), chronic myelogenous leukemia (CML) and acute myelogenous leukemia (AML); the skin cancer is selected from the group consisting of malignant melanoma, squamous cell carcinoma, basal cell carcinoma and angiosarcoma; the myeloma is preferably multiple myeloma.

The active compounds may be formulated into a form suitable for administration by any suitable route, and one or more pharmaceutically acceptable carriers are used to formulate the compositions of the present disclosure by conventional methods. Thus, the active compounds of the present disclosure may be formulated into a variety of dosage forms for oral administration, administration by injection (e.g., intravenous, intramuscular or subcutaneous), or administration by inhalation or insufflation. The compounds of the present disclosure may also be formulated into a dosage form, such as tablets, hard or soft capsules, aqueous or oily suspensions, emulsions, injections, dispersible powders or granules, suppositories, lozenges or syrups.

As a general guide, the active compound of the present disclosure is preferably in a form of a unit dose, or in a form of a single dose that can be self-administered by a patient. The unit dose of the compound or composition of the present disclosure may be in a tablet, capsule, cachet, vial, powder, granule, lozenge, suppository, regenerating powder or liquid formulation. A suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, an excipient, and the like. Depending on the method of administration, the compositions may comprise 0.1 to 99 wt.% of the active compound.

The tablet comprises the active ingredient and a non-toxic pharmaceutically acceptable excipient that is used for mixing and is suitable for the preparation of the tablet. Such an excipient may be an inert excipient, a granulating agent, a disintegrating agent, a binder and a lubricant. Such a tablet may be uncoated or may be coated by known techniques for masking the taste of the drug or delaying the disintegration and absorption of the drug in the gastrointestinal tract and thus enabling sustained release of the drug over a longer period.

An oral formulation in a soft gelatin capsule where the active ingredient is mixed with an inert solid diluent or with a water-soluble carrier or oil vehicle may also be provided. An aqueous suspension comprises an active substance and an excipient that is used for mixing and suitable for the preparation of the aqueous suspension. Such an excipient is a suspending agent, a dispersant or a wetting agent. The aqueous suspension may also comprise one or more preservatives, one or more colorants, one or more corrigents and one or more sweeteners.

An oil suspension may be formulated by suspending the active ingredient in a vegetable oil, or in a mineral oil. The oil suspension may comprise a thickening agent. The sweeteners and corrigents described above may be added to provide a palatable formulation. Antioxidants may also be added to preserve the compositions.

The pharmaceutical composition disclosed herein may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a corrigent, a preservative and an antioxidant. Such a formulation may also comprise a palliative, a preservative, a colorant and an antioxidant.

The pharmaceutical composition disclosed herein may be in a form of a sterile injectable aqueous solution. Available and acceptable vehicles or solvents include water, Ringer's solution and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer solutions and microemulsions in such a way as to maintain a constant circulating concentration of the compound disclosed herein. To maintain such a constant concentration, a continuous intravenous delivery device may be used. An example of such a device is a Deltec CADD-PLUS. TM. 5400 intravenous injection pump.

The pharmaceutical composition disclosed herein may be in a form of a sterile injectable aqueous or oil suspension for intramuscular and subcutaneous administration. The suspension can be prepared according to the prior art using those suitable dispersants or wetting agents and suspending agents mentioned above. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be employed. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

The compounds of the present disclosure can be administered in the form of dispersible powders and granules that are formulated into aqueous suspensions by adding water. These pharmaceutical compositions can be prepared by mixing the active ingredient with a dispersant or a wetting agent, a suspending agent, or one or more preservatives.

As is well known to those skilled in the art, the dose of the drug administered depends on a variety of factors, including but not limited to, the activity of the particular compound employed, the severity of the disease, the age of the patient, the weight of the patient, the health condition of the patient, the behavior of the patient, the diet of the patient, the time of administration, the route of administration, the rate of excretion, the combination of drugs, and the like. In addition, the optimal treatment regimen, such as the mode of administration, the daily dose of the compound or the type of pharmaceutically acceptable salts, can be verified according to conventional treatment regimens.

### Description of the terms

Unless otherwise stated, the terms used in the specification and claims have the following meanings.

The term "alkyl" refers to a saturated aliphatic hydrocarbon group which is a linear or branched group containing 1 to 20 carbon atoms, preferably alkyl having 1 to 12 (e.g., 1, 2, 3, 4, 5, 6, 7, 8, 9, 10, 11 and 12) carbon atoms (i.e., C₁₋₁₂ alkyl), and more preferably alkyl having 1 to 6 carbon atoms (i.e., C₁₋₆ alkyl). Non-limiting examples include methyl, ethyl, *n*-propyl, isopropyl, *n*-butyl, isobutyl, *tert*-butyl, sec-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof, etc. The alkyl may be substituted or unsubstituted. When substituted, the substituent may be substituted at any available connection site, and the substituent is preferably one or more substituents selected from the group consisting of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkylene" refers to a saturated linear or branched aliphatic hydrocarbon group, which is a residue derived from the parent alkane by removal of two hydrogen atoms from the same carbon atom or two different carbon atoms. It is a linear or branched group containing 1 to 20 carbon atoms, preferably alkylene containing 1 to 12 (e.g., 1, 2, 3, 4, 5, 6, 7, 8, 9, 10, 11 and 12) carbon atoms (i.e., C₁₋₁₂ alkylene), and more preferably alkylene containing 1 to 6 carbon atoms (i.e., C₁₋₆ alkylene). Non-limiting examples of alkylene include, but are not limited to, methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), etc. The alkylene may be substituted or unsubstituted. When substituted, the substituent may be substituted at any available connection site, and the substituent is preferably one or more substituents selected from the group consisting of alkenyl, alkynyl, alkoxy, haloalkoxy, cycloalkyloxy, heterocyclyloxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkenyl" refers to alkyl containing at least one carbon-carbon double bond in the molecule, wherein alkyl is as defined above; preferably alkenyl having 2 to 12 (e.g., 2, 3, 4, 5, 6, 7, 8, 9, 10, 11 and 12) carbon atoms (i.e., C₂₋₁₂ alkenyl), and more preferably alkenyl having 2 to 6 carbon atoms (i.e., C₂₋₆ alkenyl). Non-limiting examples include: ethenyl, propenyl, isopropenyl, butenyl and the like. Alkenyl may be substituted or unsubstituted. When substituted, the substituent is preferably one or more substituents selected from the group consisting of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkynyl" refers to alkyl containing at least one carbon-carbon triple bond in the molecule, wherein the alkyl is as defined above; preferably alkynyl having 2 to 12 (e.g., 2, 3, 4, 5, 6, 7, 8, 9, 10, 11 and 12) carbon atoms (i.e., C₂₋₁₂ alkynyl), and more preferably alkynyl having 2 to 6 carbon atoms (i.e., C₂₋₆ alkynyl). Non-limiting examples include: ethynyl, propynyl, butynyl, pentynyl, hexynyl and the like. Alkynyl may be substituted or unsubstituted. When substituted, the substituent is preferably one or more substituents selected from the group consisting of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent. The cycloalkyl ring contains 3 to 20 carbon atoms, preferably 3 to 14 (e.g., 3, 4, 5, 6, 7, 8, 9, 10, 11, 12, 13 and 14) carbon atoms (i.e., 3-14 membered cycloalkyl), preferably 3 to 8 (e.g., 3, 4, 5, 6, 7 and 8) carbon atoms (i.e., 3-8 membered cycloalkyl), and more preferably 3 to 6 carbon atoms (i.e., 3-6 membered cycloalkyl). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The term "spiro cycloalkyl" refers to a 5-20 membered polycyclic group in which monocyclic rings share one carbon atom (referred to as the spiro atom), wherein the spiro cycloalkyl may contain one or more double bonds. Preferably, the spiro cycloalkyl is 6-14 membered, and more preferably 7-10 membered (e.g., 7-membered, 8-membered, 9-membered or 10-membered). According to the number of the spiro atoms shared among the rings, the spiro cycloalkyl may be monospiro cycloalkyl or polyspiro cycloalkyl (e.g., bispiro cycloalkyl), and preferably monospiro cycloalkyl and bispiro cycloalkyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospiro cycloalkyl. Non-limiting examples of spiro cycloalkyl include:

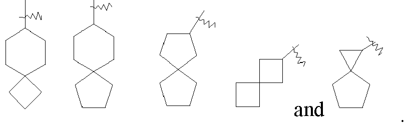

The term "fused cycloalkyl" refers to a 5-20 membered carbon polycyclic group in which each ring shares a pair of adjacent carbon atoms with the other rings in the system, wherein one or more of the rings may contain one or more double bonds. Preferably, the fused cycloalkyl is 6-14 membered, and more preferably 7-10 membered (e.g., 7-membered, 8-membered, 9-membered or 10-membered). According to the number of the formed rings, the fused cycloalkyl may be bicyclic or polycyclic fused cycloalkyl (e.g., tricyclic or tetracyclic fused cycloalkyl), preferably bicyclic or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicycloalkyl. Non-limiting examples of fused cycloalkyl include:

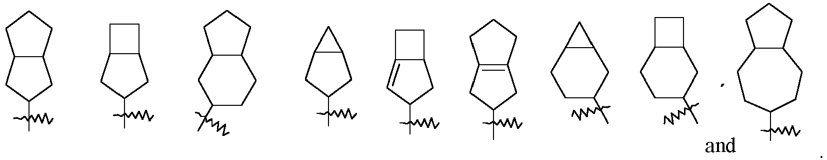

The term "bridged cycloalkyl" refers to a 5-20 membered carbon polycyclic group in which any two rings share two carbon atoms that are not directly connected to each other, wherein the bridged cycloalkyl may contain one or more double bonds. Preferably, the bridged cycloalkyl is 6-14 membered, and more preferably 7-10 membered (e.g., 7-membered, 8-membered, 9-membered or 10-membered). According to the number of the formed rings, the bridged cycloalkyl may be bicyclic or polycyclic (e.g., tricyclic or tetracyclic) bridged cycloalkyl, preferably bicyclic, tricyclic or tetracyclic bridged cycloalkyl, and more preferably bicyclic or tricyclic bridged cycloalkyl. Non-limiting examples of bridged cycloalkyl include:

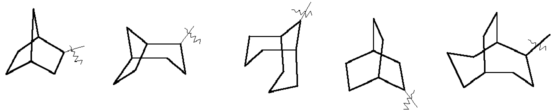

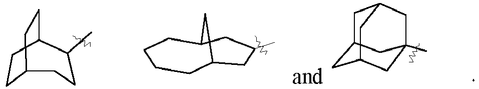

The cycloalkyl ring includes those in which the cycloalkyl described above (including monocyclic, spiro, fused and bridged rings) is fused to an aryl, heteroaryl or heterocycloalkyl ring, wherein the ring connected to the parent structure is cycloalkyl. Non-limiting examples include 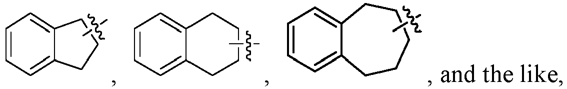 and preferably

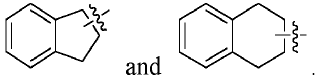

The cycloalkyl may be substituted or unsubstituted. When substituted, the substituent may be substituted at any available connection site, and the substituent is preferably one or more substituents selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkoxy" refers to -O-(alkyl), wherein the alkyl is as defined above. Non-limiting examples of alkoxy include methoxy, ethoxy, propoxy and butoxy. Alkoxy may be optionally substituted or unsubstituted. When substituted, the substituent is preferably selected from the group consisting of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heterocyclyl" refers to a saturated or partially unsaturated monocyclic or polycyclic substituent containing 3 to 20 (e.g., 3, 4, 5, 6, 7, 8, 9, 10, 11, 12, 13, 14, 15, 16, 17, 18, 19 or 20) ring atoms (i.e., 3-20 membered heterocyclyl), wherein one or more of the ring atoms is a heteroatom selected from the group consisting of nitrogen, oxygen and sulfur, and the sulfur may optionally be oxo (i.e., form sulfoxide or sulfone), but does not include a cyclic portion of -O-O-, -O-S- or -S-S-, the remaining ring atoms being carbon. The heterocyclyl preferably contains 3 to 14 (e.g., 3, 4, 5, 6, 7, 8, 9, 10, 11, 12, 13 and 14) ring atoms (i.e., 3-14 membered heterocyclyl), of which 1 to 4 (e.g., 1, 2, 3 and 4) are heteroatoms; more preferably 3 to 8 ring atoms (e.g., 3, 4, 5, 6, 7 and 8) (i.e., 3-8 membered heterocyclyl) or 6 to 14 ring atoms (e.g., 6, 7, 8, 9, 10, 11, 12, 13 and 14), of which 1 to 3 (e.g., 1, 2 and 3) are heteroatoms; more preferably 3 to 8 ring atoms, of which 1 to 3 (e.g. 1, 2 and 3) are heteroatoms; most preferably 5 or 6 ring atoms (i.e., 5- or 6-membered heterocyclyl), of which 1 to 3 are heteroatoms. Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl, and the like. Polycyclic heterocyclyl includes spiro heterocyclyl, fused heterocyclyl, and bridged heterocyclyl.

The term "spiro heterocyclyl" refers to a 5-20 membered polycyclic heterocyclyl group in which monocyclic rings share one atom (referred to as the spiro atom), wherein one or more of the ring atoms is a heteroatom selected from the group consisting of nitrogen, oxygen and sulfur, and the sulfur may optionally be oxo (i.e., form sulfoxide or sulfone), the remaining ring atoms being carbon. The spiro heterocyclyl may contain one or more double bonds. The spiro heterocyclyl is preferably 6-14 membered (e.g. 6-membered, 7-membered, 8-membered, 9-membered, 10-membered, 11-membered, 12-membered, 13-membered and 14-membered) (i.e., 6-14 membered spiro heterocyclyl), and more preferably 7-10 membered (e.g. 7-membered, 8-membered, 9-membered or 10-membered) (i.e., 7-10 membered spiro heterocyclyl). According to the number of spiro atoms shared among the rings, the spiro heterocyclyl may be monospiro heterocyclyl or polyspiro heterocyclyl (e.g., bispiro heterocyclyl), and preferably monospiro heterocyclyl and bispiro heterocyclyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospiro heterocyclyl. Non-limiting examples of spiro heterocyclyl include:

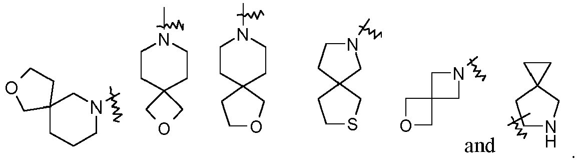

The term "fused heterocyclyl" refers to a 5 to 20 membered polycyclic heterocyclyl group in which each ring shares a pair of adjacent atoms with the other rings in the system, wherein one or more of the rings may contain one or more double bonds, one or more of the ring atoms is a heteroatom selected from the group consisting of nitrogen, oxygen and sulfur, and the sulfur may optionally be oxo (i.e., form sulfoxide or sulfone), the remaining ring atoms being carbon. The fused heterocyclyl is preferably 6-14 membered (e.g. 6-membered, 7-membered, 8-membered, 9-membered, 10-membered, 11-membered, 12-membered, 13-membered and 14-membered) (i.e., 6-14 membered fused heterocyclyl), and more preferably 7-10 membered (e.g. 7-membered, 8-membered, 9-membered or 10-membered) (i.e., 7-10 membered fused heterocyclyl). According to the number of the formed rings, the fused heterocyclyl may be bicyclic or polycyclic fused heterocyclyl (e.g., tricyclic or tetracyclic fused heterocyclyl), preferably bicyclic or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused heterocyclyl. Non-limiting examples of fused heterocyclyl include: 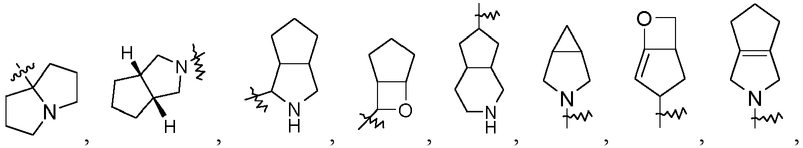 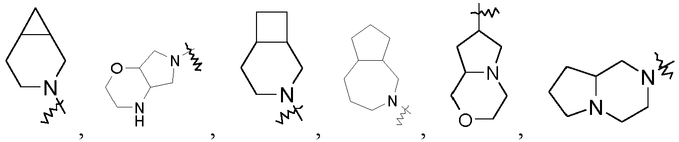 and

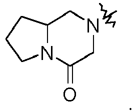

The term "bridged heterocyclyl" refers to a 5-20 membered polycyclic heterocyclyl group in which any two rings share two atoms which are not directly connected. The bridged heterocyclyl may contain one or more double bonds, wherein one or more of the ring atoms is a heteroatom selected from the group consisting of nitrogen, oxygen and sulfur, and the sulfur may optionally be oxo (i.e., form sulfoxide or sulfone), the remaining ring atoms being carbon. The bridged heterocyclyl is preferably 6-14 membered (e.g. 6-membered, 7-membered, 8-membered, 9-membered, 10-membered, 11-membered, 12-membered, 13-membered and 14-membered) (i.e., 6-14 membered bridged heterocyclyl), and more preferably 7-10 membered (e.g. 7-membered, 8-membered, 9-membered or 10-membered) (i.e., 7-10 membered bridged heterocyclyl). According to the number of the formed rings, the bridged heterocyclyl may be bicyclic or polycyclic (e.g., tricyclic or tetracyclic) bridged heterocyclyl, preferably bicyclic, tricyclic or tetracyclic bridged heterocyclyl, and more preferably bicyclic or tricyclic bridged heterocyclyl. Non-limiting examples of bridged heterocyclyl include:

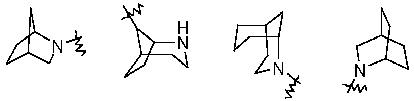

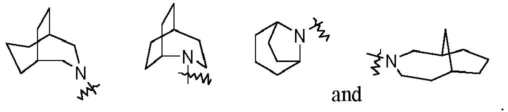

The heterocyclyl ring includes those in which the heterocyclyl described above (including monocyclic, spiro heterocyclic, fused heterocyclic and bridged heterocyclic rings) is fused to an aryl, heteroaryl or cycloalkyl ring, wherein the ring connected to the parent structure is heterocyclyl. Non-limiting examples include: 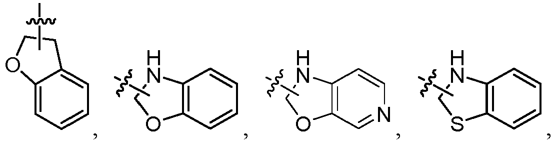 and the like.

The heteroaryl may be substituted or unsubstituted. When substituted, the substituent may be substituted at any available connection site, and the substituent is preferably one or more substituents selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "aryl" refers to a 6-14 membered, preferably 6-10 membered carbon monocyclic or fused polycyclic (fused polycyclic rings are those sharing a pair of adjacent carbon atoms) group having a conjugated π-electron system, such as phenyl and naphthyl. The aryl ring includes those in which the aryl ring described above is fused to a heteroaryl, heterocyclyl or cycloalkyl ring, wherein the ring connected to the parent structure is an aryl ring. Non-limiting examples include: 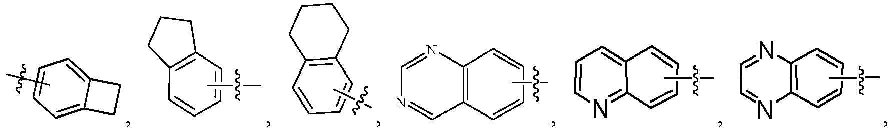 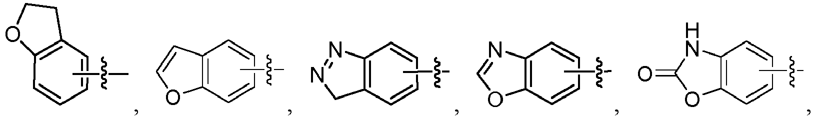 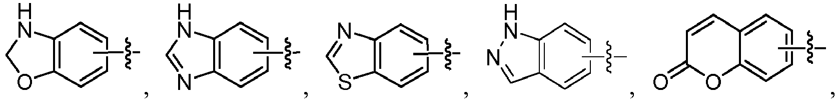 and

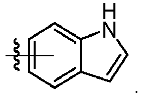

The aryl may be substituted or unsubstituted. When substituted, the substituent may be substituted at any available connection site, and the substituent is preferably one or more substituents selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heteroaryl" refers to a heteroaromatic system containing 1 to 4 (e.g., 1, 2, 3, and 4) heteroatoms and 5 to 14 ring atoms, wherein the heteroatoms are selected from the group consisting of oxygen, sulfur and nitrogen. The heteroaryl is preferably 5-10 membered (e.g., 5-membered, 6-membered, 7-membered, 8-membered, 9-membered or 10-membered) (i.e., 5-10 membered heteroaryl), further preferably 8-10 membered (e.g., 8-membered, 9-membered or 10-membered), more preferably 5- or 6-membered (i.e., 5- or 6-membered heteroaryl), such as furyl, thienyl, pyridyl, pyrrolyl, *N*-alkylpyrrolyl, pyrimidinyl, pyrazinyl, pyridazinyl, imidazolyl, pyrazolyl, triazolyl and tetrazolyl. The heteroaryl ring includes those in which the heteroaryl ring described above is fused to an aryl, heterocyclyl or cycloalkyl ring, wherein the ring connected to the parent structure is a heteroaryl ring. Non-limiting examples include: 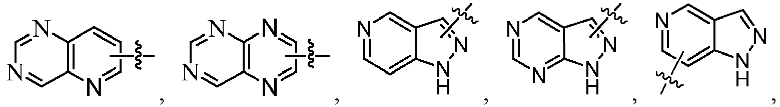 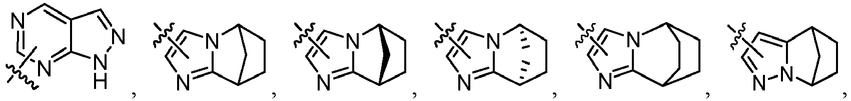 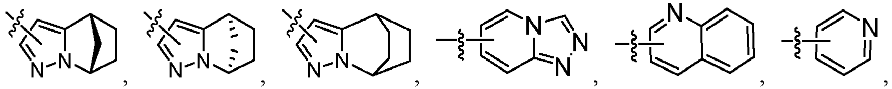 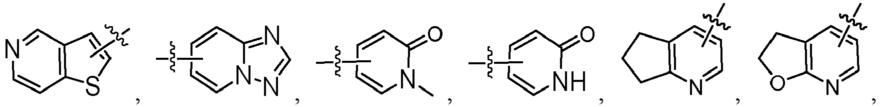 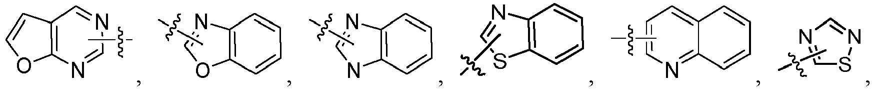 and

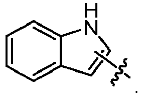

The heteroaryl may be substituted or unsubstituted. When substituted, the substituent may be substituted at any available connection site, and the substituent is preferably one or more substituents selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The cycloalkyl, heterocyclyl, aryl and heteroaryl described above include residues derived from the parent ring by removal of one hydrogen atom from a ring atom, or residues derived from the parent ring by removal of two hydrogen atoms from the same ring atom or two different ring atoms, i.e., "divalent cycloalkyl", "divalent heterocyclyl", "arylene" and "heteroarylene".

The term "amino protecting group" refers to a group that can be easily removed and is intended to protect an amino group from being changed when a reaction is conducted elsewhere in the molecule. Non-limiting examples include (trimethylsilyl)ethoxymethyl (SEM), tetrahydropyranyl, *tert*-butyloxycarbonyl (Boc), acetyl, benzyl, allyl, *p*-methylbenzenesulfonyl (Ts), p-methoxybenzyl, and the like. These groups may be optionally substituted with 1 to 3 substituents selected from the group consisting of halogen, alkoxy and nitro; the amino protecting group is preferably Boc.

The term "hydroxy protecting group" refers to a hydroxy derivative that is commonly used to block or protect hydroxyl while reactions are taking place on other functional groups of the compound. As an example, preferably, the hydroxy protecting group is, for example: triethylsilyl, triisopropylsilyl, *tert*-butyldimethylsilyl (TBS), *tert*-butyldiphenylsilyl, methyl, *tert*-butyl, allyl, benzyl, methoxymethyl (MOM), ethoxyethyl, 2-tetrahydropyranyl (THP), formyl, acetyl, benzoyl and *p*-nitrobenzoyl; the hydroxy protecting group is preferably MOM.

The term "alkynyl protecting group" refers to a group introduced on alkynyl that is easily removed and is intended to protect the active hydrogen in the acetylene or terminal alkyne from being changed when a reaction is conducted elsewhere in the molecule. Non-limiting examples include: trimethylsilyl (TMS), triethylsilyl (TES), tert-butyldimethylsilyl (TBS), triisopropylsilyl (TIPS), *tert*-butyldimethylsilyl (TBDMS), *tert*-butyldiphenylsilyl (TBDPS), methyl, *tert*-butyl, allyl, benzyl, methoxymethyl (MOM), ethoxyethyl, 2-tetrahydropyranyl (THP), formyl, acetyl, benzoyl, *p*-nitrobenzoyl, etc.; the alkynyl protecting group is preferably TIPS.

The term "cycloalkyloxy" refers to cycloalkyl-O-, wherein the cycloalkyl is as defined above.

The term "heterocyclyloxy" refers to the heterocyclyl-O-, wherein the heterocyclyl is as defined above.

The term "aryloxy" refers to aryl-O-, wherein aryl is as defined above.

The term "heteroaryloxy" refers to heteroaryl-O-, wherein heteroaryl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "deuterated alkyl" refers to alkyl substituted with one or more deuterium atoms, wherein the alkyl is as defined above.

The term "hydroxyalkyl" refers to alkyl substituted with one or more hydroxy groups, wherein the alkyl is as defined above.

The term "halogen" refers to fluorine, chlorine, bromine or iodine.

The term "hydroxy" refers to -OH.

The term "mercapto" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The term "carboxylate" refers to -C(O)O(alkyl), -C(O)O(cycloalkyl), (alkyl)C(O)O- or (cycloalkyl)C(O)O-, wherein the alkyl and cycloalkyl are as defined above.

MOM refers to methoxymethyl.

Boc refers to *tert*-butyloxycarbonyl.

TIPS refers to triisopropylsilyl.

TBS refers to *tert*-butyldimethylsilyl.

The compound disclosed herein may contain all manner of rotamers and conformationally constrained states thereof. Also included is an atropisomer. The term "atropisomer" is a stereoisomer resulting from hindered rotation about a single bond, wherein the energy difference due to steric strain or other contributing factors forms a sufficiently high rotational barrier to allow separation of the individual conformers. For example, certain compounds of the present disclosure may exist in the form of a mixture of atropisomers (e.g., an equal ratio mixture, a mixture enriched in one atropisomer) or a purified atropisomer. Non-limiting examples include:

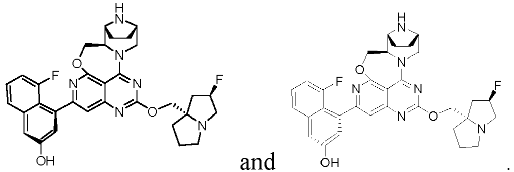

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, proton tautomers (also known as proton transfer tautomers) include interconversion via proton migration, such as keto-enol and imine-enamine, lactam-lactim isomerization. Examples of keto-enol equilibria are shown below:

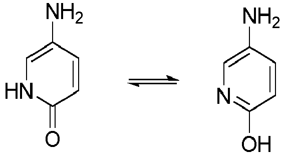

All tautomeric forms are within the scope of the present disclosure. The nomenclature of the compounds does not exclude any tautomers.

The compounds of the present disclosure may exist in specific stereoisomeric forms. The term "stereoisomer" refers to isomers that are structurally identical but differ in the arrangement of the atoms in space. It includes cis and trans (or Z and *E*) isomers, (-)- and (+)- isomers, (*R*)- and (*S*)-enantiomers, diastereomers, (*D*)- and (L)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. For all carbon-carbon double bonds, both *Z*- and *E*-forms are included, even if only one configuration is named. Optically active (-)- and (+)- isomers, (*R*)- and (*S*)-enantiomers, and (*D*)- and (*L*)-isomers can be prepared by chiral synthesis, chiral reagents or other conventional techniques. One isomer of a certain compound of the present disclosure may be prepared by asymmetric synthesis or derivatization with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. Furthermore, separation of enantiomers and diastereomers is typically accomplished by chromatography.

In the chemical structure of the compound of the present disclosure, a bond " 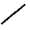 " represents an unspecified configuration, namely if chiral isomers exist in the chemical structure, the bond " 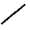 " may be " 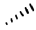 " or " 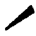 ", or contains both the configurations of " 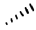 " and " 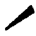 " simultaneously.

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (deuterium, T), ¹¹C, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸²Br, ¹²³I, ¹²⁴I, ¹²⁵I, ¹²⁹I and ¹³¹I, respectively, and preferably deuterium.

Compared with non-deuterated drugs, the deuterated drugs have the advantages of reduced toxic and side effects, increased stability of the drugs, enhanced curative effect, longer biological half-life of the drugs and the like. All isotopic variations of the compounds of the present disclosure, whether radioactive or not, are intended to be included within the scope of the present disclosure. Each available hydrogen atom linked to a carbon atom may be independently replaced with a deuterium atom, wherein replacement of deuterium may be partial or complete, and replacement of partial deuterium refers to replacement of at least one hydrogen with at least one deuterium.

The term "optionally" or "optional" means that the event or circumstance subsequently described may, but not necessarily, occur, and that the description includes instances where the event or circumstance occurs or does not occur. For example, "C₁₋₆ alkyl optionally substituted with halogen or cyano" means that halogen or cyano may, but not necessarily, be present, and the description includes the instance where alkyl is substituted with halogen or cyano and the instance where alkyl is not substituted with halogen and cyano.

The term "substituted" means that one or more, preferably 1-6, more preferably 1-3 hydrogen atoms in the group are independently substituted with a corresponding number of substituents. Those skilled in the art are able to determine (experimentally or theoretically) possible or impossible substitution without undue efforts. For example, it may be unstable when an amino or hydroxy group having a free hydrogen is bound to a carbon atom having an unsaturated (e.g., olefinic) bond.

The term "pharmaceutical composition" refers to a mixture containing one or more of the compounds described herein or a pharmaceutically acceptable salt or pro-drug thereof, and other chemical components, and other components, for example, pharmaceutically acceptable carriers and excipients. The purpose of the pharmaceutical composition is to promote the administration to an organism, which facilitates the absorption of the active ingredient, thereby exerting biological activities.

The "pharmaceutically acceptable salt" refers to a salt of the compound disclosed herein, which may be selected from the group consisting of inorganic and organic salts. The salts are safe and effective for use in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacological active agents, the term "therapeutically effective amount" refers to an amount of the drug or agent sufficient to achieve, or at least partially achieve, the desired effect. The determination of the therapeutically effective amount varies from person to person. It depends on the age and general condition of a subject, as well as the particular active substance used. The appropriate therapeutically effective amount in a case may be determined by those skilled in the art in light of routine tests. The term "pharmaceutically acceptable" as used herein means that those compounds, materials, compositions and/or dosage forms which are, within the scope of reasonable medical judgment, suitable for use in contact with the tissues of patients without excessive toxicity, irritation, allergic response, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration and temperature, it means that the parameter may vary by ±10%, and sometimes more preferably within ±5%. As will be appreciated by those skilled in the art, when the parameters are not critical, the numbers are generally given for illustrative purposes only and are not intended to be limiting.

### Synthesis of the compounds of the present disclosure

In order to achieve the purpose of the present disclosure, the following technical schemes are adopted in the present disclosure: 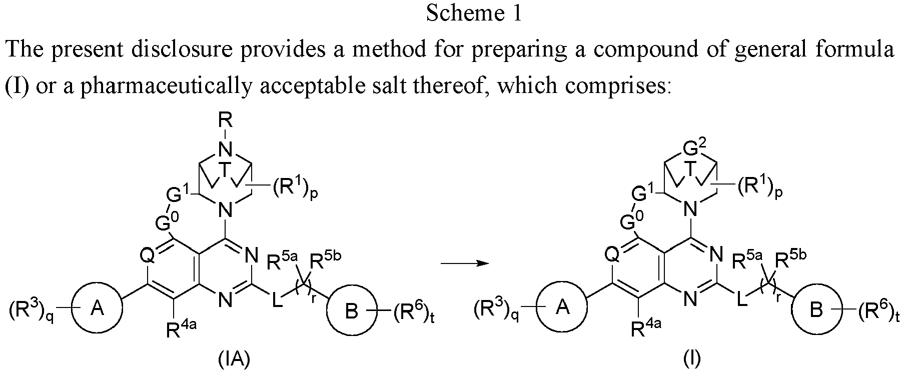 performing a deprotection reaction on a compound of general formula (IA) or a salt thereof under an acidic condition to obtain the compound of general formula (I) or the pharmaceutically acceptable salt thereof, wherein optionally, when R³ and/or R⁶ groups contain a protecting group, a step of removing the protecting group on the R³ and/or R⁶ groups under an acidic/alkaline condition is also included before, simultaneously with or after the deprotection reaction;
wherein R is an amino protecting group, and preferably Boc;
G² is NH;
G⁰, G¹, T, ring A, ring B, Q, L, R¹, R³, R^{4a}, R^{5a}, R^{5b}, R⁶, p, q, r and t are as defined in general formula (I). 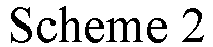 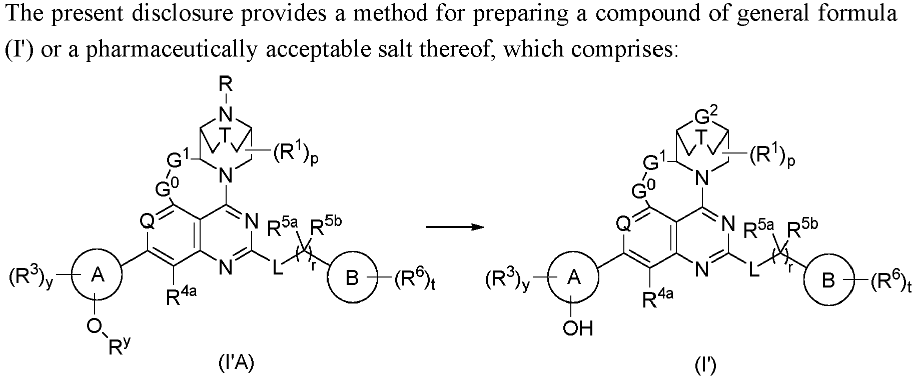
performing a deprotection reaction on a compound of general formula (I'A) or a salt thereof under an acidic condition to obtain the compound of general formula (I') or the pharmaceutically acceptable salt thereof, wherein optionally, when R³ and/or R⁶ groups contain a protecting group, a step of removing the protecting group on the R³ and/or R⁶ groups under an acidic/alkaline condition is also included before, simultaneously with or after the deprotection reaction;
wherein R is an amino protecting group, and preferably Boc;
R^{y} is a hydroxy protecting group, and preferably MOM;
y is 0, 1, 2, 3 or 4;
G² is NH;
G⁰, G¹, T, ring A, ring B, Q, L, R¹, R³, R^{4a}, R^{5a}, R^{5b}, R⁶, p, r and t are as defined in general formula (I'). 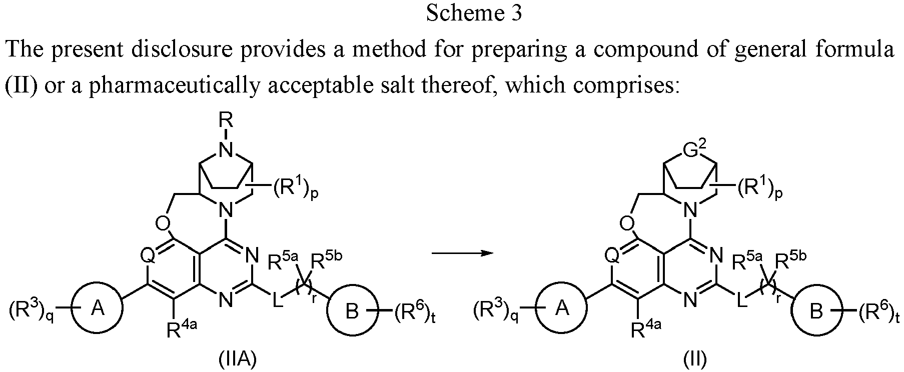
performing a deprotection reaction on a compound of general formula (IIA) or a salt thereof under an acidic condition to obtain the compound of general formula (II) or the pharmaceutically acceptable salt thereof, wherein optionally, when R³ and/or R⁶ groups contain a protecting group, a step of removing the protecting group on the R³ and/or R⁶ groups under an acidic/alkaline condition is also included before, simultaneously with or after the deprotection reaction;
wherein R is an amino protecting group, and preferably Boc;
G² is NH;
ring A, ring B, Q, L, R¹, R³, R^{4a}, R^{5a}, R^{5b}, R⁶, p, q, r and t are as defined in general formula (II).

Reagents that provide acidic conditions in the above synthesis schemes include organic and inorganic acids, wherein the organic acids include, but are not limited to, trifluoroacetic acid, formic acid, acetic acid, methanesulfonic acid, p-toluenesulfonic acid, Me₃SiCl and TMSOTf; the inorganic acids include, but are not limited to hydrogen chloride, a solution of hydrochloric acid in dioxane, hydrochloric acid, sulfuric acid, nitric acid and phosphoric acid, and preferably a solution of hydrochloric acid in dioxane.

The reagents that provide alkaline conditions in the above synthesis schemes include organic and inorganic bases, wherein the organic bases include, but are not limited to, triethylamine, *N*,*N*-diisopropylethylamine, *n*-butyllithium, lithium diisopropylamide, potassium acetate, sodium *tert*-butoxide, potassium *tert*-butoxide, tetrabutylammonium fluoride, solution of tetrabutylammonium fluoride in tetrahydrofuran or 1,8-diazabicycloundec-7-ene; the inorganic bases include, but are not limited to, sodium hydride, potassium phosphate, sodium carbonate, sodium acetate, potassium acetate, potassium carbonate, cesium carbonate, sodium hydroxide, lithium hydroxide, cesium fluoride and potassium hydroxide.

In the above synthetic schemes, when R³ and/or R⁶ contain terminal alkynyl, the terminal alkynyl can be protected with TIPS, and the reagent for removing TIPS is preferably a solution of tetrabutylammonium fluoride in tetrahydrofuran or cesium fluoride.

The reaction of the above steps is preferably performed in a solvent including, but not limited to: pyridine, ethylene glycol dimethyl ether, acetic acid, methanol, ethanol, acetonitrile, *n*-butanol, toluene, tetrahydrofuran, dichloromethane, petroleum ether, ethyl acetate, *n*-hexane, dimethyl sulfoxide, 1,4-dioxane, water, *N*,*N*-dimethylformamide, *N*,*N*-dimethylacetamide, 1,2-dibromoethane and a mixture thereof.

### DETAILED DESCRIPTION

The following examples further illustrate the present disclosure, but the present disclosure is not limited thereto.

### Examples

The structure of the compound was determined by nuclear magnetic resonance (NMR) spectroscopy and/or mass spectrometry (MS). NMR shift (δ) was given in a unit of 10⁻⁶ (ppm). NMR spectra were measured using a Bruker AVANCE-400 nuclear magnetic resonance instrument or Bruker AVANCE NEO 500M, with deuterated dimethyl sulfoxide (DMSO-*d₆*), deuterated chloroform (CDCl₃) and deuterated methanol (CD₃OD) as determination solvents and tetramethylsilane (TMS) as an internal standard.

Mass spectra (MS) were measured using Agilent 1200/1290 DAD-6110/6120 Quadrupole MS liquid chromatography-mass spectrometry system (manufacturer: Agilent; MS model: 6110/6120 Quadrupole MS),
waters ACQuity UPLC-QD/SQD (manufacturer: waters; MS model: waters ACQuity Qda Detector/waters SQ Detector) and
THERMO Ultimate 3000-Q Exactive (manufacturer: THERMO; MS model: THERMO Q Exactive).

High performance liquid chromatography (HPLC) was performed using the following HPLC instruments: Agilent HPLC 1200DAD, Agilent HPLC 1200VWD and Waters HPLC e2695-2489.

Chiral HPLC was performed on Agilent 1260 DAD HPLC.

HPLC preparation was performed using Waters 2545-2767, Waters 2767-SQ Detecor2, Shimadzu LC-20AP and Gilson GX-281 preparative chromatographs.

Chiral preparation was performed on a Shimadzu LC-20AP preparative chromatograph. A CombiFlash Rf200 (TELEDYNE ISCO) system was used for rapid preparation.

Huanghai HSGF254 or Qingdao GF254 silica gel plates of specifications 0.15 mm to 0.2 mm were adopted for thin layer chromatography (TLC) analysis and 0.4 mm to 0.5 mm for TLC separation and purification.

The silica gel column chromatography generally used 200 to 300-mesh silica gel (Huanghai, Yantai) as the carrier.

The mean inhibition of kinase and the IC₅₀ value were measured using a NovoStar microplate reader (BMG, Germany).

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions could be performed in an argon atmosphere or a nitrogen atmosphere unless otherwise specified.

The argon atmosphere or nitrogen atmosphere means that the reaction flask is linked to a balloon containing about 1 L of argon or nitrogen.

The hydrogen atmosphere means that the reaction flask is linked to a balloon containing about 1 L of hydrogen.

Parr 3916EKX hydrogenator, Qinglan QL-500 hydrogenator or HC2-SS hydrogenator was used in the pressurized hydrogenation reactions.

The hydrogenation reactions usually involved 3 cycles of vacuumization and hydrogen purge.

A CEM Discover-S 908860 microwave reactor was used in the microwave reactions.

In the examples, a solution refers to an aqueous solution unless otherwise specified.

In the examples, the reaction temperature was room temperature, i.e., 20 °C to 30 °C, unless otherwise specified.

The monitoring of the reaction progress in the examples was conducted by thin layer chromatography (TLC). The developing solvent for reactions, the eluent system for column chromatography purification and the developing solvent system for thin layer chromatography included: A: dichloromethane/methanol system, and B: n-hexane/ethyl acetate. The volume ratio of the solvents was adjusted according to the polarity of the compound, or by adding a small amount of basic or acidic reagents such as triethylamine and acetic acid.

When the compounds of the examples contained two or more chiral centers, the relative stereochemistry of these compounds was determined by NMR studies and/or X-ray diffraction. In these cases, the compounds were identified using the prefix "rel" followed by the R/S nomenclature, where R/S provides only relative stereochemical information and does not indicate absolute stereochemistry. For example, 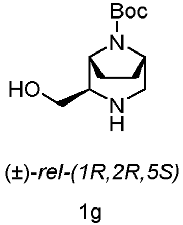 represents a 1:1 mixture of 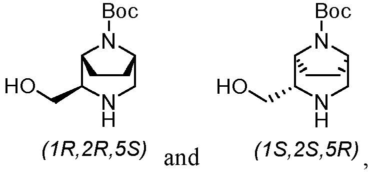 i.e., a racemate.

### Example 1

### Diastereomeric (1:1) mixture of 5-fluoro-4-((5aS,6S,9R)-12-(((2R,7aS)-2-fluorotetrahydro-1H-pyrrolizin-7a(5H)-yl)met hoxy)-5a,6,7,8,9,10-hexahydro-5H-4-oxa-3,10a,11,13,14-pentaaza-6,9-methanonaphtho [1,8-ab]heptalen-2-yl)naphthalen-2-ol 1-p1 and

### 5-fluoro-4-((5aR,6R,9S)-12-(((2R,7aS)-2-fluorotetrahydro-1H-pyrrolizin-7a(5H)-yl)met hoxy)-5a,6,7,8,9,10-hexahydro-5H-4-oxa-3,10a,11,13,14-pentaaza-6,9-methanonaphtho [1,8-ab]heptalen-2-yl)naphthalen-2-ol 1-p2

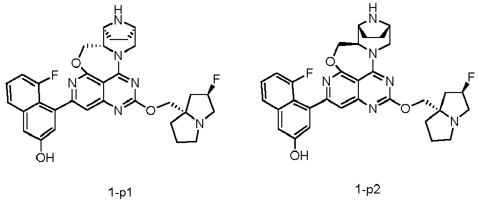

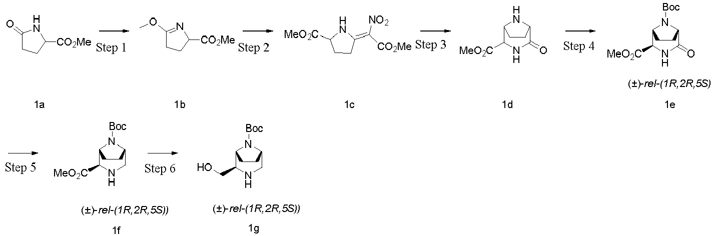

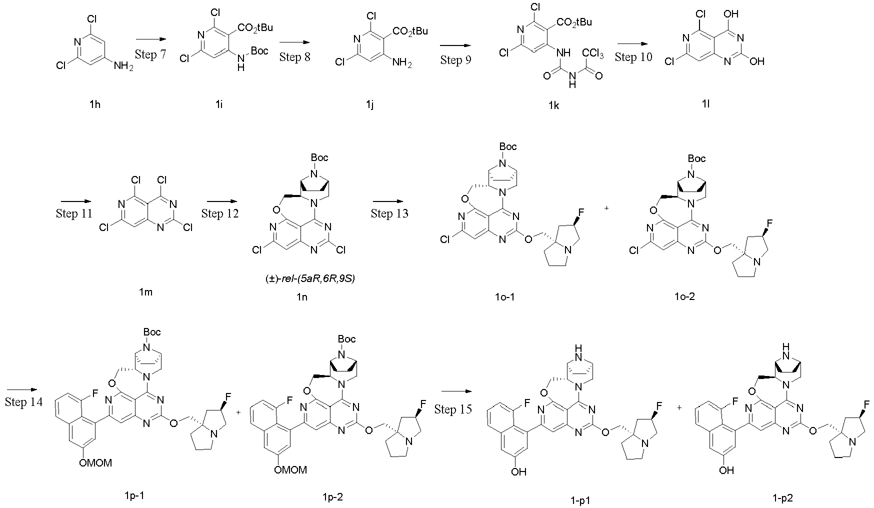

### Step 1

### Methyl (±)-5-methoxy-3,4-dihydro-2H-pyrrole-2-carboxylate 1b

Methyl (±)-2-pyrrolidone-5-formate **1a** (100 g, 698.61 mmol, Shanghai Bide) and dimethyl sulfate (110 g, 872.10 mmol) were mixed and reacted at 60 °C for 16 h, and the reaction solution was cooled to room temperature, and poured into a solution of triethylamine (100 g) and methyl *tert*-butyl ether (150 mL) under an ice bath. The resulting solution was extracted with methyl *tert*-butyl ether (300 mL × 6) and then concentrated under reduced pressure to give crude title compound **1b** (90 g, yield: 81.9%), which was directly used in the next reaction without purification.

MS m/z (ESI): 158.1 [M+1].

### Step 2

### Methyl (±)-5-(2-methoxy-1-nitro-2-oxoethylidene)pyrrolidine-2-carboxylate 1c

Crude compound **1b** (90 g, 572.64 mmol) and methyl nitroacetate (68.18 g, 572.63 mmol) were mixed, heated to 60 °C and stirred for 30 h, after the reaction solution was cooled to room temperature, the reaction solution was added with ethyl acetate (300 mL), stirred for 0.5 h and filtered, and the filter cake was dried to give title compound **1c** (70 g, yield: 50%), which was directly used in the next reaction without purification. MS m/z (ESI): 245.1 [M+1].

### Step 3

### Methyl 4-oxo-3,8-diazabicyclo[3.2.1]octane-2-carboxylate (diastereomeric mixture) 1d

Crude compound **1c** (14 g, 57.3 mmol) was dissolved in 600 mL of methanol, the reaction solution was added with a 10% palladium on carbon catalyst (wet) (14 g), purged with hydrogen three times, stirred for 48 h, and filtered through celite, and the filtrate was concentrated to give crude title compound **1d** (10 g, yield: 94.6%), which was directly used in the next reaction without purification.

MS m/z (ESI): 185.2 [M+1].

### Step 4

### 8-(tert-butyl) 2-methyl (±)-rel(1R,2R,5S)-4-oxo-3,8-diazabicyclo[3.2.1]octane-2,8-diformate 1e

Crude compound **1d** (10 g, 54.2 mmol) was dissolved in 300 mL of dichloromethane, the reaction solution was added with triethylamine (16 g, 158.12 mmol) and di-*tert*-butyl dicarbonate (11 g, 50.4 mmol, Shanghai Accela) under an ice bath, stirred for 14 h, and concentrated under reduced pressure, and the residue was purified by silica gel column chromatography with eluent system B to give title compound **1e** (3.3 g, yield: 21.3%).

MS m/z (ESI): 285.2 [M+1].

HPLC analysis: retention time: 1.02 min; purity: 98.5% (chromatography column: ACQUITY UPLC^{®}BEH, C18, 1.7 µm, 2.1 × 50 mm; mobile phase: water (10 mM ammonium bicarbonate), acetonitrile; gradient ratio: acetonitrile 10%-95%).

### Step 5

### 8-(tert-butyl) 2-methyl (±)-rel-(1R,2R,5S)-3,8-diazabicyclo[3.2.1]octane-2,8-diformate 1f

Compound **1e** (400 mg, 1.4 mmol) was dissolved in 2 mL of tetrahydrofuran, the reaction solution was added with 3.5 mL of 2 M borane dimethylsulfide complex in tetrahydrofuran, stirred for 14 h, added with methanol for quenching the reaction, then reacted at 50 °C for 14 h, and concentrated under reduced pressure, and the residue was purified by silica gel column chromatography with eluent system A to give title compound **1f** (176 mg, yield: 46.2%).

MS m/z (ESI): 271.2 [M+1].

### Step 6

### Tert-butyl (±)-rel-(1R,2R,5S)-2-(hydroxymethyl)-3,8-diazabicyclo[3.2.1]octane-8-carboxylate 1g

Compound **1f** (1 g, 3.69 µmol) was dissolved in 15 mL of tetrahydrofuran, the reaction solution was added with 4.4 mL of 1 M tetrahydrofuran solution of lithium aluminum hydride, stirred at 0 °C for 1 h, sequentially added with 0.2 mL of water, 0.2 mL of 15% aqueous sodium hydroxide solution, 0.4 mL of water and anhydrous sodium sulfate, stirred for 10 min and filtered, and the filtrate was concentrated to give title compound **1g** (430 mg, yield: 47.9%), which was directly used in the next reaction without purification.

MS m/z (ESI): 243.1 [M+1].

### Step 7

### Tert-butyl 4-((tert-butoxycarbonyl)amino)-2,6-dichloronicotinate 1i

4-amino-2,6-dichloropyridine **1h** (1 g, 6.13 mmol, Shanghai Bide) was dissolved in 1,4-dioxane (15 mL), and the reaction solution was added with 15 mL of 2 M sodium bis(trimethylsilyl)amide in tetrahydrofuran under an ice bath, stirred for 0.5 h, added with di-*tert*-butyl dicarbonate (3.3 g, 15.1 mmol), stirred for 14 h, added with saturated aqueous ammonium chloride for quenching the reaction, and extracted with ethyl acetate (30 mL × 3). The organic phases were combined, dried over anhydrous sodium sulfate, and filtered to remove the drying agent, and the filtrate was concentrated under reduced pressure to give title compound **1i** (500 mg, yield: 22.5%), which was directly used in the next reaction without purification.

MS m/z (ESI): 363.1 [M+1].

### Step 8

### Tert-butyl 4-amino-2,6-dichloronicotinate 1j

Compound **1i** (300 mg, 825.9 µmol) was dissolved in acetonitrile (8 mL), and the reaction solution was added with 0.35 mL of 4 M hydrochloride in dioxane, stirred for 2 h, adjusted the pH to neutral with 4 M aqueous sodium hydroxide solution under an ice bath, and extracted with ethyl acetate (10 mL × 3). The organic phases were combined, dried over anhydrous sodium sulfate, and filtered to remove the drying agent, and the filtrate was concentrated under reduced pressure to give crude title compound **1j** (62 mg, yield: 28.5%), which was directly used in the next reaction without purification.

MS m/z (ESI): 363.1 [M+1].

### Step 9

### Tert-butyl 2,6-dichloro-4-(3-(2,2,2-trichloroacetyl)ureido)nicotinate 1k

Crude compound **1j** (240 mg, 912.13 µmol) was dissolved in tetrahydrofuran (10 mL), and the reaction solution was added with trichloroacetylisocyanate (260 mg, 1.38 mmol), stirred for 30 min, and concentrated under reduced pressure to give crude title compound **1k** (411 mg, yield: 99.7%), which was directly used in the next reaction without purification.

MS m/z (ESI): 449.9 [M+1].

### Step 10

### 5,7-dichloropyrido[4,3-d]pyrimidine-2,4-diol 11

Crude compound **1k** (410 mg, 913 µmol) was dissolved in 7 M methanolic ammonia (10 mL), the reaction solution was stirred for 1 h, and concentrated under reduced pressure, and the residue was added with methyl *tert*-butyl ether (10 mL), stirred for 0.5 h and filtered, and the filter cake was dried to give crude title compound **11** (200 mg, yield: 94.3%), which was directly used in the next reaction without purification.

MS m/z (ESI): 232.1 [M+1].

### Step 11

### 2,4,5,7-tetrachloropyrido[4,3-d]pyrimidine 1m

Crude compound **11** (150 mg, 646.4 µmol) was dissolved in phosphorus oxychloride (3 mL), and the reaction solution was added with *N, N*-diisopropylethylamine (420 mg, 3.2 mmol), stirred at 110 °C for 3 h, cooled to room temperature and concentrated under reduced pressure to give crude title compound **1m** (170 mg, yield: 97.7%), which was directly used in the next step without purification.

MS m/z (ESI): 267.8 [M+1].

### Step 12

### Tert-butyl

### (±)-rel-(5aR,6R,9S)-2,12-dichloro-5a,6,7,8,9,10-hexahydro-5H-4-oxa-3,10a,11,13,14-p entaaza-6,9-methanonaphtho[1,8-ab]heptalen-14-carboxylate1n

Compound **1m** (104 mg, 386.7 µmol) and N,N diisopropylethylamine (750 mg, 5.8 mmol) were dissolved in 7 mL of dichloromethane, the reaction solution was added with **1g** (45 mg, 185.7 mmol) at -40 °C, stirred for 2 h while maintaining the temperature, and concentrated under reduced pressure, and the residue was purified by silica gel column chromatography with eluent system B to give title compound **1n**(30 mg, yield: 39.3%).

MS m/z (ESI): 474.2 [M+1].

### Step 13

### Diastereomeric mixture of tert-butyl

### (5aS,6S,9R)-2-chloro-12-(((2R,7aS)-2-fluorotetrahydro-1H-pyrrolizin-7a(5H)-yl)metho xy)-5a,6,7,8,9,10-hexahydro-5H-4-oxa-3,10a,11,13,14-pentaaza-6,9-methanonaphtho[1, 8-ab]heptalen-14-carboxylate 10-1 and

### tert-butyl (5aR,6R,9S)-2-chloro-12-(((2R,7aS)-2-fluorotetrahydro-1H-pyrrolizin-7a(5H)-yl)metho xy)-5a,6,7,8,9,10-hexahydro-5H-4-oxa-3,10a,11,13,14-pentaaza-6,9-methanonaphtho[l, 8-ab]heptalen-14-carboxylate 1o-2

Compound **1n**(30 mg, 68.4 µmol) was dissolved in 1,4-dioxane (2 mL), the reaction solution was added with ((2*R*,7*aS*)-2-fluorotetrahydro-1*H*-pyrrolizin-7*a*(5*H*)-yl)methanol (22 mg, 138.1 µmol, WuXi AppTec), 0.1 mL of 2 M sodium bis(trimethylsilyl)amide in tetrahydrofuran and 4A molecular sieves (300 mg), stirred at 90 °C for 14 h, cooled to room temperature and concentrated under reduced pressure, and the residue was purified by silica gel column chromatography with eluent system A to give a diastereomeric (1:1) mixture of title compounds **1o-1** and **1o-2** (14 mg, yield: 36.4%).

MS m/z (ESI): 561.2 [M+1].

### Step 14

### Diastereomeric mixture of tert-butyl (5aS,6S,9R)-2-(8-fluoro-3-(methoxymethoxy)naphthalen-1-yl)-12-(((2R,7aS)-2-fluorote trahydro-1H-pyrrolizin-7a(5H)-yl)methoxy)-5a,6,7,8,9,10-hexahydro-5H-4-oxa-3,10a,1 1,13, 14-pentaaza-6,9-methanonaphtho[1,8-ab]heptalen-14-carboxylate 1p-1 and

### tert-butyl (5aR,6R,9S)-2-(8-fluoro-3-(methoxymethoxy)naphthalen-1-yl)-12-(((2R,7aS)-2-fluorote trahydro-1H-pyrrolizin-7a(5H)-yl)methoxy)-5a,6,7,8,9,10-hexahydro-5H-4-oxa-3,10a,1 1,13,14-pentaaza-6,9-methanonaphtho[1,8-ab]heptalen-14-carboxylate 1p-2

The diastereomeric (1:1) mixture of compounds **1o-1** and **1o-2** (20 mg, 35.6 µmol), 2-(8-fluoro-3-(methoxymethoxy)naphthalen-1-yl)-4,4,5,5-tetramethy1-1,3,2-dioxaborola ne (24 mg, 72.2 µmol, prepared using the method disclosed in Example 282 on page 522 of the specification in Patent Application "WO2021/041671"), tetrakis(triphenylphosphine)palladium (6 mg, 5.19 µmol, adamas) and cesium carbonate (58 mg, 178 µmol) were dissolved in 3 mL of a mixed solution of 1,4-dioxane and water (V:V = 5:1). The reaction solution was reacted at 100 °C for 14 h under nitrogen atmosphere, and concentrated under reduced pressure, and the residue was purified by silica gel column chromatography with eluent system A to give a diastereomeric (1:1) mixture of title compounds **1p-1** and **1p-2** (5 mg, yield: 19.2%).

MS m/z (ESI): 731.2 [M+1].

### Step 15

### Diastereomeric mixture of 5-fluoro-4-((5aS,6S,9R)-12-(((2R,7aS)-2-fluorotetrahydro-1H-pyrrolizin-7a(5H)-yl)met hoxy)-5a,6,7,8,9,10-hexahydro-5H-4-oxa-3,10a,11,13,14-pentaaza-6,9-methanonaphtho [1,8-ab]heptalen-2-yl)naphthalen-2-ol 1-p1 And

### 5-fluoro-4-((5aR,6R,9S)-12-(((2R,7aS)-2-fluorotetrahydro-1H-pyrrolizin-7a(5H)-yl)met hoxy)-5a,6,7,8,9,10-hexahydro-5H-4-oxa-3,10a,11,13,14-pentaaza-6,9-methanonaphtho [1,8-ab]heptalen-2-yl)naphthalen-2-ol 1-p2

The diastereomeric (1:1) mixture of compounds **1p-1** and **1p-2** (5 mg, 6.8 µmol) was dissolved in ethyl acetate (1 mL), and the reaction solution was added with 0.5 mL of 4 M hydrochloride in dioxane, reacted at 0 °C for 1 h, concentrated under reduced pressure and then purified by high performance liquid chromatography (Waters-2545, chromatography column: SharpSil-T C18, 30 × 150 mm, 5 µm; mobile phase: aqueous phase (10 mmol/L ammonium bicarbonate) and acetonitrile, gradient ratio: acetonitrile 38%-45%, flow rate: 30 mL/min) to give the diastereomeric (1:1) mixture of title compounds **1-p1** and **1-p2** (1 mg, yield: 25%).

MS m/z (ESI): 587.2 [M+1].

HPLC analysis: retention time: 1.12 min; purity: 96.3% (chromatography column: ACQUITY UPLC^{®}BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.57 (dd, 1H), 7.39 (tt, 1H), 7.29 - 7.25 (m, 1H), 7.16 (d, 1H), 7.11 (d, 1H), 6.92 (ddd, 1H), 5.38 (dd, 1H), 4.51 - 4.44 (m, 1H), 4.31 (t, 1H), 4.23 (t, 1H), 4.14 (s, 1H), 3.75 (s, 1H), 3.67 (d, 1H), 3.49 - 3.45 (m, 1H), 3.26 - 3.18 (m, 2H), 3.06 (s, 1H), 2.36 - 1.84 (m, 12H), 1.62 (s, 1H).

### Example 2

### Diastereomeric (1:1) mixture of 5-ethyl-4-((5aS,6S,9R)-12-(((2R,7aS)-2-fluorotetrahydro-1H-pyrrolizin-7a(5H)-yl)meth oxy)-5a,6,7,8,9,10-hexahydro-5H-4-oxa-3,10a,11,13,14-pentaaza-6,9-methanonaphtho[ 1,8-ab]heptalen-2-yl)naphthalen-2-ol 2-p1 and

### 5-Ethyl-4-((5aR,6R,9S)-12-(((2R,7aS)-2-fluorotetrahydro-1H-pyrrolizin-7a(5H)-yl)met hoxy)-5a,6,7,8,9,10-hexahydro-5H-4-oxa-3,10a,11,13,14-pentaaza-6,9-methanonaphtho [1,8-ab]heptalen-2-yl)naphthalen-2-ol 2-p2

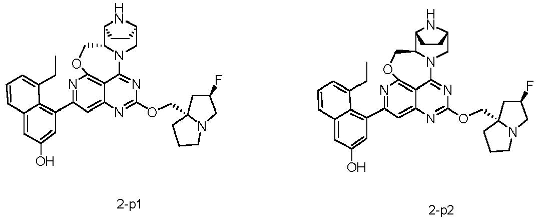

Using the synthetic route in Example 1, the starting material 2-(8-fluoro-3-(methoxymethoxy)naphthalen-1-yl)-4,4,5,5-tetramethyl-1,3,2-dioxaborola ne of step 14 was replaced with 2-(8-ethyl-3-(methoxymethoxy)naphthalen-1-yl)-4,4, 5, 5-tetramethyl-1,3,2-dioxaborolan e (prepared using the method disclosed in intermediate 21 on page 111 of the specification in Patent Application "WO2021/041671") to give the diastereomeric (1:1) mixture of title compounds **2-p1** and **2-p2** (0.81 mg, yield: 7.18%).

MS m/z (ESI): 597.2 [M+1].

¹H NMR (500 MHz, CD₃OD): δ 7.60 (d, 1H), 7.36 (d, 1H), 7.26 - 7.19 (m, 2H), 7.17 (d, 1H), 7.05 (s, 1H), 5.36 (t, 1H), 4.47 (d, 2H), 4.33 - 4.10 (m, SH), 3.76 - 3.68 (m, 4H), 3.68 (s, 2H), 3.19 (t, 2H), 2.80 (t, 1H), 2.56 - 2.24 (m, 4H), 1.99 - 1.79 (m, SH), 1.63 (t, 3H).

### Example 3

### 5-Fluoro-4-((5aS,6S,9R)-1-fluoro-12-(((2R,7aS)-2-fluorotetrahydro-1H-pyrrolizin-7a(5 H)-yl)methoxy)-5a,6,7,8,9,10-hexahydro-5H-4-oxa-3,10a,11,13,14-pentaaza-6,9-metha nonaphtho[1,8-ab]heptalen-2-yl)naphthalen-2-ol 3-p1

### 5-Fluoro-4-((5aR,6R,9S)-1-fluoro-12-(((2R,7aS)-2-fluorotetrahydro-1H-pyrrolizin-7a(5 H)-yl)methoxy)-5a,6,7,8,9,10-hexahydro-5H-4-oxa-3,10a,11,13,14-pentaaza-6,9-metha nonaphtho[1,8-ab]heptalen-2-yl)naphthalen-2-ol 3-p2

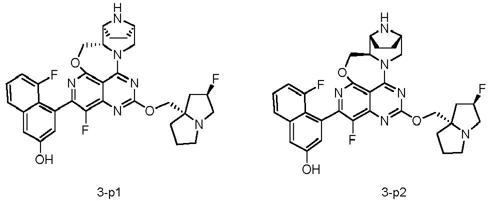

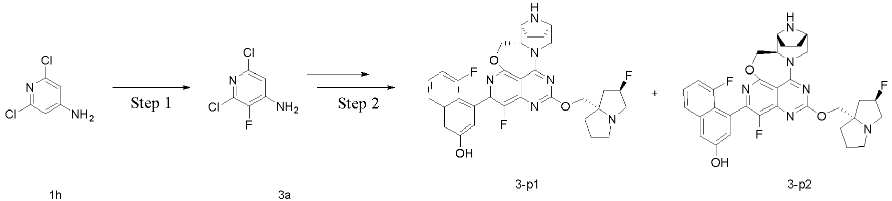

### Step 1

### 2,6-dichloro-3-fluoropyridin-4-amine 3a

Compound **1h** (5 g, 30.6 mmol) was dissolved in 20 mL of *N,N*-dimethylformamide and 20 mL of acetonitrile, the reaction solution was added with 1-chloromethyl-4-fluoro-1,4-diazabicyclo[2.2.2]octane bis(tetrafluoroborate) (13 g, 36.8 mmol), reacted at 80 °C for 0.5 h, and concentrated under reduced pressure, and the residue was purified by silica gel column chromatography with eluent system B to give title compound **3a** (2.2 g, yield: 39.6%).

MS m/z (ESI): 180.9 [M+1].

### Step 2

### 5-Fluoro-4-((5aS,6S,9R)-1-fluoro-12-(((2R,7aS)-2-fluorotetrahydro-1H-pyrrolizin-7a(5 H)-yl)methoxy)-5a,6,7,8,9,10-hexahydro-5H-4-oxa-3,10a, 11, 13, 14-pentaaza-6,9-metha nonaphtho[1,8-ab]heptalen-2-yl)naphthalen-2-ol 3-p1

### 5-Fluoro-4-((5aR,6R,9S)-1-fluoro-12-(((2R,7aS)-2-fluorotetrahydro-1H-pyrrolizin-7a(5 H)-yl)methoxy)-5a,6,7,8,9,10-hexahydro-5H-4-oxa-3,10a,11,13,14-pentaaza-6,9-metha nonaphtho[1,8-ab]heptalen-2-yl)naphthalen-2-ol 3-p2

Using the synthetic route in Example 1, the starting material compound **1h** of step 7 was replaced with compound **3a** to give the diastereomeric (1:1) mixture of title compounds **3-p1** and **3-p2** (20 mg, yield: 32.1%).

MS m/z (ESI): 605.2 [M+1].

¹H NMR (500 MHz, CD₃OD): δ 7.57 (dd, 1H), 7.41 - 7.34 (m, 1H), 7.30 (d, 1H), 7.18 (d, 1H), 7.08 (d, 1H), 5.38 (s, 1H), 5.27 (s, 1H), 4.59 (d, 4H), 4.15 (d, 1H), 3.73 (s, 1H), 3.64 (d, 1H), 3.46 (q, 1H), 3.05 (br, 1H), 2.37 (d, 1H), 2.28 (s, 1H), 2.20 (dd, 2H), 2.05 - 2.00 (m, 2H), 1.95 - 1.83 (m, 3H), 1.60 (s, 1H), 1.15 - 1.10 (m, 3H).

The diastereomeric mixture of compounds **3-p1** and **3-p2** was subjected to chiral resolution (Shimadzu LC-20AP, chromatography column: DAICEL CHIRALPAK^{®}IC, 25 × 250 mm, 10 µm; mobile phase A: n-hexane, mobile phase B: ethanol), gradient ratio: A:B: 30:70, flow rate: 30 mL/min) to give title compounds **3-p1** (6 mg, yield: 9.6%) and **3-p2** (5 mg, yield: 8.0%).

Single configuration compound (shorter retention time) **3-p2:** (5 mg, yield: 8.0%).

MS m/z (ESI): 605.2 [M+1].

Chiral HPLC analysis: retention time: 8.52 min, purity: 99% (chromatography column: DAICEL CHIRALPAK^{®}IC, 250 × 4.6 mm, 5 µm; mobile phase: n-hexane and ethanol (containing 0.2% diethylamine), flow rate: 1.0 mL/min).

¹H NMR (500 MHz, CD₃OD): δ 7.57 (dd, 1H), 7.41 - 7.34 (m, 1H), 7.30 (d, 1H), 7.18 (d, 1H), 7.08 (d, 1H), 5.38 (s, 1H), 5.27 (s, 1H), 4.59 (d, 4H), 4.15 (d, 1H), 3.73 (s, 1H), 3.64 (d, 1H), 3.46 (q, 1H), 3.05 (br, 1H), 2.37 (d, 1H), 2.28 (s, 1H), 2.20 (dd, 2H), 2.05 - 2.00 (m, 2H), 1.95 - 1.83 (m, 3H), 1.60 (s, 1H), 1.15 - 1.10 (m, 3H).

Single configuration compound (longer retention time) **3-p1**: (6 mg, yield: 9.6%).

MS m/z (ESI): 605.2 [M+1].

Chiral HPLC analysis: retention time: 11.45 min, purity: 99% (chromatography column: DAICEL CHIRALPAK^{®}IC, 250 × 4.6 mm, 5 µm; mobile phase: n-hexane and ethanol (containing 0.2% diethylamine), flow rate: 1.0 mL/min).

¹H NMR (500 MHz, CD₃OD): δ 7.56 (dd, 1H), 7.41 - 7.34 (m, 1H), 7.30 (d, 1H), 7.18 - 7.08 (d, 1H), 6.99 - 6.87 (m, 1H), 5.38 (s, 1H), 5.27 (s, 1H), 4.59 (d, 4H), 4.15 (d, 1H), 3.73 (s, 1H), 3.64 (d, 1H), 3.46 (q, 1H), 3.05 (br, 1H), 2.37 (d, 1H), 2.28 (s, 1H), 2.20 (dd, 2H), 2.05 - 2.00 (m, 2H), 1.95 - 1.83 (m, 3H), 1.60 (s, 1H), 1.15 - 1.10 (m, 3H).

### Example 4

### 5-Ethyl-4-((5aS,6S,9R)-1-fluoro-12-(((2R,7aS)-2-fluorotetrahydro-1H-pyrrolizin-7a(5H )-yl)methoxy)-5a,6,7,8,9,10-hexahydro-5H-4-oxa-3,10a,11,13,14-pentaaza-6,9-methano naphtho[1,8-ab]heptalen-2-yl)naphthalen-2-ol 4-p1

### 5-Ethyl-4-((5aR,6R,9S)-1-fluoro-12-(((2R,7aS)-2-fluorotetrahydro-1H-pyrrolizin-7a(5H )-yl)methoxy)-5a,6,7,8,9,10-hexahydro-5H-4-oxa-3,10a,11,13,14-pentaaza-6,9-methano naphtho[1,8-ab]heptalen-2-yl)naphthalen-2-ol 4-p2

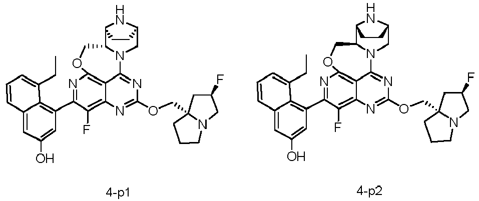

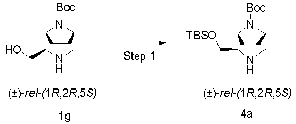

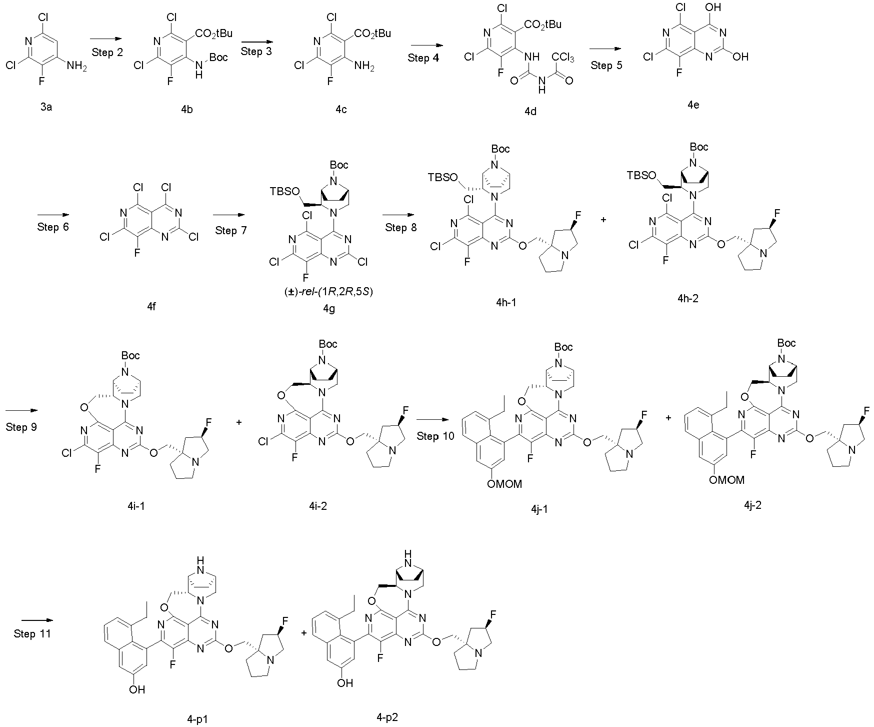

### Step 1

### Tert-butyl

### (±)-rel-(1R,2R,5S)-2-(((tert-butyldimethylsilyl)oxy)methyl)-3,8-diazabicyclo[3.2.1]octa ne-8-carboxylate 4a

Compound **1g** (8.8 g, 36.3 mmol), *tert*-butyldimethylsilyl chloride (16 g, 106.1558 mmol) and 4-dimethylaminopyridine (4 g, 32.4739 mmol) were dissolved in 200 mL of dichloromethane, the reaction solution was added with triethylamine (15 g, 148.23 mmol, 21.4286 mL), stirred for 16 h, and concentrated under reduced pressure, and the residue was purified by silica gel column chromatography with eluent system B to give title compound **4a** (8 g, yield: 61.7%).

MS m/z (ESI): 357.1 [M+1].

### Step 2

### Tert-butyl 4-((tert-butoxycarbonyl)amino)-2,6-dichloro-5-fluoronicotinate 4b

Compound **3a** (1.8 g, 9.94 mmol) was dissolved in tetrahydrofuran (50 mL), the reaction solution was added with 20 mL of 2 M sodium bis(trimethylsilyl)amide in tetrahydrofuran under an ice bath, stirred for 0.5 h, added with di-tert-butyl dicarbonate (6.5 g, 29.7 mmol), stirred for 14 h, then added with saturated aqueous ammonium chloride for quenching the reaction, and extracted with ethyl acetate (50 mL × 3). The organic phases were combined, dried over anhydrous sodium sulfate, and filtered to remove the drying agent, the filtrate was concentrated under reduced pressure, and the residue was purified with eluent system B to give title compound **4b** (1 g, yield: 26.3%), which was directly used in the next reaction without purification.

MS m/z (ESI): 381.1 [M+1].

### Step 3

### Tert-butyl 4-amino-2,6-dichloro-5-fluoronicotinate 4c

Compound **4b** (1 g, 2.62 mmol) was dissolved in ethyl acetate (8 mL), and the reaction solution was added with 3 mL of 4 M hydrochloride in dioxane, stirred for 2 h, adjusted the pH to neutral with 4 M aqueous sodium hydroxide solution under an ice bath, and extracted with ethyl acetate (10 mL × 3). The organic phases were combined, dried over anhydrous sodium sulfate, and filtered to remove the drying agent, the filtrate was concentrated under reduced pressure, and the residue was purified with eluent system B to give crude title compound **4c** (500 mg, yield: 67.8%).

MS m/z (ESI): 281.1 [M+1].

### Step 4

### Tert-butyl 2,6-dichloro-5-fluoro-4-(3-(2,2,2-trichloroacetyl)ureido)nicotinate 4d

Crude compound **4c** (500 mg, 1.77 mmol) was dissolved in tetrahydrofuran (10 mL), and the reaction solution was added with trichloroacetylisocyanate (670 mg, 3.55 mmol), stirred for 30 min, and concentrated under reduced pressure to give crude title compound **4d** (835 mg, yield: 99.7%), which was directly used in the next reaction without purification.

MS m/z (ESI): 467.9 [M+1].

### Step 5

### 5,7-dichloro-8-fluoro-pyrido[4,3-d]pyrimidine-2,4-diol 4e

Crude compound **4d** (835 mg, 1.77 mmol) was dissolved in 7 M methanolic ammonia (10 mL), the reaction solution was stirred for 1 h, and concentrated under reduced pressure, the residue was added with methyl *tert*-butyl ether (10 mL), stirred for 0.5 h and filtered, and the filter cake was dried to give crude title compound **4e** (400 mg, yield: 89.9%), which was directly used in the next reaction without purification.

MS m/z (ESI): 249.9 [M+1].

### Step 6

### 2,4,5,7-tetrachloro-8-fluoro-pyrido[4,3-d]pyrimidine 4f

Crude compound **4e** (300 mg, 1.19 mmol) was dissolved in phosphorus oxychloride (6 mL), the reaction solution was added with *N,N-*diisopropylethylamine (800 mg, 6.19 mmol), stirred at 110 °C for 3 h, cooled to room temperature and concentrated under reduced pressure to give crude title compound **4f** (344 mg, yield: 97.7%), which was directly used in the next step without purification.

MS m/z (ESI): 285.8 [M+1].

### Step 7

### Tert-butyl (±)-rel-(1R,2R,5S)-2-(((tert-butyldimethylsilyl)oxy)methyl)-3-(2,5,7-trichloro-8-fluorop yrido[4,3-d]pyrimidin-4-yl)-3,8-diazabicyclo[3.2.1]octane-8-carboxylate 4g

Compound **4f** (1.0 g, 3.48 mmol) and N,N-diisopropylethylamine (0.9 g, 6.9 mmol) were dissolved in 15 mL of dichloromethane, the reaction solution was added with **4a** (1.25 g, 3.5 mmol) at -78 °C, stirred for 1 h while maintaining the temperature and then allowed to return to room temperature and reacted for 16 h, and concentrated under reduced pressure, and the residue was purified with eluent system B to give crude title compound **4g** (1.56 g, yield: 73.7%).

MS m/z (ESI): 606.2 [M+1].

### Step 8

### Diastereomeric mixture of tert-butyl (1S,2S,5R)-2-(((tert-butyldimethylsilyl)oxy)methyl)-3-(5,7-dichloro-8-fluoro-2-(((2R,7a S)-2-fluorotetrahydro-1H-pyrrolizin-7a(5H)-yl)methoxy)pyrido[4,3-d]pyrimidin-4-yl)-3 ,8-diazabicyclo[3.2.1]octane-8-carboxylate 4h-1 and

### tert-butyl (1R,2R,5S)-2-(((tert-butyldimethylsilyl)oxy)methyl)-3-(5,7-dichloro-8-fluoro-2-(((2R,7 aS)-2-fluorotetrahydro-1H-pyrrolizin-7a(5H)-yl)methoxy)pyrido[4,3-d]pyrimidin-4-yl)-3,8-diazabicyclo[3.2.1]octane-8-carboxylate 4h-2

Compound **4g** (1.4 g, 2.3 mmol) was dissolved in 1,4-dioxane (20 mL), the reaction solution was added with ((2*R*,7*aS*)-2-fluorotetrahydro-1*H*-pyrrolizin-7*a*(5*H*)-yl)methanol (650 mg, 4.08 mmol, WuXi AppTec), *N,N*-diisopropylethylamine (1.5 g, 11.6 mmol) and 4A molecular sieves (1.4 g), stirred at 105 °C for 6 h, cooled to room temperature and filtered, and concentrated under reduced pressure to give the diastereomeric mixture of crude title compounds **4h-1** and **4h-2** (1.68 g, yield: 99.8%), which was directly used in the next step without purification.

MS m/z (ESI): 729.2 [M+1].

### Step 9

### Diastereomeric mixture of tert-butyl (5aS,6S,9R)-2-chloro-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a, 11, 13, 14-pentaaza-6,9-methanon aphtho[1,8-ab]heptalen-14-carboxylate 4i-1 and

### tert-butyl (5aR,6R,9S)-2-chloro-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanon aphtho[1,8-ab]heptalen-14-carboxylate 4i-2

The diastereomeric mixture of crude compounds **4h-1** and **4h-2** (1.68 g, 2.3mmol) was added into tetrabutylammonium fluoride (2.59 g, 11.51 mmol), the reaction solution was stirred at room temperature for 16 h, and concentrated under reduced pressure, and the residue was purified with eluent system B to give the diastereomeric mixture of title compounds **4i-1** and **4i-2** (1.0 g, yield: 75.0%).

MS m/z (ESI): 579.2 [M+1].

### Step 10

### Diastereomeric (1:1) mixture of tert-butyl (5aS,6S,9R)-2-(8-ethyl-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa -3,10a,11,13,14-pentaaza-6,9-methanonaphtho[1,8-ab]heptalen-14-carboxylate 4j-1 and

### tert-butyl (5aR,6R,9S)-2-(8-ethyl-3-(methoxymethoxy)naphthalen-1-yl)-1-fluoro-12-(((2R,7aS)-2-fluorotetrahydro-1H-pyrrolizin-7a(5H)-yl)methoxy)-5a,6,7,8,9,10-hexahydro-5H-4-oxa -3,10a,11,13,14-pentaaza-6,9-methanonaphtho[1,8-ab]heptalen-14-carboxylate 4j-2

The diastereomeric mixture of compounds **4i-1** and **4i-2** (1.9 g, 3.28 mmol), 2-(8-ethyl-3-(methoxymethoxy)naphthalen-1-yl)-4,4, 5, 5-tetramethyl-1,3,2-dioxaborolan e (1.5 g, 4.38 mmol, prepared using the method disclosed in intermediate 21 on page 111 of the specification in Patent Application "WO2021/041671"), tetrakis(triphenylphosphine)palladium (1.16 g, 1 mmol, adamas) and cesium carbonate (4.7 g, 14.4 mmol) were dissolved in 36 mL of a mixed solution of 1,4-dioxane and water (V:V = 5:1). The reaction solution was reacted at 100 °C for 6 h under nitrogen atmosphere, and concentrated under reduced pressure to give a diastereomeric (1:1) mixture of crude title compounds **4j-1** and **4j-2** (2.5 g, yield: 100%).

MS m/z (ESI): 759.2 [M+1].

### Step 11

### 5-Ethyl-4-((5aS,6S,9R)-1-fluoro-12-(((2R,7aS)-2-fluorotetrahydro-1H-pyrrolizin-7a(5H )-yl)methoxy)-5a,6,7,8,9,10-hexahydro-5H-4-oxa-3,10a,11,13,14-pentaaza-6,9-methano naphtho[1,8-ab]heptalen-2-yl)naphthalen-2-ol 4-p1

### 5-Ethyl-4-((5aR,6R,9S)-1-fluoro-12-(((2R,7aS)-2-fluorotetrahydro-1H-pyrrolizin-7a(5H )-yl)methoxy)-5a,6,7,8,9,10-hexahydro-5H-4-oxa-3,10a,11,13,14-pentaaza-6,9-methano naphtho[1,8-ab]heptalen-2-yl)naphthalen-2-ol 4-p2

The diastereomeric mixture of crude compounds **4j-1** and **4j-2** (2.4 g, 3.16 mol) was dissolved in ethyl acetate (40 mL), the reaction solution was added with 17 mL of 4 M hydrochloride in dioxane, reacted at 0 °C for 2 h, and concentrated under reduced pressure, and the residue was purified by high performance liquid chromatography (Waters-2545, chromatography column: SharpSil-T C18, 30 × 150 mm, 5 µm; mobile phase: aqueous phase (10 mmol/L ammonium bicarbonate) and acetonitrile, gradient ratio: acetonitrile 38%-45%, flow rate: 30 mL/min) to give the diastereomeric (1:1) mixture of title compounds **4-p1** and **4-p2** (620 mg, yield: 32.6%).

MS m/z (ESI): 615.2 [M+1].

¹H NMR (500 MHz, CD₃OD): δ 7.63 (d, 1H), 7.37 (q, 1H), 7.29 (q, 1H), 7.18 (dd, 1H), 7.08 (t, 1H), 5.48 - 5.29 (m, 2H), 4.55 - 4.28 (m, 4H), 4.17 (dd, 1H), 3.84 - 3.75 (m, 1H), 3.70 (t, 1H), 3.47 (q, 1H), 3.31 - 3.07 (m, 3H), 2.57 - 2.17 (m, 5H), 2.15 - 1.79 (m, 7H), 1.63 (t, 1H), 1.08 - 0.87 (m, 3H).

The diastereomeric mixture of compounds **4-p1** and **4-p2** was subjected to chiral resolution (Shimadzu LC-20AP, chromatography column: DAICEL CHIRALPAK^{®}IC, 25 × 250 mm, 10 µm; mobile phase A: n-hexane, mobile phase B: ethanol (0.1% 7 M NH₃ in MeOH)), gradient ratio: A:B: 30:70, flow rate: 30 mL/min) to give title compounds **4-p1** (50 mg, yield: 35.7%) and **4-p2** (50 mg, yield: 35.7%).

Single configuration compound (shorter retention time) **4-p2:** (50 mg, yield: 35.7%). MS m/z (ESI): 615.2 [M+1].

Chiral HPLC analysis: retention time: 9.85 min, purity: 99% (chromatography column: DAICEL CHIRALPAK^{®}IC, 250 × 4.6 mm, 5 µm; mobile phase: n-hexane and ethanol (containing 0.2% diethylamine), flow rate: 1.0 mL/min).

¹H NMR (500 MHz, CD₃OD): δ 7.61 - 7.59 (m, 1H), 7.36 - 7.32 (m, 1H), 7.26 - 7.25 (m, 1H), 7.18 - 7.13 (m 1H), 7.05 - 6.95 (m, 1H), 5.37 - 5.22 (m, 2H), 5.09 - 5.00 (m, 1H), 4.61 - 4.56 (m, 1H), 4.49 - 4.41 (m, 1H), 4.30 (dd, 1H), 4.24 - 4.18 (m, 1H), 4.16 - 4.09 (m, 1H), 3.72 (dd, 1H), 3.62 (dd, 1H), 3.27 - 3.17 (m, 3H), 3.01 (td, 1H), 2.48 (dt, 1H), 2.40 - 2.10 (m, 5H), 1.99 (td, 2H), 1.94 - 1.75 (m, 4H), 0.99 - 0.88 m, 3H).

Single configuration compound (longer retention time) **4-p1**: (50 mg, yield: 35.7%). MS m/z (ESI): 615.2 [M+1].

Chiral HPLC analysis: retention time: 16.0 min, purity: 99% (chromatography column: DAICEL CHIRALPAK^{®}IC, 250 × 4.6 mm, 5 µm; mobile phase: *n*-hexane and ethanol (containing 0.2% diethylamine), flow rate: 1.0 mL/min).

¹H NMR (500 MHz, CD₃OD): δ 7.61 - 7.59 (m, 1H), 7.36 - 7.32 (m, 1H), 7.26 - 7.25 (m, 1H), 7.18 - 7.13 (m 1H), 7.05 - 6.94 (m, 1H), 5.36 - 5.33 (m, 2H), 5.10 - 5.01 (m, 1H), 4.61 - 4.56 (m, 1H), 4.49 - 4.41 (m, 1H), 4.30 (dd, 1H), 4.24 - 4.22 (m, 1H), 4.16 - 4.10 (m, 1H), 3.73 - 3.72 (m, 1H), 3.64 - 3.61 (m, 1H), 3.26 - 3.20 (m, 3H), 3.04 - 2.99 (m, 1H), 2.48 (dt, 1H), 2.38 - 2.17 (m, 5H), 2.03 - 1.96 (m, 2H), 1.93 - 1.78 (m, 4H), 0.99 - 0.88 m, 3H).

### Example 5

### Diastereomeric (1:1) mixture of 5-ethyl-4-((5aS,6S,9R)-1-fluoro-12-(((2R,7aR)-2-fluorotetrahydro-1H-pyrrolizin-7a(5H )-yl)methoxy)-5a,6,7,8,9,10-hexahydro-5H-4-oxa-3,10a,11,13,14-pentaaza-6,9-methano naphtho[1,8-ab]heptalen-2-yl)naphthalen-2-ol 5-p1 and

### 5-ethyl-4-((5aR,6R,9S)-1-fluoro-12-(((2R,7aR)-2-fluorotetrahydro-1H-pyrrolizin-7a(5H )-yl)methoxy)-5a,6,7,8,9,10-hexahydro-5H-4-oxa-3,10a,11,13,14-pentaaza-6,9-methano naphtho[1,8-ab]heptalen-2-yl)naphthalen-2-ol 5-p2

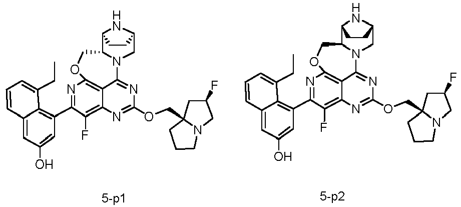

Using the synthetic route from step 8 to step 11 in Example 4, the starting material ((2*R*,7*aS*)-2-fluorotetrahydro-1*H*-pyrrolizin-7*a*(5*H*)-yl)methanol of step 8 was replaced with ((2*R*,7*aR*)-2-fluorotetrahydro-1*H*-pyrrolizin-7*a*(5*H*)-yl)methanol (prepared using the method disclosed in intermediate B-21 on page 132 of the specification in Patent Application "WO2020/146613") to give the diastereomeric (1:1) mixture of title compounds **5-p1** and **5-p2.**

MS m/z (ESI): 615.2 [M+1].

### Example 6

### 3-Chloro-4-cyclopropyl-5-((5aS,6S,9R)-1-fluoro-12-(((2R,7aS)-2-fluorotetrahydro-1H-p yrrolizin-7a(5H)-yl)methoxy)-5a,6,7,8,9,10-hexahydro-5H-4-oxa-3,10a,11,13,14-penta aza-6,9-methanonaphtho[1,8-ab]heptalen-2-yl)phenol 6-p1

### 3-Chloro-4-cyclopropyl-5-((5aR,6R,9S)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pent aaza-6,9-methanonaphtho[1,8-ab]heptalen-2-yl)phenol 6-p2

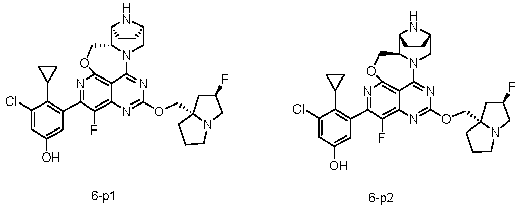

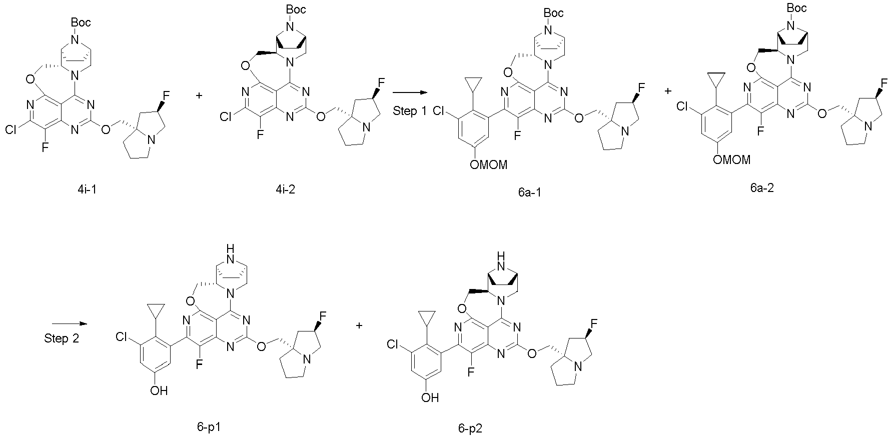

### Step 1

### Diastereomeric mixture of tert-butyl (SaS,6S,9R)-2-(3-chloro-2-cyclopropyl-5-(methoxymethoxy)phenyl)-1-fluoro-12-(((2R, 7aS)-2-fluorotetrahydro-1H-pyrrolizin-7a(5H)-yl)methoxy)-5a,6,7,8,9,10-hexahydro-5 H-4-oxa-3,10a,11,13,14-pentaaza-6,9-methanonaphtho[1,8-ab]heptalen-14-carboxylate 6a-1 and

### tert-butyl (5aR,6R,9S)-2-(3-chloro-2-cyclopropyl-5-(methoxymethoxy)phenyl)-1-fluoro-12-(((2R, 7aS)-2-fluorotetrahydro-1H-pyrrolizin-7a(5H)-yl)methoxy)-5a,6,7,8,9,10-hexahydro-5 H-4-oxa-3,10a,11,13,14-pentaaza-6,9-methanonaphtho[1,8-ab]heptalen-14-carboxylate 6a-2

The diastereomeric (1:1) mixture of compounds **4i-1** and **4i-2** (190 mg, 328 µmol), 2-(3-chloro-2-cyclopropyl-5-(methoxymethoxy)phenyl)-4,4,5,5-tetramethyl-1,3,2-dioxa borolane (144.4 mg, 426 µmol, prepared using the method disclosed in Example 283 on page 526 of the specification in Patent Application "WO2021/041671"), tetrakis(triphenylphosphine)palladium (75.8 mg, 65.6 µmol, adamas) and cesium carbonate (320.7 mg, 984 µmol) were dissolved in 10 mL of a mixed solution of 1,4-dioxane and water (V:V = 5:1). The reaction solution was reacted at 100 °C for 6 h under nitrogen atmosphere, and concentrated under reduced pressure to give a diastereomeric (1:1) mixture of crude title compounds **6a-1** and **6a-2** (240 mg, yield: 97.1%).

MS m/z (ESI): 755.2 [M+1].

### Step 2

### 3-Chloro-4-cyclopropyl-5-((5aS,6S,9R)-1-fluoro-12-(((2R,7aS)-2-fluorotetrahydro-1H-p yrrolizin-7a(5H)-yl)methoxy)-5a,6,7,8,9,10-hexahydro-5H-4-oxa-3,10a,11,13,14-penta aza-6,9-methanonaphtho[1,8-ab]heptalen-2-yl)phenol 6-p1

### 3-Chloro-4-cyclopropyl-5-((5aR,6R,9S)-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pent aaza-6,9-methanonaphtho[1,8-ab]heptalen-2-yl)phenol 6-p2

The diastereomeric (1:1) mixture of crude compounds **6a-1** and **6a-2** (240 mg, 327 µmol) was dissolved in acetonitrile (5 mL), and the reaction solution was added with 2 mL of 4 M hydrochloride in dioxane, stirred for 2 h, and concentrated under reduced pressure, and the residue was purified by high performance liquid chromatography (Waters-2545, chromatography column: SharpSil-T C18, 30 × 150 mm, 5 µm; mobile phase: aqueous phase (10 mmol/L ammonium bicarbonate) and acetonitrile, gradient ratio: acetonitrile 38%-45%, flow rate: 30 mL/min) to give the diastereomeric (1:1) mixture of title compounds **6-p1** and **6-p2** (75 mg, yield: 37.6%).

MS m/z (ESI): 611.2 [M+1].

¹H NMR (500 MHz, CD₃OD): δ 6.96 (dd, 1H), 6.77 (d, 1H), 5.38 (s, 1H), 5.28 (s, 1H), 5.06 (d, 1H), 4.61 (d, 1H), 4.45 (dd, 1H), 4.31 (dd, 1H), 4.24 (dd, 1H), 4.14 (d, 1H), 3.74 (d, 1H), 3.65 (s, 1H), 3.24 (dd, 5H), 3.08 - 3.01 (m, 1H), 2.41 - 2.32 (m, 1H), 2.32 - 2.19 (m, 2H), 2.16 (d, 1H), 2.02 (m, 3H), 1.92 (dd, 4H), 1.86 - 1.82 (m, 2H).

The diastereomeric mixture of compounds **6-p1** and **6-p2** was subjected to chiral resolution (Shimadzu LC-20AP, chromatography column: DAICEL CHIRALPAK^{®}IC, 25 × 250 mm, 10 µm; mobile phase A: n-hexane, mobile phase B: ethanol (0.1% 7 M NH₃ in MeOH)), gradient ratio: A:B: 30:70, flow rate: 30 mL/min) to give title compounds **6-p1** (26 mg, yield: 13%) and **6-p2** (32 mg, yield: 16%).

Single configuration compound (shorter retention time) **6-p2:** (32 mg, yield: 16%).

MS m/z (ESI): 611.2 [M+1].

Chiral HPLC analysis: retention time: 5.74 min, purity: 99% (chromatography column: DAICEL CHIRALPAK^{®}IC, 250 × 4.6 mm, 5 µm; mobile phase: n-hexane and ethanol (containing 0.2% diethylamine), flow rate: 1.0 mL/min).

¹H NMR (500 MHz, CD₃OD): δ 6.96 (d, 1H), 6.77 (d, 1H), 5.39 (d, 1H), 5.28 (s, 1H), 5.06 (dd, 1H), 4.61 (dd, 1H), 4.45 (dd, 1H), 4.32 (d, 1H), 4.23 (d, 1H), 4.14 (d, 1H), 3.74 (dd, 1H), 3.68 - 3.63 (m, 1H), 3.25 (tq, 5H), 3.05 (td, 1H), 2.40 - 2.11 (m, 4H), 2.07 - 1.74 (m, 9H).

Single configuration compound (longer retention time) **6-p1**: (26 mg, yield: 13%).

MS m/z (ESI): 611.2 [M+1].

Chiral HPLC analysis: retention time: 9.9 min, purity: 99% (chromatography column: DAICEL CHIRALPAK^{®}IC, 250 × 4.6 mm, 5 µm; mobile phase: n-hexane and ethanol (containing 0.2% diethylamine), flow rate: 1.0 mL/min).

¹H NMR (500 MHz, CD₃OD): δ 6.96 (d, 1H), 6.77 (d, 1H), 5.39 (s, 1H), 5.28 (s, 1H), 5.07 (dd, 1H), 4.63 - 4.59 (m, 1H), 4.45 (dd, 1H), 4.31 (d, 1H), 4.25 (d, 1H), 4.15 (dd, 1H), 3.74 (d, 1H), 3.66 (dd, 1H), 3.32 - 3.17 (m, 5H), 3.05 (dt, 1H), 2.40 - 2.12 (m, 4H), 2.08 - 1.76 (m, 9H).

### Example 7

### Diastereomeric (1:1) mixture of 5-ethyl-4-((5aS,6S,9R)-1-fluoro-12-((tetrahydro-1H-pyrrolizin-7a(5H)-yl)methoxy)-5a, 6,7,8,9,10-hexahydro-5H-4-oxa-3,10a,11,13,14-pentaaza-6,9-methanonaphtho[1,8-ab]h eptalen-2-yl)naphthalen-2-ol 7-p1 and

### 5-ethyl-4-((5aR,6R,9S)-1-fluoro-12-((tetrahydro-1H-pyrrolizin-7a(5H)-yl)methoxy)-5a, 6,7,8,9,10-hexahydro-5H-4-oxa-3,10a,11,13,14-pentaaza-6,9-methanonaphtho[1,8-ab]h eptalen-2-yl)naphthalen-2-ol 7-p2

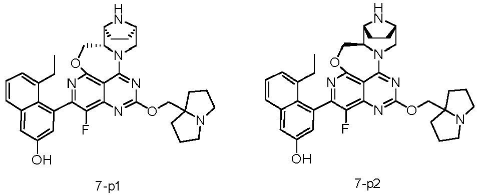

Using the synthetic route from step 8 to step 11 in Example 4, the starting material ((2*R*,7*aS*)-2-fluorotetrahydro-1*H*-pyrrolizin-7*a*(5*H*)-yl)methanol of step 8 was replaced with (hexahydro-1*H*-pyrrolizin-7*a*-yl)methanol (WuXi AppTec) to give the diastereomeric mixture of title compounds **7-p1** and **7-p2.**

MS m/z (ESI): 597.2 [M+1].

### Example 8

### 5,6-Difluoro-4-((5aS,6S,9R)-1-fluoro-12-(((2R,7aS)-2-fluorotetrahydro-1H-pyrrolizin-7 a(5H)-yl)methoxy)-5a,6,7,8,9,10-hexahydro-5H-4-oxa-3,10a,11,13,14-pentaaza-6,9-me thanonaphtho[1,8-ab]heptalen-2-yl)naphthalen-2-ol 8-p1

### 5,6-Difluoro-4-((5aR,6R,9S)-1-fluoro-12-(((2R,7aS)-2-fluorotetrahydro-1H-pyrrolizin-7 a(5H)-yl)methoxy)-5a,6,7,8,9,10-hexahydro-5H-4-oxa-3,10a,11,13,14-pentaaza-6,9-me thanonaphtho[1,8-ab]heptalen-2-yl)naphthalen-2-ol 8-p2

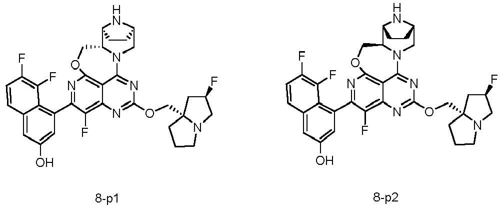

Using the synthetic route from step 10 to step 11 in Example 4, the starting material 2-(8-ethyl-3-(methoxymethoxy)naphthalen-1-yl)-4,4,5,5-tetramethyl-1,3,2-dioxaborolan e of step 10 was replaced with 2-(7,8-difluoro-3-(methoxymethoxy)naphthalen-1-yl)-4,4,5,5-tetramethyl-1,3,2-dioxabo rolane (prepared using the method disclosed in Example 246 on page 437 of the specification in Patent Application "WO2021/041671") to give the diastereomeric (1:1) mixture of title compounds **8-p1** and **8-p2** (40 mg, yield: 37.9%).

MS m/z (ESI): 623.2 [M+1].

¹H NMR (500 MHz, CD₃OD): δ 7.56 (dd, 1H), 7.41 - 7.34 (m, 1H), 7.30 (d, 1H), 7.18 - 7.08 (d, 1H), 5.38 (s, 1H), 5.27 (s, 1H), 4.59 (d, 4H), 4.15 (d, 1H), 3.73 (s, 1H), 3.64 (d, 1H), 3.46 (q, 1H), 3.05 (br, 1H), 2.37 (d, 1H), 2.28 (s, 1H), 2.20 (dd, 2H), 2.05 - 2.00 (m, 2H), 1.95 - 1.83 (m, 3H), 1.60 (s, 1H), 1.15 - 1.10 (m, 3H).

The diastereomeric mixture of compounds **8-p1** and **8-p2** was subjected to chiral resolution (Shimadzu LC-20AP, chromatography column: DAICEL CHIRALPAK^{®}IC, 25 × 250 mm, 10 µm; mobile phase A: *n*-hexane, mobile phase B: ethanol (0.1% 7 M NH₃ in MeOH)), gradient ratio: A:B: 40:60, flow rate: 30 mL/min) to give title compounds **8-p1** (16 mg, yield: 15.1%) and **8-p2** (19 mg, yield: 17.9%).

Single configuration compound (shorter retention time) **8-p2:** (19 mg, yield: 17.9%). MS m/z (ESI): 623.2 [M+1].

Chiral HPLC analysis: retention time: 6.95 min, purity: 99% (chromatography column: DAICEL CHIRALPAK^{®}IC, 250 × 4.6 mm, 5 µm; mobile phase: n-hexane and ethanol (containing 0.2% diethylamine), flow rate: 1.0 mL/min).

¹H NMR (500 MHz, CD₃OD): δ 7.56 (dd, 1H), 7.41 - 7.34 (m, 1H), 7.30 (d, 1H), 7.18 - 7.08 (d, 1H), 5.38 (s, 1H), 5.27 (s, 1H), 4.59 (d, 4H), 4.15 (d, 1H), 3.73 (s, 1H), 3.64 (d, 1H), 3.46 (q, 1H), 3.05 (br, 1H), 2.37 (d, 1H), 2.28 (s, 1H), 2.20 (dd, 2H), 2.05 - 2.00 (m, 2H), 1.95 - 1.83 (m, 3H), 1.60 (s, 1H), 1.15 - 1.10 (m, 3H).

Single configuration compound (longer retention time) **8-p1**: (16 mg, yield: 17.9%). MS m/z (ESI): 623.2 [M+1].

Chiral HPLC analysis: retention time: 9.86 min, purity: 99% (chromatography column: DAICEL CHIRALPAK^{®}IC, 250 × 4.6 mm, 5 µm; mobile phase: n-hexane and ethanol (containing 0.2% diethylamine), flow rate: 1.0 mL/min).

¹H NMR (500 MHz, CD₃OD): δ 7.58 (dd, 1H), 7.41 - 7.34 (m, 1H), 7.30 (d, 1H), 7.18 - 7.08 (d, 1H), 5.39 (s, 1H), 5.27 (s, 1H), 4.59 (d, 4H), 4.15 (d, 1H), 3.73 (s, 1H), 3.64 (d, 1H), 3.46 (q, 1H), 3.05 (br, 1H), 2.37 (d, 1H), 2.28 (s, 1H), 2.20 (dd, 2H), 2.05 - 2.00 (m, 2H), 1.95 - 1.83 (m, 3H), 1.60 (s, 1H), 1.15 - 1.10 (m, 3H).

### Example 9

### 5-Ethyl-6-fluoro-4-((5aS,6S,9R)-1-fluoro-12-(((2R,7aS)-2-fluorotetrahydro-1H-pyrrolizi n-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol 9-p1

### 5-Ethyl-6-fluoro-4-((5aR,6R,9S)-1-fluoro-12-(((2R,7aS)-2-fluorotetrahydro-1H-pyrroliz in-7a(5H)-yl)methoxy)-5a,6,7,8,9,10-hexahydro-5H-4-oxa-3,10a,11,13,14-pentaaza-6,9 -methanonaphtho[1,8-ab]heptalen-2-yl)naphthalen-2-ol 9-p2

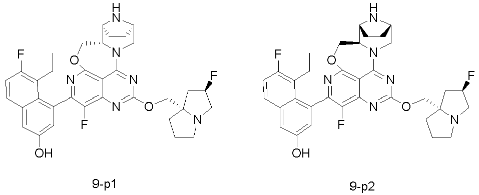

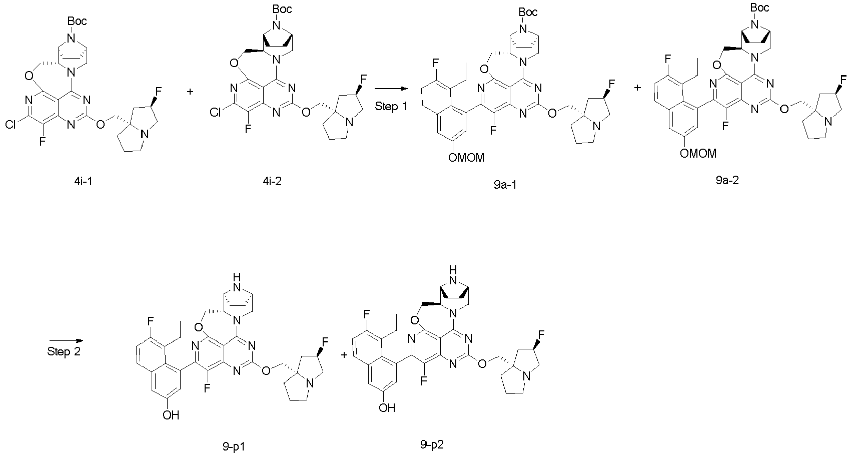

### Step 1

### Diastereomeric (1:1) mixture of tert-butyl (5aS,6S,9R)-2-(8-ethyl-7-fluoro-3-(methoxymethoxy)naphthalen-1-yl)-1-fluoro-12-(((2 R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-14-carboxylat e 9a-1 and

### tert-butyl (5aR,6R,9S)-2-(8-ethyl-7-fluoro-3-(methoxymethoxy)naphthalen-1-yl)-1-fluoro-12-(((2 R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanonaphtho[1,8-ab]heptalen-14-carboxylat e 9a-2

The diastereomeric mixture of compounds **4i-1** and **4i-2** (300 mg, 518.1 µmol), 2-(8-ethyl-7-fluoro-3 -(methoxymethoxy)naphthalen-1-yl)-4,4, 5, 5-tetramethyl-1,3,2-dio xaborolane (280 mg, 777.2 µmol, prepared using the method disclosed in intermediate 18 on page 104 of the specification in Patent Application "WO2021/041671"), tetrakis(triphenylphosphine)palladium (120 mg, 103.8 µmol) and cesium carbonate (506 mg, 1.55 mmol) were dissolved in 6 mL of a mixed solution of 1,4-dioxane and water (V:V = 5:1). The reaction solution was reacted at 100 °C for 6 h under nitrogen atmosphere, and concentrated under reduced pressure to give a diastereomeric (1:1) mixture of title compounds **9a-1** and **9a-2** (400 mg), which was directly used in the next reaction without purification.

MS m/z (ESI): 777.2 [M+1].

### Step 2

### 5-Ethyl-6-fluoro-4-((5aS,6S,9R)-1-fluoro-12-(((2R,7aS)-2-fluorotetrahydro-1H-pyrrolizi n-7a(5H)-yl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol 9-p1

### 5-Ethyl-6-fluoro-4-((5aR,6R,9S)-1-fluoro-12-(((2R,7aS)-2-fluorotetrahydro-1H-pyrroliz in-7a(5H)-yl)methoxy)-5a,6,7,8,9,10-hexahydro-5H-4-oxa-3,10a,11,13,14-pentaaza-6,9 -methanonaphtho[1,8-ab]heptalen-2-yl)naphthalen-2-ol 9-p2

The diastereomeric mixture of crude compounds **9a-1** and **9a-2** (160 mg, 205.9 µmol) was dissolved in ethyl acetate (5 mL), the reaction solution was added with 1 mL of 4 M hydrochloride in dioxane, reacted at 0 °C for 2 h, and concentrated under reduced pressure, and the residue was purified by high performance liquid chromatography (Waters-2545, chromatography column: SharpSil-T C18, 30 × 150 mm, 5 µm; mobile phase: aqueous phase (10 mmol/L ammonium bicarbonate) and acetonitrile, gradient ratio: acetonitrile 38%-45%, flow rate: 30 mL/min) to give the diastereomeric (1:1) mixture of title compounds **9-p1** and **9-p2** (10 mg, yield: 7.2%).

MS m/z (ESI): 633.2 [M+1].

¹H NMR (500 MHz, CD₃OD): δ 7.67 (ddd, 1H), 7.32 - 7.21 (m, 2H), 7.11 - 7.01 (m, 1H), 5.38 - 5.35 (m, 2H), 5.11 - 5.03 (m, 1H), 4.64 - 4.59 (m, 1H), 4.52 - 4.46 (m, 1H), 4.34 - 4.29 (m, 1H), 4.25 (dd, 1H), 4.18 - 4.12 (m, 1H), 3.74 (br, 1H), 3.65 (br, 1H), 3.26 - 3.23 (m, 3H), 3.05 - 3.01 (m, 1H), 2.61 - 1.81 (m, 12H), 0.94 - 0.82 (m, 3H).

The diastereomeric mixture of compounds **9-p1** and **9-p2** was subjected to chiral resolution (Shimadzu LC-20AP, chromatography column: DAICEL CHIRALPAK^{®}IC, 25 × 250 mm, 10 µm; mobile phase A: *n*-hexane, mobile phase B: ethanol (0.1% 7 M NH₃ in MeOH)), gradient ratio: A:B: 40:60, flow rate: 30 mL/min) to give title compounds **9-p1** (26 mg, yield: 43.3%) and **9-p2** (26 mg, yield: 43.3%).

Single configuration compound (shorter retention time) **9-p2:** (26 mg, yield: 43.3%). MS m/z (ESI): 633.2 [M+1].

Chiral HPLC analysis: retention time: 7.89 min, purity: 99% (chromatography column: DAICEL CHIRALPAK^{®}IC, 250 × 4.6 mm, 5 µm; mobile phase: n-hexane and ethanol (containing 0.2% diethylamine), flow rate: 1.0 mL/min).

¹H NMR (500 MHz, CD₃OD): δ 7.67 (ddd, 1H), 7.32 - 7.21 (m, 2H), 7.11 - 7.01 (m, 1H), 5.38 - 5.27 (m, 2H), 5.11 - 5.03 (m, 1H), 4.64 - 4.59 (m, 1H), 4.52 - 4.44 (m, 1H), 4.33 (d, 1H), 4.24 (dd, 1H), 4.18 - 4.12 (m, 1H), 3.75 (br, 1H), 3.66 (br, 1H), 3.27 - 3.18 (m, 3H), 3.05 - 3.03 (m, 1H), 2.60 - 1.81 (m, 12H), 0.93 - 0.82 (m, 3H).

Single configuration compound (longer retention time) **9-p1**: (26 mg, yield: 43.3%). MS m/z (ESI): 633.2 [M+1].

Chiral HPLC analysis: retention time: 13.8 min, purity: 99% (chromatography column: DAICEL CHIRALPAK^{®}IC, 250 × 4.6 mm, 5 µm; mobile phase: n-hexane and ethanol (containing 0.2% diethylamine), flow rate: 1.0 mL/min).

¹H NMR (500 MHz, CD₃OD): δ 7.67 (ddd, 1H), 7.32 - 7.21 (m, 2H), 7.11 - 7.01 (m, 1H), 5.38 - 5.35 (m, 2H), 5.11 - 5.03 (m, 1H), 4.64 - 4.59 (m, 1H), 4.52 - 4.46 (m, 1H), 4.34 - 4.29 (m, 1H), 4.25 (dd, 1H), 4.18 - 4.12 (m, 1H), 3.74 (br, 1H), 3.65 (br, 1H), 3.26 - 3.23 (m, 3H), 3.05 - 3.01 (m, 1H), 2.61 - 1.81 (m, 12H), 0.94 - 0.82 m, 3H).

### Example 10

### Diastereomeric (1:1) mixture of 5-ethynyl-6-fluoro-4-((5aS,6S,9R)-1-fluoro-12-(((2R,7aS)-2-fluorotetrahydro-1H-pyrrol izin-7a(5H)-yl)methoxy)-5a,6,7,8,9,10-hexahydro-5H-4-oxa-3,10a,11,13,14-pentaaza-6 ,9-methanonaphtho[1,8-ab]heptalen-2-yl)naphthalen-2-ol 10-p1 and

### 5-ethynyl-6-fluoro-4-((5aR,6R,9S)-1-fluoro-12-(((2R,7aS)-2-fluorotetrahydro-1H-pyrrol izin-7a(5H)-yl)methoxy)-5a,6,7,8,9,10-hexahydro-5H-4-oxa-3,10a,11,13,14-pentaaza-6 ,9-methanonaphtho[1,8-ab]heptalen-2-yl)naphthalen-2-ol 10-p2

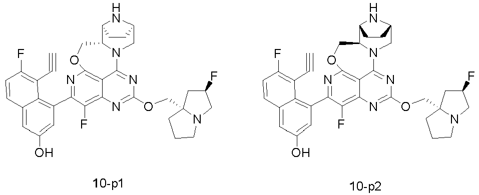

Using the synthetic route from step 10 to step 11 in Example 4, the starting material 2-(8-ethyl-3-(methoxymethoxy)naphthalen-1-yl)-4,4,5,5-tetramethyl-1,3,2-dioxaborolan e of step 10 was replaced with ((2-fluoro-6-(methoxymethoxy)-8-(4,4,5,5-tetramethyl-1,3,2-dioxaborolan-2-yl)naphtha len-1-yl)ethynyl)triisopropylsilane (prepared using the method disclosed in intermediate 15 on page 96 of the specification in Patent Application "WO2021/041671") to give the diastereomeric (1:1) mixture of title compounds **10-p1** and **10-p2** (2 mg, yield: 3.5%).

MS m/z (ESI): 629.2 [M+1].

¹H NMR (500 MHz, DMSO-d6): δ 7.93 (dd, 1H), 7.86 (s, 1H), 7.41 (t, 1H), 7.27 (s, 1H), 5.40 - 5.22 (m, 2H), 4.78 (d, 1H), 4.42 (dd, 1H), 4.14 (dd, 1H), 4.06 - 3.99 (m, 2H), 3.62 - 3.53 (m, 2H), 3.20 - 3.03 (m, 4H), 2.89 - 2.81 (m, 1H), 2.20 - 1.96 (m, 4H), 1.90 - 1.52 (m, 9H), 0.94 (t, 3H).

### Example 11

### Diastereomeric (1:1) mixture of 5-ethyl-4-((5aS,6S,9R)-1-fluoro-13-(((2R,7aS)-2-fluorotetrahydro-1H-pyrrolizin-7a(5H) -yl)methoxy)-5a,6,7,8,9,10-hexahydro-5H-6,9-epiminoazepino[2',1':3,4][1,4]oxazepino [5,6,7-de]quinazolin-2-yl)naphthalen-2-ol 11-p1 and

### 5-ethyl-4-((5aR,6R,9S)-1-fluoro-13-(((2R,7aS)-2-fluorotetrahydro-1H-pyrrolizin-7a(5H )-yl)methoxy)-5a,6,7,8,9,10-hexahydro-5H-6,9-epiminoazepino[2',1':3,4][1,4]oxazepin o[5,6,7-de]quinazolin-2-yl)naphthalen-2-ol 11-p2

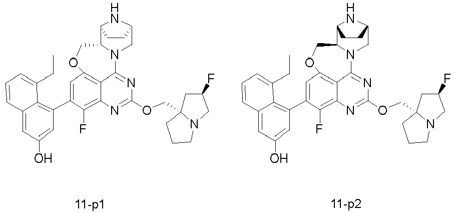

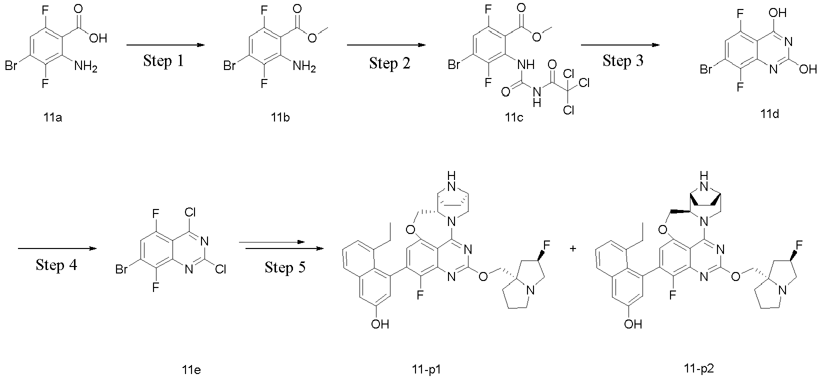

### Step 1

### Methyl 2-amino-4-bromo-3,6-difluorobenzoate 11b

2-amino-4-bromo-3,6-difluorobenzoic acid **11a** (9 g, 35.71 mmol, prepared using the method disclosed on page 110 of the specification in Patent Application "WO2018206539 A1") was dissolved in dichloromethane (100 mL) and methanol (10 mL), the reaction solution was added dropwise with 35.71 mL of 2 M trimethylsilyl diazomethane in n-hexane under an ice bath, then stirred at room temperature for 2 h after the dropwise addition was completed, and concentrated under reduced pressure, and the residue was purified with eluent system B to give title compound **11b** (6.8 g, yield: 71.5%).

MS m/z (ESI): 265.9 [M+1].

### Step 2

### Methyl 4-bromo-3,6-difluoro-2-(3-(2,2,2-trichloroacetyl)ureido)benzoate 11c

Compound **11b** (2 g, 7.51 mmol) was dissolved in the solvent tetrahydrofuran (30 mL), and the reaction solution was added with trichloroacetyl isocyanate (1.42 g, 7.5373 mmol, Jiangsu Aikon) in portions, stirred for 2 h, and concentrated under reduced pressure to give crude title compound **11c** (3.4 g, yield: 99%), which was directly used in the next reaction without purification.

MS m/z (ESI): 452.9 [M+1].

### Step 3

### 7-bromo-5,8-difluoroquinazoline-2,4-diol 11d

Crude compound **11c** (3.4 g, 7.48 mmol) was dissolved in 30 mL of 7 M methanolic ammonia, the reaction solution was stirred for 2 h, concentrated under reduced pressure to remove most of the solvent, added with 20 mL of methyl *tert*-butyl ether and slurried, and filtered, and the filter cake was washed with methyl *tert*-butyl ether and dried to give crude title compound **11d** (2 g, yield: 96.4%), which was directly used in the next reaction without purification.

MS m/z (ESI): 276.9 [M+1].

### Step 4

### 7-bromo-2,4-dichloro-5,8-difluoroquinazoline 11e

Compound **11d** (500 mg, 1.80 mmol) was dissolved in the solvent phosphorus oxychloride (5 mL), and the reaction solution was stirred at 100 °C for 3 h, and concentrated under reduced pressure to give crude title compound **11e** (500 mg, yield: 88.2%), which was directly used in the next reaction without purification.

MS m/z (ESI): 312.9 [M+1].

### Step 5

### Diastereomeric mixture of 5-ethyl-4-((5aS,6S,9R)-1-fluoro-13-(((2R,7aS)-2-fluorotetrahydro-1H-pyrrolizin-7a(5H) -yl)methoxy)-5a,6,7,8,9,10-hexahydro-5H-6,9-epiminoazepino[2',1':3,4][1,4]oxazepino [5,6,7-de]quinazolin-2-yl)naphthalen-2-ol 11-p1 and

### 5-ethyl-4-((5aR,6R,9S)-1-fluoro-13-(((2R,7aS)-2-fluorotetrahydro-1H-pyrrolizin-7a(5H )-yl)methoxy)-5a,6,7,8,9,10-hexahydro-5H-6,9-epiminoazepino[2',1':3,4][1,4]oxazepin o[5,6,7-de]quinazolin-2-yl)naphthalen-2-ol 11-p2

Using the synthetic route from step 7 to step 11 in Example 4, the starting compound **4f** of step 7 was replaced with compound **11e** to give the diastereomeric (1:1) mixture of title compounds **11-p1** and **11-p2** (5 mg, yield: 15.4%).

MS m/z (ESI): 614.2 [M+1].

¹H NMR (500 MHz, CD₃OD): δ 7.64 - 7.59 (m, 1H), 7.35 (td, 1H), 7.24 (dt, 1H), 7.15 (dd, 1H), 6.97 - 6.88 (m, 1H), 6.84 - 6.80 (m, 1H), 5.45 - 5.35 (m, 2H), 5.18 - 5.09 (m, 1H), 4.58 - 4.49 (m, 1H), 4.45 - 4.40 (m, 1H), 4.38 - 4.29 (m, 2H), 4.19 - 4.10 (m, 1H), 3.76 (s, 1H), 3.65 - 3.60 (m, 1H), 3.52 - 3.40 (m, 2H), 3.29 - 3.15 (m, 2H), 2.56 - 1.79 (m, 12H), 0.97 - 0.86 (m, 3H).

### Example 12

### 5-Ethyl-4-((5aR,6S,9R)-1-fluoro-12-(((2R,7aS)-2-fluorotetrahydro-1H-pyrrolizin-7a(5H )-yl)methoxy)-5a,6,7,8,9,10-hexahydro-5H-4-oxa-3,10a,11,13,14-pentaaza-6,9-methano naphtho[1,8-ab]heptalen-2-yl)naphthalen-2-ol 12-p1

### 5-Ethyl-4-((5aS,6R,9S)-1-fluoro-12-(((2R,7aS)-2-fluorotetrahydro-1H-pyrrolizin-7a(5H )-yl)methoxy)-5a,6,7,8,9,10-hexahydro-5H-4-oxa-3,10a,11,13,14-pentaaza-6,9-methano naphtho[1,8-ab]heptalen-2-yl)naphthalen-2-ol 12-p2

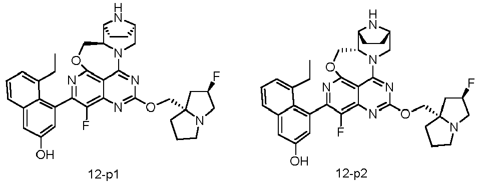

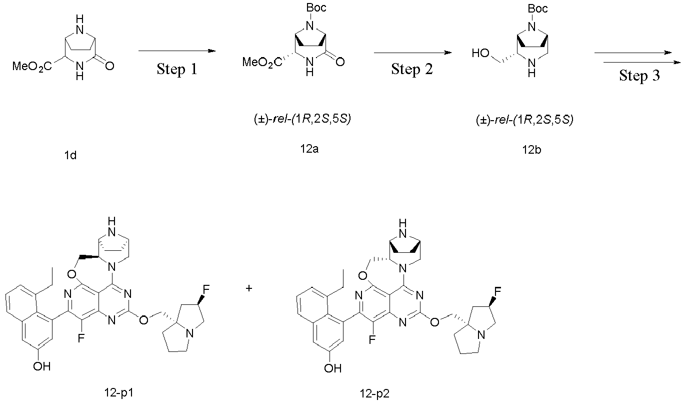

### Step 1

### 8-(tert-butyl) 2-methyl

(±)-*rel*-(1*R*,2*S*,5*S*)-4-oxo-3,8-diazabicyclo[3.2.1]octane-2,8-diformate **12a** Crude compound **1d** (10 g, 54.2 mmol) was dissolved in 300 mL of dichloromethane, the reaction solution was added with triethylamine (16 g, 158.12 mmol) and di-tert-butyl dicarbonate (11 g, 50.4 mmol, Shanghai Accela) under an ice bath, stirred for 14 h, and concentrated under reduced pressure, and the residue was purified by silica gel column chromatography with eluent system B to give title compound **12a** (1.6 g, yield: 10.3%).

MS m/z (ESI): 285.2 [M+1].

HPLC analysis: retention time: 0.94 min; purity: 98.5% (chromatography column: ACQUITY UPLC^{®}BEH, C18, 1.7 µm, 2.1 × 50 mm; mobile phase: water (10 mM ammonium bicarbonate), acetonitrile; gradient ratio: acetonitrile 10%-95%).

### Step 2

### Tert-butyl (±)-rel-(1R,2S,5S)-2-(hydroxymethyl)-3,8-diazabicyclo[3.2.1]octane-8-carboxylate 12b

Compound **12a** (3.15 g, 11.09 mmol) was dissolved in tetrahydrofuran (10 mL), the reaction solution was added with lithium aluminum hydride (1.05 g, 31 mmol) under an ice bath, stirred for 2 h, sequentially added with 1.7 mL of water and 1.7 mL of 15% aqueous sodium hydroxide solution for quenching the reaction, added with anhydrous sodium sulfate and dried, and filtered to remove the drying agent, and the filtrate was concentrated under reduced pressure to give crude title compound **12b** (2.6 g, yield: 96.6%), which was directly used in the next reaction without purification.

MS m/z (ESI): 243.2 [M+1].

### Step 3

### 5-Ethyl-4-((5aR,6S,9R)-1-fluoro-12-(((2R,7aS)-2-fluorotetrahydro-1H-pyrrolizin-7a(5H )-yl)methoxy)-5a,6,7,8,9,10-hexahydro-5H-4-oxa-3,10a, 11,13,14-pentaaza-6,9-methano naphtho[1,8-ab]heptalen-2-yl)naphthalen-2-ol 12-p1

### 5-Ethyl-4-((5aS,6R,9S)-1-fluoro-12-(((2R,7aS)-2-fluorotetrahydro-1H-pyrrolizin-7a(5H )-yl)methoxy)-5a,6,7,8,9,10-hexahydro-5H-4-oxa-3,10a,11,13,14-pentaaza-6,9-methano naphtho[1,8-ab]heptalen-2-yl)naphthalen-2-ol 12-p2

Using the synthetic route from step 1 to step 11 in Example 4, the starting compound **1g** of step 1 was replaced with compound **12b** to give title compounds **12-p1** and **12-p2** (3 mg, 3 mg, yields: 37%, 37%).

Single configuration compound (shorter retention time): (3 mg, yield: 37%).

MS m/z (ESI): 615.2 [M+1].

HPLC analysis: retention time: 1.24 min; purity: 90% (chromatography column: ACQUITY UPLC^{®}BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.63 (d, 1H), 7.40 - 7.35 (m, 1H), 7.30 - 7.28 (m, 1H), 7.21 - 7.16 (m, 1H), 7.12 - 6.95 (m, 1H), 5.47 (d, 1H), 5.36 (t, 1H), 5.15 - 5.07 (m, 1H), 4.69 - 4.44 (m, 3H), 4.23 (d, 1H), 3.92 - 3.76 (m, 2H), 3.62 (br, 2H),2.62 - 2.27 (m, 7H), 2.24 - 1.85 (m, 8H), 0.95 - 0.88 (m, 3H).

Single configuration compound (longer retention time): (3 mg, yield: 37%).

MS m/z (ESI): 615.2 [M+1].

HPLC analysis: retention time: 1.28 min; purity: 90% (chromatography column: ACQUITY UPLC^{®}BEH, C18, 1.7 µm, 2.1 × 50 mm; mobile phase: water (10 mM ammonium bicarbonate), acetonitrile; gradient ratio: acetonitrile 10%-95%).

¹H NMR (500 MHz, CD₃OD): δ 7.63 (d, 1H), 7.38 (td, 1H), 7.29 (t, 1H), 7.19 (t, 1H), 7.12 - 6.95 (m, 1H), 5.52 (d, 1H), 5.36 (t, 1H), 4.67 - 4.53 (m, 4H), 4.44 (t, 1H), 3.98 - 3.67 (m, 6H), 2.55 - 2.42 (m, 2H), 2.40 - 2.34 (m, 1H), 2.30 - 2.28 (m, 2H), 2.21 - 2.03 (m, 2H), 2.02 - 18.4 (m, 3H), 1.05 - 0.89 (m, 6H).

### Example 13

### Diastereomeric (1:1) mixture of 5-ethynyl-4-((5aS,6S,9R)-1-fluoro-12-(((2R,7aS)-2-fluorotetrahydro-1H-pyrrolizin-7a(5 H)-yl)methoxy)-5a,6,7,8,9,10-hexahydro-5H-4-oxa-3,10a,11,13,14-pentaaza-6,9-metha nonaphtho[1,8-ab]heptalen-2-yl)naphthalen-2-ol 13-p1 and

### 5-ethynyl-4-((5aR,6R,9S)-1-fluoro-12-(((2R,7aS)-2-fluorotetrahydro-1H-pyrrolizin-7a(5 H)-yl)methoxy)-5a,6,7,8,9,10-hexahydro-5H-4-oxa-3,10a,11,13,14-pentaaza-6,9-metha nonaphtho[1,8-ab]heptalen-2-yl)naphthalen-2-ol 13-p2

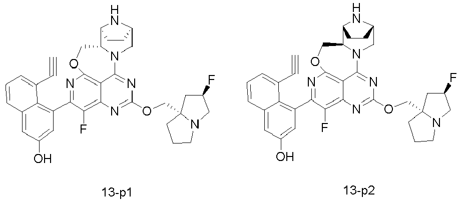

Using the synthetic route from step 10 to step 11 in Example 4, the starting material 2-(8-ethyl-3-(methoxymethoxy)naphthalen-1-yl)-4,4,5,5-tetramethyl-1,3,2-dioxaborolan e of step 10 was replaced with triisopropyl((6-(methoxymethoxy)-8-(4,4, 5, 5-tetramethyl-1,3,2-dioxaborolan-2-yl)napht halen-1-yl)ethynyl)silane (prepared using the method disclosed in intermediate 17 on page 103 of the specification in Patent Application "WO2021/041671") to give the diastereomeric (1:1) mixture of title compounds **13-p1** and **13-p2** (8 mg, yield: 14.1%). MS m/z (ESI): 611.2 [M+1].

¹H NMR (500 MHz, DMSO-d6): δ 7.63 (d, 1H), 7.37 (q, 1H), 7.29 (q, 1H), 7.18 (dd, 1H), 7.08 (t, 1H), 5.40 - 5.22 (m, 2H), 4.78 (d, 1H), 4.42 (dd, 1H), 4.14 (dd, 1H), 4.06 - 3.99 (m, 2H), 3.62 - 3.53 (m, 2H), 3.20 - 3.03 (m, 4H), 2.89 - 2.81 (m, 1H), 2.20 - 1.96 (m, 4H), 1.90 - 1.52 (m, 6H), 0.94 (t, 3H).

### Example 14

### 3-Chloro-5-((5aS,6S,9R)-1-fluoro-12-(((2R,7aS)-2-fluorotetrahydro-1H-pyrrolizin-7a(5 H)-yl)methoxy)-5a,6,7,8,9,10-hexahydro-5H-4-oxa-3,10a,11,13,14-pentaaza-6,9-metha nonaphtho[1,8-ab]heptalen-2-yl)-4-(trifluoromethyl)phenol 14-p1

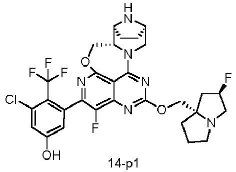

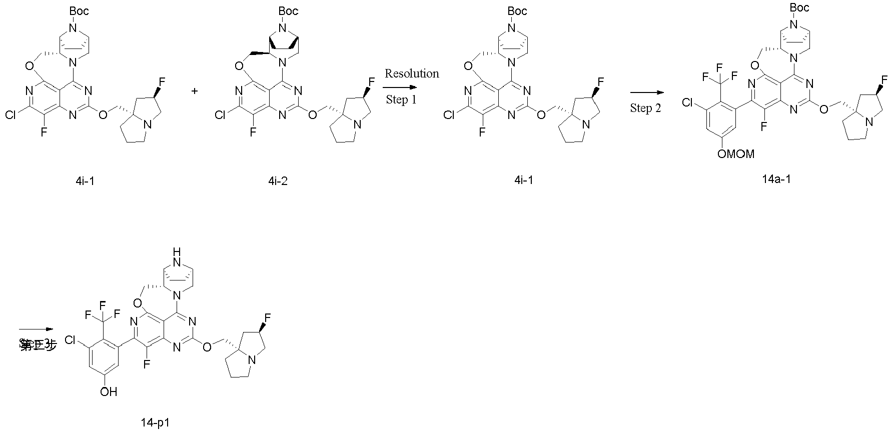

### Step 1

### Diastereomeric mixture of tert-butyl (5aS,6S,9R)-2-chloro-1-fluoro-12-(((2R,7aS)-2-fluorotetrahydro-1H-pyrrolizin-7a(5H)-yl)methoxy)-5a,6,7,8,9,10-hexahydro-5H-4-oxa-3,10a,11,13,14-pentaaza-6,9-methanon aphtho[1,8-ab]heptalen-14-carboxylate 4i-1

The diastereomeric mixture of compounds **4i-1** and **4i-2** (29.6 g, 51.1 mmol) was subjected to chiral resolution (Waters SFC 150, chromatography column: DAICEL CHIRALPAK^{®}IC, 25 × 250 mm, 10 µm; mobile phase A: Supercritical CO₂, mobile phase B: ethanol, gradient ratio: A:B: 45:55, flow rate: 110 mL/min) to give title compound **4i-1** (7.8 g, yield: 26.3%).

Chiral HPLC analysis: retention time: 3.199 min, purity: 99% (chromatography column: DAICEL CHIRALPAK^{®}OZ, 100 × 3 mm, 3 µm; mobile phase: Supercritical CO₂ and ethanol (containing 0.1% diethylamine), flow rate: 2.0 mL/min).

### Step 2

### Tert-butyl (5aS,6S,9R)-2-(3-chloro-5-(methoxymethoxy)-2-(trifluoromethyl)phenyl)-1-fluoro-12-(( (2R,7aS)-2-fluorotetrahydro-1H-pyrrolizin-7a(5H)-yl)methoxy)-5a,6,7,8,9,10-hexahydr o-5H-4-oxa-3,10a,11,13,14-pentaaza-6,9-methanonaphtho[1,8-ab]heptalen-14-carboxyl ate 14a-1

Compound **4i-1** (60 mg, 103.6 µmol), 2-(3-chloro-2-trifluoromethyl-5-(methoxymethoxy)phenyl) -4,4,5,5-tetramethyl-1,3,2-dioxaborolane (75 mg, 204.6 µmol, prepared using the method disclosed in Example 284 on page 530 of the specification in Patent Application "WO2021/041671"), tetrakis(triphenylphosphine)palladium (24 mg, 10.7 µmol, adamas) and cesium carbonate (101 mg, 309.9 µmol) were dissolved in 3 mL of a mixed solution of 1,4-dioxane and water (V:V = 5:1). The reaction solution was reacted at 100 °C for 6 h under nitrogen atmosphere, and concentrated under reduced pressure to give crude title compound **14a-1** (81 mg, yield: 99%).

MS m/z (ESI): 783.2 [M+1].

### Step 3

### 3-Chloro-5-((5aS,6S,9R)-1-fluoro-12-(((2R,7aS)-2-fluorotetrahydro-1H-pyrrolizin-7a(5 H)-yl)methoxy)-5a,6,7,8,9,10-hexahydro-5H-4-oxa-3,10a, 11, 13, 14-pentaaza-6,9-metha nonaphtho[1,8-ab]heptalen-2-yl)-4-(trifluoromethyl)phenol 14-p1

The product crude compound **14a-1** (80 mg, 102.1 µmol) of step 2 described above was dissolved in acetonitrile (1 mL), the reaction solution was added with 0.5 mL of 4 M hydrochloride in dioxane, stirred for 2 h, and concentrated under reduced pressure, and the residue was purified by high performance liquid chromatography (Waters-2545, chromatography column: SharpSil-T C18, 30 × 150 mm, 5 µm; mobile phase: aqueous phase (10 mmol/L ammonium bicarbonate) and acetonitrile, gradient ratio: acetonitrile 38%-45%, flow rate: 30 mL/min) to give title compound **14-p1**(26 mg, yield: 39.8%).

MS m/z (ESI): 639.2 [M+1].

¹H NMR (500 MHz, CD₃OD): δ 7.11 (d, 1H), 6.73 (d, 1H), 5.44 - 5.26 (m, 1H), 5.06 (d, 1H), 4.61 (d, 1H), 4.45 (s, 1H), 4.33 (d, 1H), 4.27 (d, 1H), 4.14 (s, 1H), 3.74 (d, 1H), 3.66 (d, 1H), 3.22 (d, 1H), 3.07 (d, 1H), 2.44 - 2.12 (m, 4H), 2.04 (dq, 3H), 1.89 (ddd, 6H).

### Example 15

### 5-Ethyl-6-fluoro-4-((5aS,6S,9R)-1-fluoro-12-((1-(((R)-2-(hydroxymethyl)pyrrolidin-1-y l)methyl)cyclopropyl)methoxy)-5a,6,7,8,9,10-hexahydro-5H-4-oxa-3,10a,11,13,14-pent aaza-6,9-methanonaphtho[1,8-ab]heptalen-2-yl)naphthalen-2-ol 15

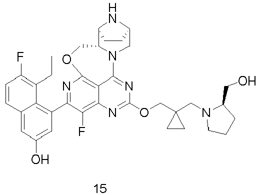

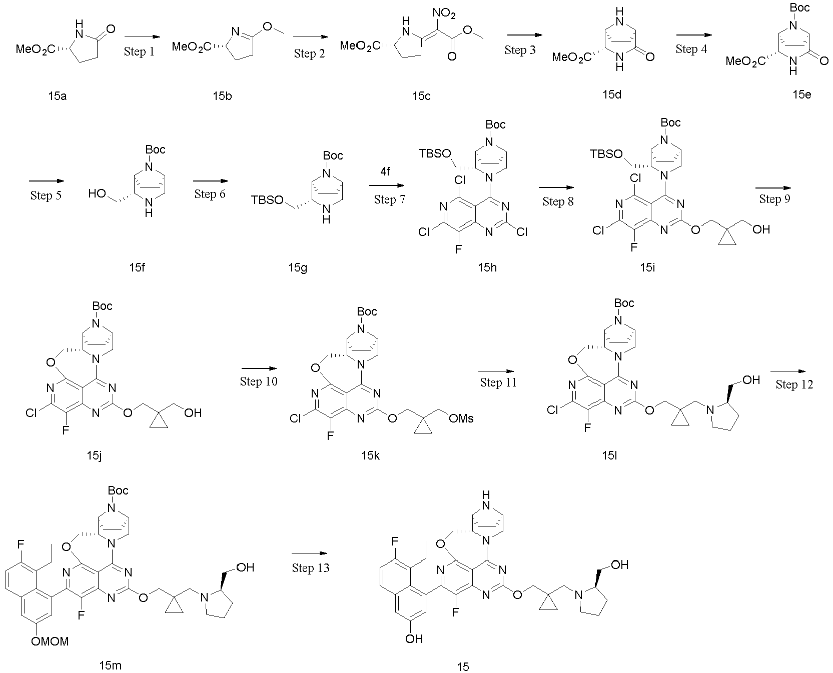

### Step 1

### Methyl (R)-5-methoxy-3,4-dihydro-2H-pyrrole-2-carboxylate 15b

Methyl (*R*)-2-pyrrolidone-5-formate **15a** (20 g, 139.7 mmol, Shanghai Bide) and dimethyl sulfate (22.1 g, 175.2 mmol) were mixed and reacted at 60 °C for 22 h, and the reaction solution was cooled to room temperature, and poured into a solution of triethylamine (20 g) and methyl *tert*-butyl ether (30 mL) under an ice bath. The resulting solution was extracted with methyl *tert*-butyl ether (60 mL × 6) and then concentrated under reduced pressure to give crude title compound **15b** (16.3 g, yield: 74.2%), which was directly used in the next reaction without purification.

MS m/z (ESI): 158.1 [M+1].

### Step 2

### Methyl (R)-5-(2-methoxy-1-nitro-2-oxoethylidene)pyrrolidine-2-carboxylate 15c

Crude compound **15b** (16.3 g, 103.7 mmol) and methyl nitroacetate (13.6 g, 114.2 mmol, Shanghai Accela) were mixed, heated to 60 °C and stirred for 24 h, after the reaction solution was cooled to room temperature, the reaction solution was concentrated under reduced pressure, and the residue was purified by silica gel column chromatography with eluent system B to give title compound **15c** (8.37 g, yield: 33%).

MS m/z (ESI): 245.1 [M+1].

### Step 3

### Methyl (1S,2S,5R)-4-oxo-3,8-diazabicyclo[3.2.1]octane-2-carboxylate 15d

Compound **15c** (7.5 g, 30.7 mmol) was dissolved in 150 mL of methanol, the reaction solution was added with a 10% palladium on carbon catalyst (wet) (1.5 g), purged with hydrogen three times, heated to 50 °C and stirred for 24 h, then cooled to room temperature, and filtered through celite, the filter cake was washed with methanol, and the filtrate was concentrated under reduced pressure to give crude title compound **15d** (5.6 g), which was directly used in the next reaction without purification.

MS m/z (ESI): 185.2 [M+1].

### Step 4

### 8-(Tert-butyl) 2-methyl (1S,2S,5R)-4-oxo-3,8-diazabicyclo[3.2.1]octane-2,8-dicarboxylate 15e

Crude compound **15d** (5.65 g, 30.4 mmol) was dissolved in 60 mL of dichloromethane, the reaction solution was added with triethylamine (6.2 g, 61.27 mmol) and di-tert-butyl dicarbonate (6.6 g, 30.24 mmol) under an ice bath, stirred for 14 h, and concentrated under reduced pressure, and the residue was purified by silica gel column chromatography with eluent system B to give title compound **15e** (3 g, yield: 34.7%).

MS m/z (ESI): 285.2 [M+1].

### Step 5

### Tert-butyl (1S,2S,5R)-2-(hydroxymethyl)-3,8-diazabicyclo[3.2.1]octane-8-carboxylate 15f

Compound **15e** (3 g, 10.55 mmol) was dissolved in 30 mL of tetrahydrofuran, the reaction solution was added dropwise with 32 mL of 1 M lithium aluminum hydride in tetrahydrofuran under an ice bath, allowed to return to room temperature, stirred for 4 h, sequentially added with 1.05 mL of water, 1.05 mL of 15% sodium hydroxide solution and 3.15 mL of water under an ice bath, then allowed to return to room temperature and stirred for 15 min, added with anhydrous magnesium sulfate (1 g), stirred for 15 min, and filtered, and the filtrate was concentrated under reduced pressure to give crude title compound **15f** (2.4 g), which was directly used in the next reaction without purification. MS m/z (ESI): 243.2 [M+1].

### Step 6

### Tert-butyl (1S,2S,5R)-2-(((tert-butyldimethylsilyl)oxy)methyl)-3,8-diazabicyclo[3.2.1]octane-8-car boxylate 15g

Crude compound **15f** (2.4 g, 9.9 mmol) was dissolved in 25 mL of dichloromethane, the reaction solution was added with *tert*-butyldimethylsilyl chloride (4.48 g, 29.7 mmol) and 4-dimethylaminopyridine (122 mg, 990 µmol),added dropwise with triethylamine (4 g, 39.5 mmol), stirred for 16 h, added with 20 mL of water, and extracted with dichloromethane (20 mL × 2). The organic phases were combined, washed with a saturated sodium chloride solution and concentrated under reduced pressure, and the residue was purified by silica gel column chromatography with eluent system B to give title compound **15g** (1.95 g, yield: 55.2%).

MS m/z (ESI): 357.1 [M+1].

### Step 7

### Tert-butyl (1S,2S,5R)-2-(((tert-butyldimethylsilyl)oxy)methyl)-3-(2,5,7-trichloro-8-fluoropyrido[4, 3-d]pyrimidin-4-yl)-3,8-diazabicyclo[3.2.1]octane-8-carboxylate 15h

Compound **4f** (50 g, 174 mmol) and N,N-diisopropylethylamine (65 g, 503 mmol) were dissolved in 75 mL of dichloromethane, the reaction solution was added dropwise with a solution of **15g** (60 g, 168.3 mmol) in dichloromethane (250 mL) at -78 °C, naturally allowed to return to room temperature for 4 h, and concentrated under reduced pressure, and the residue was purified with eluent system B to give title compound **15h** (86 g, yield: 84%).

MS m/z (ESI): 606.2 [M+1].

### Step 8

### Tert-butyl (1S,2S,5R)-2-(((tert-butyldimethylsilyl)oxy)methyl)-3-(5,7-dichloro-8-fluoro-2-((1-(hyd roxymethyl)cyclopropyl)methoxy)pyrido[4,3-d]pyrimidin-4-yl)-3,8-diazabicyclo[3.2.1] octane-8-carboxylate 15i

Compound **15h** (5 g, 8.23 mmol) was dissolved in 1,4-dioxane (80 mL), and the reaction solution was added with 1,1-bis(hydroxymethyl)cyclopropane (1.3 g, 12.7 mmol, Shanghai Accela), cesium carbonate (5.4 g, 16.6 mmol) and 4A molecular sieves (5 g), stirred at 110 °C for 14 h, cooled to room temperature, then filtered, and concentrated under reduced pressure to give crude title compound **15i** (5.5 g), which was directly used in the next step without purification.

MS m/z (ESI): 672.2 [M+1].

### Step 9

### Tert-butyl (5aS,6S,9R)-2-chloro-1-fluoro-12-((1-(hydroxymethyl)cyclopropyl)methoxy)-5a,6,7,8,9 , 10-hexahydro-5H-4-oxa-3,10a,11,13,14-pentaaza-6,9-methanonaphtho[1,8-ab]heptalen -14-carboxylate 15j

Crude compound **15i** (5.5 g, 8.2 mmol) was dissolved in tetrahydrofuran (80 mL), and the reaction solution was added with tetrabutylammonium fluoride (5.57 g, 24.7 mmol) and *N*,*N*-diisopropylethylamine (5.33 g, 41.2 mmol), stirred for 1 h, then heated to 60 °C and stirred for 2 h, cooled to room temperature, added with water (80 mL) for dilution, and extracted with ethyl acetate (50 mL × 2). The organic phases were combined and concentrated under reduced pressure, and the residue was purified by silica gel column chromatography with eluent system A to give title compound **15j** (3 g, yield: 69.7%).

MS m/z (ESI): 522.2 [M+1].

### Step 10

### Tert-butyl (4aS,6S,9R-2-chloro-1-fluoro-12-((1-(((methylsulfonyl)oxy)methyl)cyclopropyl)methox y)-5a,6,7,8,9,10-hexahydro-5H-4-oxa-3,10a,11,13,14-pentaaza-6,9-methanonaphtho[1,8 -ab]heptalen-14-carboxylate 15k

Compound **15j** (200 mg, 344.8 µmol) and *N,N-*diisopropylethylamine (112 mg, 866.5 µmol) were dissolved in dichloromethane (10 mL), and the reaction solution was added with methanesulfonyl chloride (60 mg, 523.7 µmol) under an ice bath, stirred for 1 h while maintaining the temperature, added with a saturated ammonium chloride solution for quenching the reaction, and extracted with dichloromethane (5 mL × 2). The organic phases were combined, dried over anhydrous sodium sulfate, and filtered to remove the drying agent, and the filtrate was concentrated under reduced pressure to give crude title compound **15k** (206 mg), which was directly used in the next reaction without purification.

MS m/z (ESI): 600.2 [M+1].

### Step 11

### Tert-butyl (5aS,6S,9R)-2-chloro-1-fluoro-12-((1-(((R)-2-(hydroxymethyl)pyrrolidin-1-yl)methyl)c yclopropyl)methoxy)-5a,6,7,8,9,10-hexahydro-5H-4-oxa-3,10a,11,13,14-pentaaza-6,9-methanonaphtho[1,8-ab]heptalen-14-carboxylate 15l

Crude compound **15k** (206 mg, 343.3 µmol) was dissolved in acetonitrile (10 mL), the reaction solution was added with *L*-prolinol (53 mg, 524 µmol, Shanghai Accela), anhydrous potassium carbonate (143 mg, 1.03 mmol) and sodium iodide (103 mg, 687 µmol),heated to 80 °C and stirred for 2 h, cooled to room temperature, added with water (20 mL) for dilution, and extracted with ethyl acetate (10 mL × 2). The organic phases were combined and concentrated under reduced pressure to give crude title compound **15l** (200 mg), which was directly used in the next reaction without purification.

MS m/z (ESI): 605.2 [M+1].

### Step 12

### Tert-butyl (5aS,6S,9R)-2-(8-ethyl-7-fluoro-3-(methoxymethoxy)naphthalen-1-yl)-1-fluoro-12-(1-(( (R)-2-(hydroxymethyl)pyrrolidin-1-yl)methyl)cyclopropyl)methoxy)-5a,6,7,8,9,10-hexa hydro-5H-4-oxa-3,10a,11,13,14-pentaaza-6,9-methanonaphtho[1,8-ab]heptalen-14-carb oxylate 15m

Compound **15l** (100 mg, 165 µmol), 2-(8-ethyl-7-fluoro-3 -(methoxymethoxy)naphthalen-1-yl)-4,4, 5, 5-tetramethyl-1,3,2-dio xaborolane (84 mg, 233 µmol),tetrakis(triphenylphosphine)palladium (39 mg, 33.7 µmol) and cesium carbonate (108 mg, 331 µmol) were dissolved in 11 mL of a mixed solution of 1,4-dioxane and water (V:V = 10:1). The reaction solution was reacted at 100 °C for 4 h under nitrogen atmosphere and concentrated under reduced pressure, and the residue was purified by thin layer chromatography with developing solvent system A to give title compound **15m** (50 mg, yield: 37.6%).

MS m/z (ESI): 803.2 [M+1].

### Step 13

### 5-Ethyl-6-fluoro-4-((5aS,6S,9R)-1-fluoro-12-((1-(((R)-2-(hydroxymethyl)pyrrolidin-1-y l)methyl)cyclopropyl)methoxy)-5a,6,7,8,9,10-hexahydro-5H-4-oxa-3,10a,11,13,14-pent aaza-6,9-methanonaphtho[1,8-ab]heptalen-2-yl)naphthalen-2-ol 15

Compound **15m** (50 mg, 62.2 µmol) was dissolved in ethyl acetate (2 mL), the reaction solution was added with 0.5 mL of 4 M hydrochloride in dioxane under an ice bath, reacted for 2 h while maintaining the temperature, and concentrated under reduced pressure, and the residue was purified by high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **15** (10 mg, yield: 24.3%).

MS m/z (ESI): 659.2 [M+1].

¹H NMR (500 MHz, CD₃OD): δ 7.67 (ddd, 1H), 7.30 (d, 1H), 7.27 - 7.21 (m, 1H), 7.06 (dd, 1H), 5.36 (t, 1H), 5.06 (dd, 1H), 4.78 (s, 1H), 4.65 - 4.60 (m, 1H), 4.48 (ddd, 1H), 4.15 (dd, 2H), 3.71 (d, 2H), 3.57 - 3.38 (m, 4H), 3.24 (t, 1H), 2.51 - 2.34 (m, 2H), 2.22 (q, 2H), 2.03 - 1.76 (m, 7H), 1.71 (s, 1H), 1.62 (s, 1H), 0.96 - 0.82 (m, 4H), 0.67 (s, 1H), 0.55 (s, 1H).

### Example 16

### 5-Ethyl-6-fluoro-4-((5aS,6S,9R)-1-fluoro-12-((1-(((R)-2-(hydroxymethyl)-2,5-dihydro-1 H-pyrrol-1-yl)methyl)cyclopropyl)methoxy)-5a,6,7,8,9,10-hexahydro-5H-4-oxa-3,10a, 1 1,13,14-pentaaza-6,9-methanonaphtho[1,8-ab]heptalen-2-yl)naphthalen-2-ol 16

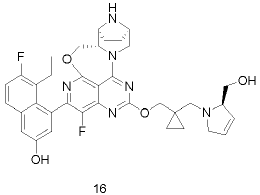

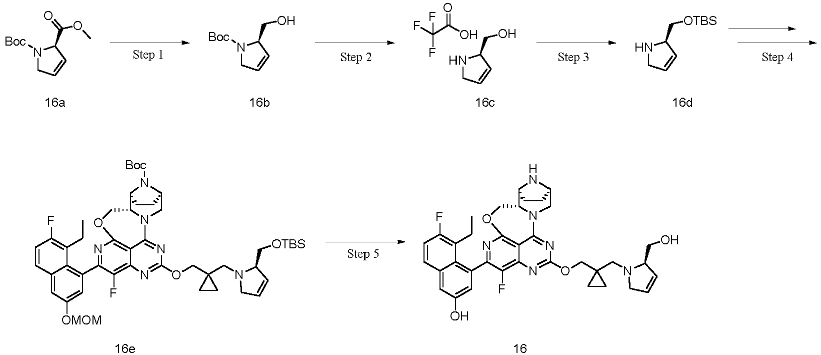

### Step 1

### Tert-butyl (R)-2-(hydroxymethyl)-2,5-dihydro-1H-pyrrole-1-carboxylate 16b

1-(*Tert*-butyl) 2-methyl (*R*)-2,5-dihydro-1*H*-pyrrole-1,2-dicarboxylate **16a** (543 mg, 2.39 mmol, WuXi AppTec Co. Ltd.) was dissolved in tetrahydrofuran (10 mL), the reaction solution was added with 1 M lithium aluminum hydride in tetrahydrofuran (24 mL) under an ice bath, reacted for 2 h, added with sodium sulfate decahydrate for quenching the reaction, and filtered through celite, and the filtrate was concentrated under reduced pressure to give crude title compound **16b** (421 mg, yield: 88.4%), which was directly used in the next reaction without purification.

MS m/z (ESI): 144.2 [M-55].

### Step 2

### (R)-(2,5-dihydro-1H-pyrrol-2-yl)methanol 2,2,2-trifluoroacetate 16c

Crude compound **16b** (421 mg, 2.1 mmol) was dissolved in dichloromethane (10 mL), the reaction solution was added with trifluoroacetic acid (1.68 g, 1.1 mL) under an ice bath, stirred for 1 h, and concentrated under reduced pressure to give crude title compound **16c** (500 mg), which was directly used in the next reaction without purification.

MS m/z (ESI): 100.2 [M+1].

### Step 3

### (R)-2-(((tert-butyldimethylsilyl)oxy)methyl)-2,5-dihydro-1H-pyrrole 16d

Crude compound **16c** (450 mg, 2.1 mmol) was dissolved in 10 mL of dichloromethane, and the reaction solution was added with *tert*-butyldimethylsilyl chloride (226 mg, 2.74 mmol), added dropwise with triethylamine (1.1 g, 10.8 mmol), stirred for 16 h, added with 20 mL of water, and extracted with dichloromethane (10 mL × 2). The organic phases were combined, washed with a saturated sodium chloride solution and concentrated under reduced pressure, and the residue was purified by silica gel column chromatography with eluent system B to give title compound **16d** (110 mg, yield: 24.4%).

MS m/z (ESI): 214.1 [M+1].

### Step 4

### Tert-butyl (5aS,6S,9R)-12-((1-((2-(((tert-butyldimethylsilyl)oxy)methyl)-2,5-dihydro-1H-pyrrol-1-yl)methyl)cyclopropyl)methoxy)-2-(8-ethyl-7-fluoro-3-(methoxymethoxy)naphthalen-1 -yl)-1-fluoro-5a,6,7,8,9,10-hexahydro-5H-4-oxa-3,10a,11,13,14-pentaaza-6,9-methanon aphtho[1,8-ab]heptalen-14-carboxylate 16e

Using the synthetic route from step 11 to step 12 in Example 15, the starting material L-prolinol of step 11 was replaced with compound **16d** to give crude title compound **16e** (40 mg), which was directly used in the next reaction without purification.

MS m/z (ESI): 915.2 [M+1].

### Step 5

### 5-Ethyl-6-fluoro-4-((5aS,6S,9R)-1-fluoro-12-((1-(((R)-2-(hydroxymethyl)-2,5-dihydro-1 H-pyrrol-1-yl)methyl)cyclopropyl)methoxy)-5a,6,7,8,9,10-hexahydro-5H-4-oxa-3,10a, 1 1,13,14-pentaaza-6,9-methanonaphtho[1,8-ab]heptalen-2-yl)naphthalen-2-ol 16

Crude compound **16e** (40 mg, 43.7 µmol) was dissolved in ethyl acetate (2 mL), the reaction solution was added with 0.5 mL of 4 M hydrochloride in dioxane under an ice bath, reacted for 2 h while maintaining the temperature, and concentrated under reduced pressure, and the residue was purified by high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **16** (8 mg, yield: 27.8%).

MS m/z (ESI): 657.2 [M+1].

¹H NMR (500 MHz, CD₃OD): δ 7.67 (ddd, 1H), 7.34 - 7.21 (m, 2H), 7.06 (dd, 1H), 5.95 - 5.86 (m, 1H), 5.79 (d, 1H), 5.08 (dd, 1H), 4.81 (s, 1H), 4.62 (dd, 1H), 4.49 (ddd, 1H), 4.17 (dd, 1H), 4.11 (d, 2H), 3.86 - 3.66 (m, 2H), 3.59 (dd, 1H), 3.48 (d, 2H), 3.27 (d, 1H), 2.58 (dt, 1H), 2.48 (p, 1H), 2.22 (dt, 1H), 2.05 (d, 1H), 2.01 - 1.79 (m, 4H), 1.62 (t, 1H), 0.96 - 0.82 (m, 3H), 0.75 (s, 2H), 0.68 (d, 1H), 0.56 (d, 1H).

### Example 17

### 5-Ethyl-6-fluoro-4-((5aS,6S,9R)-1-fluoro-12-((1-(((S)-2-(hydroxymethyl)pyrrolidin-1-yl )methyl)cyclopropyl)methoxy)-5a,6,7,8,9,10-hexahydro-5H-4-oxa-3,10a,11,13,14-pent aaza-6,9-methanonaphtho[1,8-ab]heptalen-2-yl)naphthalen-2-ol 17

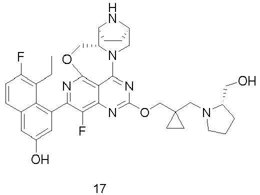

Using the synthetic route in Example 15, the starting material L-prolinol of step 11 was replaced with D-prolinol to give title compound **17** (10 mg, yield: 24.3%).

MS m/z (ESI): 659.2 [M+1].

¹H NMR (500 MHz, CD₃OD): δ7.67 - 7.65 (m, 1H), 7.30 - 7.24 (m, 2H), 7.11 (s, 1H),7.02 (s, 1H), 5.05 - 5.01 (m, 1H), 4.75 - 4.73 (m, 1H), 4.63 - 4.59 (m, 1H), 4.48 - 4.46 (m, 1H), 4.17 - 4.13 (m, 2H), 3.76 - 3.74 (m, 1H), 3.65 - 3.64 (m, 1H), 3.57 - 3.54 (m, 1H), 3.47 - 3.39 (m, 3H), 3.36 - 3.23 (m, 1H), 2.70 - 2.68 (m, 1H), 2.60 - 2.47 (m, 2H), 2.45 - 2.10 (m, 2H), 2.05 - 1.78 (m, 7H), 1.74 - 1.69 (m, 1H), 0.94 - 0.91 (m, 1H), 0.86 - 0.84 (m, 2H), 0.74 - 0.71 (m, 2H), 0.63 - 0.65 (m, 1H), 0.56 - 0.53 (m, 1H).

### Example 18

### 5-Ethyl-6-fluoro-4-((5aS,6S,9R)-1-fluoro-12-((1-(((S)-2-(hydroxymethyl)-2,5-dihydro-1 H-pyrrol-1-yl)methyl)cyclopropyl)methoxy)-5a,6,7,8,9,10-hexahydro-5H-4-oxa-3,10a, 1 1,13,14-pentaaza-6,9-methanonaphtho[1,8-ab]heptalen-2-yl)naphthalen-2-ol 18

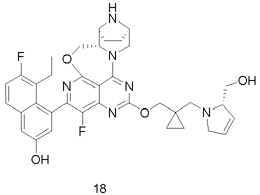

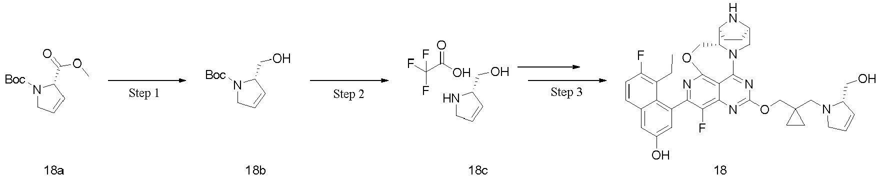

### Step 1

### Tert-butyl (S)-2-(hydroxymethyl)-2,5-dihydro-1H-pyrrole-1-carboxylate 18a

1-(Tert-butyl) 2-methyl (*R*)-2,5-dihydro-1*H*-pyrrole-1,2-dicarboxylate **18a** (520 mg, 2.28 mmol, WuXi AppTec Co. Ltd.) was dissolved in tetrahydrofuran (5 mL), the reaction solution was added with 1 M lithium aluminum hydride in tetrahydrofuran (24 mL) under an ice bath, reacted for 2 h, added with sodium sulfate decahydrate for quenching the reaction, and filtered through celite, and the filtrate was concentrated under reduced pressure to give crude title compound **18b** (421 mg), which was directly used in the next reaction without purification.

MS m/z (ESI): 144.2 [M-55].

### Step 2

### (S)-(2,5-dihydro-1H-pyrrol-2-yl)methanol 2,2,2-trifluoroacetate 18c

Crude compound **18b** (162 mg, 813 µmol) was dissolved in dichloromethane (5 mL), the reaction solution was added with trifluoroacetic acid (1 g, 8.77 mmol) under an ice bath, stirred for 1 h, and concentrated under reduced pressure to give crude title compound **18c** (260 mg), which was directly used in the next reaction without purification.

MS m/z (ESI): 100.2 [M+1].

### Step 3

### 5-Ethyl-6-fluoro-4-((5aS,6S,9R)-1-fluoro-12-((1-(((S)-2-(hydroxymethyl)-2,5-dihydro-1 H-pyrrol-1-yl)methyl)cyclopropyl)methoxy)-5a,6,7,8,9,10-hexahydro-5H-4-oxa-3,10a, 1 1,13,14-pentaaza-6,9-methanonaphtho[1,8-ab]heptalen-2-yl)naphthalen-2-ol 18

Using the synthetic route in Example 15, the starting material L-prolinol of step 11 was replaced with compound **18c** to give title compound **18** (2 mg, yield: 12.1%).

MS m/z (ESI): 657.2 [M+1].

¹H NMR (500 MHz, CD₃OD): δ 7.70 - 7.63 (m, 1H), 7.31 - 7.21 (m, 2H), 7.11 - 7.01 (m, 1H), 5.90 - 5.84 (m, 1H), 5.77 (d, 1H), 5.11 - 5.04 (m, 1H), 4.83 - 4.79 (m, 1H), 4.61 (d, 1H), 4.48 (td, 1H), 4.15 (dd, 1H), 4.09 (d, 1H), 4.00 (d, 1H), 3.76 (s, 1H), 3.66 (s, 1H), 3.64 - 3.54 (m, 2H), 3.45 (d, 1H), 3.25 (d, 1H), 2.49 (dd, 2H), 2.21 (t, 2H), 2.05 (s, 1H), 1.87 (d, 4H), 1.63 (d, 1H), 0.92 - 085 (m, 3H), 0.71 (s, 1H), 0.63 (d, 1H), 0.51 (d, 1H).

### Example 19

### 5 -Ethyl-6-fluoro-4-((5aS,6S,9R)-1-fluoro-12-((1-(((1S,2R,5R)-2-(hydroxymethyl)-3-aza bicyclo[3.1.0]hex-3-yl)methyl)cyclopropyl)methoxy)-5a,6,7,8,9,10-hexahydro-5H-4-ox a-3,10a,11,13,14-pentaaza-6,9-methanonaphtho[1,8-ab]heptalen-2-yl)naphthalen-2-ol 19

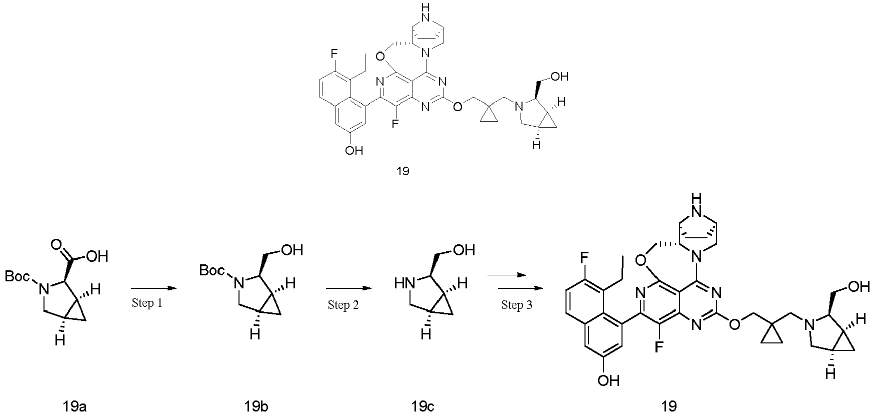

### Step 1

Tert-butyl (1*S*,2*R*,5*R*)-2-(hydroxymethyl)-3-azabicyclo[3.1.0]hexane-3-carboxylate **19b** (1*S*,2*R*,5*R*)-3-(*tert*-butoxycarbonyl)-3-azabicyclo[3.1.0]hexane-2-carboxylic acid **19a** (100 mg, 440 µmol, Nanjing PharmaBlock) was dissolved in tetrahydrofuran (2 mL), the reaction solution was added with a borane-tetrahydrofuran complex (0.55 mL, 1 M in THF) under an ice bath, stirred for 14 h, added with a borane-dimethylsulfide complex (0.2 mL, 10 M in THF), heated to 60 °C and reacted for 1 h, cooled to room temperature, added with methanol for quenching the reaction, and concentrated under reduced pressure to give crude title compound **19b** (94 mg), which was directly used in the next reaction without purification.

### Step 2

### ((1S,2R,5R)-3-azabicyclo[3.1.0]hex-2-yl)methanol 19c

Crude compound **19b** (94 mg, 440 µmol) was dissolved in dichloromethane (1.5 mL), the reaction solution was added with trifluoroacetic acid (0.5 mL) under an ice bath, stirred for 1 h, and concentrated under reduced pressure, the residue was dissolved in methanol, added with anhydrous potassium carbonate, stirred for 10 min, and then filtered, and the filtrate was concentrated under reduced pressure to give crude title compound **19c** (30 mg), which was directly used in the next reaction without purification.

MS m/z (ESI): 114.2 [M+1].

### Step 3

### 5 -Ethyl-6-fluoro-4-((5aS,6S,9R)-1 -fluoro-1 2-((1-(((1S,2R,5R)-2-(hydroxymethyl)-3-aza bicyclo[3.1.0]hex-3-yl)methyl)cyclopropyl)methoxy)-5a,6,7,8,9,10-hexahydro-5H-4-ox a-3,10a,11,13,14-pentaaza-6,9-methanonaphtho[1,8-ab]heptalen-2-yl)naphthalen-2-ol 19

Using the synthetic route in Example 15, the starting material *L*-prolinol of step 11 was replaced with compound **19c** to give title compound **19** (10 mg, yield: 20%).

MS m/z (ESI): 671.2 [M+1].

¹H NMR (500 MHz, CD₃OD): δ 7.67 (dd, 1H), 7.32 - 7.20 (m, 2H), 7.06 (dd, 1H), 5.15 - 4.94 (m, 2H), 4.69 - 4.61 (m, 1H), 4.48 (td, 1H), 4.16 (dd, 1H), 3.90 - 3.73 (m, 2H), 3.71 - 3.59 (m, 2H), 3.46 (dd, 1H), 3.30 - 3.13 (m, 2H), 2.68 (s, 1H), 2.58 (s, 1H), 2.49 (dd, 1H), 2.39 (s, 1H), 2.22 (q, 1H), 2.01 - .71 (m, 4H), 1.59 (s, 3H), 1.43 (d, 1H), 0.89 (dt, 4H), 0.56 (q, 2H), 0.39 (d, 1H), 0.27 (t, 1H).

### Example 20

### 5 -Ethyl-6-fluoro-4-((5aS,6S,9R)-1-fluoro-12-((1-(((1S,2S,5R)-2-(hydroxymethyl)-3-aza bicyclo[3.1.0]hex-3-yl)methyl)cyclopropyl)methoxy)-5a,6,7,8,9,10-hexahydro-5H-4-ox a-3,10a,11,13,14-pentaaza-6,9-methanonaphtho[1,8-ab]heptalen-2-yl)naphthalen-2-ol 20

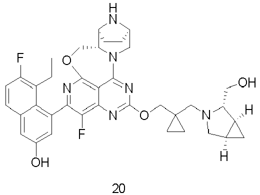

Using the synthetic route in Example 19, the starting material compound **19a** of step 1 was replaced with (1*S*,2*S*,5*R*)-3-(*tert*-butoxycarbonyl)-3-azabicyclo[3.1.0]hexane-2-carboxylic acid (Nanjing PharmaBlock) to give title compound **19** (2 mg, yield: 12.1%).

MS m/z (ESI): 671.2 [M+1].

¹H NMR (500 MHz, CD₃OD): δ 7.67 (ddd, 1H), 7.32 - 7.21 (m, 2H), 7.06 (dd, 1H), 5.13 - 5.01 (m, 1H), 4.66 - 4.58 (m, 2H), 4.54 - 4.43 (m, 2H), 4.30 (t, 1H), 4.16 (d, 1H), 3.65 m, 4H), 3.53 - 3.43 (m, 2H), 3.26 (d, 2H), 2.93 (m, 3H), 2.53 (m, 2H), 2.27 - 2.18 (m, 2H), 1.89 (m, 3H), 1.63 (s, 1H), 1.50 - 1.42 (m, 1H), 0.99 - 0.81 (m, 3H), 0.67 (s, 1H), 0.51 (s, 1H), 0.39 (s, 1H).

### Example 21

### 5 -Ethyl-6-fluoro-4-((5aS,6S,9R)-1-fluoro-12-((1-(((1R,2R,5S)-2-(hydroxymethyl)-3-aza bicyclo[3.1.0]hex-3-yl)methyl)cyclopropyl)methoxy)-5a,6,7,8,9,10-hexahydro-5H-4-ox a-3,10a,11,13,14-pentaaza-6,9-methanonaphtho[1,8-ab]heptalen-2-yl)naphthalen-2-ol 21

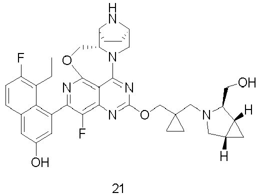

Using the synthetic route in Example 19, the starting material compound **19a** of step 1 was replaced with (1*R*,2*R*,5*S*)-3-(*tert*-butoxycarbonyl)-3-azabicyclo[3.1.0]hexane-2-carboxylic acid (Nanjing PharmaBlock) to give title compound **21** (2 mg, yield: 11%).

MS m/z (ESI): 671.2 [M+1].

¹H NMR (500 MHz, CD₃OD): δ 7.67 (td, 1H), 7.32 - 7.20 (m, 2H), 7.06 (dd, 1H), 5.07 (dd, 2H), 4.66 - 4.56 (m, 2H), 4.54 - 4.41 (m, 2H), 4.41 - 4.34 (m, 1H), 4.16 (dd, 1H), 3.78 (s, 1H), 3.68 (s, 1H), 3.61 (s, 1H), 3.47 (s, 1H), 3.27 (d, 1H), 2.97 - 2.85 (m, 2H), 2.58 (dd, 1H), 2.48 (d, 1H), 2.23 (dt, 2H), 2.05 (s, 1H), 1.99 - 1.83 (m, 3H), 1.62 (s, 1H), 1.46 (s, 2H), 0.89 (dt, 3H), 0.67 (s, 2H), 0.51 (s, 1H), 0.38 (s, 1H).

### Example 22

### 5 -Ethyl-6-fluoro-4-((5aS,6S,9R)-1-fluoro-12-((1-(((1R,2S,5S)-2-(hydroxymethyl)-3-aza bicyclo[3.1.0]hex-3-yl)methyl)cyclopropyl)methoxy)-5a,6,7,8,9,10-hexahydro-5H-4-ox a-3,10a,11,13,14-pentaaza-6,9-methanonaphtho[1,8-ab]heptalen-2-yl)naphthalen-2-ol 22

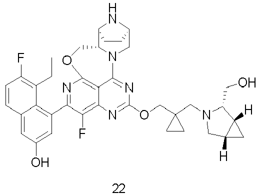

Using the synthetic route in Example 19, the starting material compound **19a** of step 1 was replaced with (1*R*,2*S*,5*S*)-3-(*tert*-butoxycarbonyl)-3-azabicyclo[3.1.0]hexane-2-carboxylic acid (Nanjing PharmaBlock) to give title compound **22** (10 mg, yield: 24.2%).

MS m/z (ESI): 671.2 [M+1].

¹H NMR (500 MHz, CD₃OD): δ 7.67 (ddd, 1H), 7.33 - 7.20 (m, 2H), 7.06 (dd, 1H), 5.07 (dd, 2H), 4.80 (d, 1H), 4.49 (td, 1H), 4.16 (dd, 1H), 3.93 (s, 1H), 3.79 (s, 1H), 3.72 - 3.58 (m, 2H), 3.47 (d, 1H), 3.30 - 3.16 (m, 2H), 2.69 (d, 1H), 2.58 (dd, 1H), 2.48 (h, 2H), 2.23 (dt, 1H), 2.05 (d, 1H), 2.00 - 1.71 (m, 4H), 1.69 - 1.55 (m, 2H), 1.45 (s, 1H), 0.89 (dt, 4H), 0.57 (d, 2H), 0.40 (d, 1H), 0.29 (s, 1H).

### Example 23

### 5-Ethyl-6-fluoro-4-((5aS,6S,9R)-1-fluoro-12-((1-((1-(hydroxymethyl)-3-azabicyclo[3.1. 0]hex-3-yl)methyl)cyclopropyl)methoxy)-5a,6,7,8,9,10-hexahydro-5H-4-oxa-3,10a, 11, 13,14-pentaaza-6,9-methanonaphtho[1,8-ab]heptalen-2-yl)naphthalen-2-ol (diastereomeric mixture) 23

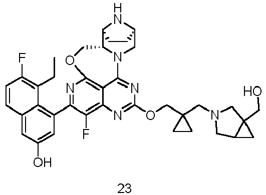

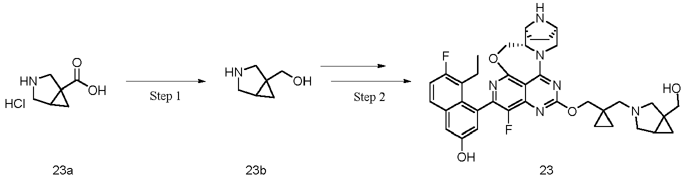

### Step 1

### (±)-(3-azabicyclo[3.1.0]hex-1-yl)methanol 23b

(±)-3-azabicyclo[3.1.0]hexane-1-carboxylic acid hydrochloride **23a** (100 mg, 611.2 µmol, Shanghai Accela) was dissolved in tetrahydrofuran (5 mL), and the reaction solution was added with a borane-tetrahydrofuran complex (3 mL, 1 M in THF) under an ice bath, stirred for 14 h, heated to 60 °C and reacted for 1 h, cooled to room temperature, added with methanol for quenching the reaction, and concentrated under reduced pressure to give crude title compound **23b** (70 mg), which was directly used in the next reaction without purification.

MS m/z (ESI): 114.2 [M+1].

### Step 2

### 5-Ethyl-6-fluoro-4-((5aS,6S,9R)-1-fluoro-12-((1-((1-(hydroxymethyl)-3-azabicyclo[3.1. 0]hex-3-yl)methyl)cyclopropyl)methoxy)-5a,6,7,8,9,10-hexahydro-5H-4-oxa-3,10a, 11, 13,14-pentaaza-6,9-methanonaphtho[1,8-ab]heptalen-2-yl)naphthalen-2-ol (diastereomeric mixture) 23

Using the synthetic route in Example 15, the starting material L-prolinol of step 11 was replaced with compound **23b** to give title compound **23** (10 mg, yield: 24.2%).

MS m/z (ESI): 671.2 [M+1].

¹H NMR (500 MHz, CD₃OD): δ7.68 - 7.66 (m, 1H), 7.30 - 7.26 (m, 2H), 7.11 (s, 1H),7.02 (s, 1H),5.07 - 5.01 (m, 1H),4.61 - 4.59 (m, 1H), 4.48 - 4.35(m, 3H), 4.18 - 4.16 (m, 1H), 3.77 - 3.76 (m, 1H), 3.67 - 3.65 (m, 2H), 3.57 - 3.54 (m, 1H), 3.27 - 3.17 (m, 3H), 2.57 - 2.46(m, 5H), 2.26 - 2.24 (m, 1H),1.93 - 1.85 (m, 4H), 1.35 - 1.26 (m, 3H), 0.94 - 0.92 (m, 2H), 0.86 - 0.83 (m, 2H), 0.68 - 0.65 (m, 1H), 0.51 - 0.48(m, 2H).

### Example 24

### 5-Ethyl-6-fluoro-4-((5aS,6S,9R)-1-fluoro-12-((3-(hydroxymethyl)tetrahydro-1H-pyrroli zin-7a(5H)-yl)methoxy)-5a,6,7,8,9,10-hexahydro-5H-4-oxa-3,10a, 11, 13, 14-pentaaza-6, 9-methanonaphtho[1,8-ab]heptalen-2-yl)naphthalen-2-ol (diastereomeric mixture) 24

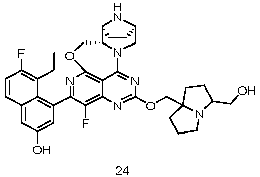

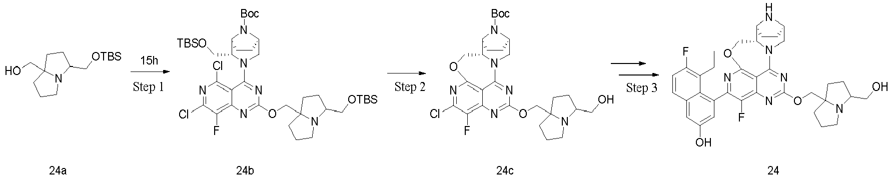

### Step 1

### Tert-butyl (1S,2S,5R)-2-(((tert-butyldimethylsilyl)oxy)methyl)-3-(2-((3-(((tert-butyldimethylsilyl) oxy)methyl)tetrahydro-1H-pyrrolizin-7a(5H)-yl)methoxy)-5,7-dichloro-8-fluoropyrido[ 4,3-d]pyrimidin-4-yl)-3,8-diazabicyclo[3.2.1]octane-8-carboxylate (diastereomeric mixture) 24b

Compound **15h** (300 mg, 494.2 µmol) was dissolved in tetrahydrofuran (5 mL), and the reaction solution was added with (3-(((*tert*-butyldimethylsilyl)oxy)methyl)tetrahydro-1*H*-pyrrolizin-7*a*(5*H*)-yl)methanol

(diastereomeric mixture) **24a** (142 mg, 497.3 µmol, prepared using the method disclosed in intermediate B-3 on page 115 of the specification in Patent Application "WO2020/146613"), added with 0.3 mL of 2 M sodium bis(trimethylsilyl)amide in tetrahydrofuran under an ice bath, stirred for 1 h, and concentrated under reduced pressure to give crude title compound **24b** (420 mg), which was directly used in the next reaction without purification.

MS m/z (ESI): 855.2 [M+1].

### Step 2

### Tert-butyl (5aS,6S,9R)-2-chloro-1-fluoro-12-((3-(hydroxymethyl)tetrahydro-1H-pyrrolizin-7a(5H) -yl)methoxy)-5a,6,7,8,9,10-hexahydro-5H-4-oxa-3,10a, 11,13,14-pentaaza-6,9-methano naphtho[1,8-ab]heptalen-14-carboxylate (diastereomeric mixture) 24c

Crude compound **24b** (423 mg, 494.1 µmol) was dissolved in tetrahydrofuran (5 mL), and the reaction solution was added with tetrabutylammonium fluoride (556 mg, 2.46 mmol), heated to 60 °C and stirred for 1 h, cooled to room temperature, added with water (10 mL) for dilution, and extracted with ethyl acetate (10 mL × 2). The organic phases were combined and concentrated under reduced pressure, and the residue was purified by silica gel column chromatography with eluent system A to give title compound **24c** (106 mg, yield: 36.2%).

MS m/z (ESI): 591.2 [M+1].

### Step 3

### 5-Ethyl-6-fluoro-4-((5aS,6S,9R)-1-fluoro-12-((3-(hydroxymethyl)tetrahydro-1H-pyrroli zin-7a(5H)-yl)methoxy)-5a,6,7,8,9,10-hexahydro-5H-4-oxa-3,10a,11,13,14-pentaaza-6, 9-methanonaphtho[1,8-ab]heptalen-2-yl)naphthalen-2-ol (diastereomeric mixture) 24

Using the synthetic route of step 12 and step 13 in Example 15, the starting material compound **15l** of step 12 was replaced with compound **24c** to give title compound **24** (30 mg, yield: 34.6%).

MS m/z (ESI): 645.2 [M+1].

¹H NMR (500 MHz, CD₃OD): δ 7.70 - 7.64 (m, 1H), 7.30 (d, 1H), 7.25 (td, 1H), 7.06 (m, 1H), 5.08 (dd, 1H), 4.62 (dd, 1H), 4.48 (td, 1H), 4.42 (s, 1H), 4.37 - 4.30 (m, 1H), 4.16 (dd, 1H), 3.86 (dd, 1H), 3.78 (d, 1H), 3.75 (s, 1H), 3.66 (s, 1H), 3.50 - 3.45 (m, 2H), 3.30 - 3.18 (m, 1H), 3.11 (s, 1H), 2.97 (s, 1H), 2.58 (dd, 1H), 2.49 (dd, 1H), 2.22 (dd, 2H), 2.07 (s, 1H), 1.95 - 1.78 (m, 7H), 1.63 (m, 1H), 0.88 (m, 3H).

### Example 25

### ((S)-1-((1-((((5aS,6S,9R)-2-(8-ethyl-7-fluoro-3-hydroxynaphthalen-1-yl)-1-fluoro-5a,6, 7,8,9,10-hexahydro-5H-4-oxa-3,10a,11,13,14-pentaaza-6,9-methanonaphtho[1,8-ab]hep talen-12-yl)oxy)methyl)cyclopropyl)methyl)pyrrolidin-2-yl)methyl dimethylcarbamate 25

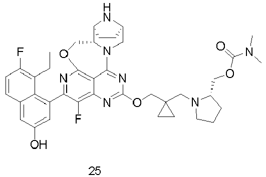

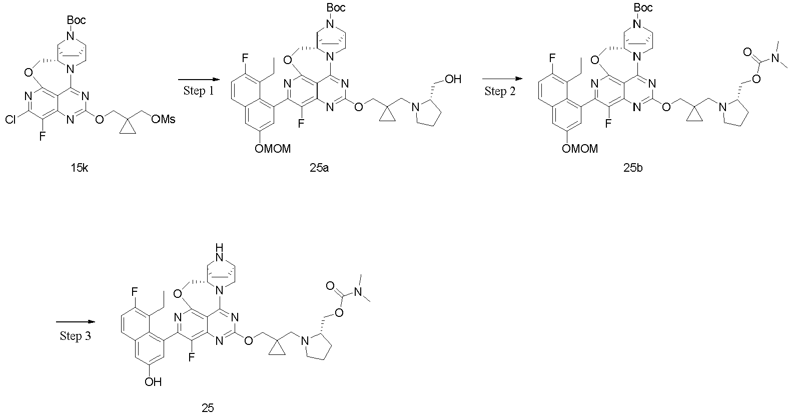

### Step 1

### Tert-butyl (5aS,6S,9R)-2-(8-ethyl-7-fluoro-3-(methoxymethoxy)naphthalen-1-yl)-1-fluoro-12-(1-(( (S)-2-(hydroxymethyl)pyrrolidin-1-yl)methyl)cyclopropyl)methoxy)-5a,6,7,8,9,10-hexa hydro-5H-4-oxa-3,10a,11,13,14-pentaaza-6,9-methanonaphtho[1,8-ab]heptalen-14-carb oxylate 25a

Using the synthetic route in Example 15, the starting material *L*-prolinol of step 11 was replaced with *D*-prolinol, with the use of the method of step 11 and step 12, to give title compound **25a** (184 mg, yield: 52.2%).

MS m/z (ESI): 803.2 [M+1].

### Step 2

### Tert-butyl (5aS,6S,9R)-12-((1-(((S)-2-(((dimethylaminocarbonyl)oxy)methyl)pyrrolidin-1-yl)meth yl)cyclopropyl)methoxy)-2-(8-ethyl-7-fluoro-3-(methoxymethoxy)naphthalen-1-yl)-1-fl uoro-5a,6,7,8,9,10-hexahydro-5H-4-oxa-3,10a,11,13,14-pentaaza-6,9-methanonaphtho[ 1,8-ab]heptalen-14-carboxylate 25b

Compound **25a** (120 mg, 149.5 µmol) and triethylamine (90 mg, 889.4 µmol) were dissolved in 5 mL of tetrahydrofuran, and the reaction solution was added with 4-nitrophenyl chloroformate (90 mg, 446.5 µmol),stirred for 5 h and then added with dimethylamine hydrochloride (242 mg, 3 mmol), stirred for 15 min, added with 10 mL of ethyl acetate for dilution, and sequentially washed with water and a saturated sodium chloride solution. The organic phase was concentrated under reduced pressure, and the residue was purified by silica gel column chromatography with eluent system B to give title compound **25b** (100 mg, yield: 76.5%).

MS m/z (ESI): 874.2 [M+1].

### Step 3

### ((S)-1-((1-((((5aS,6S,9R)-2-(8-ethyl-7-fluoro-3-hydroxynaphthalen-1-yl)-1-fluoro-5a,6, 7,8,9,10-hexahydro-5H-4-oxa-3,10a,11,13,14-pentaaza-6,9-methanonaphtho[1,8-ab]hep talen-12-yl)oxy)methyl)cyclopropyl)methyl)pyrrolidin-2-yl)methyl dimethylcarbamate 25

Compound **25b** (100 mg, 114.4 µmol) was dissolved in ethyl acetate (2 mL), the reaction solution was added with 0.5 mL of 4 M hydrochloride in dioxane under an ice bath, reacted for 2 h while maintaining the temperature, and concentrated under reduced pressure, and the residue was purified by high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **25** (25 mg, yield: 29.9%).

MS m/z (ESI): 730.2 [M+1].

¹H NMR (500 MHz, CD₃OD):δ 7.67 (t, 1H), 7.29 (q, 1H), 7.27 - 7.22 (m, 1H), 7.07 (dd, 1H), 5.14 - 5.04 (m, 1H), 4.61 (dd, 1H), 4.51 (td, 1H), 4.16 (dd, 1H), 4.05 - 3.92 (m, 2H), 3.80 (d, 2H), 3.65 (s, 1H), 3.50 (t, 2H), 3.29 - 3.18 (m, 1H), 2.88 (s, 3H), 2.78 (s, 3H), 2.61 - 2.42 (m, 2H), 2.37 - 2.18 (m, 2H), 1.92 (s, 5H), 1.79 (s, 3H), 1.66 - 1.53 (m, 2H), 0.88 (m, 3H), 0.69 (s, 2H), 0.60 (d, 1H), 0.47 (d, 1H).

### Biological Evaluation

### Test Example 1: Biological Evaluation of Inhibition Experiment for ERK Phosphorylation of AGS Cells (HTRF Method)

### I. Purpose

In the experiment, the inhibition effect of the compounds on ERK phosphorylation of the cells was detected, and the inhibition effect of the compounds disclosed herein on the KRAS target was evaluated according to the IC₅₀.

### II. Method

AGS cells (Nanjing Cobioer, CBP60476) were cultured in RPMI1640 (Hyclone, SH30809.01) complete medium containing 10% fetal bovine serum. On the first day of the experiment, the AGS cells were seeded into a 96-well plate with complete medium at a density of 40,000 cells/well to form 190 µL of cell suspension per well. The plate was incubated overnight in a cell incubator at 37 °C with 5% CO₂.

The next day, 10 µL of serially-diluted test compound prepared from the complete medium was added to each well. The final concentrations of the compound were 9 concentration points obtained by 5-fold serial dilution from 10 µM. A blank control containing 0.5% DMSO was set. The well plate was incubated in a cell incubator at 37 °C with 5% CO₂ for 1 h. After completion of incubation, the 96-well cell culture plate was taken out, the medium was removed by pipetting, 200 µL PBS (Shanghai BasalMedia Technologies Co., Ltd., B320) was added to each well, and the cells were washed once. PBS was removed by pipetting, 50 µL of lysis buffer (Cisbio, 64KL1FDF) containing a blocking solution (blocking reagent, Cisbio, 64KB1AAC) was added to each well, and the well plate was lysed on a shaker for 40 min at room temperature with shaking. After lysis, the lysate was pipetted and mixed well, 16 µL of lysate was transferred to two HTRF 96-well assay plates (Cisbio, 66PL96100) per well, and then 4 µL of premixed phosphorylated ERK1/2 antibody solution (Cisbio, 64AERPEG) or 4 µL of premixed total ERK1/2 antibody solution (Cisbio, 64NRKPEG) was added to these two plates. The microplate was sealed with a sealing membrane, centrifuged for 1 min in a microplate centrifuge, and incubated overnight at room temperature in the dark. On the third day, the fluorescence values at an excitation wavelength of 337 nm and at emission wavelengths of 665 nm and 620 nm were read using an ENVISION multifunctional microplate reader (PerkinElmer, ENVISION).

### III. Data analysis

IC₅₀ values for inhibitory activity of the compounds were calculated using Graphpad Prism software based on compound concentrations and phosphorylated ERK/total ERK ratio, and the results are shown in Table 1 below.

**Table 1. Inhibitory activity data for ERK phosphorylation of AGS cells**

| Compound No. | AGS/IC₅₀ (nM) |
|---|---|
| Diastereomeric (1:1) mixture of **1-p1** and **1-p2** | 94.1 |
| Diastereomeric (1:1) mixture of **2-p1** and **2-p2** | 6.6 |
| **3-p1** | 17.0 |
| Diastereomeric (1:1) mixture of **4-p1** and **4-p2** | 1.35 |
| **4-p2** | 86.5 |
| **4-p1** | 2.0 |
| **6-p1** | 1.2 |
| **8-p1** | 1.4 |
| **9-p2** | 60.4 |
| **9-p1** | 0.4 |
| Diastereomeric (1:1) mixture of **11-p1** and **11-p2** | 15.6 |
| Compound with shorter retention time in **12-p1** and **12-p2** | 1.1 |
| Compound with longer retention time in **12-p1** and **12-p2** | 57.4 |
| **14-p1** | 20.2 |
| **15** | 13.4 |
| **16** | 11.6 |
| **17** | 2.8 |
| **18** | 10.5 |
| **19** | 31.1 |
| **20** | 14.0 |
| **21** | 8.1 |
| **22** | 10.2 |
| **23** | 4.0 |
| **25** | 23.5 |

Conclusion: the compounds disclosed herein have a better inhibition effect on ERK phosphorylation of AGS cells.

### Test Example 2: Biological Evaluation of Inhibition Experiment for 3D Proliferation of GP2d and AGS Cells

### I. Purpose

The inhibition effect of the compounds disclosed herein on the KRAS target was evaluated by testing the 3D proliferation inhibition effect of the compounds disclosed herein on GP2d and AGS cells.

### II. Method

GP2d cells (Nanjing Cobioer, CBP60010) were cultured in a complete medium, namely DMEM/high glucose medium (Hyclone, SH30243.01) containing 10% fetal bovine serum (Corning, 35-076-CV). On the first day of the experiment, GP2d cells were seeded into a 96-well low adsorption plate (Corning, CLS7007-24EA) with complete medium at a density of 1,000 cells/well to form 90 µL of cell suspension per well. The plate was centrifuged at 2,000 rpm for 5 min at room temperature and then incubated overnight in a cell incubator at 37 °C with 5% CO₂.

AGS cells (Nanjing Cobioer, CBP60476) were cultured in a complete medium, namely RPMI1640 medium (Hyclone, SH30809.01) containing 10% fetal bovine serum (Corning, 35-076-CV). On the first day of the experiment, the AGS cells were seeded into a 96-well low adsorption plate (Corning, CLS7007-24EA) at a density of 1,000 cells/well using complete medium to form 90 µL of cell suspension per well. The plate was centrifuged at 2,000rpm for 5 min at room temperature and then incubated overnight in a cell incubator at 37 °C with 5% CO₂.

The next day, 10 µL of serially-diluted test compound prepared from the complete medium was added to each well. The final concentrations of the compound of GP2d cells were 9 concentration points obtained by 3-fold serial dilution from 1 µM, and the final concentrations of the compound of AGS cells were 9 concentration points obtained by 3-fold serial dilution from 10 µM. A blank control containing 0.5% DMSO was set. The well plate was incubated in a cell incubator at 37 °C with 5% CO₂ for 120 h. On the seventh day, the 96-well cell culture plate was taken out, 50 µL CellTiter-Glo^{®} 3D reagent (Promega, G9682) was added into each well, the plate was shaken at room temperature for 25 min, pipetted and mixed well, 50 µL of the solution was transferred into a white impermeable 96-well plate (PE, 6005290), and the luminescence signal values were read using a multifunctional microplate reader (PerkinElmer, ENVISION).

### III. Data analysis

IC₅₀ values for inhibitory activity of the compounds were calculated using Graphpad Prism software, and the results are shown in Table 2 below.

**Table 2. Inhibitory activity data for 3D proliferation of AGS and GP2d cells**

| Compound No. | AGS /IC₅₀ (nM) | GP2d /IC₅₀ (nM) |
|---|---|---|
| Diastereomeric (1:1) mixture of **2-p1** and **2-p2** | - | 19.9 |
| **3-p1** | 95.3 | 13.0 |
| **4-p1** | 14.5 | 0.8 |
| **6-p1** | 32.9 | 7.2 |
| **8-p1** | 50.3 | 1.3 |
| **9-p1** | 5.8 | 0.9 |
| Diastereomeric (1:1) mixture of **11-p1** and **11-p2** | - | 62.5 |
| Compound with shorter retention time in **12-p1** and **12-p2** | 25.8 | 4.4 |
| **15** | - | 7.2 |
| **16** | - | 10.1 |
| **17** | 52.2 | 4.9 |
| **18** | - | 39.3 |
| **19** | - | 28.9 |
| **20** | - | 8.8 |
| **21** | - | 14.7 |
| **22** | - | 9.6 |
| **23** | 108.3 | 8.8 |
| **25** | - | 48.5 |

Conclusion: the compounds disclosed herein have a better inhibition effect on 3D proliferation of AGS and GP2d cells.

### Test Example 3: SPR Method for Detecting Affinity of Compounds Disclosed Herein for KRAS Protein Subtype G12D or WT

Biotinylated Avi-KRAS-WT or Avi-KRAS-G12D was diluted to 20 µg/mL with 1 × HBS-P+ (Cat. # BR1006-71) buffer containing 100 mM MgCl₂, and then flowed through SA (Cat. # BR1005-31) biosensing chip channel 2 for 420 s to obtain a coupling level of approximately 5000-7000 RU. Then samples of small molecular compounds were injected for 120 s in an ascending order, and then were dissociated for 720 s. The experiment employed a single-cycle kinetic mode. Reaction signals were detected in real time using a Biacore 8K instrument to obtain association and dissociation curves. After the experiment ended, data analysis was performed using Biacore 8K evaluation software, and affinity data were obtained by performing data fitting using a 1:1 model.

**Table 3. Affinity data of compounds for KRAS protein subtype G12D or WT**

| Compound No. | G12D KD (unit mol/L) | WT KD (unit mol/L) |
|---|---|---|
| Diastereomeric (1:1) mixture of **2-p1** and **2-p2** | 0.4 E-09 | 32.4 E-09 |
| **4-p1** | 0.14E-09 | 3.4E-09 |
| **6-p1** | 0.1E-09 | 7.0E-09 |
| **8-p1** | 0.1E-09 | 3.8E-09 |
| **9-p1** | 0.03E-09 | 1.54E-09 |
| Compound with shorter retention time in **12-p1** and **12-p2** | 0.3E-09 | 7.6E-09 |

Conclusion: the compounds disclosed herein have a better affinity for KRAS protein subtype G12D or WT.

## Claims

1. A compound of general formula (I) or a pharmaceutically acceptable salt thereof: 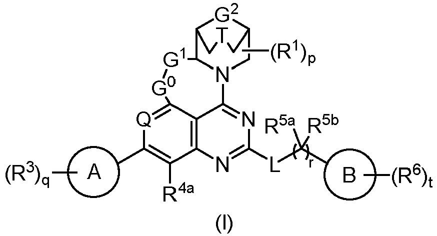 wherein:
G⁰ is selected from the group consisting of O, S, S(O), S(O)₂, CR^{G0a}R^{G0b} and NR^{G0c};
G¹ is selected from the group consisting of CR^{G1a}R^{G1b}, CR^{G1a}R^{G1b}CR^{G1c}R^{G1d}, C=O and
C(O)CR^{G1a}R^{G1b};
G² is NR^{d};
T is a chemical bond or is selected from the group consisting of CR^{a}R^{b}, NR^{T} and O;
Q is N or CR^{2a};
ring A is aryl or heteroaryl;
ring B is selected from the group consisting of cycloalkyl, heterocyclyl, aryl and
heteroaryl;
L is selected from the group consisting of a single bond, O and NR^{e};
R^{a}, R^{b}, R^{G0a}, R^{G0b}, R^{G1a}, R^{G1b}, R^{G1c} and R^{G1d} are identical or different and are each independently selected from the group consisting of a hydrogen atom, halogen, alkyl, alkoxy, haloalkyl, haloalkoxy, cyano, amino, nitro, hydroxy, hydroxyalkyl, cycloalkyl and heterocyclyl; or, R^{G1a} and R^{G1b}, together with the carbon atom to which they are attached, form cycloalkyl; or, R^{G1c} and R^{G1d}, together with the carbon atom to which they are attached, form cycloalkyl;
each R¹ is identical or different and is independently selected from the group consisting of a hydrogen atom, halogen, alkyl, alkenyl, alkynyl, alkoxy, haloalkyl, haloalkoxy, cyano, amino, -(CH₂)ᵤ-NR^{f}R^{g}, hydroxy and hydroxyalkyl;
R^{2a} and R^{4a} are identical or different and are each independently selected from the group consisting of a hydrogen atom, halogen, alkyl, alkenyl, alkynyl, alkoxy, haloalkyl, haloalkoxy, cyano, amino, -(CH₂)ᵥ-NR^{h}Rⁱ, hydroxy, hydroxyalkyl and cycloalkyl;
each R³ and R⁶ are identical or different and are each independently selected from the group consisting of a hydrogen atom, halogen, alkyl, alkenyl, alkynyl, alkoxy, haloalkyl, haloalkoxy, cyano, amino, -(CH₂)_{w}-NR^{j}R^{k}, -(CH₂)_{w1}-(O)_{z1}-C(O)NR^{j1}R^{k1}, -(CH₂)_{w2}-(O)_{z2}-C(O)OR^{j2}, nitro, hydroxy, hydroxyalkyl, cycloalkyl, heterocyclyl, aryl and heteroaryl;
R^{5a} and R^{5b} are identical or different and are each independently selected from the group consisting of a hydrogen atom, halogen, alkyl, haloalkyl, cyano, hydroxy and hydroxyalkyl; or
R^{5a} and R^{5b}, together with the carbon atom to which they are attached, form cycloalkyl or heterocyclyl, and the cycloalkyl or heterocyclyl is independently optionally substituted with one or more of the identical or different substituents selected from the group consisting of halogen, alkyl, haloalkyl, alkoxy, haloalkoxy, cyano, amino, hydroxy and hydroxyalkyl;
R^{G0c}, R^{T}, R^{d}, R^{e}, R^{f}, R^{g}, R^{h}, Rⁱ, R^{j}, R^{k}, R^{j1}, R^{k1} and R^{j2} are identical or different and are each independently selected from the group consisting of a hydrogen atom, alkyl, alkenyl, alkynyl, haloalkyl, hydroxyalkyl, cycloalkyl, heterocyclyl, aryl and heteroaryl;
u, v, w, w1 and w2 are identical or different and are each independently selected from the group consisting of 0, 1, 2 and 3;
z1 is 0 or 1;
z2 is 0 or 1;
r is 0, 1, 2 or 3;
p is 0, 1, 2, 3, 4 or 5;
q is 0, 1, 2, 3, 4 or 5; and
t is 0, 1, 2, 3, 4 or 5.

2. The compound or the pharmaceutically acceptable salt thereof according to claim 1, wherein G¹ is CH₂.

3. The compound or the pharmaceutically acceptable salt thereof according to claim 1 or 2, wherein G⁰ is O.

4. The compound or the pharmaceutically acceptable salt thereof according to any one of claims 1 to 3, being a compound of general formula (II) or a pharmaceutically acceptable salt thereof: 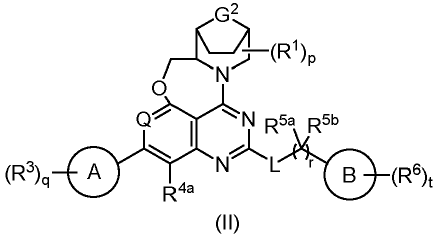 wherein ring A, ring B, G², Q, L, R¹, R³, R^{4a}, R^{5a}, R^{5b}, R⁶, p, q, r and t are as defined in claim 1.

5. The compound or the pharmaceutically acceptable salt thereof according to any one of claims 1 to 4, wherein Q is N.

6. The compound or the pharmaceutically acceptable salt thereof according to any one of claims 1 to 5, wherein ring A is naphthyl.

7. The compound or the pharmaceutically acceptable salt thereof according to any one of claims 1 to 6, wherein ring B is 3-8 membered heterocyclyl.

8. The compound or the pharmaceutically acceptable salt thereof according to any one of claims 1 to 7, wherein R^{4a} is a hydrogen atom or halogen.

9. The compound or the pharmaceutically acceptable salt thereof according to any one of claims 1 to 8, wherein G² is NH.

10. The compound or the pharmaceutically acceptable salt thereof according to any one of claims 1 to 9, wherein L is O.

11. The compound or the pharmaceutically acceptable salt thereof according to any one of claims 1 to 10, wherein R¹ is a hydrogen atom.

12. The compound or the pharmaceutically acceptable salt thereof according to any one of claims 1 to 11, wherein each R³ is identical or different and is independently selected from the group consisting of a hydrogen atom, halogen, C₁₋₆ alkyl, C₂₋₆ alkynyl, C₁₋₆ haloalkyl, hydroxy, C₁₋₆ hydroxyalkyl and 3-8 membered cycloalkyl.

13. The compound or the pharmaceutically acceptable salt thereof according to any one of claims 1 to 12, wherein R^{5a} and R^{5b} are hydrogen atoms; or R^{5a} and R^{5b}, together with the carbon atom to which they are attached, form 3-6 membered cycloalkyl.

14. The compound or the pharmaceutically acceptable salt thereof according to any one of claims 1 to 13, wherein each R⁶ is identical or different and is independently selected from the group consisting of a hydrogen atom, halogen, C₁₋₆ hydroxyalkyl and -CH₂-O-C(O)NR^{j1}R^{k1}, and R^{j1} and R^{k1} are identical or different and are each independently a hydrogen atom or C₁₋₆ alkyl.

15. The compound or the pharmaceutically acceptable salt thereof according to any one of claims 1 to 14, being the following compound:

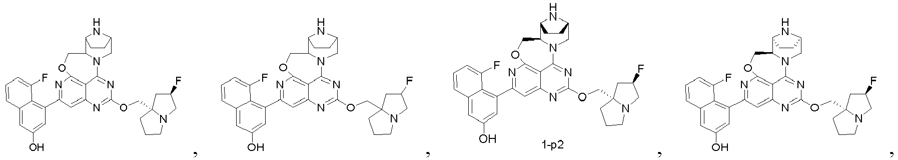

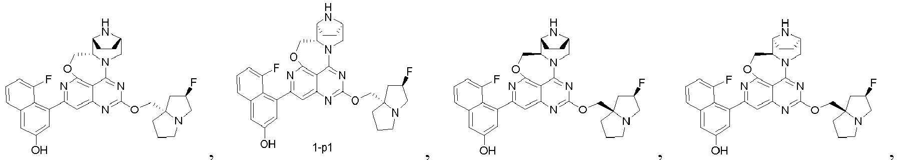

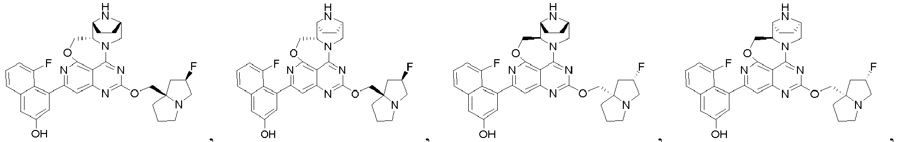

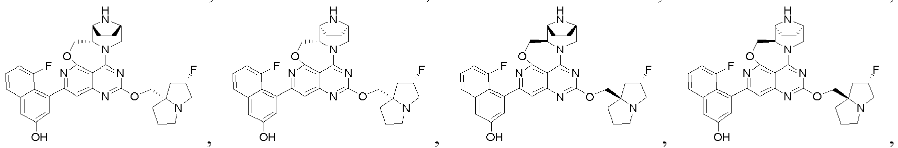

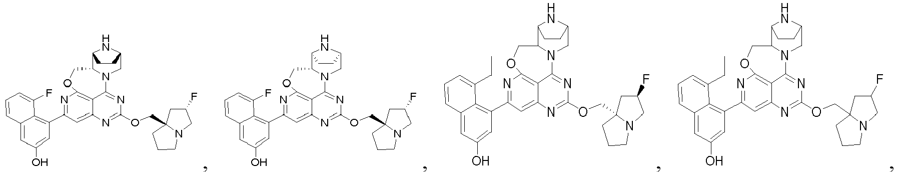

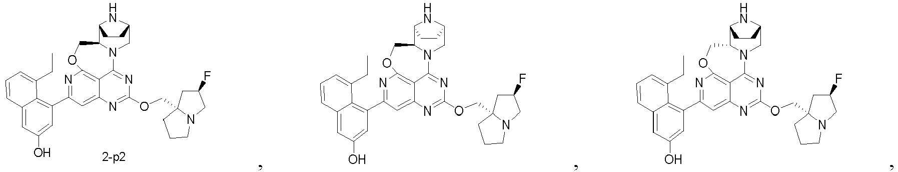

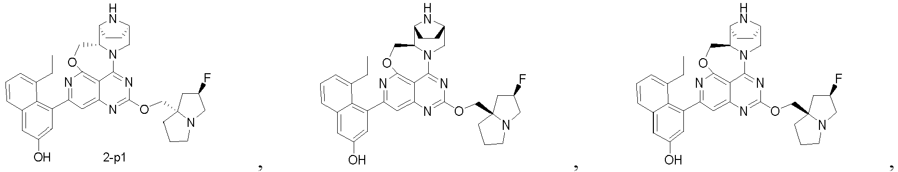

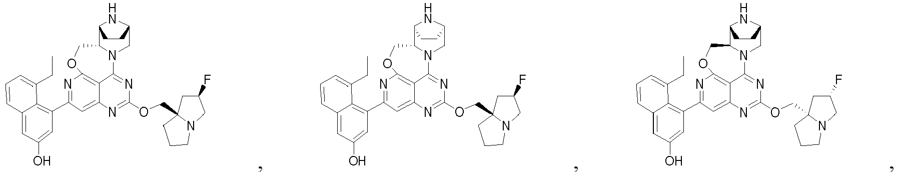

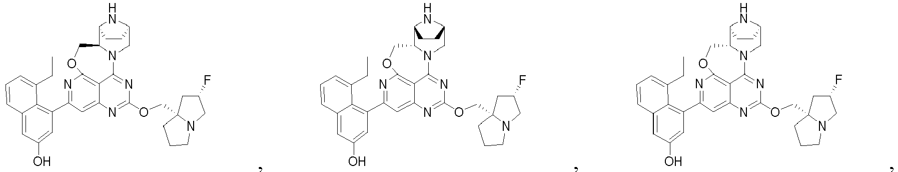

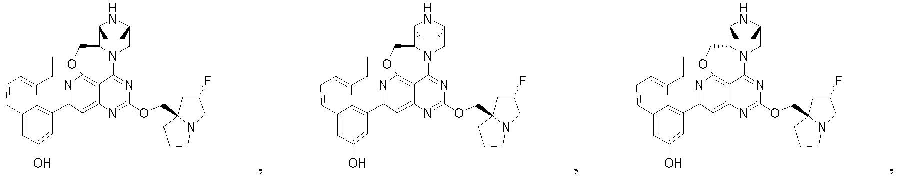

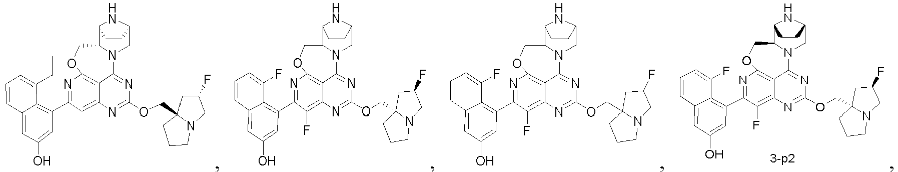

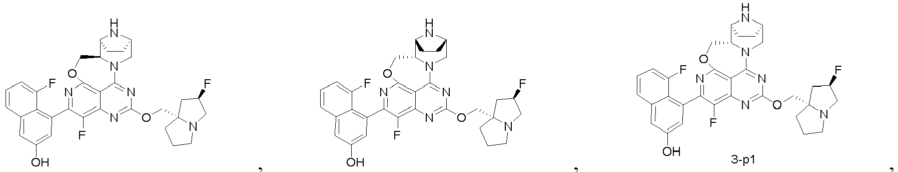

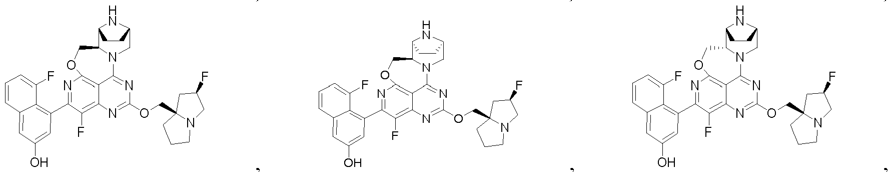

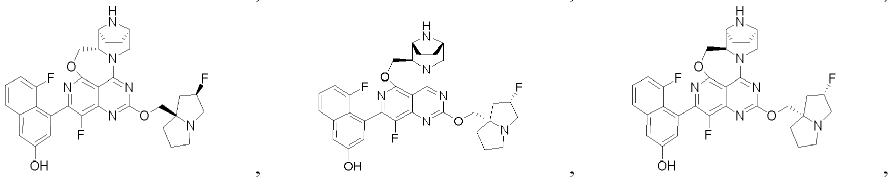

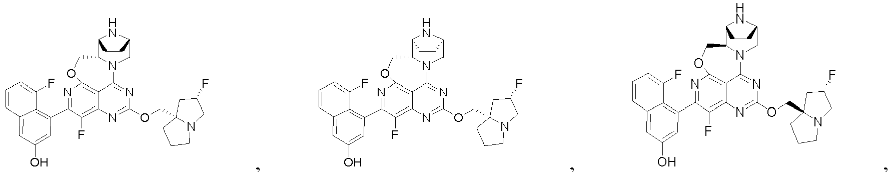

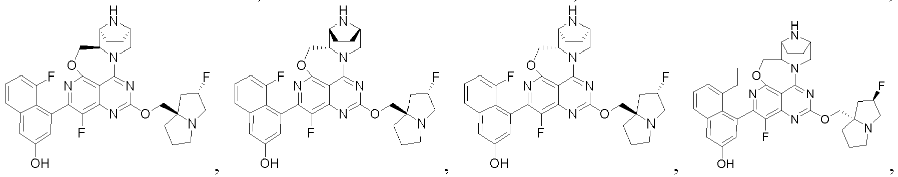

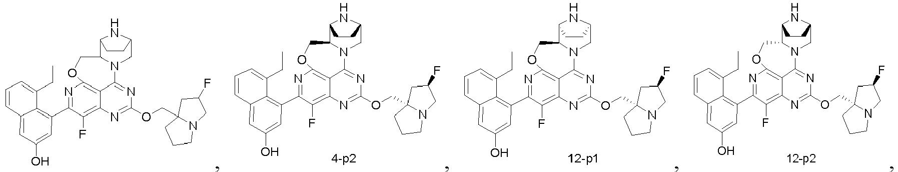

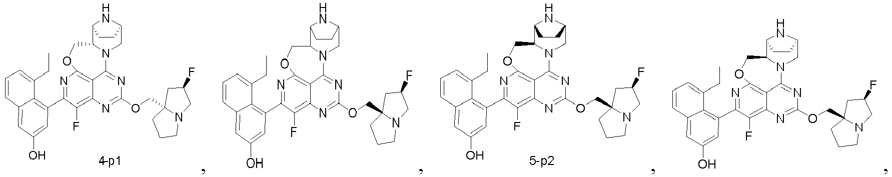

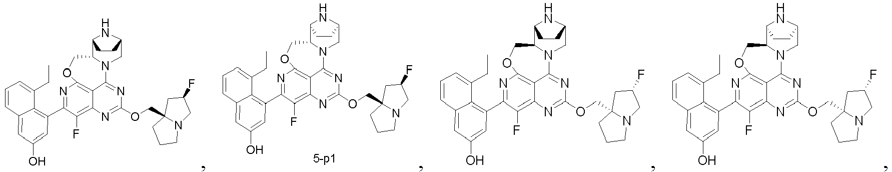

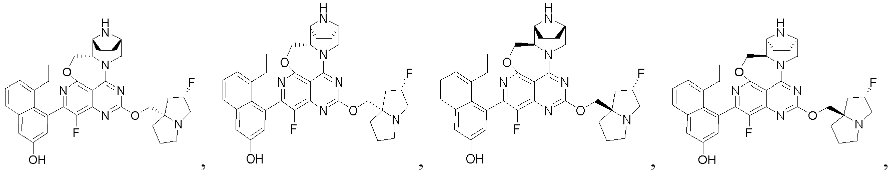

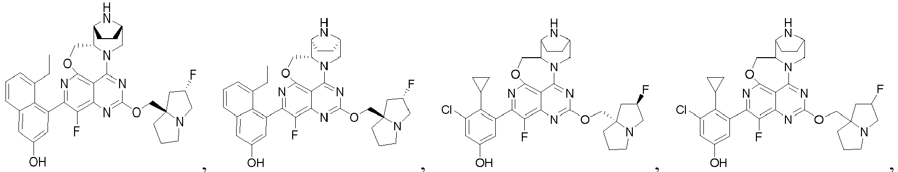

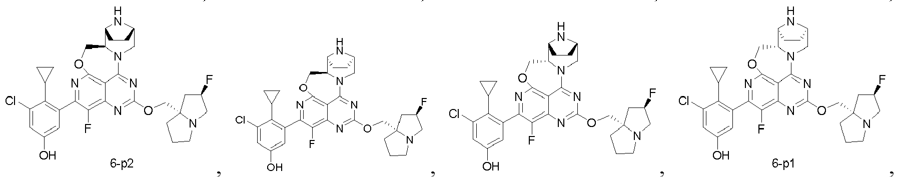

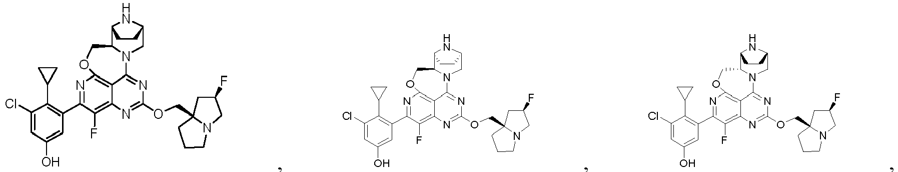

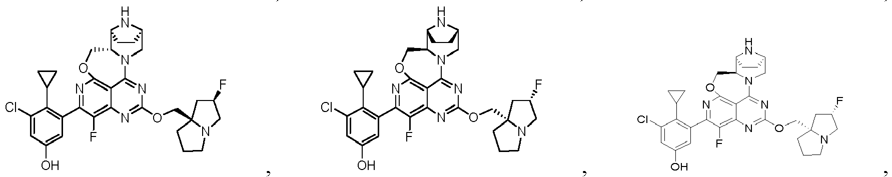

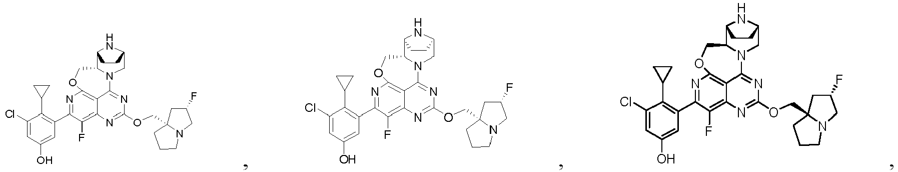

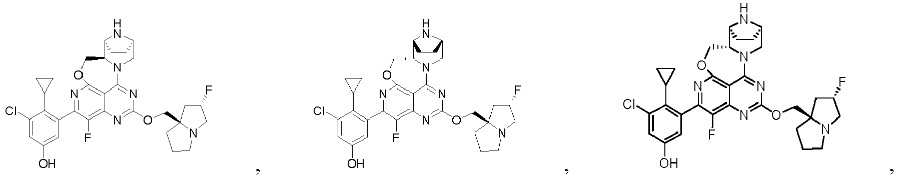

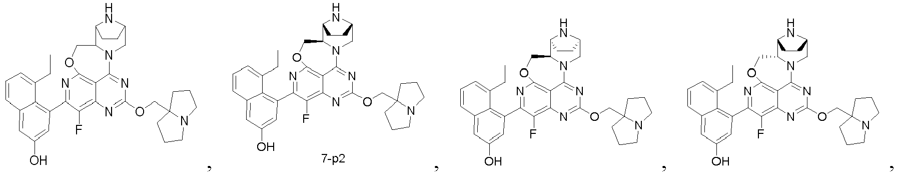

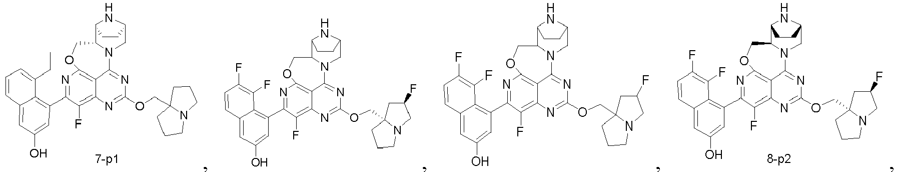

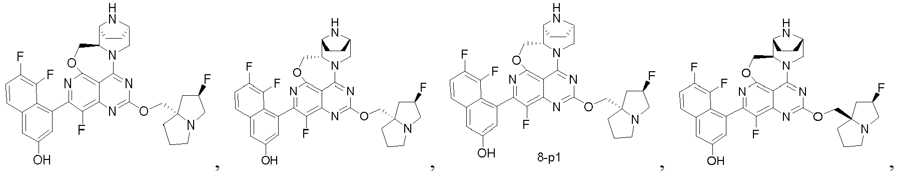

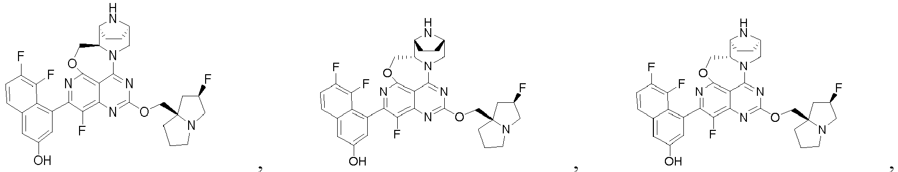

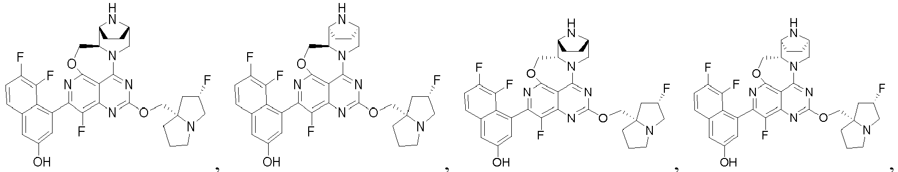

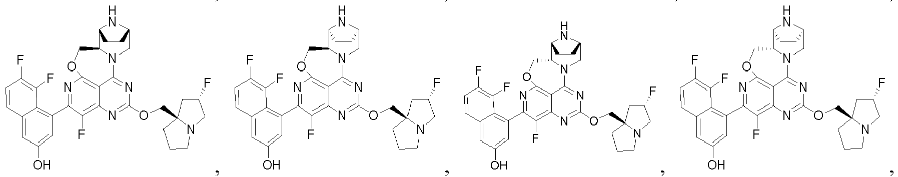

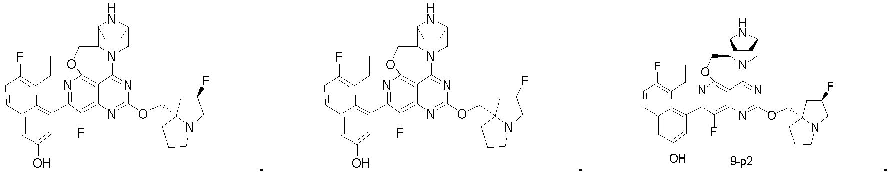

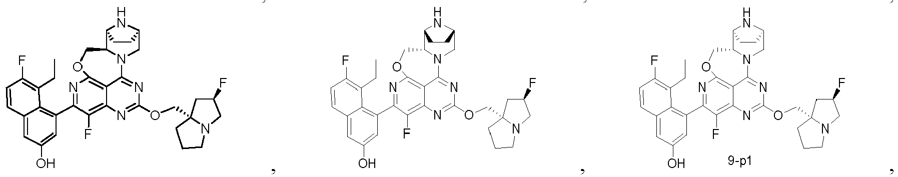

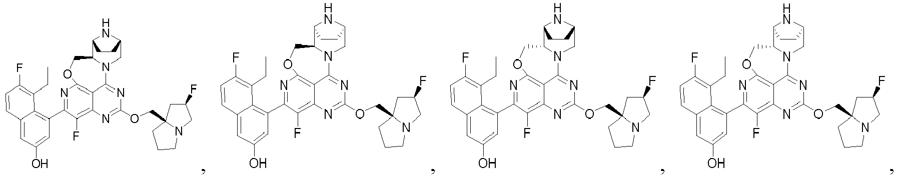

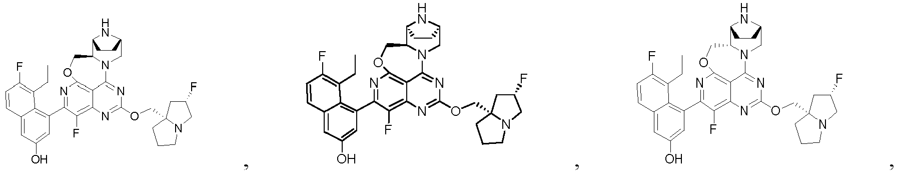

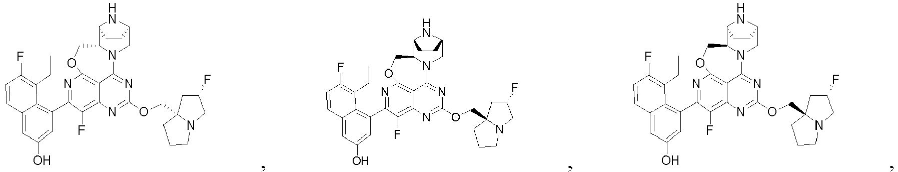

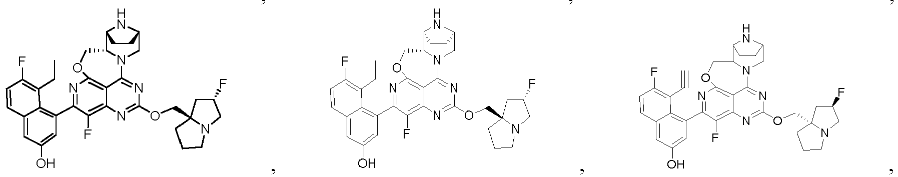

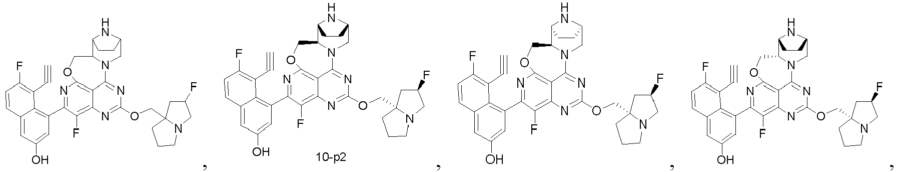

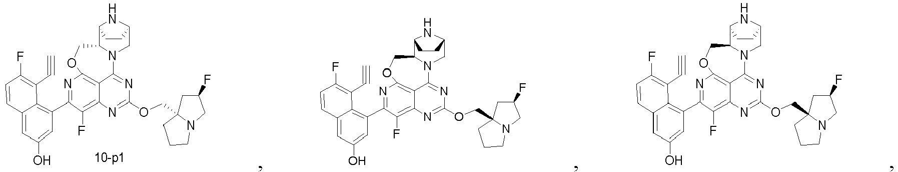

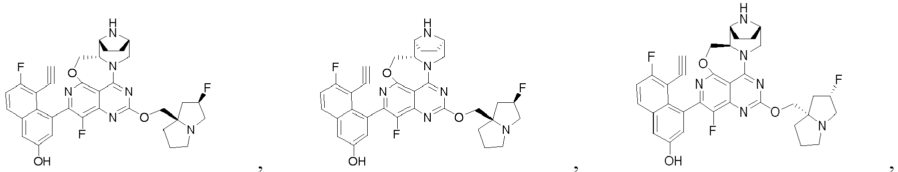

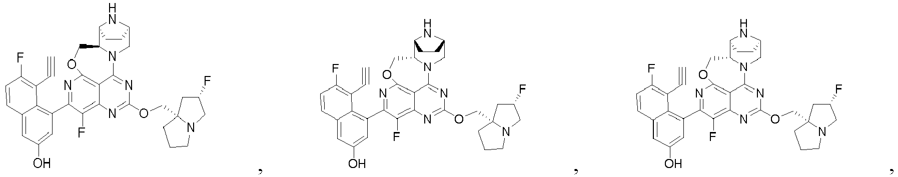

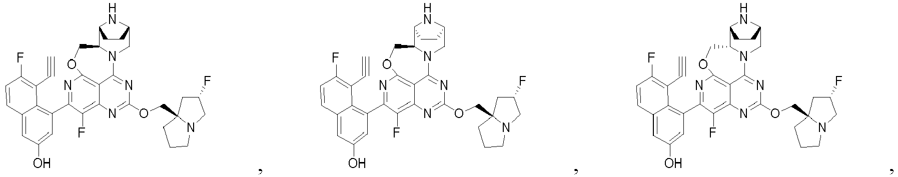

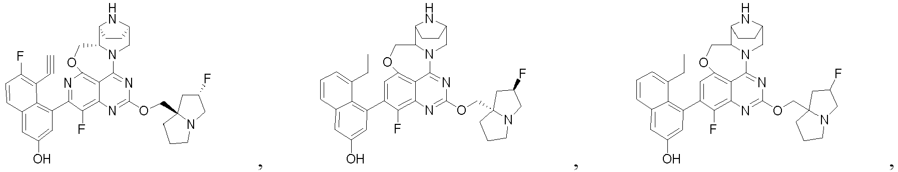

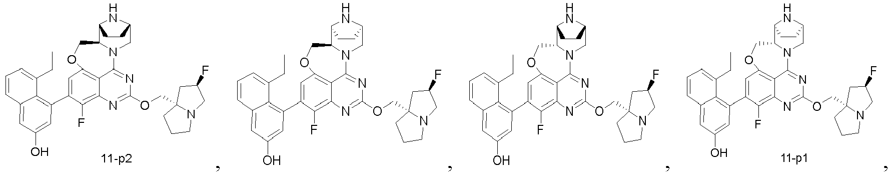

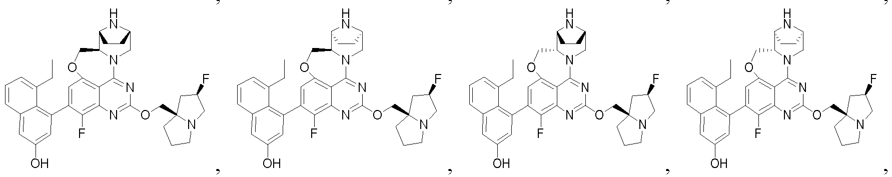

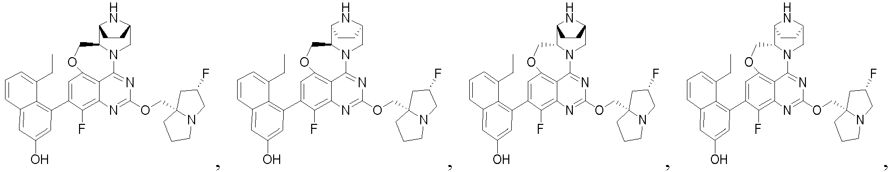

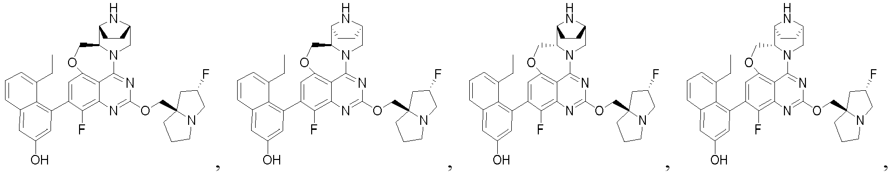

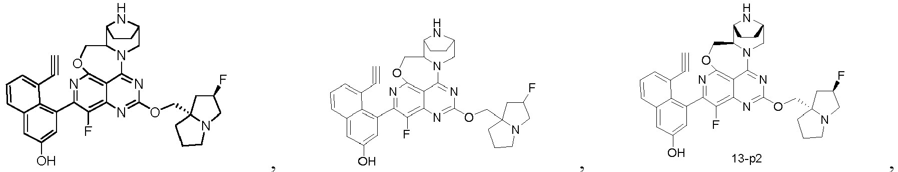

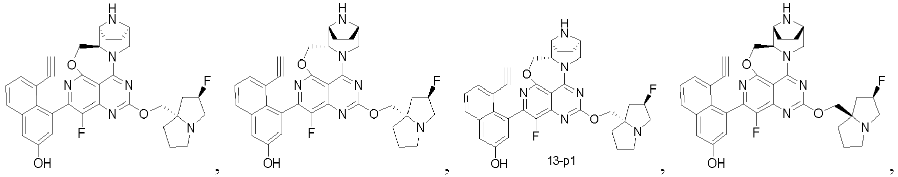

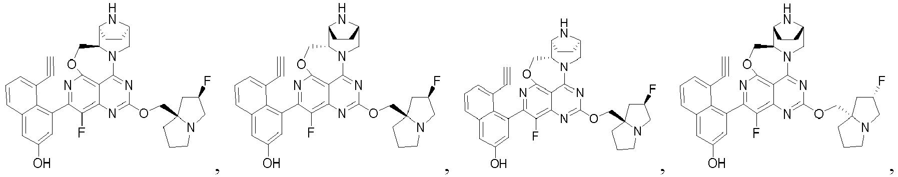

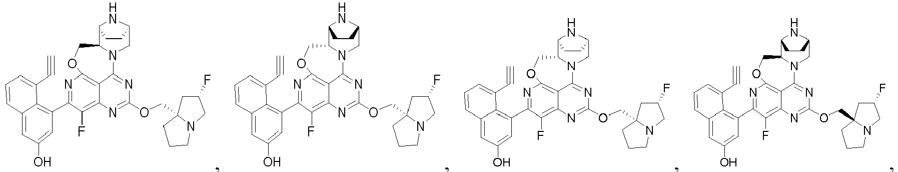

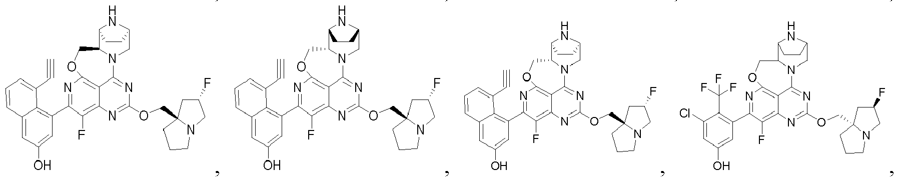

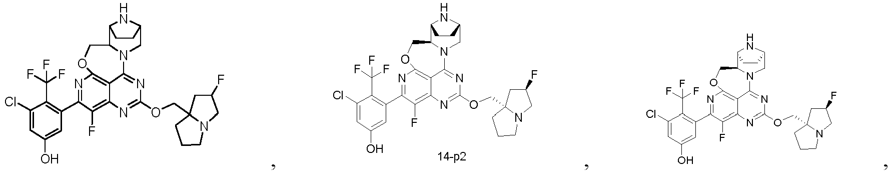

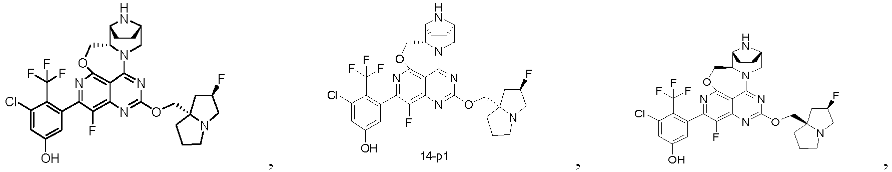

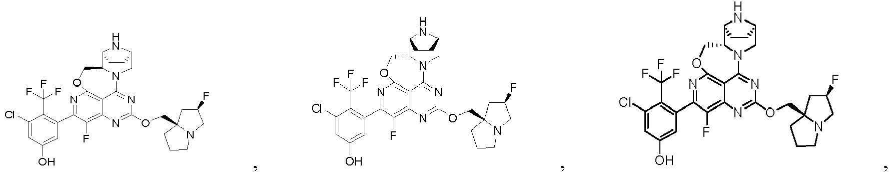

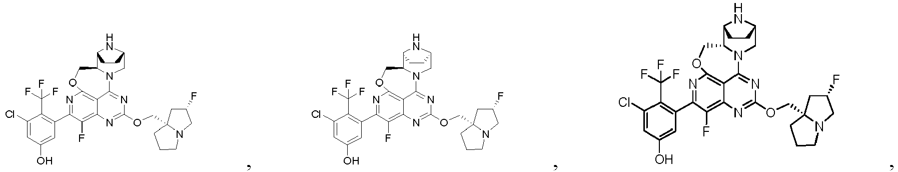

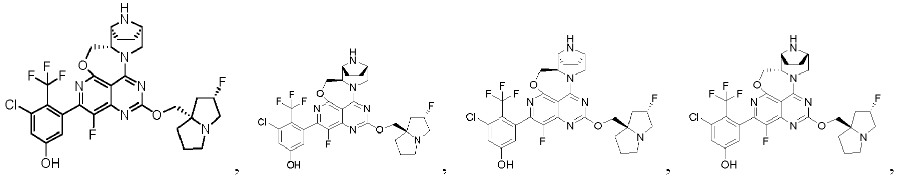

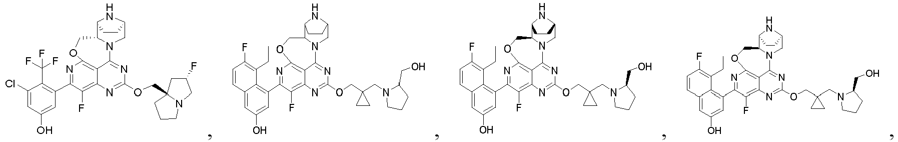

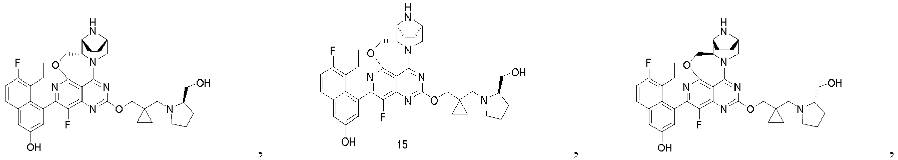

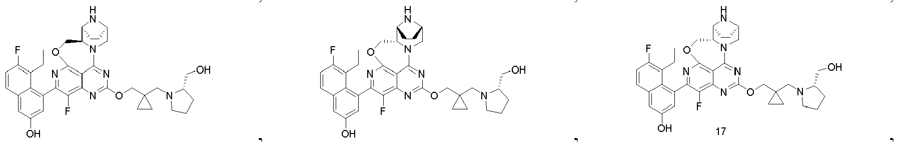

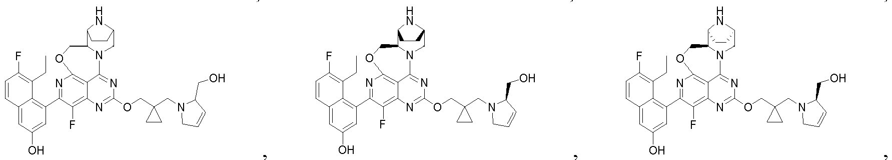

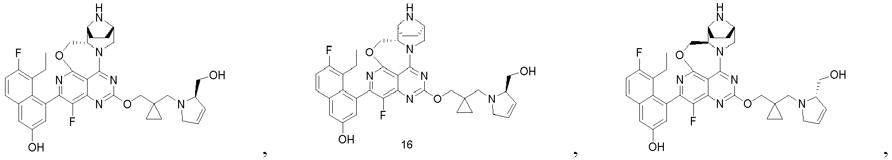

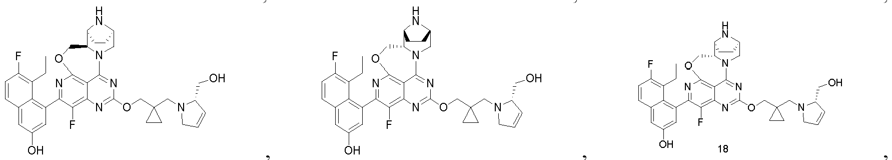

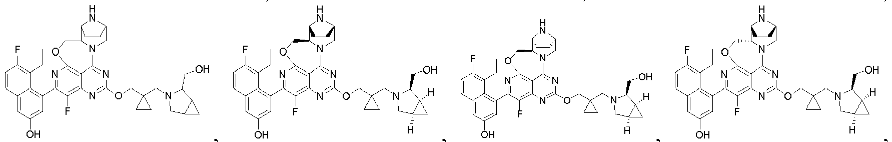

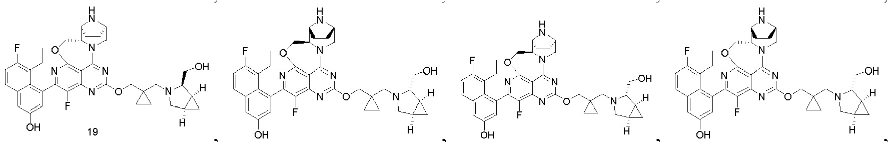

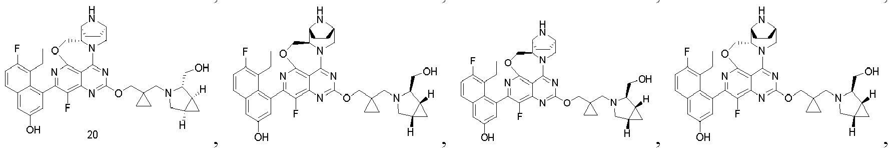

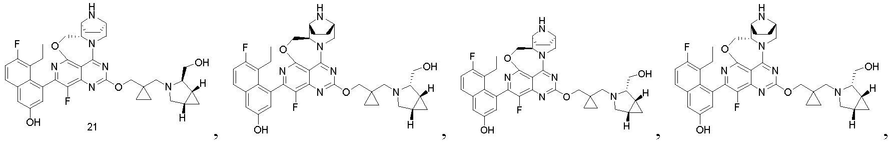

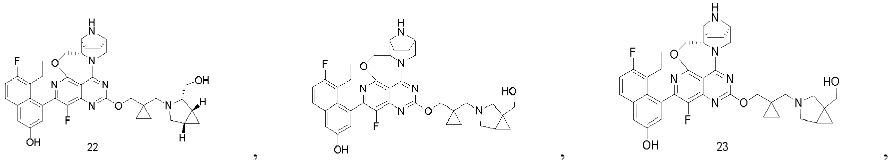

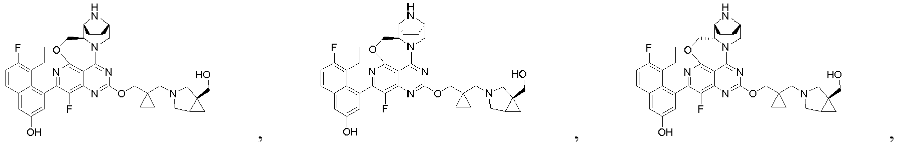

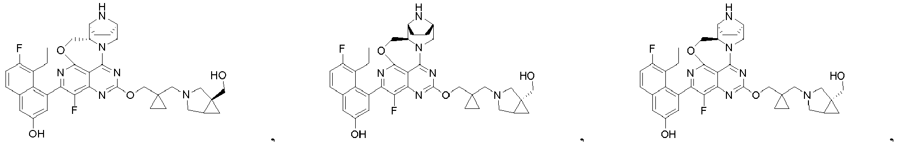

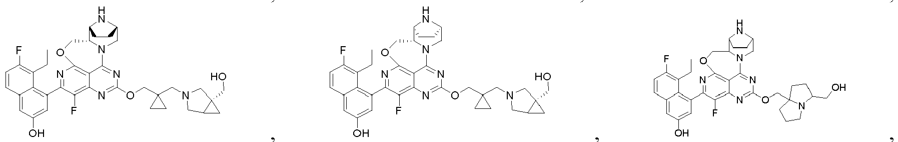

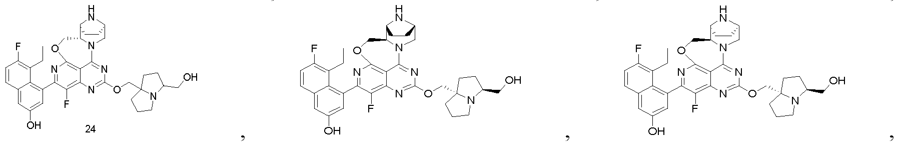

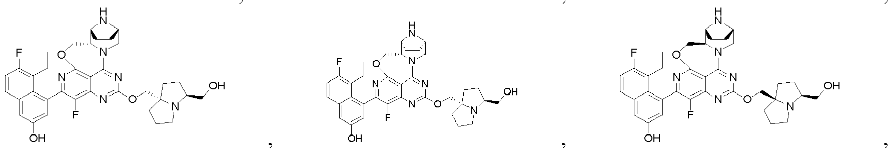

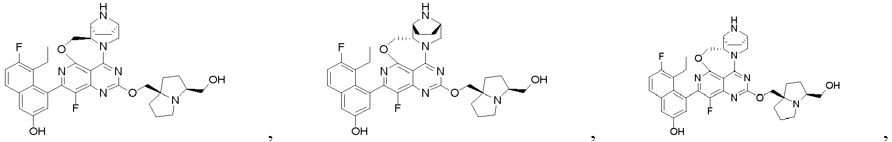

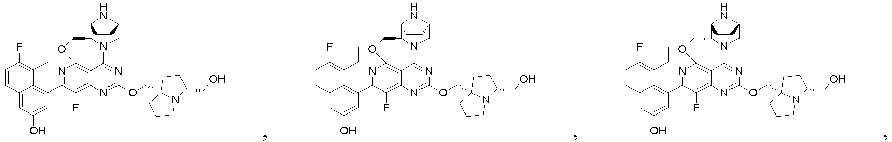

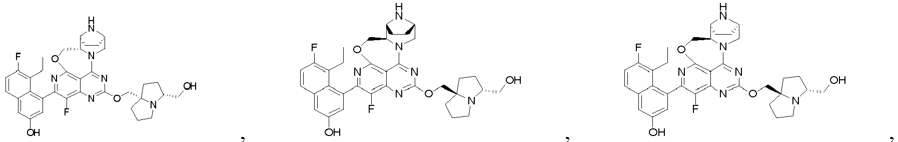

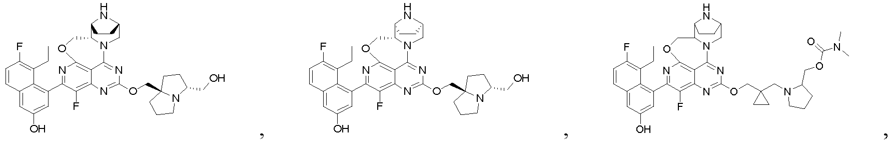

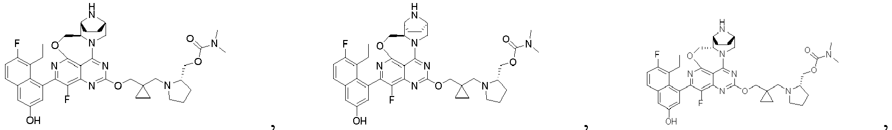

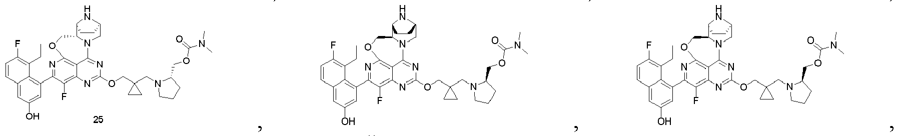

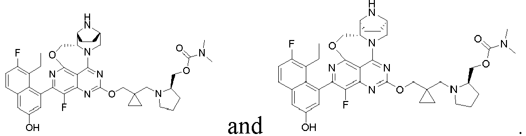

16. A compound of general formula (I'A) or a salt thereof: 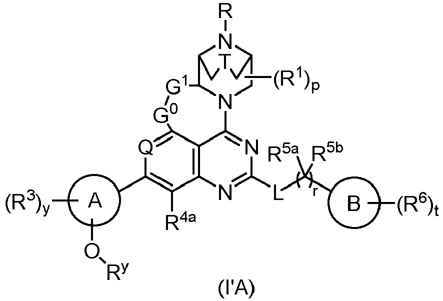 wherein,
R is an amino protecting group, and preferably Boc;
R^{y} is a hydroxy protecting group, and preferably MOM;
y is 0, 1, 2, 3 or 4;
G⁰, G¹, T, ring A, ring B, Q, L, R¹, R³, R^{4a}, R^{5a}, R^{5b}, R⁶, p, r and t are as defined in claim 1.

17. A compound selected from the group consisting of the following compounds:

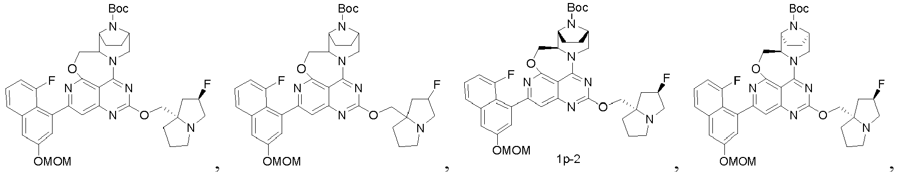

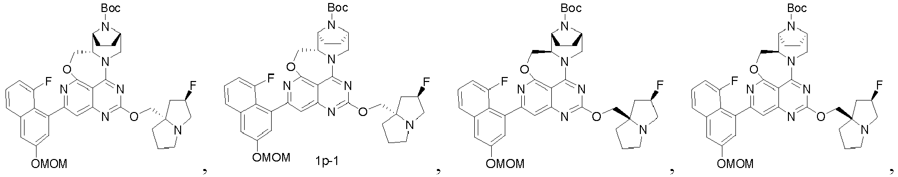

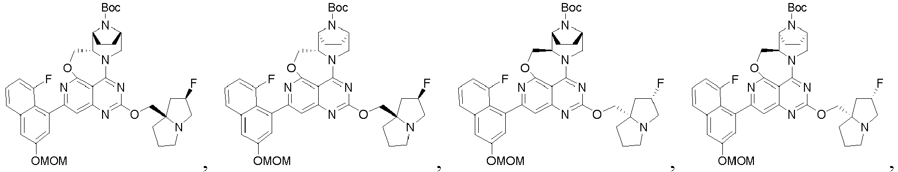

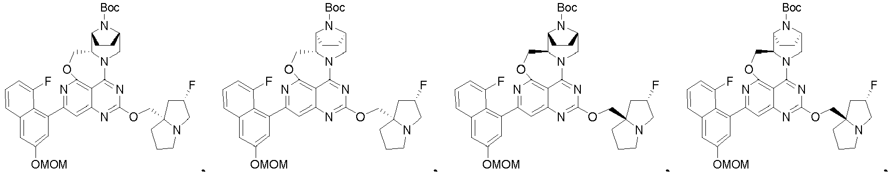

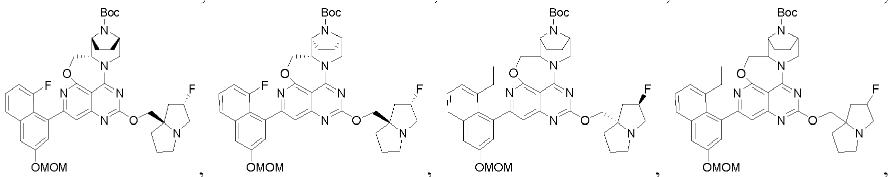

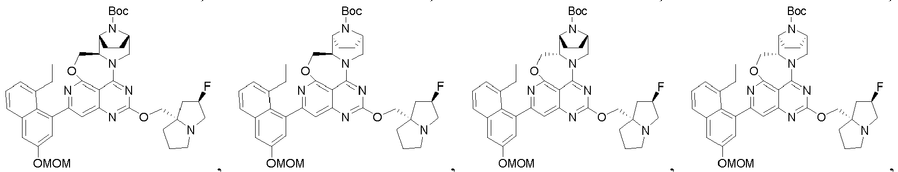

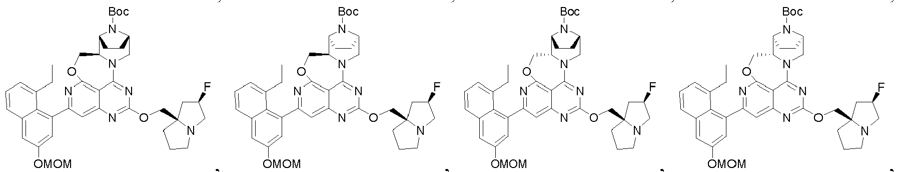

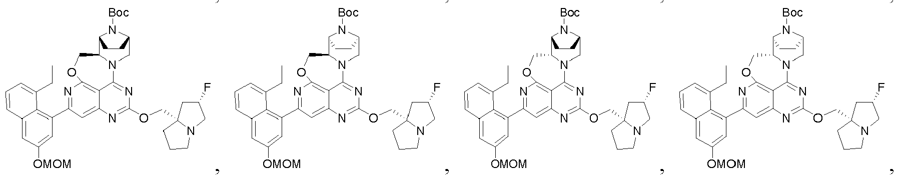

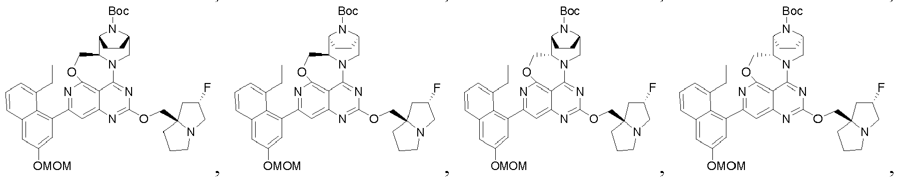

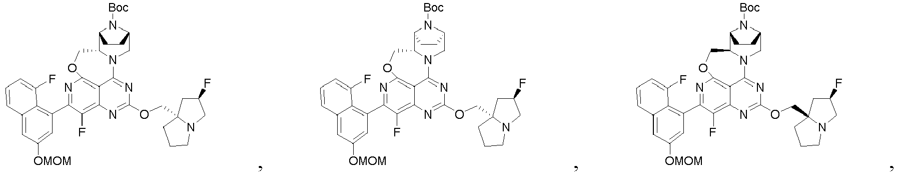

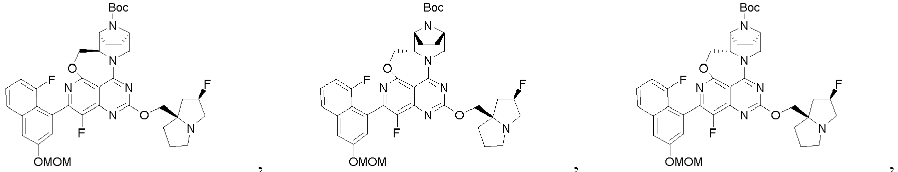

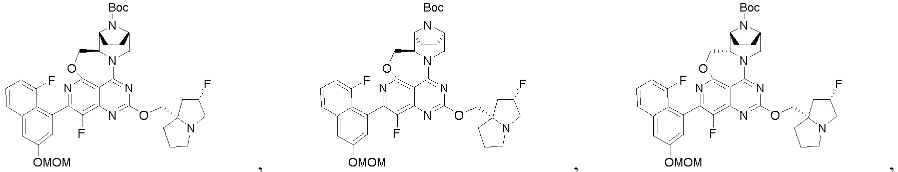

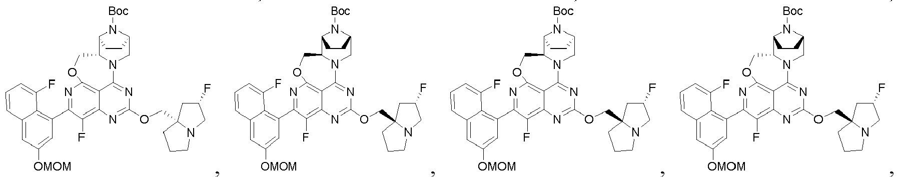

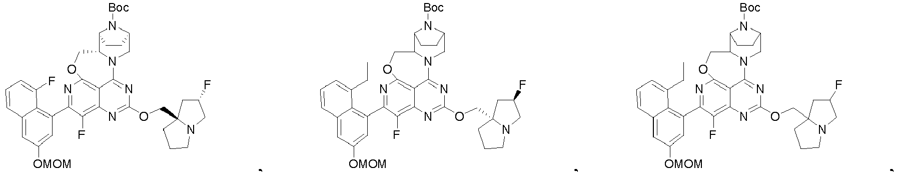

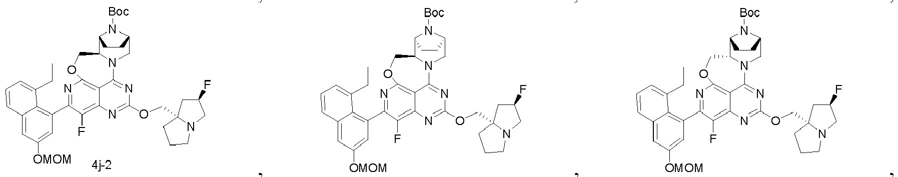

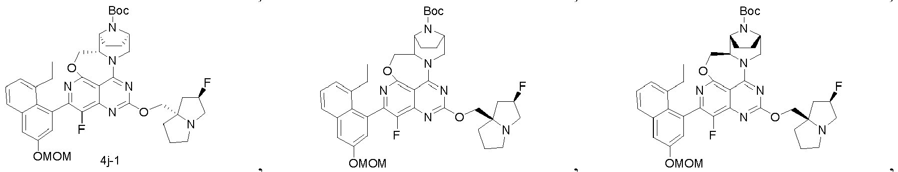

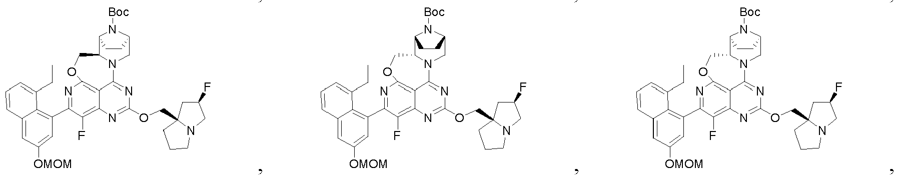

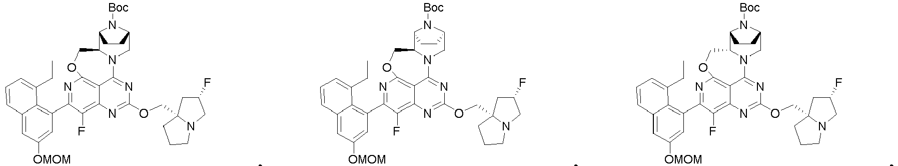

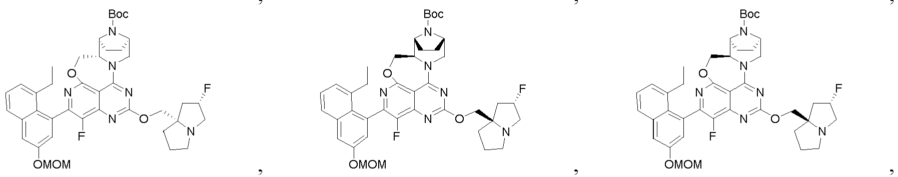

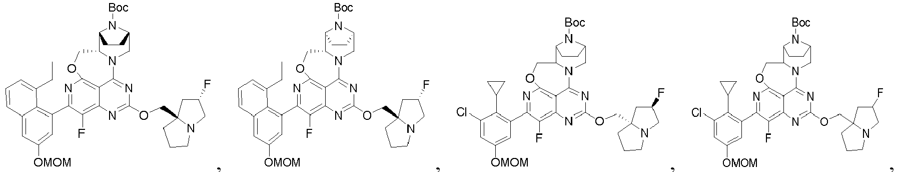

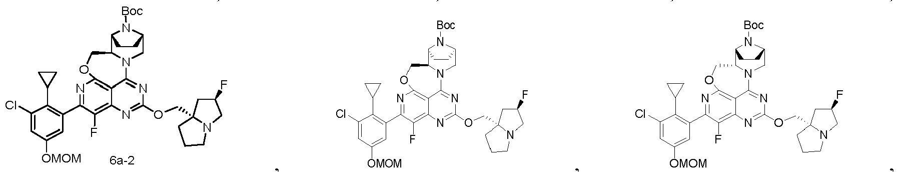

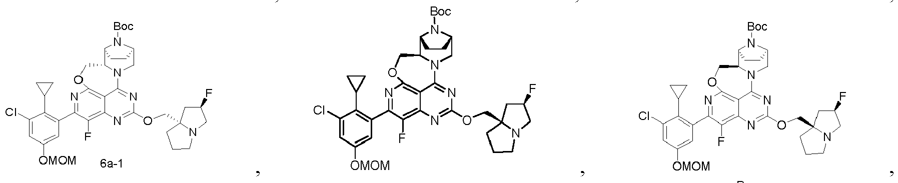

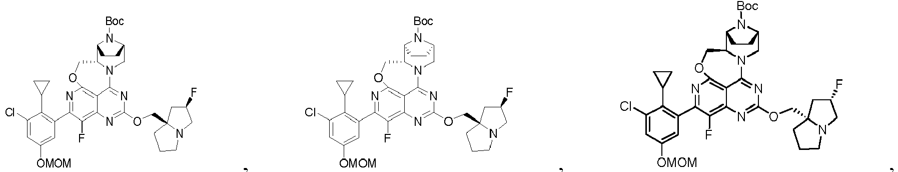

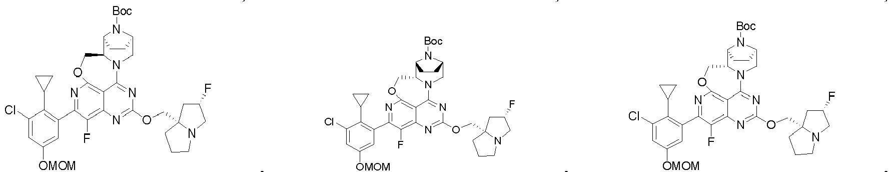

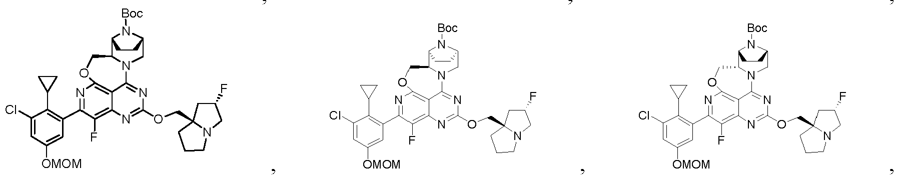

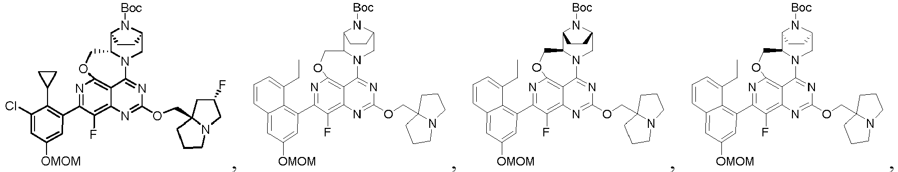

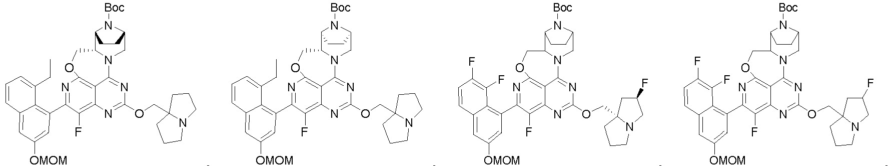

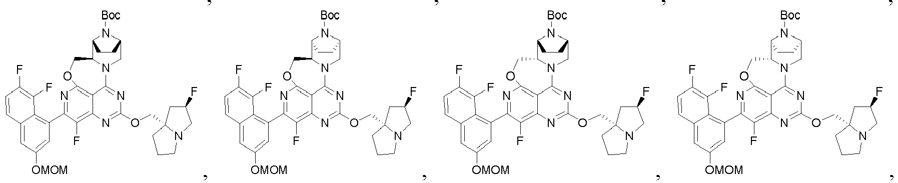

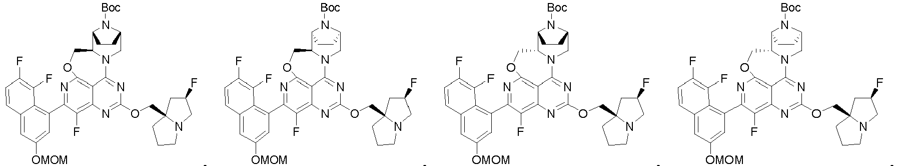

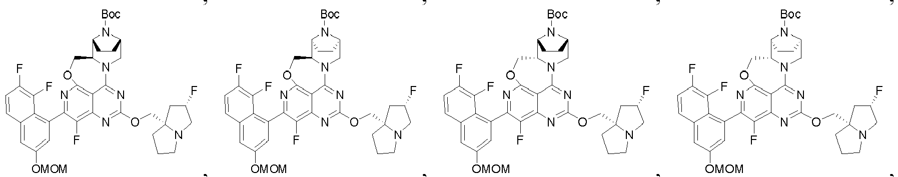

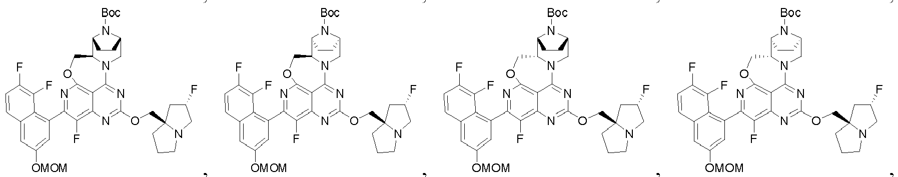

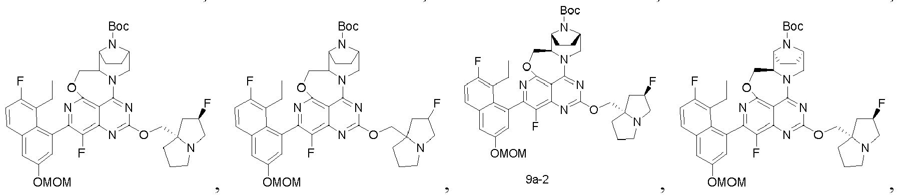

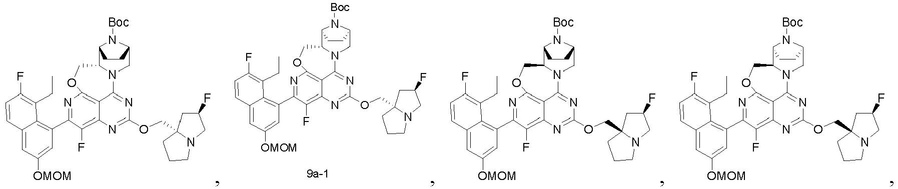

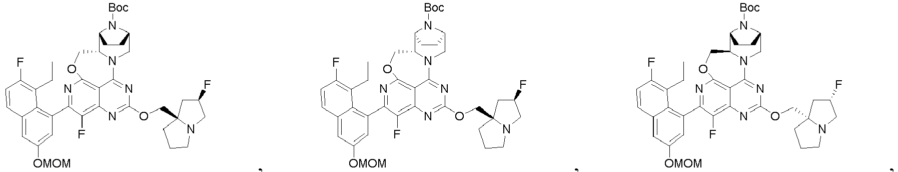

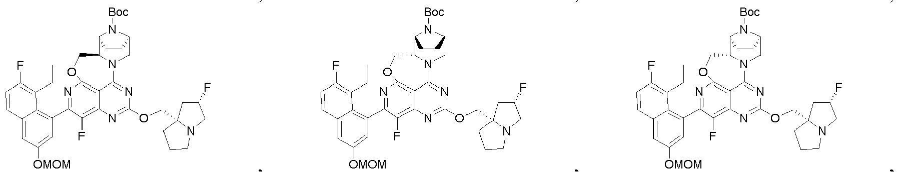

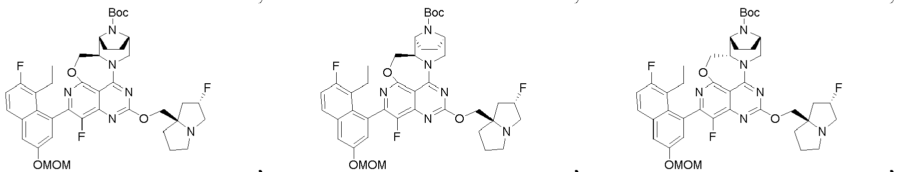

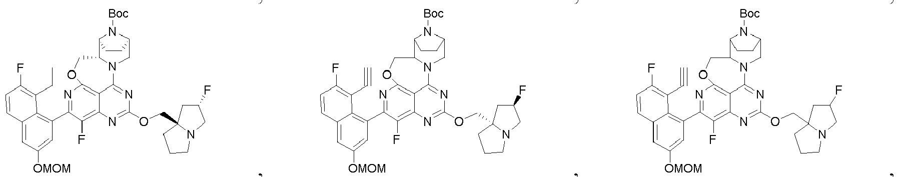

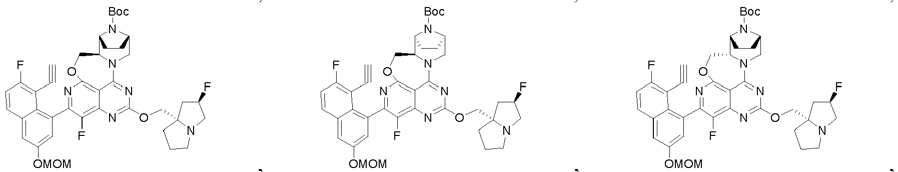

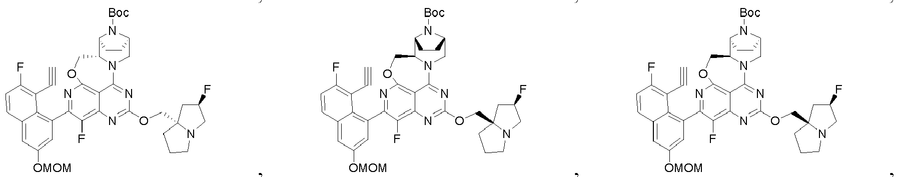

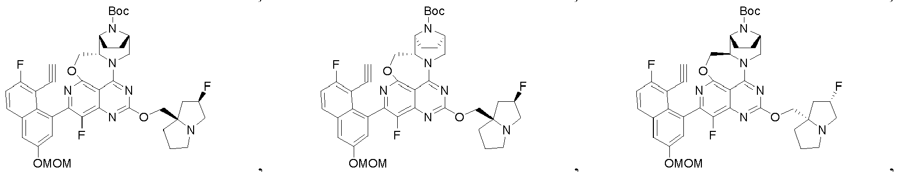

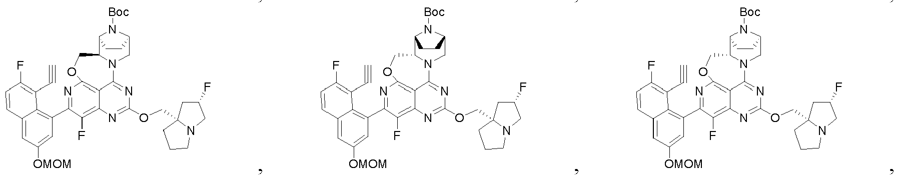

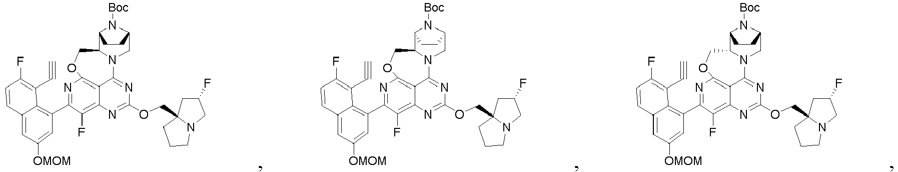

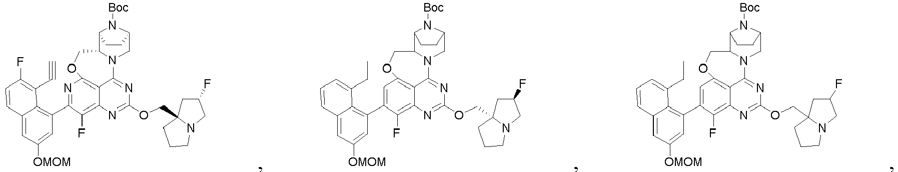

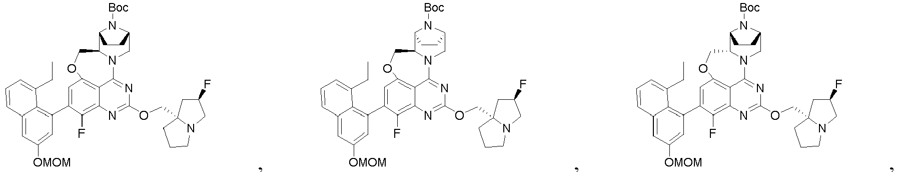

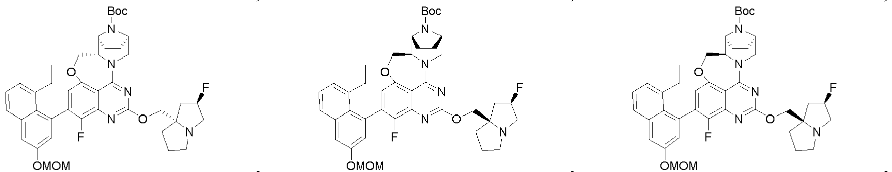

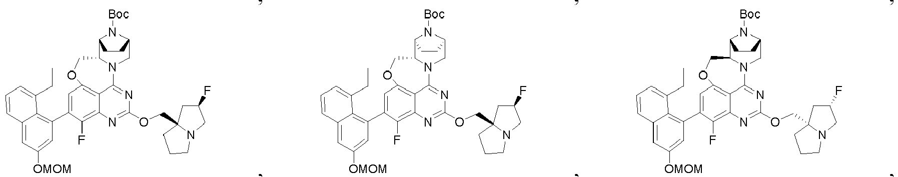

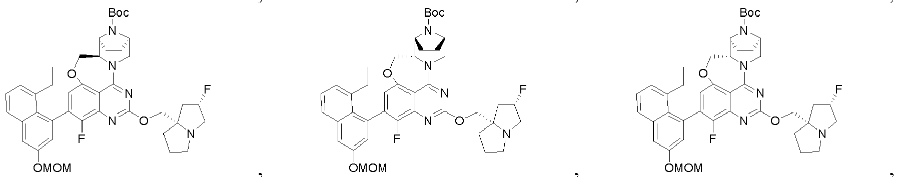

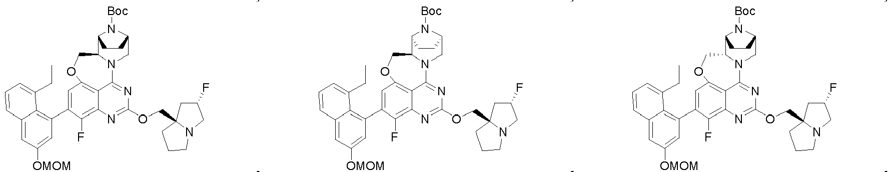

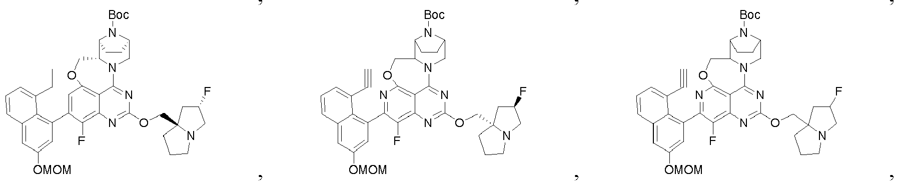

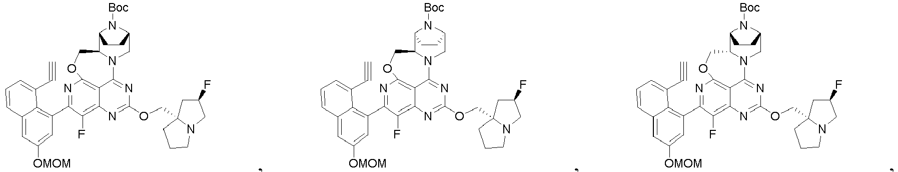

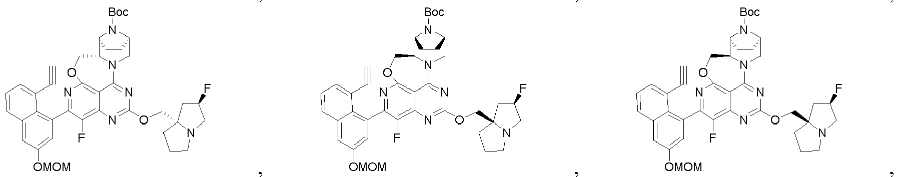

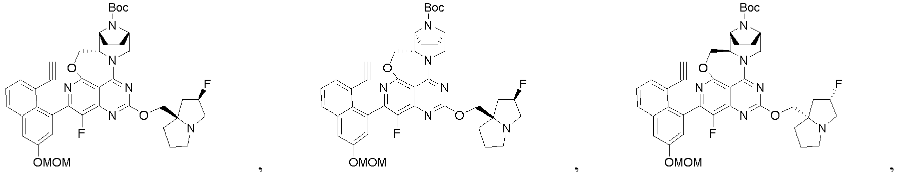

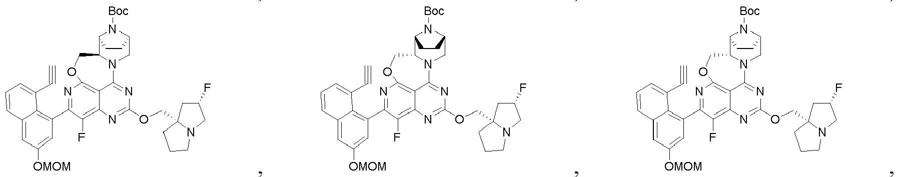

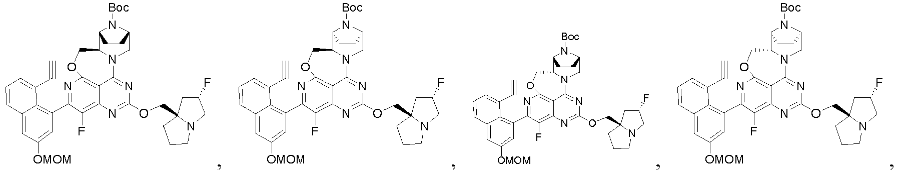

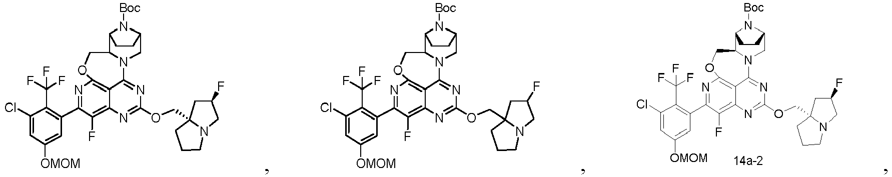

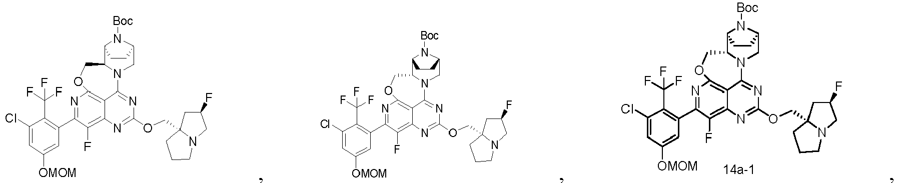

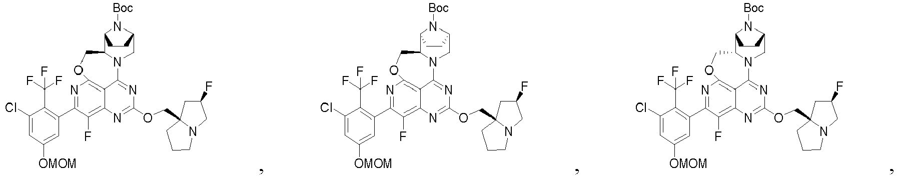

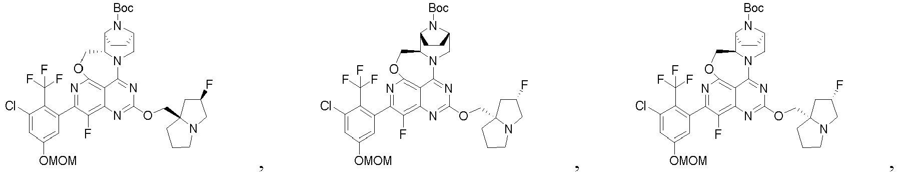

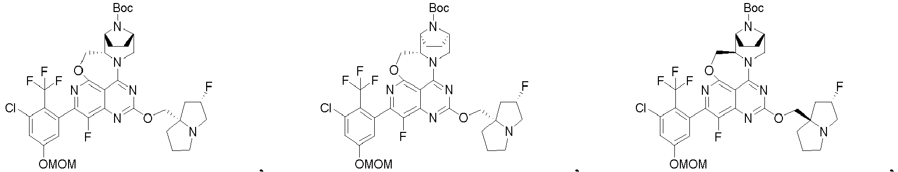

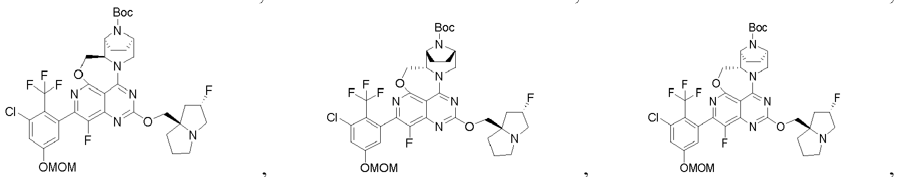

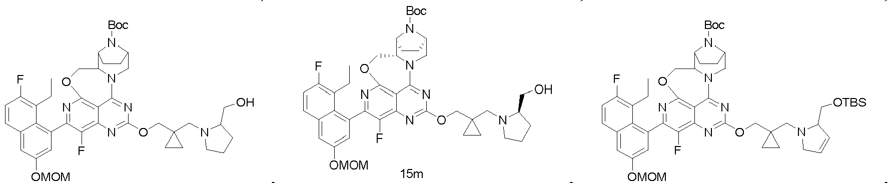

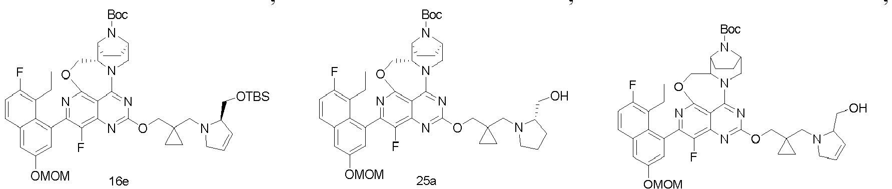

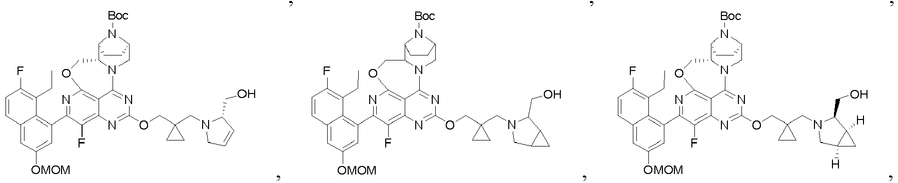

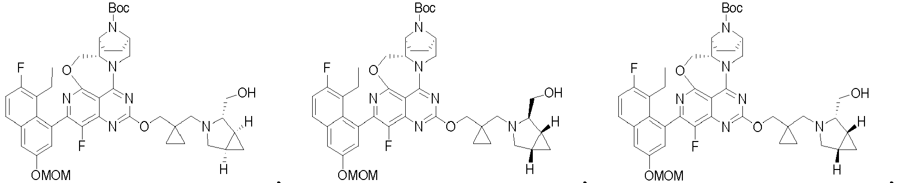

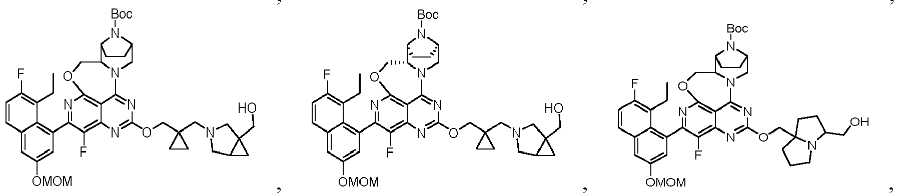

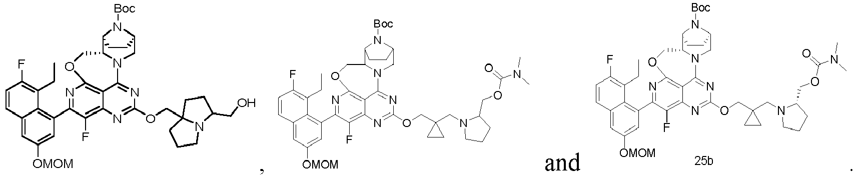

18. A method for preparing a compound of general formula (I') or a pharmaceutically acceptable salt thereof, comprising: 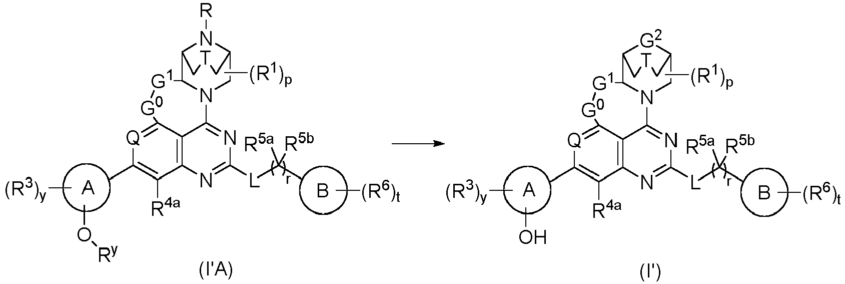
performing a deprotection reaction on a compound of general formula (I'A) or a salt thereof to obtain the compound of general formula (I') or the pharmaceutically acceptable salt thereof, wherein optionally, when R³ and/or R⁶ groups contain a protecting group, a step of removing the protecting group on the R³ and/or R⁶ groups is also included before, simultaneously with or after the deprotection reaction;
wherein,
R is an amino protecting group, and preferably Boc;
R^{y} is a hydroxy protecting group, and preferably MOM;
y is 0, 1, 2, 3 or 4;
G² is NH;
G⁰, G¹, T, ring A, ring B, Q, L, R¹, R³, R^{4a}, R^{5a}, R^{5b}, R⁶, p, r and t are as defined in claim 1.

19. A pharmaceutical composition, comprising the compound or the pharmaceutically acceptable salt thereof according to any one of claims 1 to 15, and one or more pharmaceutically acceptable carriers, diluents or excipients.

20. Use of the compound or the pharmaceutically acceptable salt thereof according to any one of claims 1 to 15 or the pharmaceutical composition according to claim 19 in the preparation of a medicament for inhibiting KRAS G12D.

21. Use of the compound or the pharmaceutically acceptable salt thereof according to any one of claims 1 to 15 or the pharmaceutical composition according to claim 19 in the preparation of a medicament for treating and/or preventing a disease or condition wherein the disease or condition is cancer.

22. Use of the compound or the pharmaceutically acceptable salt thereof according to any one of claims 1 to 15 or the pharmaceutical composition according to claim 19 in the preparation of a medicament for treating and/or preventing a disease or condition, wherein the disease or condition is selected from the group consisting of brain cancer, thyroid cancer, head and neck cancer, nasopharyngeal cancer, laryngeal cancer, oral cancer, salivary gland cancer, esophageal cancer, gastric cancer, lung cancer, liver cancer, kidney cancer, pancreatic cancer, gallbladder cancer, bile duct cancer, colorectal cancer, small intestine cancer, gastrointestinal stromal tumor, urothelial cancer, urinary tract cancer, bladder cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, lipoma, bone cancer, soft tissue sarcoma, neurofibroma, neuroglioma, neuroblastoma and glioblastoma, and preferably is selected from the group consisting of pancreatic cancer, colorectal cancer and non-small cell lung cancer.
